(19) Europäisches Patentamt
European Patent Office
Office européen des brevets

(11) Publication number: 0 071 908 B1

(12) **EUROPEAN PATENT SPECIFICATION**

(45) Date of publication of patent specification: 27.09.89

(21) Application number: 82106901.0

(22) Date of filing: 30.07.82

(51) Int. Cl.⁴: **C 07 D 487/04,**
C 07 D 519/00, A 61 K 31/40
// (C07D487/04, 209:00, 205:00),(C07D519/00, 487:00, 487:00)

(54) **1-, and 1,1-disubstituted-6-substituted-2-carbamimidoyl-1-carbadethiapen-2-em-3-carboxylic acids, a process for preparing and an antibiotic composition containing the same.**

(30) Priority: **03.08.81 US 289345**
**03.08.81 US 289599**
**03.08.81 US 289600**

(43) Date of publication of application:
**16.02.83 Bulletin 83/07**

(45) Publication of the grant of the patent:
**27.09.89 Bulletin 89/39**

(84) Designated Contracting States:
**AT BE CH DE FR GB IT LI LU NL SE**

(56) References cited:
**EP-A-0 010 316**
**EP-A-0 010 317**
**EP-A-0 017 992**
**EP-A-0 030 032**
**EP-A-0 038 869**
**EP-A-0 050 334**
**EP-A-0 060 077**

**The file contains technical information submitted after the application was filed and not included in this specification**

(73) Proprietor: **MERCK & CO. INC.**
**126, East Lincoln Avenue P.O. Box 2000**
**Rahway New Jersey 07065-0900 (US)**

(72) Inventor: **Christensen, Burton G.**
**770 Anderson Avenue Apt. 19H**
**Cliffside Park New Jersey 07010 (US)**
Inventor: **Leanza, William J.**
**20 Rutherford Place**
**Berkeley Heights New Jersey 07922 (US)**
Inventor: **Ratcliffe, Ronald W.**
**234 Matawan Avenue**
**Matawan New Jersey 07747 (US)**
Inventor: **Shih, David H.**
**2 Colby Court**
**Manalapan New Jersey 07726 (US)**

(74) Representative: **Abitz, Walter, Dr.-Ing. et al**
**Abitz, Morf, Gritschneder, Freiherr von Wittgenstein Postfach 86 01 09**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 071 908 B1

## Description

Background of the Invention

This invention relates to 1-, and 1,1-disubstituted-6-substituted-2-carbamimidoyl-1-carbadethiapen-2-em-3-carboxylic acids (I) and the pharmaceutically acceptable salt, ester and amide derivatives thereof which are useful as antibiotics:

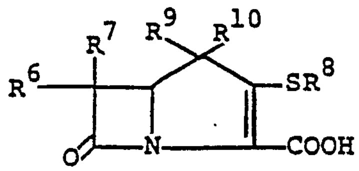

I

$R^6$ and $R^7$ and $R^9$ and $R^{10}$ are independently hydrogen ($R^9$ and $R^{10}$ are not both hydrogen) or are selected from from the group consisting of substituted and unsubstituted: alkyl, alkenyl, and alkynyl having 1—10 carbon atoms; cycloalkyl, cycloalkylalkyl, and alkylcycloalkyl having 3—6 carbon atoms in the cycloalkyl ring and 1—6 carbon atoms in the alkyl moiety; phenyl, phenylalkyl, phenylalkenyl, and phenylalkynyl wherein the aliphatic portion has 1—6 carbon atoms; or $R^6$ and $R^7$ are independently pyridyl or $R^6$ and $R^7$, and $R^9$ and $R^{10}$ may be joined to form a cyclicalkyl having, together with the carbon atom to which they are attached, 3—6 carbon atoms; wherein the substituent or substituents on $R^6$, $R^7$, $R^9$ or $R^{10}$ are independently selected from chloro, fluoro, hydroxy, bromo, alkylthio having 1—6 carbon atoms, and alkoxyl having 1—6 carbon atoms; and wherein $R^7$ can additionally be hydroxyl, methoxyl, mercapto, chloro, fluoro and bromo; and

when $R^7$ is hydrogen, $CH_3$ or $OCH_3$, $R^6$ can additionally have carboxyl, hydroxyimino, ureido or amino as substituents on the above-mentioned alkyl, cycloalkyl, cycloalkylalkyl and phenylalkyl groups; and

$R^8$ is carbamimidoyl selected from the group consisting of:

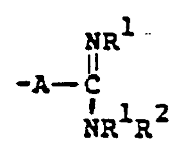 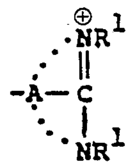 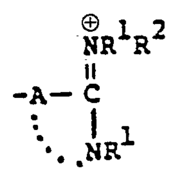

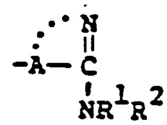 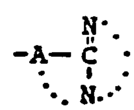 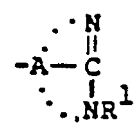

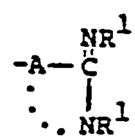 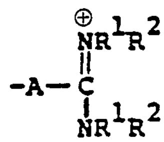 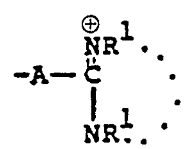

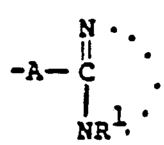 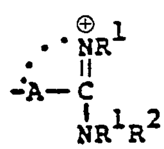 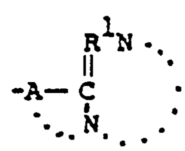

wherein: A is a single, direct bond, or A, the cyclic or acyclic connector, is selected from the group consisting of alkylene, alkenylene, and alkynylene having 1—10 carbon atoms which may be interrupted by a hetero atom selected from O, S or N, or by phenylene, cycloalkylene, cycloalkenylene, heterocyclylene or heteroarylene wherein such cyclic interruptions comprise 3—6 ring atoms selected from C, O, S and N; cycloalkylene having 3—10 carbon atoms; cycloalkenylene having 3—6 carbon atoms; and phenylene; $R^1$ and $R^2$ are independently selected from hydrogen and the previously defined, but monovalent, values for the group A; cycloalkylalkyl wherein the cycloalkyl moiety comprises 3 to 6 carbon atoms and the alkyl moiety comprises 1 to 6 carbon atoms; alkylcycloalkyl wherein the alkyl moiety comprises 1 to 6 carbon atoms and the cycloalkyl moiety comprises 3 to 6 carbon atoms; phenylalkyl having 7—10 carbon atoms;

wherein, if $R^1$ and $R^2$ are independently selected from straight and branched alkyl having from 1 to 6 carbon atoms; cycloalkyl having 3 to 6 carbon atoms; phenyl; or one of the above-defined cycloalkylalkyl, alkylcycloalkyl and phenylalkyl groups, each of those radicals can be substituted by amino, hydroxy, cyano,

carboxyl, nitro, chloro, bromo, fluoro, alkoxy and alkylthio having from 1 to 6 carbon atoms, mercapto, perhaloalkyl having 1 to 3 carbon atoms, guanidino, amidino or sulfamoyl; and

wherein A can additionally be selected from cycloalkylalkylene wherein the cycloalkyl moiety comprises 3 to 6 carbon atoms and the alkylene moiety comprises 1 to 10 carbon atoms; alkylcycloalkylene wherein the alkyl moiety comprises 1 to 6 carbon atoms and the cycloalkylene moiety comprises 3 to 6 carbon atoms; cycloalkenylalkylene wherein the cycloalkenyl moiety comprises 3 to 10 carbon atoms and the alkylene moiety comprises 1 to 6 carbon atoms; naphthylene; phenylalkylene and alkylphenylene wherein the alkyl has 1 to 6 carbon atoms; and

wherein the dotted lines indicate provision for cyclic structures formed by the joinder of the indicated nitrogen atom and the connector group A and by the joinder of the indicated nitrogen atoms; with the exception of such compounds

wherein:

a) $R^6$ is H, and
$R^7$ is $CH_2OH$ $R^8$ is 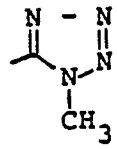
$R^9$ is $CH_3$
$R^{10}$ is H

b) $R^6$ is H
$R^7$ is 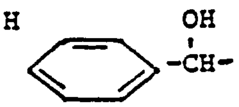 and
$R^9$ is $CH_3$ $R^8$ is $CH_2CH_2C(=NH)-NH_2$
$R^{10}$ is 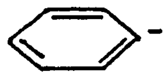

c) $R^6$ is H
$R^7$ is $CH_3-CH(OH)-$ and
$R^9$ is $CH_3$ $R^8$ is 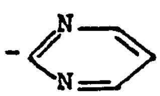
$R^{10}$ is $CH_3$

d) $R^6$ is H
$R^7$ is $CH_3-CH(OH)-$
$R^9$ and $R^{10}$ are joined to form a cyclopropyl ring
and
$R^8$ is 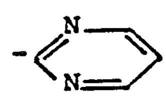.

Similar compounds are described in EP—A—10317, EP—A—30032, EP—A—17992 and EP—A—60077.

It should be noted that the final products of this invention (I) can exist in either neutral or zwitterionic (internal salt) forms. In the zwitterionic form, the basic function is protonated and positively charged and the carboxyl group is deprotonated and negatively charged. The zwitterionic form is the predominant species under most conditions and is in equilibrium with a minor amount of the uncharged, neutral species. The equilibrium process is conveniently visualized as an internal acid-base neutralization. The neutral and zwitterionic forms are shown below.

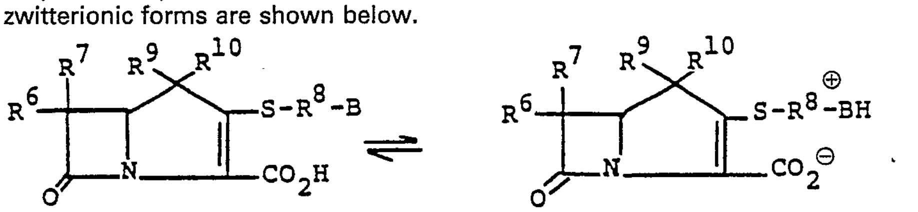

Neutral form

Zwitterionic or Internal salt form

wherein B is the carbamimidoyl group.

Further, the final products of this invention I wherein $R^8$ contains a positively charged quaternary nitrogen function such as the "carbamimidinium" can exist as zwitterionic (internal salt) forms or as external salt forms. The preferred form of this product group is the zwitterionic or internal salt form. These forms are shown below:

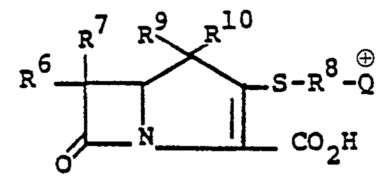

Zwitterionic (internal salt) form

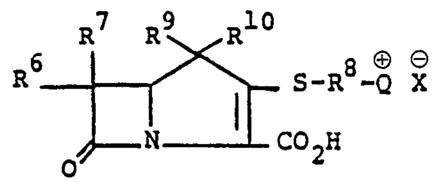

External salt form

wherein Q represents the quaternized nitrogen group, and wherein X is a pharmaceutically acceptable anion such as those listed in U.S. Patent 4,194,047, issued 3/18/80, which is incorporated herein by reference.

This invention also relates to the carboxyl derivatives of I which are antibiotics and which may be represented by the following generic structure (I):

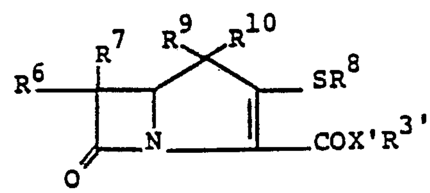

wherein X' is oxygen, sulphur or NR' (R' = H or lower alkyl having 1—6 carbon atoms); and $R^{3'}$ is, hydrogen, or, *inter alia* is representatively selected to provide the pharmaceutically acceptable salt, ester, anhydride ($R^{3'}$ is acyl), and amide moieties known in bicyclic β-lactam antibiotic art; $R^{3'}$ may also be a readily removable blocking group. The definition of $R^{3'}$ is given in greater detail below.

This invention also relates to processes for the preparation of such compounds I; and pharmaceutical compositions comprising such compounds.

There is a continuing need for new antibiotics. For unfortunately, there is no static effectiveness of any given antibiotic because continued wide scale usage selectively gives rise to resistant strains of pathogens. In addition, the known antibiotics suffer from the disadvantage of being effective only against certain types of microorganisms. Accordingly, the search for new antibiotics continues.

Thus, it is an object of the present invention to provide a novel class of antibiotics which are useful in animal and human therapy and in inanimate systems. These antibiotics are active against a broad range of pathogens which representatively include both Gram positive bacteria such as *S. aureus, Strep. pyogenes,* and *B. subtilis,* and Gram negative bacteria such as *E. coli, Pseudomonas, Proteus morganii, Serratia,* and *Klebsiella.* Further objects of this invention are to provide chemical processes for the preparation of such antibiotics and their nontoxic, pharmaceutically acceptable salts; and pharmaceutical compositions comprising such antibiotics.

Detailed Description of the Invention

The compounds of the present invention (I, above) are conveniently prepared by the following scheme:

DIAGRAM I

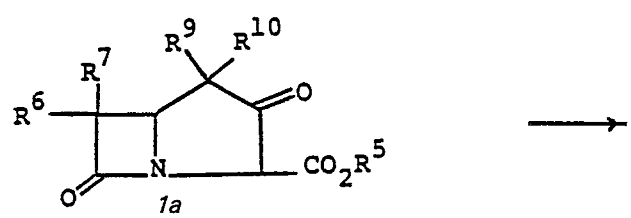

1a

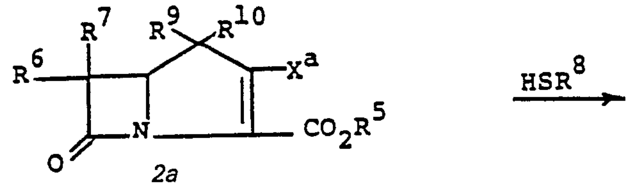


*2a*

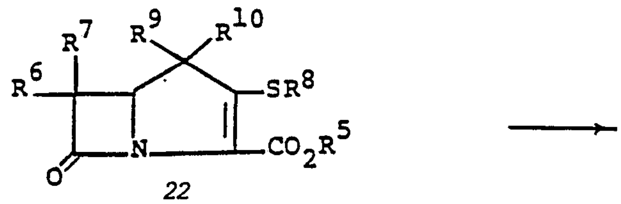

*22*

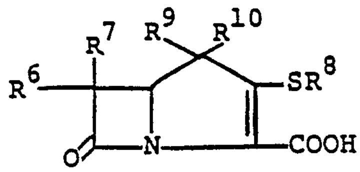

I

In words relative to the above reaction scheme, Diagram I, the step *1a* to *2a* to establish leaving group $X_a$ is accomplished by acrylating the bicyclic keto ester *1a* with an acylating agent $RX^a$ such as p-toluenesulfonic acid anhydride, p-nitrophenylsulfonic acid anhydride, 2,4,6-triisopropylphenylsulfonic acid anhydride, methanesulfonic acid anhydride, trifluoromethane sulfonic acid anhydride, diphenyl chlorophosphate, toluenesulfonyl chloride, p-bromophenylsulfonyl chloride, or the like; wherein $X^a$ is the corresponding leaving group such as toluene sulfonyloxy, p-nitrophenylsulfonyloxy, benzenesulfonyloxy, diphenylphosphoryl, and other leaving groups which are established by conventional procedures and are well known in the art. Typically, the above acylation to establish leaving group $X^a$ is conducted in a solvent such as methylene chloride, acetonitrile or dimethylformamide, in the presence of a base such as diisopropylethylamine, triethylamine, 4-dimethylaminopyridine or the like at a temperature of from −20 to 40°C for from 0.1 to 5 hours. The leaving group $X^a$ of intermediate *2a* can also be halogen. The halogen leaving group is established by treating *1a* with a halogenating agent such as $\phi_3PCl_2$, $\phi_3PBr_2$, $(\phi O)_3PBr_2$, oxalyl chloride or the like in a solvent such as $CH_2Cl_2$, $CH_3CN$, THF, or the like in the presence of a base such as diisopropylethylamine, triethylamine, or 4-dimethylaminopyridine or the like. [$\phi$ = phenyl.]

The reaction *2a* to *22* is accomplished by treating *2a* in a solvent such as dioxane, dimethylformamide, dimethylsulfoxide, acetonitrile, hexamethylphosphoramide, or the like, in the presence of an approximately equivalent to excess of the mercaptan reagent $HSR^8$, wherein $R^8$ is defined above, in the presence of a base such as sodium hydrogen carbonate, potassium carbonate, triethylamine, diisopropylethylamine, or the like at a temperature of from −40 to 25°C for from 30 sec. to 1 hour.

The final deblocking step *22* to *I* is accomplished by conventional procedures such as solvolysis or hydrogenation. The conditions of deblocking *22* to *I* are thus: typically *22* in a solvent such as tetrahydrofuran-water, tetrahydrofuran-ethanol-water, dioxane-water, dioxane-ethanol-water, n-butanol-water, or the like containing pH 7 morpholinopropanesulfonic acid-sodium hydroxide buffer, pH 7 phosphate buffer, dipotassium hydrogen phosphate, sodium bicarbonate, or the like, is treated under a hydrogen pressure of from 1 to 4 atmospheres in the presence of a catalyst such as platinum oxide, palladium on charcoal, or palladium hydroxide on charcoal, or the like, at a temperature of from 0 to 50°C for from 0.25 to 4 hours to provide I. Photolysis, when $R^5$ is a group such as o-nitrobenzyl, for example, may also be used for deblocking.

Relative to Diagram I, the bicyclic keto ester *1a* may be obtained by the following scheme, Diagram II.

DIAGRAM II

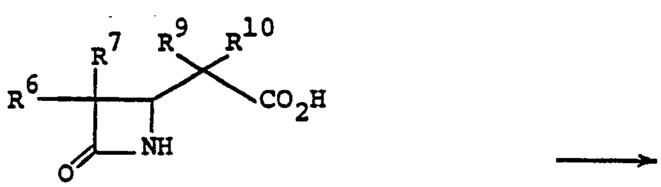

*3*

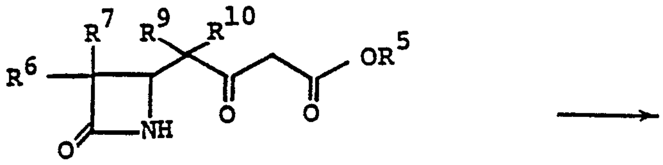

4

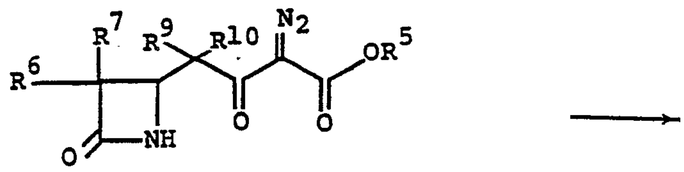

5

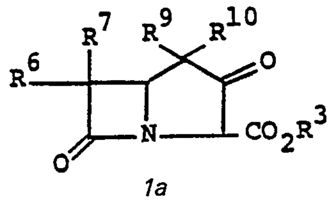

1a

The addition *3* to *4* is accomplished by treating *3* with 1,1'-carbonyldiimidazole, or the like, in a solvent such as tetrahydrofuran (THF), dimethoxyethane, acetonitrile or the like, at a temperature of from 0 to 70°C, followed by the addition of 1.1 to 3.0 equivalent of (R $O_2CCH_2CO_2)_2Mg$, at a temperature of from 0 to 70°C for from 1 to 48 hours. The group $R^5$ is a pharmaceutically acceptable ester moiety or a readily removable carboxyl protecting group such as p-nitrobenzyl, benzyl, or the like.

The diazo species *5* is prepared from *4* by treating *4* in a solvent such as $CH_3CN$, $CH_2Cl_2$, THF, or the like with an azide such as p-carboxybenzenesulfonylazide, p-toluenesulfonylazide, methanesulfonylazide, or the like in the presence of a base such as triethylamine, pyridine, diethylamine or the like for from 1 to 50 hours at 0—50°C.

Cyclization (*5* to *1a*) is accomplished by treating *5* in a solvent such as benzene, toluene, THF, cyclohexane, ethylacetate or the like at a temperature of from 25 to 110°C for from 1—5 hours in the presence of a catalyst such as *bis* (acetylacetonato)Cu(II)[Cu(acac)$_2$], $CuSO_4$, Cu powder $Rh_2(OAc)_4$ or $Pd(OAc)_2$. Alternatively, the cyclization may be accomplished by irradiating *6* through a pyrex filter (a wave length greater than 300 nm) in a solvent such as benzene, $CCl_4$, diethylether, or the like, at a temperature of from 0—25°C for from 0.5 to 2 hours. ["OAc" = acetate.]

Relative to Diagram II, the following scheme, Diagram III, crosses at intermediate *3* ($R^9$ substituted):

DIAGRAM III

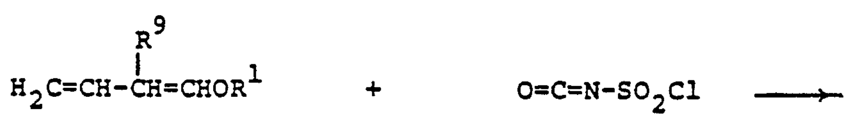

1' 2'

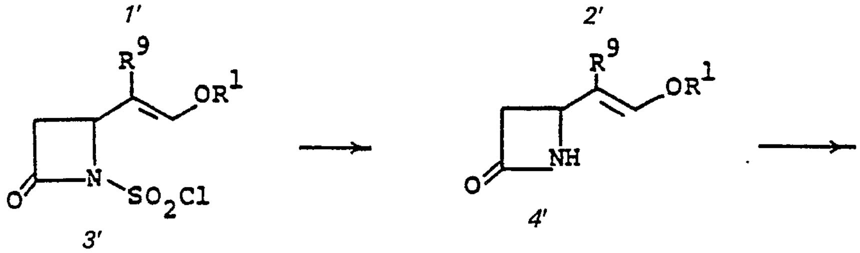

3' 4'

DIAGRAM III

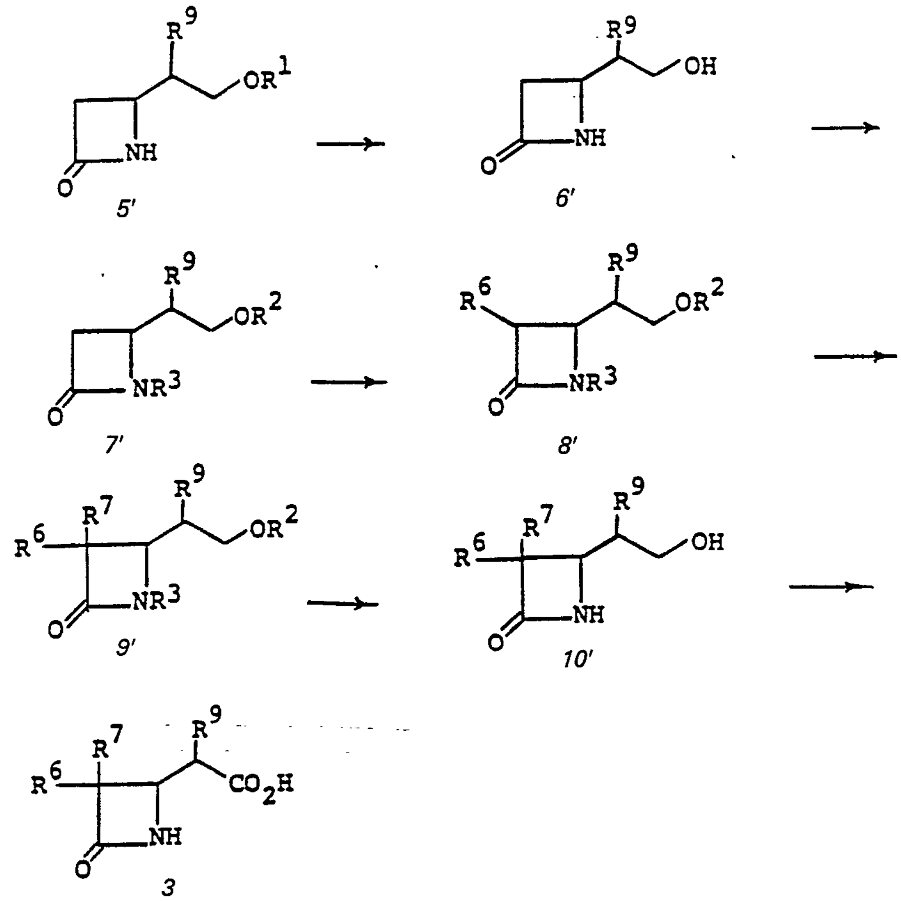

5'  6'  7'  8'  9'  10'  3

In words relative to the above diagram, the 4-(1,2-substituted-vinyl)azetidine-2-one, *4'*, is prepared by reacting an $R^1$-oxybutadiene, 1', with chlorosulfonylisocyanate 2'. The reaction is conducted without solvent or may be run in solvent such as diethyl ether, ethyl acetate, chloroform, methylene chloride, or the like, at a temperature of from −78°C to 25°C for from a few minutes to 1 hour to provide *3'*. The radical $R^1$ is an easily removable acyl blocking group such as alkanoyl or aralkanoyl which bears no functional group or groups which might interfere with the desired course of reaction (*1' + 2'* to *3' to 4'*), and $R_9$ is as defined in I. Intermediate species *3'* is converted to the sulfinamide by reduction which is then hydrolyzed to *4'* at pH 6—8. Typically the reaction solution comprising *3'* is contacted (5—30 minutes) with an aqueous solution (at 0—25°C) of a reducing agent such as sodum sulfite, thiophenol, or the like, at pH 6—8 to provide *4'*.

The reaction *4'* to *5'* is a reduction, and is preferably achieved by hydrogenation in a solvent such as ethyl acetate, ether, dioxane, tetrahydrofuran (THF), ethanol or the like at 0 to 25°C for from 5 minutes to 2 hours under 1 to 10 atmospheres of hydrogen in the presence of a hydrogenation catalyst such as a platinum metal or oxide thereof such as 10% Pd/C or the like.

The deblocking reaction *5'* to *6'* is usually desirable when $R^1$ is acyl to permit the later alkylation, *7'* to *8'*. The preferred deblocking procedure is by alcoholysis wherein the solvent is a lower alkanol such as methanol, ethanol or the like in the presence of the corresponding alkali metal alkoxide, such as sodium methoxide. Typically, the reaction is conducted for from 5 minutes to 1 hour at a temperature of from −10° to 25°C.

Blocking groups $R^3$ and $R^2$ are established (*6'* to *7'*) to provide a suitably protected species for alkylation (*7'* to *8'* to *9'*). There is no criticality in the choice of blocking groups, provided only that they do not interfere with the intended alkylation. $R^3$ may be hydrogen, a triorganosilyl group such as trimethylsilyl or the like, or a cyclic ether such as 2-tetrahydropyranyl. $R^2$ may also be cyclic ether such as 2-tetrahydropyranyl; alternatively $R^3$ and $R^2$ may be joined together to form protected species such as *7a*:

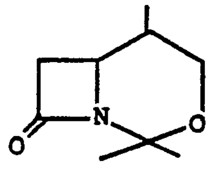

*7a*

For example, species such as *7a* are conveniently prepared by treating *6'* with 2,2-dimethoxypropane in the presence of a catalyst such as boron trifluoride etherate, toluene sulphonic acid, or the like in a solvent such as methylene chloride, ether, chloroform, dioxane or the like at a temperature of from −10°C to 35°C for from a few minutes to 1 hour. Species *7'* can be mono- or dialkylated at ring position *3*. Alkylation of *7'* provides *8'*. Typically, *7'* is treated with a strong base such as lithium diisopropyl amide, sodium hydride, phenyl lithium or butyl lithium and the like in a solvent such as tetrahydrofuran (THF), ether, dimethoxyethane and the like at a temperature of from −80°C to 0°C, whereupon the alkylating agent of choice, $R^6X$, is added ($R^6$ is as described above and X is chloro, iodo or bromo; alternatively the alkylating agent may be $R^6$-tosylate, $R^6$-mesylate or an aldehyde or ketone such as acetone and the like) to provide monoalkylated species *8'*. When desired, dialkylated species *9'* may be obtained from *8'* by repeating the alkylating procedure, *7'* to *8'*.

The de-blocking reaction *9'* to *10'* is typically conducted by acid hydrolysis such as aqueous acetic acid at a temperature of from 25°C to 75°C for from 5 minutes to 3 hours.

The acid *3* is prepared by treating 10' with an oxidizing agent such as Jones reagent in acetone or the like at a temperature of from 0—25°C for from 5 minutes to 1 hour.

The disubstituted azetidinone carboxylic acid *3* may be prepared from 3-substituted 1,4-butadiene (Diagram IV).

DIAGRAM IV

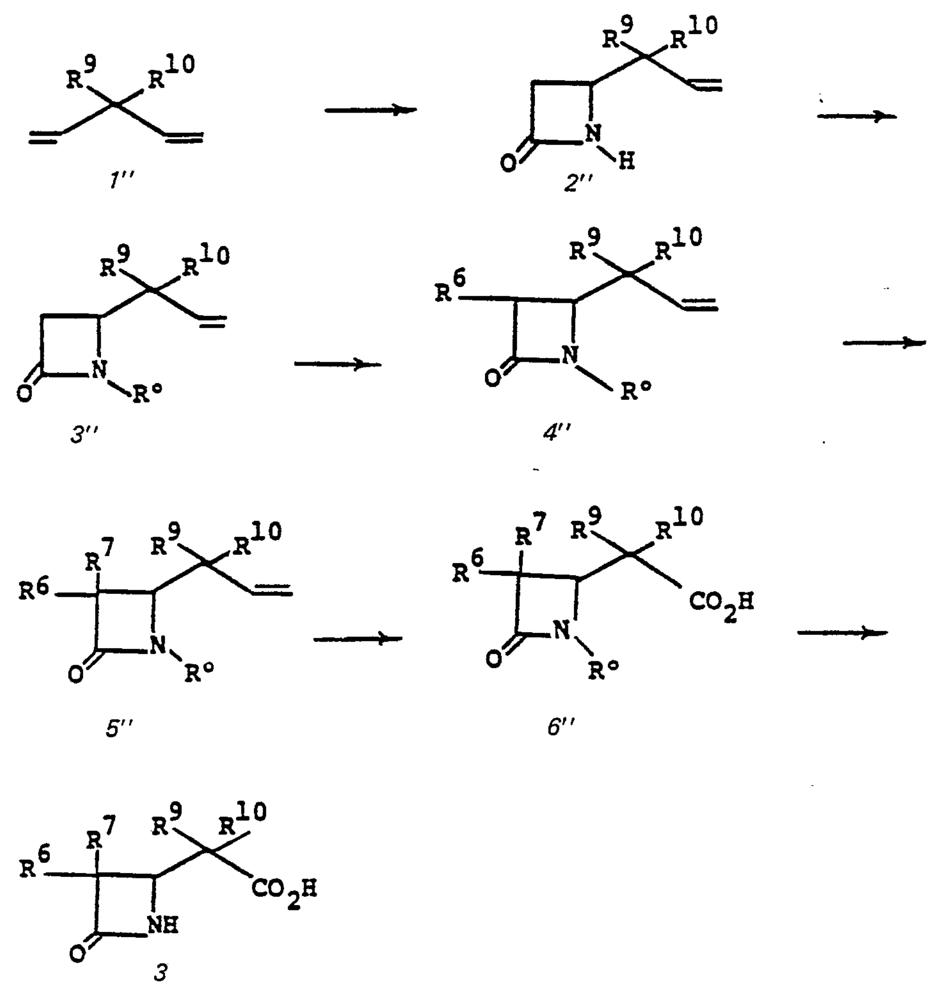

1″ 2″ 3″ 4″ 5″ 6″ 3

In words relative to Diagram IV, the substituted azetidinone *2″* is prepared by reacting a 3-substituted 1,4-pentadiene *1″* with chlorosulfonylisocyanate at 25°C to 60°C in a pressure bottle for 3—12 days. The resulting mixture is hydrolyzed with aqueous sodium sulfite solution between pH 6.5—7.5 at 0°C to 25°C for from 5 min to 60 min.

Azetidinone *2″* is transformed (*2″* to *3″*) to establish the protecting group $R^0$ which may be a triorganosilyl group, such as t-butyldimethylsilyl, t-butyldiphenylsilyl, triphenylsilyl, isopropyldimethylsilyl, for example, or may be 3,4-dimethoxybenzyl, for example. Silyl protection is preferred, and typically $R^0$ is established by treating *2″* in a solvent such as dimethylformamide, acetonitrile, hexamethylphosphoramide, tetrahydrofuran or the like with a silylating agent such as t-butyldimethylchlorosilane, t-butyldiphenylchlorosilane, triphenylchlorosilane, or the like at a temperature of from −20° to 25°C for from 0.5 to 24 hours in the presence of a base such as triethylamine, diisopropylethylamine.

Alkylation of *3''* provides *4''*. Typically, *3''* is treated with a strong base such as lithium diisopropylamide, sodium hydride, phenyl lithium, butyl lithium, or the like in a solvent such as tetrahydrofuran, ether, dimethoxyethane or the like at a temperature of from −80°C to 0°C, whereupon the alkylating agent of choice, $R^6X/R^7X$ is introduced ($R^6/R^7$ are described above and X is chloro, iodo or bromo; alternatively the alkylating agent may be $R^6$-tosylate, $R^6$-mesylate, an aldehyde, or a ketone such as acetone, or the like) to provide monoalkylated species *4''*. When desired, dialkylated species *5''* may be obtained from *4''* by repeating the alkylating procedure, *4''* to *5''*.

The oxidation *5''* to *6''* is accomplished by treating *5''* in a solvent such as methylenechloride, methanol, or the like, with ozone, at a temperature of from −100° to 0°C for from 0.1 to 4 hours, followed by treating the crude product with an oxidizing agent such as m-chloroperbenzoic acid, hydrogen peroxide, peracetic acid, or the like, at a temperature of from 0°C to 100°C for from 1 to 100 hours. Deprotection of *6''* by acid hydrolysis in 6.0 *N* HCl in MeOH at temperature of from 0 to 25°C for from 10 min to 3 hours gives *3*.

With respect to starting reagent *1''*, its preparation is generally described in *J. Amer. Chem. Soc., 74,* 661 (1952) by E. B. Reid and T. E. Gompf, *J. Org. Chem., 23,* 1063 (1958) by R. Ciola and K. L. Burwell, Jr., and Belgium Patent 632,193 (1963) by R. Polster and E. Scharf. The Diagram V summarizes the preparation of *1''*.

DIAGRAM V

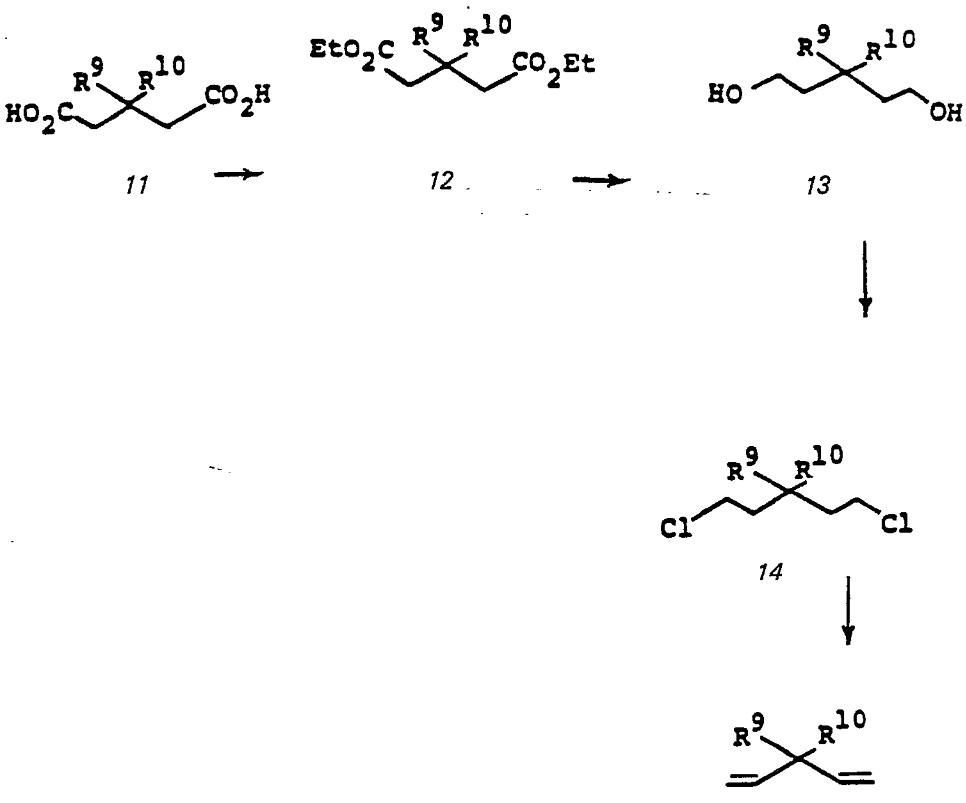

*11* *12* *13*

*14*

*1''*

In words relative to Diagram V, the diester 12 is prepared by treating the diacid 11 with thionyl chloride at reflux for two hours followed by reacting with ethanol at 80°C for 4 hours. Reduction of the diester 12 with lithium aluminum hydride in ether at reflux for 4 hours followed by hydrolysis with 10% NaOH gives diol *13* which on further reaction with thionyl chloride gives dichloride *14*. Reaction of the dichloride *14* with base such as 2-methylquinoline, DBU, or sodium hydroxide in polyethylene glycol gives the expected 3-substituted 1,4-pentadiene *1''*.

Alkylating and Acylating Reagents for Establishing $R^6$ and $R^7$

The establishment of $R^6$ and $R^7$ by alkylation has been shown. There is a second scheme for establishing $R^6$ and $R^7$. It involves direct acylation followed by reduction. These schemes are conveniently compared and consolidated, below (Diagram Va), and, there following, is a representative list of suitable alkylating and acylating reagents for establishing $R^6$ and $R^7$.

DIAGRAM Va

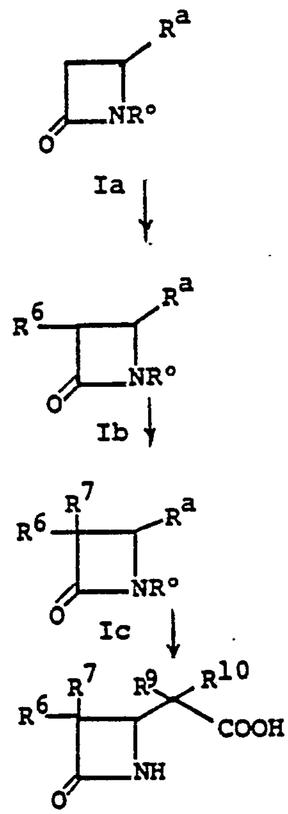


3

wherein $R^a$ comprises the following five classes:

1) —$CR^9R^{10}COOR$; R is a protecting group such as methyl, ethyl, p-nitrobenzyl, benzyl, triorganosilyl, or the like; $R^9$ and $R^{10}$ are as previously defined ($R^9$ and $R^{10}$ are H);

2) —$CR^9R^{10}CH{=}CH_2$; $R^9$ and $R^{10}$ are as previously defined;

3) —$CR^9R^{10}CH_2OR^o$; $R^9$ and $R^{10}$ are as previously defined; $R^o$ is triorganosilyl, such as trimethylsilyl, t-butyldimethylsilyl, or the like;

4) —$CH_2C(SR)_3$; R is alkyl, aryl, or aralkyl; wherein the alkyl has 1—6 carbon atoms, and the aryl is phenyl;

5) $CH_2C(SR)_2$ $SiR_3$; wherein R is as defined above for Ic, 4.

The intermediates Ic(2-5) above, are disclosed in EPO Patent Application Serial Number 81,108,420.1 which is fully incorporated herein by reference.

Conversion of Ic, classes 1—5 to *3* are achieved by the following reactions:

1) *Ic, Class 1.* The starting material is first treated with 1.0 to 1.5 eq. of 6.0*N* HCl in methanol at room temperature for from 10 minutes to 2 hours to remove the triorganosilyl protecting group $R^o$, followed by treating the mixture with 2.0 to 3.0 eq. of 2.5*N* NaOH at room temperature for 30 minutes to 8 hours then acidified with HCl to give *3*.

2) *Ic, Class 2.* This conversion is previously described in Diagram IV, *5''* to *6''* to *3*.

3) *Ic, Class 3 to 3:* The triorganosilyl protecting group is removed by acid hydrolysis with 6.0*N* HCl in methanol at room temperature for 10 minutes to 2 hours. The free hydroxyl group of Ic is oxidized by Jone's reagent in acetone at room temperature for 10 minutes to 1 hour to yield *3*.

4) *Ic, Class 4.* This conversion is achieved by treating the starting material with a Lewis acid, such as mercuric chloride, silver tetrafluoroborate, or the like, in a solvent such as methanol at 0° to 60°C for from 1 to 30 minutes. After the mixture is quenched with sodium bicarbonate, the methyl ester of *3* is obtained. Hydrolysis of the ester with 2.5*N* NaOH in methanol at room temperature for 30 minutes to 8 hours, followed by acidification with HCl gives *3*.

5) *Ic, Class 5.* The starting material is first converted to silyl lactone intermediate by reacting with mercuric oxide/mercuric chloride in a solvent such as methanol at reflux for 0.5—3 hours. The silyl ketone so obtained is then treated with an oxidizing agent, such as m-perbenzoic acid, peracetic acid, hydrogen peroxide or the like in a solvent such as methylene chloride, chloroform, carbon tetrachloride or the like, at reflux for 0.5 to 24 hours to afford *3*.

In words relative to Diagram Va, starting material Ia can be mono-, or dialkylated at ring position 3. Alkylation of Ia provides Ic. Typically, Ia is treated with a strong base such as lithium diisopropylamide,

lithium 2,2,6,6-tetramethylpiperidide, potassium hydride, lithium hexamethyldisilazane, phenyllithium or the like in a solvent such as tetrahydrofuran (THF), hexamethylphosphoramide, ether, dimethoxyethane, and the like at a temperature of from −80°C to 0°C whereupon the alkylating agent of choice, $R^6X°$ is added (X° is chloro, iodo or bromo); alternatively the alkylating agent may be $R^6$-tosylate, $R^6$-mesylate or an aldehyde or ketone such as acetaldehyde to provide monoalkylated species Ib. When desired, dialkylated species Ic may be obtained from Ib by repeating the alkylating procedures Ia → Ib.

The eventual 6-substituents (nomenclature relative to final, bicyclic structure) can also be established by direct acylation using an acylating agent such as N-acyl imdazole or the like. Such N-acyl imidazole acylating reagents are listed below. Also given below is a detailed description of this second approach for establishing, $R^6$ and $R^7$.

The following list is representative of useful *alkylating* agents for establishing $R^6$ and $R^7$, according to the above scheme Ia → Ib → Ic (this will be referred to as Scheme I, to be distinguished from Scheme II, below, which involves *acylation*):

<u>Alkylating Agents</u>

$CH_3CHO$

$ØCH_2CHO$ Ø = phenyl

$ØCH_2CH_2CHO$

$CH_2O$

$CH_3I$

$ØCH_2Br$

$CH_3COCH_3$

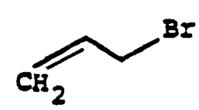

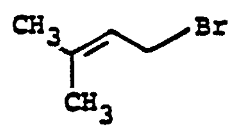

$CH_3OCH_2CHO$

$CH_3CH_2I$

$(CH_3)_2CHI$

$N_3CH_2CHO$

$(CH_3)_2NCH_2CHO$

$RO_2CCH_2Br$ R = $CH_3$, benzyl, p-nitrobenzyl

$CF_3CF_2CHO$

$RO_2CCH_2CHO$ R = $CH_3$, benzyl, p-nitrobenzyl

$CH_3CH(CH_3)CHO$,

$CH_3(CH_3)CHCH_2CHO$,

$CH_3CH_2CHO$ ,

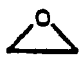 ,

$CF_3CHO$,

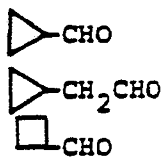

$[(CH_3)_3C](CH_3)_2SiOCH_2\overset{O}{\overset{\|}{C}}H$

$F_2CH\overset{O}{\overset{\|}{C}}H$

$FCH_2\overset{O}{\overset{\|}{C}}H$

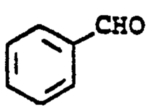

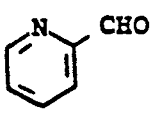

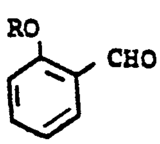 R = protecting group

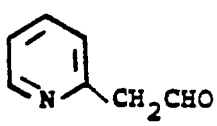

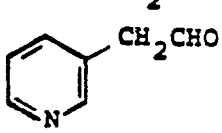

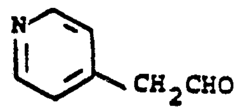

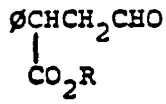

R is removable carboxyl protecting group, such as benzyl.

As mentioned above, the 6-substituents may also be established by acylation. Utilization of such acylating agents may be demonstrated in the following manner with regard to a preferred starting material Ib or Ic:

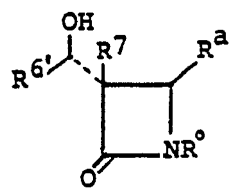

Ib/Ic

wherein $R^7$, $R^a$ and $R^o$ are as defined above. $R^{6'}$ is defined relative to the definition of $R^6$ and in that sense is the balance of the previously identified group $R^6$. In other words, for purposes of this definition $R^{6'}CH(OH)— = R^6$. An especially preferred material Ib is when $R^7$ is hydrogen and $R^{6'}$ is methyl. Basically, such 1'-hydroxy $R^{6'}$ species Ib are prepared according to the following scheme:

SCHEME II

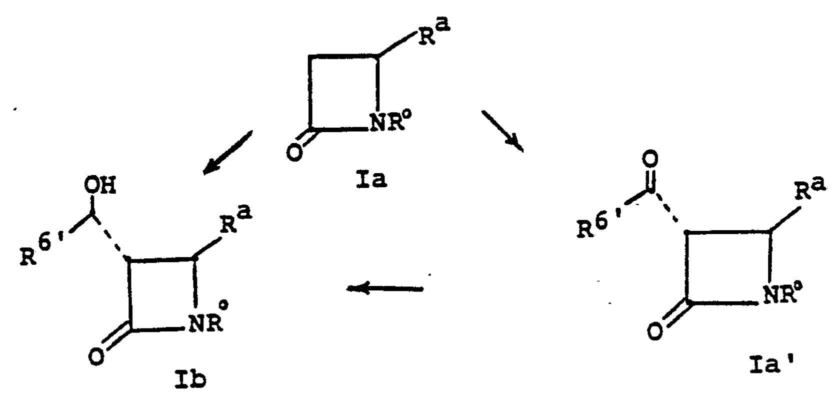

Ia

Ib

Ia'

The alkylation Ia → Ib, Scheme II, is accomplished as previously described, by treating Ia in a solvent such as tetrahydrofuran, dimethoxyethane, diethylether, hexamethylphosphoramide, at a temperature of from −100° to −20°C with a strong base such as lithium diisopropylamide, lithium hexamethyldisilazide, lithium 2,2,6,6-tetramethylpiperidide, potassium hydride or the like followed by the addition of an equivalent to 10 fold excess of an aldehyde. This reaction gives a mixture of isomers from which the desired *trans*-R form Ib can be conveniently separated by chromatography or crystallization.

Intermediate Ia may proceed directly to Ib as indicated above, or it may take the circuitous path *via* Ia'. The direct acylation, to Ia' is accomplished by treating Ia with two or more equivalents of a base such as lithium diisopropylamide, lithium hexamethyldisilazide, lithium 2,2,6,6-tetramethylpiperidide, in a solvent such as tetrahydrofuran, diethylether, or dimethoxyethane, for example, at a temperature of from −100 to −20°C with an acylating agent such as N-acyl imidazole or the like. Addition of the Ia plus base mixture to the acylating agent is preferred.

Representative acylating agents for this scheme Ia → Ia' → Ib are listed below.

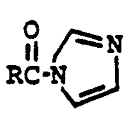 ; R=$CH_3$, $ClCH_2$, $CH_3CH_2$, $N_3CH_2$, $CH_3OCH_2$,

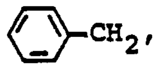, 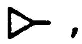, 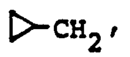, 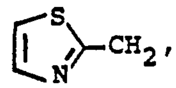

$R\overset{O}{\overset{\|}{C}}-SCH_2CH_3$; R=$CF_3$, $CF_2H$, $CH_2$=CH, 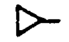,

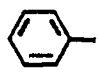, 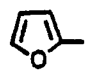

Further with respect to Scheme II, the reduction, Ia′→ Ib is accomplished by contacting the ketone with a reducing agent such as potassium tri(sec-butyl)borohydride, lithium tri(sec-butyl)borohydride, sodium borohydride, sodium tris(methoxyethoxy)aluminum hydride, lithium aluminum hydride or the like in a solvent such as diethylether, tetrahydrofuran, toluene, i-propanol or the like at a temperature of from −78°C to 25°C. The reaction can conveniently be conducted in the presence of an added complexing salt such as potassium iodide, magnesium bromide or the like.

In a similar manner, unresolved Ib (*cis* and *trans*) may be oxidized to Ia′ for reduction to Ib as indicated above:

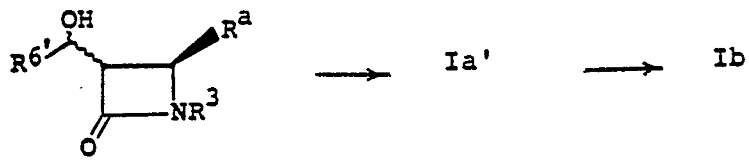

The oxidation is accomplished with an oxidizing agent such as dipyridine chromium (VI) oxide, trifluoroacetic anhydride-dimethylsulfoxide-triethylamine, pyridinium dichromate, acetic anhydride-dimethylsulfoxide in a solvent such as methylene chloride, acetonitrile, or the like at a temperature of from −78 to 25°C for from 5 minutes to 5 hours.

The foregoing schemes describe the synthesis of racemic 1,1-disubstituted-6-substituted-2-carbamimidoyl-1-carbadethiapen-2-em-3-carboxylic acids I.

To achieve a given chiral synthesis of I, the racemic azetidinone carboxylic acid 3 is resolved according to conventional optical resolution techniques, such as fractional crystallization of optically active ammonium salts, esters or chromatographic separation of such esters.

Alternatively, the chiral azetidinone intermediates *3—6* (Diagram II) may also be conveniently prepared *via* alkylation of the corresponding unsubstituted chiral azetidinone. The preparation of chiral precursor *24* is known; see: EPO Serial Number 80102338.3 (filed April 30, 1980); EPO Patent Application Serial Number 81,102,270.6. "Process for The Preparation of 1-Carbapenems; and Intermediates *via* silyl-substituted Dithioacetals"; and Serial Number 81,102,269.8 "Process for The Preparation of 1-Carbapenems, and Intermediates *via* Trithioorthoacetates"; which are incorporated herein by reference. Diagram VI summerizes these reactions:

DIAGRAM VI

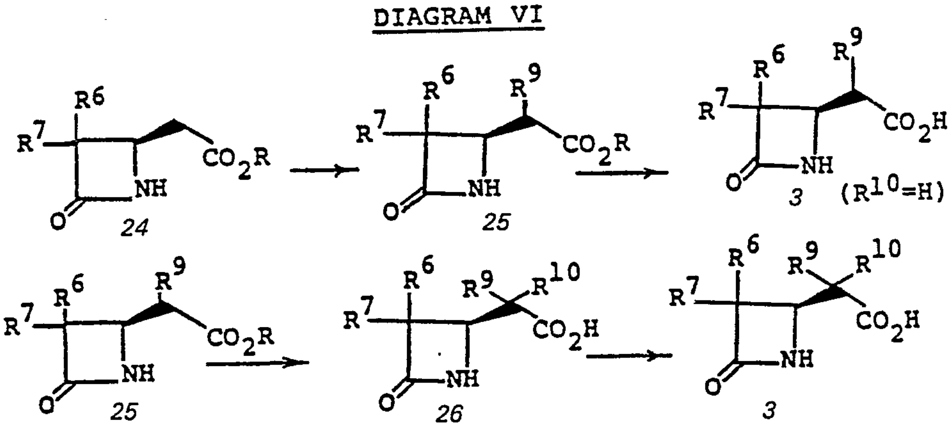

24, 25, 3 ($R^{10}$=H)

25, 26, 3

In words relative to Diagram VI, the azetidinone carboxylate *24* (R=$CH_3$) is treated with 2—2.5 eq of a base such as lithium diisopropylamide or the like in a solvent such as THF, ether or the like at a temperature of from −78°C to −20°C for from 10 minutes to 30 minutes to form a dianion intermediate. The dianion so obtained is then treated with 2 to 100 eq. of a reagent ($R^9X$, $R^{10}X$, X is a leaving group) calculated to establish $R^9/R^{10}$ (such reagents include halides, sulfonates and sulfates, for example: $R^9$-halides, such as iodomethane, iodoethane, $R^9$-sulfonates, or $R^9$-sulfates such as dimethylsulfate, or the like) at −78° to

−25°C for from 0.5 minutes to 3 hours; the reaction is then quenched with 1.0N hydrochloric acid to give chiral species *25*. By repeating the preceding procedure using *25* as starting material, the desired disubstituted azetidinone *26* is obtained. Hydrolysis of *25* or *26* in the presence of 1.0 eq. of NaOH, followed by acidic work up gives chiral 1-substituted and 1,1-disubstituted *3*.

$HSR^8$ Reagents

Relative to the foregoing description of the invention, suitable carbamimidoyl and carbamimidinium mercaptans $HSR^8$ which are utilized in the transformation *2a* to *22* are listed below. Wherein $R^8$ is:

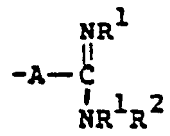
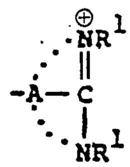
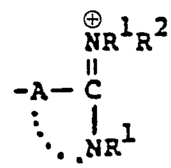
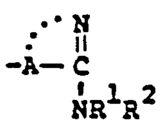
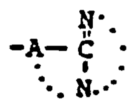
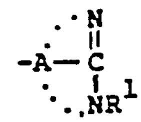
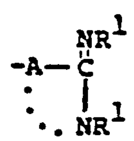
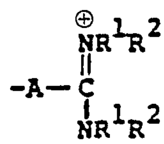
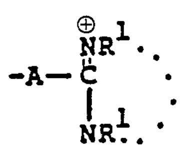
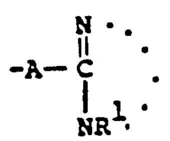
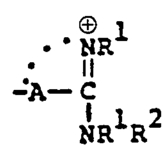
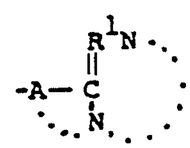

and wherein H, $R^1$ and $R^2$ are as initially defined under $R^8$.

Particularly preferred groups under the definition of $R^1/R^2$ are: hydrogen; substituted and unsubstituted: straight and branched loweralkyl having from 1 to 6 carbon atoms; cycloalkyl having from 3 to 6 carbon atoms, cycloalkylalkyl wherein the cycloalkyl moiety comprises 3 to 6 carbon atoms and the alkyl moiety comprises 1 to 6 carbon atoms; phenyl; benzyl; the substituents on the above-named radicals are selected from fluoro, hydroxy, mercapto, alkoxy and alkylthio having from 1 to 3 carbon atoms.

In defining the bivalent, cyclic or acyclic connector group "A", it is to be noted that the recited radicals of definition are to be read both left to right and right to left. Thus, the preferred connecting groups "A" are selected from: loweralkylene having from 1—6 carbon atoms; cycloalkylene having from 3—10 carbon atoms; cycloalkylalkylene wherein the cycloalkyl moiety comprises 3 to 6 carbon atoms and the alkylene moiety comprises 1 to 10 carbon atoms; alkylcycloalkylene wherein the alkyl moiety comprises 1 to 6 carbon atoms and the cycloalkylene moiety comprises 3 to 6 carbon atoms; loweralkenylene having from 2—10 carbon atoms, cycloalkenylene having from 3 to 10 carbon atoms, cycloalkenylalkylene wherein the cycloalkenyl moiety comprises 3 to 10 carbon atoms; and the alkylene moiety comprises 1 to 6 carbon atoms; alkynylene having from 2 to 10 carbon atoms; phenylene and naphthylene; phenyl-$C_{1-6}$alkylene and $C_{1-6}$alkylphenylene; benzyl, phenethyl and alkylheteroalkyl, wherein the hetero atoms are sulfur, oxygen and nitrogen and the alkyl moiety has 1 to 3 carbon atoms.

A particularly preferred class of connecting groups "A" are selected from: straight and branched loweralkylene having from 1 to 6 carbon atoms, cycloalkylene having from 3 to 6 carbon atoms; phenylene and alkylheteroalkyl wherein the alkyl moiety comprises 1 to 3 carbon atoms and the hetero atoms are sulfur, oxygen and nitrogen.

Representative examples of such preferred —$SR^8$ groups (represented as $HSR^8$) are:

EXAMPLES

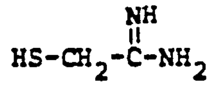

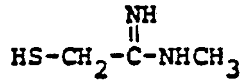

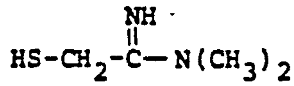

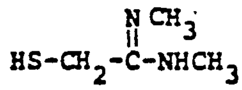

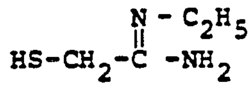

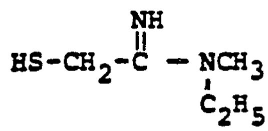

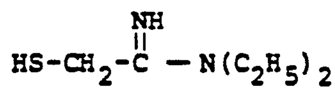

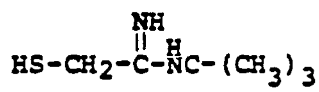

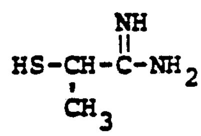

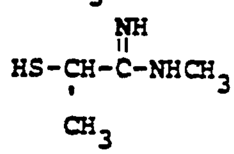

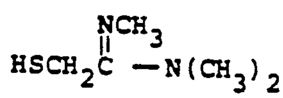

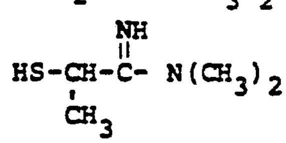

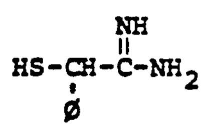

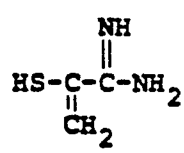

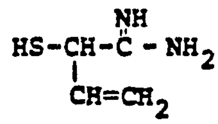

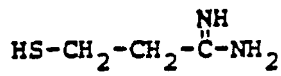

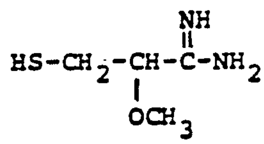

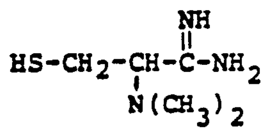

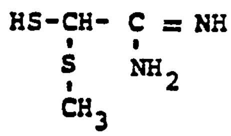

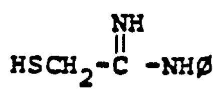

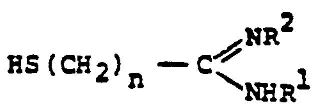

$n = 2\text{-}5$, $R^2 = H$, $CH_3$

$R^1 = H$, $CH_3$

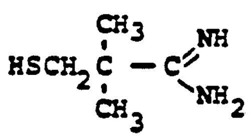

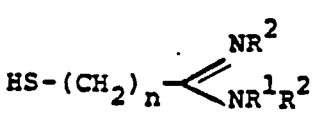

$n = 2\ \text{-}5$, $R^1$, $R^2 = H$, $CH_3$

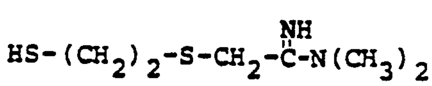

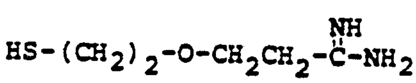

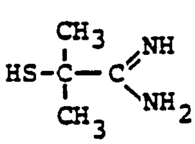

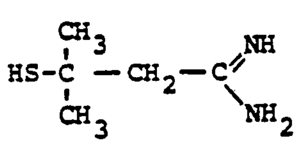

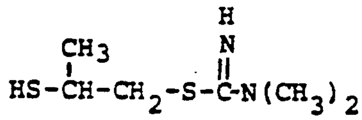

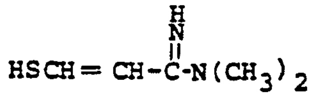

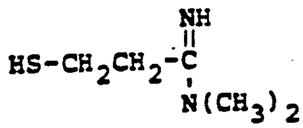

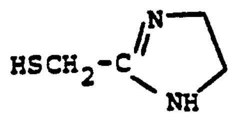

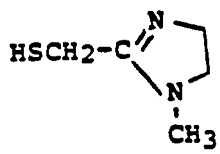

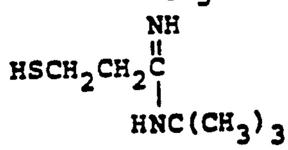

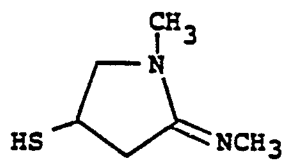

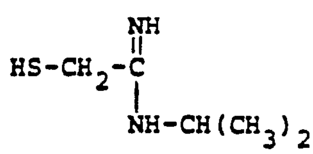

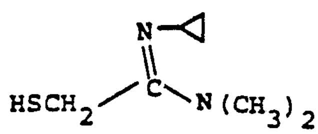

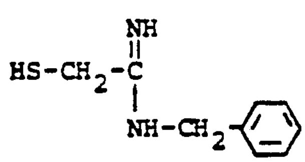

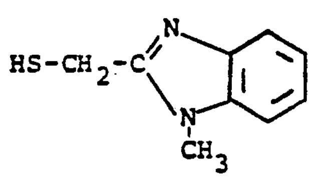

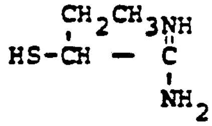

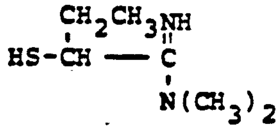

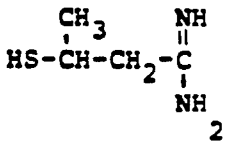

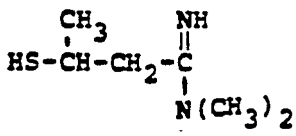

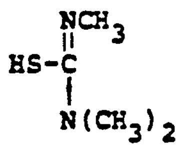

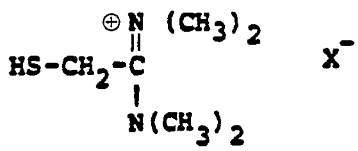

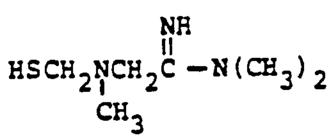

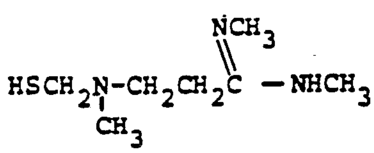

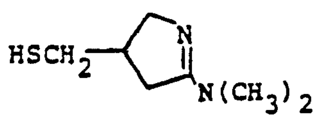

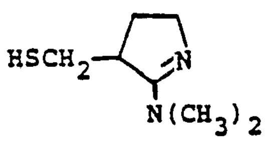

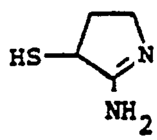

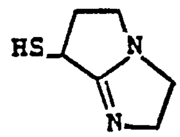

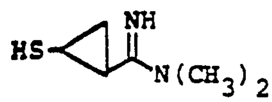

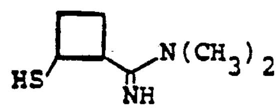

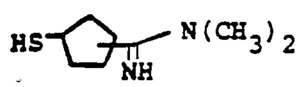

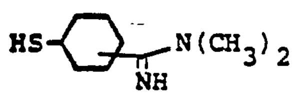

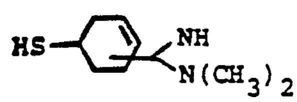

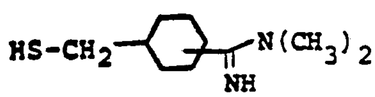

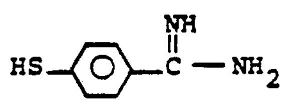

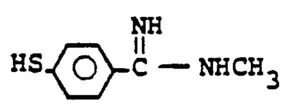

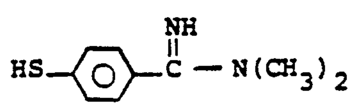

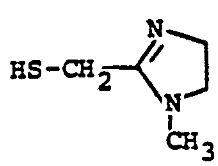

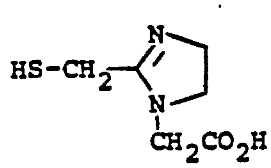

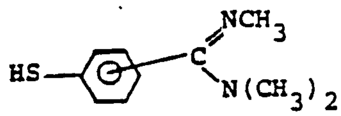

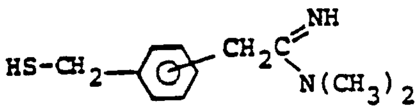

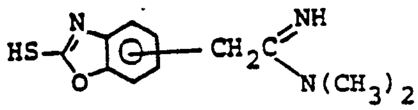

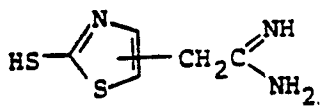

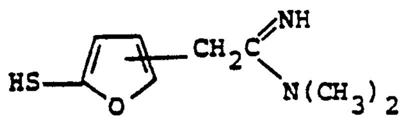

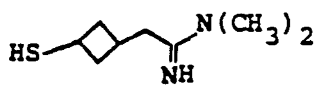

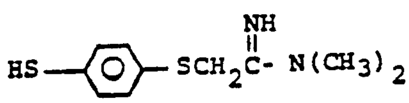

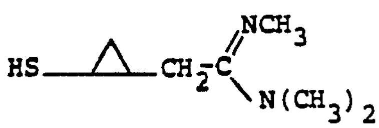

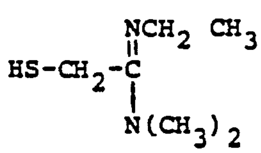

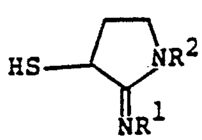

$R^1$ = H, CH3
$R^2$ = H, $CH_3$

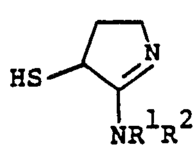

$R^1$ = H, $CH_3$
$R^2$ = H, $CH_3$

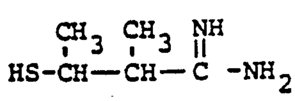

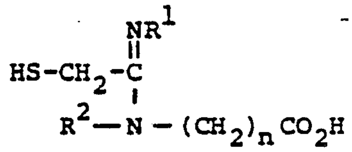

$R^1$ = H, $CH_3$
$R^2$ = H, $CH_3$
n = 1 or 2

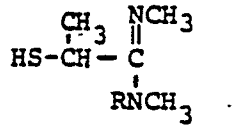

R = H, $CH_3$

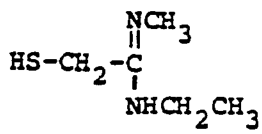

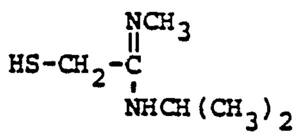

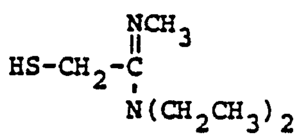

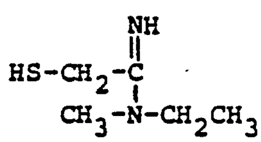

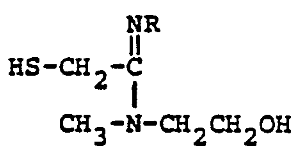

R = $CH_2CH_3$, $CH_3$

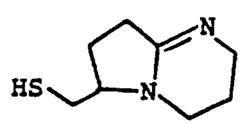

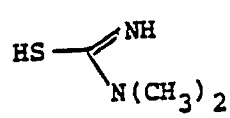

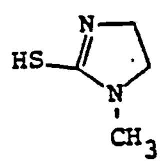

As noted above, the compounds of the present invention may also generally be represented by the following structural formula:

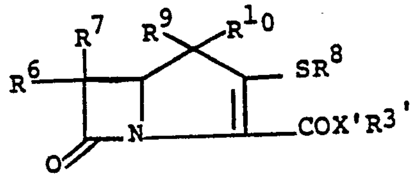

I

wherein X′ is oxygen, sulfur or NR′ (R′ is hydrogen or loweralkyl having from 1 to 6 carbon atoms); and $R^{3'}$ is hydrogen, or, *inter alia*, is representatively selected to provide the pharmaceutically acceptable salt, ester, anhydride ($R^{3'}$ is acyl), and amide moieties known in the bicyclic β-lactam antibiotic art; $R^{3'}$ may also be a readily removable blocking group.

Identification of the Radical —COX′$R^{3'}$

In the generic representation of the compounds of the present invention (I, above), the radical represented by —COX′$R^{3'}$ is, *inter alia*, —COOH (X′ is oxygen and $R^{3'}$ is hydrogen) and all radicals known to be effective as pharmaceutically acceptable ester, anhydride ($R^{3'}$ is acyl) and amide radicals in the bicyclic β-lactam antibiotic art, such as the cephalosporins and penicillins and nuclear analogues thereof.

Suitable, but representative, blocking esters $R^{3'}$ (X = O) include those selected from the following list which is representative:

(i) $R^{3'} = CR^aR^bR^c$ wherein at least of $R^a$, $R^b$ and $R^c$ is an electrondonor, e.g., *p*-methoxyphenyl. The remaining $R^a$, $R^b$ and $R^c$ groups may be hydrogen or organic substituting groups. Suitable ester groups of this type include *p*-methoxybenzyloxycarbonyl.

(ii) $R^{3'} = CR^aR^bR^c$ wherein at least one of $R^a$, $R^b$ and $R^c$ is an electron-attracting group, e.g., *p*-nitrophenyl, trichloromethyl, and o-nitrophenyl. Suitable esters of this tyhpe include *p*-nitrobenzyloxycarbonyl, and 2,2,2-trichloroethoxycarbonyl.

(iii) $R^{3'} = CR^aR^bR^c$ wherein at least two of $R^a$, $R^b$ and $R^c$ are hydrocarbon such as alkyl, e.g., methyl or ethyl, or aryl, e.g., phenyl and the remaining $R^a$, $R^b$ and $R^c$ group, if there is one, is hydrogen. Suitable esters of this type include t-butyloxycarbonyl, diphenylmethoxycarbonyl and triphenylmethoxycarbonyl.

Silyl esters. This category of blocking groups, may conveniently be prepared from a halosilane of the formula: $R^4_3SiX'$ wherein X′ is a halogen such as chloro or bromo and $R^4$ is alkyl, having 1—6 carbon atoms, phenyl, or phenylalkyl.

Pharmaceutically acceptable carboxyl derivatives of the present invention are those derived by reacting I with alcohols, acylating reagents and the like. For example, esters and amides of interest are the above-listed starting materials and final products having the —COX′$R^{3'}$ group at the 3-position; wherein X′ is oxygen, sulfur or NR′ (R′ is H or $R^{3'}$), and $R^{3'}$ is alkyl having 1—6 carbon atoms, straight or branched, such as methyl, ethyl, t-butyl, and the like; carbonylmethyl, including phenacyl; aminoalkyl including 2-methylaminoethyl, 2-diethylaminoethyl; alkanoyloxyalkyl wherein the alkanoyloxy portion is straight or branched and has 1—6 carbon atoms and the alkylportion has 1—6 carbon atoms, such as pivaloyloxymethyl; haloalkyl wherein halo is chloro, and the alkyl portion is straight or branched having 1—6 carbon atoms, e.g., 2,2,2-trichloroethyl; alkenyl having 2—4 carbon atoms, such as 2-propenyl, 3-butenyl, and 4-butenyl; aralkyl and lower alkoxyl- and nitro-substituted aralkyl such as benzyl, benzhydryl, o-nitrobenzyl, p-methoxybenzyl, and p-nitrobenzyl; phthalidyl; benzyloxyalkyl having 8—10 carbon atomes such as benzyloxymethyl, and (4-nitro) benzyloxymethyl.

In addition to the esters (and thio esters) listed above, amides are also embraced by the present invention, i.e., wherein X′ is the

R′
|
—N—

group. Representatives of such amides are those wherein R′ is selected from the group consisting of hydrogen and alkyl such as methyl and ethyl.

The most preferred —COX′$R^{3'}$ radicals of the present invention are those wherein (relative to Structure I above), X′ is oxygen and $R^{3'}$ is hydrogen; loweralkyl having 1—4 carbon atoms; lower alkenyl such as 3-methylbutenyl, 4-butenyl and the like; benzyl and substituted benzyl such as p-nitrobenzyl; pivaloyloxymethyl, 3-phthalidyl; and phenacyl.

PREFERRED VALUES FOR $R^9$ and $R^{10}$

In the generic structure (I):

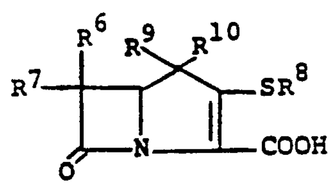

I

The following are preferred for $R^9$ and $R^{10}$:

$CH_2CH_3$

$CH(CH_3)_2$

$CH_2C_6H_5$

$CH_2F$

$CHF_2$

$CF_3$

$CH_2-Br$

$CH_2-OH$

$CH_2OMe$

$CH_2SMe$

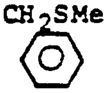

$-CH=CH_2$

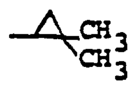

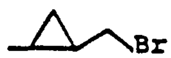

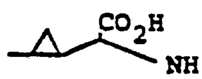

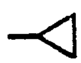

PREFERRED VALUES FOR $R^6$ and $R^7$

In the generic structure (I):

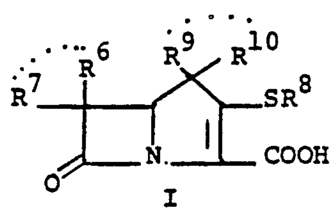

I

The preferred values for $R^6$ and $R^7$ independently are selected from:

$CH_3CH_2$

$CH_3$

OH
$CH_3CH_2-CH$

OH
$CH_2FCH$

OH
$CF_3CH$

$CH_2OH$

SH
$CH_3CH$

OH
$CH_3CH_2CH_2-CH$

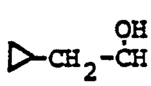

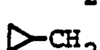

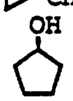

$CH_2NH_2$

$CH_2CHF_2$

$R^6 + R^7$

$CH_2=$

$CH_3CH_2OH$

$(CH_3)_2\overset{O}{\overset{\|}{C}}$

$(CH_3)_2\overset{OH}{C}$

$CH_3CF_2$

$ØCH_2$

$(CH_3)_2CH\overset{OH}{C}H$

$CH_3\overset{NH_2}{C}H$

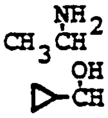

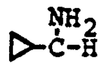

$ØCH_2-\overset{OH}{C}H$

$ØCH_2-\overset{OH}{C}H$

$CO_2Me$

$CONH_2$

$\overset{O}{\overset{\|}{C}}NHCH_3$

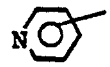

$CH_3\overset{SCH_3}{C}H$

HOCH=

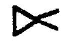

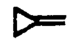

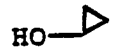

An especially preferred substitution at position 6 finds $R^7$=H, and $R^6$ selected from

$FCH_2CH(OH)-$, $CH_3CH_2CH(OH)-$, $CH_3CH_2-$, $(CH_3)_2CH-$, 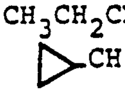CH(OH)-, and $(CH_3)_2C(OH)-$.

Preferred embodiments of the present invention employ the 1-hydroxyethyl substituent at ring position 6. The foregoing description is repeated below to demonstrate these embodiments. All symbolism is as previously defined. These embodiments are both mono- and di-substituted as ring position 1.

DIAGRAM I

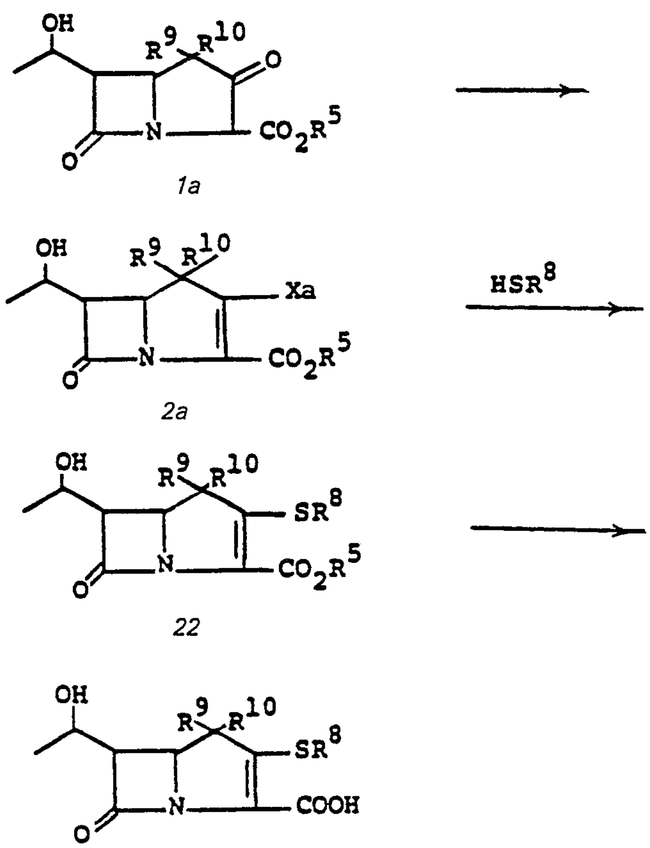


1a

2a

22

I

In words relative to the above reaction scheme, Diagram I, the step *1a* to *2a* to establish leaving group $X^a$ is accomplished by acylating the bicyclic keto ester 1a with an acylating agent $RX^a$ such as p-toluenesulfonic acid anhydride, p-nitrophenylsulfonic acid anhydride, 2,4,6-triisopropylphenylsulfonic acid anhydride, 2,4,6-triisopropylphenylsulfonic acid anhydride, methanesulfonic acid anhydride, trifluoromethane sulfonic acid anhydride, diphenyl chlorophosphate, toluenesulfonyl chloride, p-bromophenylsulfonyl chloride, or the like; wherein $X^a$ is the corresponding leaving group such as toluene sulfonyloxy, p-nitrophenylsulfonyloxy, benzenesulfonyloxy, diphenylphosphoryl, and other leaving groups which are established by conventional procedures and are well known in the art. Typically, the above acylation to establish leaving group $X^a$ is conducted in a solvent such as methylene chloride, acetonitrile or dimethylformamide, in the presence of a base such as diisopropylethylamine, triethylamine, 4-dimethylaminopyridine or the like at a temperature of from −20 to 40°C for from 0.1 to 5 hours. The leaving group $X^a$ of intermediate *2a* can also be halogen. The halogen leaving group is established by treating *1a* with a halogenating agent such as $\phi_3PCl_2$, $\phi_3PBr_2$, $(\phi O)_3PBr_2$, oxalyl chloride or the like in a solvent such as $CH_2Cl_2$, $CH_3CN$, THF, or the like in the presence of a base such as diisopropylethylamine, triethylamine, or 4-dimethylaminopyridine or the like. [$\phi$ = phenyl.]

The reaction *2a* to *22* is accomplished by treating *2a* in a solvent such as dioxane, dimethylformamide, dimethylsulfoxide, acetonitrile, hexamethylphosphoramide, or the like, in the presence of an approximately equivalent to excess of the mercaptan reagent $HSR^8$, wherein $R^8$ is defined above, in the presence of a base such as sodium hydrogen carbonate, potassium carbonate, triethylamine, diisopropylethylamine, or the like at a temperature of from −40 to 25°C for from 30 sec. to 1 hour.

The final deblocking step *22* to *I* is accomplished by conventional procedures such as solvolysis or hydrogenation. The conditions of deblocking *22* to *I* are thus: typically *22* in a solvent such as tetrahydrofuran-water, tetrahydrofuran-ethanol-water, dioxane-water, dioxane-ethanol-water, n-butanol-water, or the like containing pH 7 morpholinopropanesulfonic acid-sodium hydroxide buffer, pH 7 phosphate buffer, dipotassium hydrogen phosphate, sodium bicarbonate, or the like, is treated under a hydrogen pressure of from 1 to 4 atmospheres in the presence of a catalyst such as platinum oxide, palladium on charcoal, or palladium hydroxide on charcoal, or the like at a temperature of from 0 to 50°C for from 0.25 to 4 hours to provide I. Photolysis, when $R^5$ is a group such as o-nitrobenzyl, for example, may also be used for deblocking.

Relative to Diagram I, the bicyclic keto ester *1a* may be obtained by the following scheme, Diagram II.

DIAGRAM II

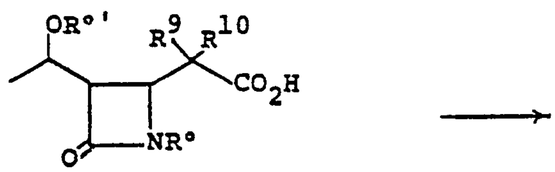

3

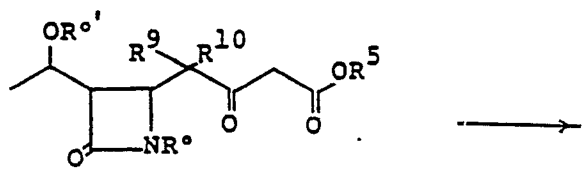

4

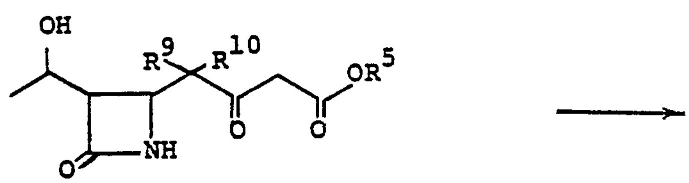

5

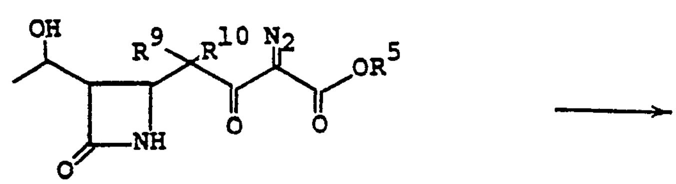

6

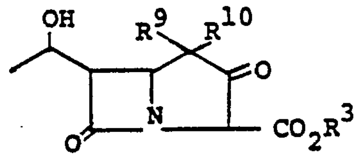

*1a*

The addition *3* to *4* is accomplished by treating 3 with 1,1'-carbonyldiimidazole, or the like, in a solvent such as tetrahydrofuran (THF), dimethoxyethane, acetonitrile or the like, at a temperature of from 0 to 70°C, followed by the addition of 1.1 to 3.0 equivalent of $(R\ O_2CCH_2CO_2)_2Mg$, at a temperature of from 0 to 70°C for from 1 to 48 hours. The addition *3* to *4* can also be achieved by using unprotected starting material *3* ($R^{o\prime} = R^{o} = H$, or partially protected *3*: $R^{o} = H$, $R^{o\prime}$ = triorganosilyl). The group $R^5$ is a pharmaceutically acceptable ester moiety or a readily removable carboxyl protecting group such as p-nitrobenzyl, benzyl, or the like. The term "triorganosilyl" embraces those conventionally employed; wherein the organo moiety is independently selected from alkyl having 1—6 carbon atoms, phenyl, and phenylalkyl.

Removal of protecting groups $R^{o\prime}$ and $R^{o}$ (*4* to *5*) (when $R^{o}$ and $R^{o\prime}$ are triorganosilyl such as t-butyldimethylsilyl) is accomplished by acidic aqueous hydrolysis of *4* in a solvent such as methanol, ethanol, tetrahydrofuran, dioxane, or the like, in the presence of an acid such as hydrochloric, sulfuric, acetic or the like at a temperature of from 0 to 100°C for from 0.5 to 18 hours.

The diazo species *6* is prepared from *5* by treating *5* in a solvent such as $CH_3CN$, $CH_2Cl_2$, THF, or the like with an azide such a p-carboxybenzenesulfonylazide, p-toluenesulfonylazide, methanesulfonylazide, or the like in the presence of a base such as triethylamine, pyridine, diethylamine or the like for from 1 to 50 hours at 0—50°C.

Cyclization (*6* to *1a*) is accomplished by treating *6* in a solvent such as benzene, toluene, THF, cyclohexane, ethylacetate or the like at a temperature of from 25 to 110°C for from 1—5 hours in the presence of a catalyst such as *bis* (acetylacetonato)Cu(II)[$Cu(acac)_2$], $CuSO_4$, Cu powder, $Rh_2(OAc)_4$ or $Pd(OAc)_2$. Alternatively, the cyclization may be accomplished by irradiating *6* through a pyrex filter (a wave length greater than 300 nm) in a solvent such as benzene, $CCl_4$, diethylether, or the like, at a temperature of from 0—25°C for from 0.5 to 2 hours. ("OAc" = acetate.]

Relative to Diagram II, the following scheme, Diagram III, crosses at intermediate *3*.

DIAGRAM III

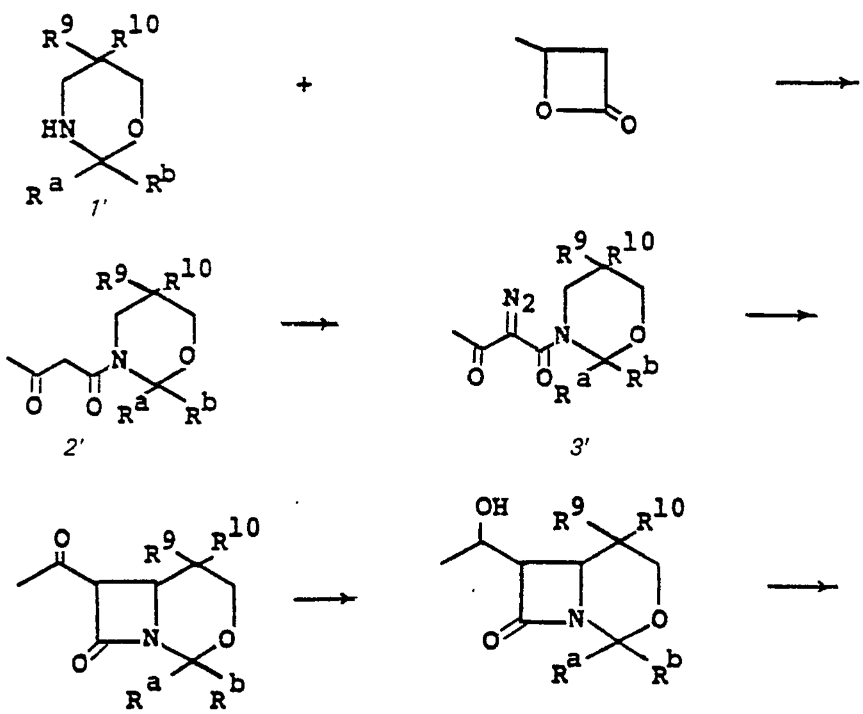

*1'*

*2'*

*3'*

*4'*

*5'*

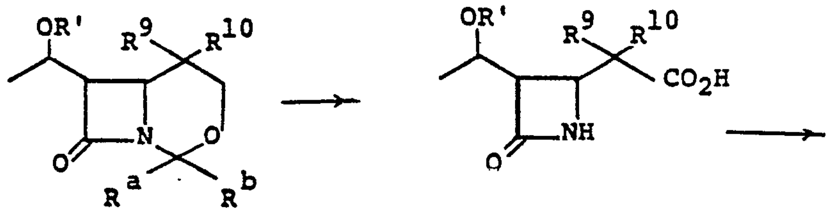

6' 7'

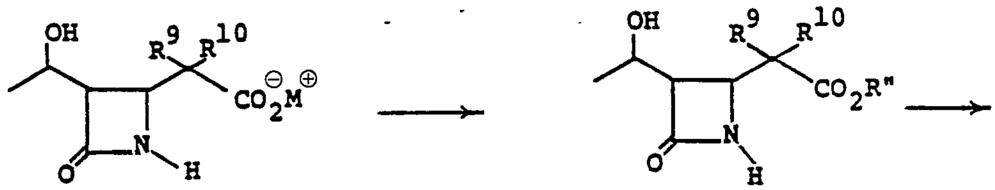

8' 9'

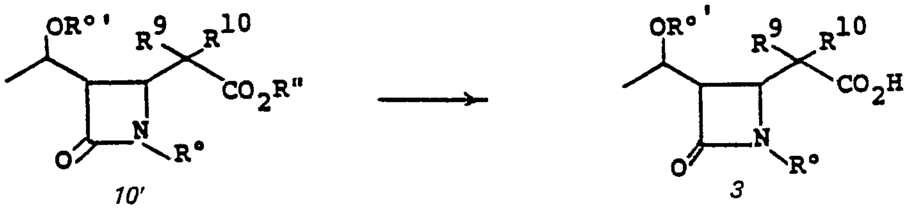

10' 3

In words relative to Diagram III for the preparation of *3*, 5,5-disubstituted 1,3-oxazine *1'* (wherein $R^9$ and $R^{10}$ are as defined in I; $R^a$ and $R^b$ are independently selected from the group of loweralkyl having 1—6 carbon atoms such as methyl, ethyl, and the like, or $R^a$ and $R^b$ are joined to form a spirocycloalkyl, such as spirocyclohexyl, and the like) is treated with diketene in a solvent such as ethanol, methanol or the like at a temperature of from −10°C to 50°C for from 1 hr to 6 hrs to give adduct *2'*.

The β-ketoamide *2'* is diazotized with a diazotizing agent such as p-toluenesulfonylazide, methanesulfonylazide, p-carboxylbenzenesulfonylazide, or the like in a solvent such as methylene chloride, acetonitrile, tetrahydrofuran or the like in the presence of a base such as triethylamine, pyridine, diethylamine, or the like for from 10 min, to 4 hours at 0°—50°C to give diazo species *3'*.

The cyclization (*3'* to *4'*) is accomplished by treating *3'* in a solvent such as benzene, toluene, THF, cyclohexane, ethylacetate or the like at a temperature of from 25 to 110°C., for 10 min to 5 hours in the presence of a catalyst such as copper(II) sulfate, copper powder, rhodium acetate, palladium acetate, or the like. Alternatively, the cyclization may be accomplished by irradiating *3'* through a pyrex filter (a wave length greater than 300 nm) in a solvent such as benzene, $CCl_4$, diethylether, or the like at a temperature of from 0—25°C for from 0.5 to 2 hours.

Treatment of ketone *4'* with a reducing agent such as sodium borohydride, lithium borohydride, or the like in a solvent such as THF, ethylether, or the like at a temperature of from 0 to 25°C for from 0.5 to 5 hours affords alcohol *5'*. The free hydroxy group of *5'* is protected with an acid stable blocking group R' such as p-nitrobenzyloxycarbonyl, o-nitrobenzoxycarbonyl, or the like by treating *5'* with 1 to 2 equivalents of chloroformate such as p-nitrobenzylchloroformate in a solvent such as DMF, THF, methylene chloride, or the like in the presence of a base such as p-dimethylaminopyridine, pyridine, triethylamine, or the like at a temperature of from −20 to 60°C for from 0.5 to 6 hours.

The conversion *6'* to *7'* is obtained by oxidation. The most preferred oxidation reaction is achieved by suspending *6'* in a solvent such as acetone, benzene, hexane, or the like at a temperature of from 0°C to 50°C and treating with an oxidizing agent such as Jones reagent. Alternatively, compound *7'* may be prepared from *6'* by reaction with 50% trifluoroacetic acid/water at 0° to 50°C for from 10 min to 1 hr. to give the intermediate alcohol which is then oxidized with Jones reagent to *8'*. The partially protected *7'* (R' = $CO_2$PNB) can also be used as the starting material for the chain extension reaction (*3* to *4*, Diagram II). However, because of restricted solubility of *7'* in organic solvent, it is preferable to use a soluble intermediate such as N,O-bis-organosilyl protected species *3* ($R^{o\prime}$ = $R^o$ = triorganosilyl) or O-silyl protected *3* ($R^o$ = H, R = triorganosilyl). The exchange of protecting group of *7'* is accomplished by hydrolysis of *7'* in an alkaline media such as aqueous NaOH, KOH or the like to provide carboxylic acid salt *8'* followed by esterification of *8'* with p-nitrobenzylbromide in DMF at room temperature for 1—8 hrs to give *9'* (R'' is p-nitrobenzyl).

Treatment of *9'* with triorganosilyl chloride such as t-butyldimethylchlorosilane, trimethylchlorosilane, or the like in the presence of a strong base such as triethylamine, diisopropylethylamine or the like in a solvent such as DMF, methylene chloride, at −20 to 50°C for 0.5 to 8 hrs affords N,O-bis-protected species *10'* ($R^{o\prime}$ = $R^o$ = triorganosilyl). Selective O-silylation of *9'* to give *10'* ($R^{o\prime}$ = triorganosilyl, $R^o$ = H) is accom-

plished by using a weak base such as imidazole to replace the strong base in the proceeding silylation reaction. [Typically, the "organo" moiety in the reagent triorganosilyl is independently selected from alkyl, aryl, or aralkyl; wherein the alkyl has 1—6 carbon atoms and the aryl is phenyl.]

Hydrogenolysis of *10'* ($R^{o\prime} = R^{o}$ are triorganosilyl, such as t-butyldimethylsilyl) in the presence of a noble metal catalyst such as 10% Pd/C, $PtO_2$, or the like in a solvent such as ethylacetate, benzene, or the like at room temperature for from 30 min. to 3 hrs affords N,O-diprotected free acid *3*.

With respect to starting material *1'* (Diagram III), its preparation is summarized in Diagram IV.

DIAGRAM IV

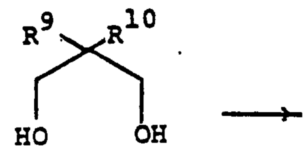

*11'*

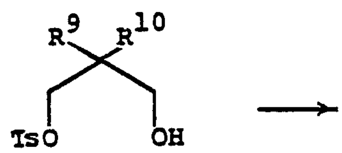

*12'*

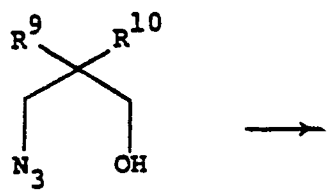

*13'*

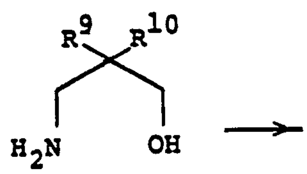

*14'*

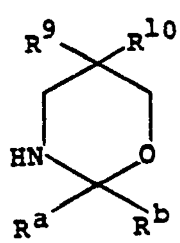

*1'*.

In words relative to Diagram IV, the substituted 1,3-oxazine *1'* (wherein $R^9$, $R^{10}$, $R^a$ and $R^b$ are as defined in Diagram III) is prepared by treating 3,3-disubstituted 1,5-pentanediol *11'* with 0.5 to 1.0 equivalents of p-toluenesulfonylchloride in a solvent such as DMF, THF, methylene chloride, pyridine, or the like in the presence of base such as triethylamine, pyridine or the like at a temperature of from 0 to 50°C for from 0.5 to 5 hours. The mono-tosyl alcohol *12'* is treated with 1 to 5 equivalents of sodium azide in a reaction medium such as polyethylene glycol (m.w. 200 to 600) at a temperature of from 90 to 140°C and the product, azido alcohol *13'*, is obtained by continuing collection of the distillate from the reacting mixture in 1 to 8 hrs. Reduction of the azido alcohol to the corresponding amino alcohol (*13'* to *14'*) is accomplished by hydrogenation of *13'* under 1 to 50 atm. of hydrogen in a solvent such as cyclohexane, methanol, ethylacetate, or the like in the presence of a catalyst such as 10% palladium on charcoal, palladium oxide, platinum or the like at a temperature of from 0 to 50°C for from 2 to 20 hours. Condensation of *14'* with a ketone such as cyclohexanone, cyclopentanone, acetone, or the like in a solvent such as cyclohexane, benzene, toluene, or the like with azeotropic removal of water by an apparatus such as Dean-Stark trap at reflux for 0.5 to 6 hours affords the desired substituted 1,3-oxazine *1'*. The ketone in condensation with *14'* may generically be represented as

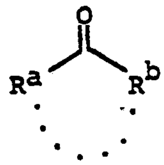

wherein $R^a$ and $R^b$ are as previously defined and the dotted line indicates that $R^a$ and $R^b$ may be joined.

Alternatively, the azetidinone carboxylic acid *3* may be prepared from 3-substituted 1,4-butadiene (Diagram V).

DIAGRAM V

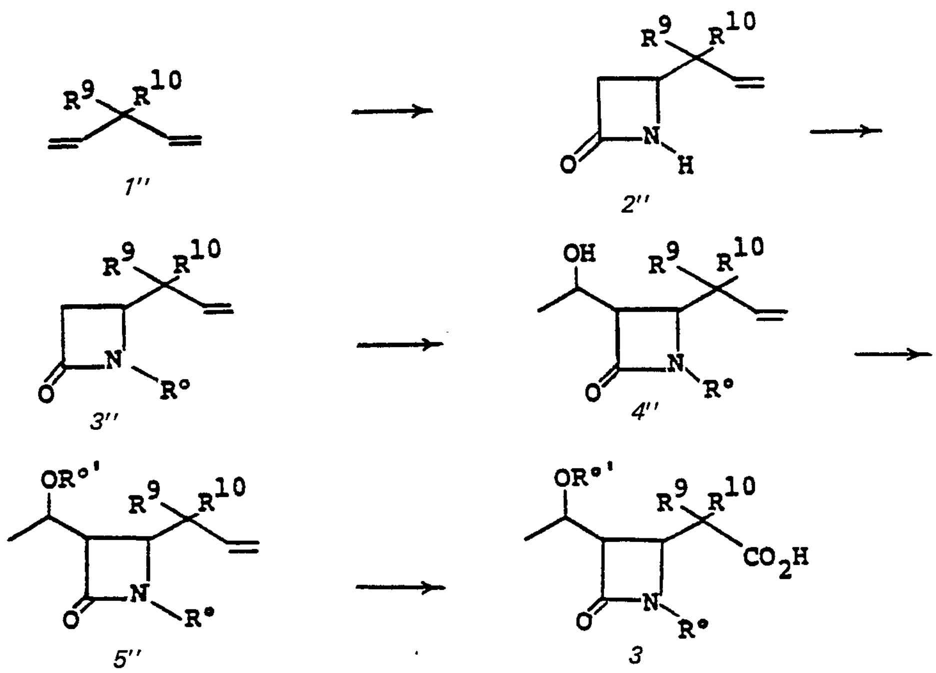

*1''* *2''* *3''* *4''* *5''* *3*

In words relative to Diagram V, the substituted azetidinone *2''* is prepared by reacting a 3-substituted 1,4-pentadiene *1''* with chlorosulfonylisocyanate at 25°C to 60°C in a pressure bottle for 3—12 days. The resulting mixture is hydrolyzed with aqueous sodium sulfite solution between pH 6.5—7.5 at 0°C to 25°C for from 5 min. to 60 min.

Azetidinone *2''* is transformed (*2''* to *3''*) to establish the protecting group R° which may be a triorganosilyl group, such as t-butyldimethylsilyl, t-butyldiphenylsilyl, triphenylsilyl, isopropyldimethylsilyl, for example, or may be 3,4-dimethoxybenzyl, for example. Silyl protection is preferred, and typically R° is established by treating *2''* in a solvent such as dimethylformamide, acetonitrile, hexamethylphosphoramide, tetrahydrofuran or the like with a silylating agent such as t-butyldimethylchlorosilane, t-butyldiphenylchlorosilane, triphenylchlorosilane, or the like at a temperature of from −20° to 25°C for from 0.5 to 24 hours in the presence of a base such as triethylamine, diisopropylethylamine.

Alkylation of *3''* provides *4''*. Typically, *3''* is treated with a strong base such as lithium diisopropylamide, sodium hydride, phenyl lithium, butyl lithium, or the like in a solvent such as tetrahydrofuran, ether, dimethoxyethane or the like at a temperature of from −80°C to 0°C, whereupon the alkylating agent of choice, acetaldehyde is introduced.

The free hydroxyl group of *4''* is protected by a triorganosilyl group such as t-butyldimethylsilyl by treating *4''* with t-butyldimethylchlorosilane and p-dimethylaminopyridine in a solvent such as DMF, acetonitrile, $CH_2Cl_2$ or the like at −20 to 60°C for from 0.5 to 8 hours.

The oxidation *5''* to *3* is accomplished by treating 5'' in a solvent such as methylenechloride, methanol, or the like, with ozone, at a temperature of from −100° to 0°C for from 0.1 to 4 hours, followed by treating the crude product with an oxidizing agent such as m-chloroperbenzoic acid, hydrogen peroxide, peracetic

acid, or the like, at a temperature of from 0°C to 100°C for from 1 to 100 hours. $R^{\circ\prime}$ and $R^{\circ}$ are readily removable protecting groups such as triorganosilyl.

With respect to starting reagent *1''*, its preparation is generally described in *J. Amer. Chem. Soc., 74,* 661 (1952) by E. B. Reid and T. E. Gompf, *J. Org. Chem., 23,* 1063 (1958) by R. Ciola and K. L. Burwell, Jr., and Belgium Patent 632,193 (1963) by R. Polster and E. Scharf. The following scheme summarizes the preparation of *1''*.

DIAGRAM VI

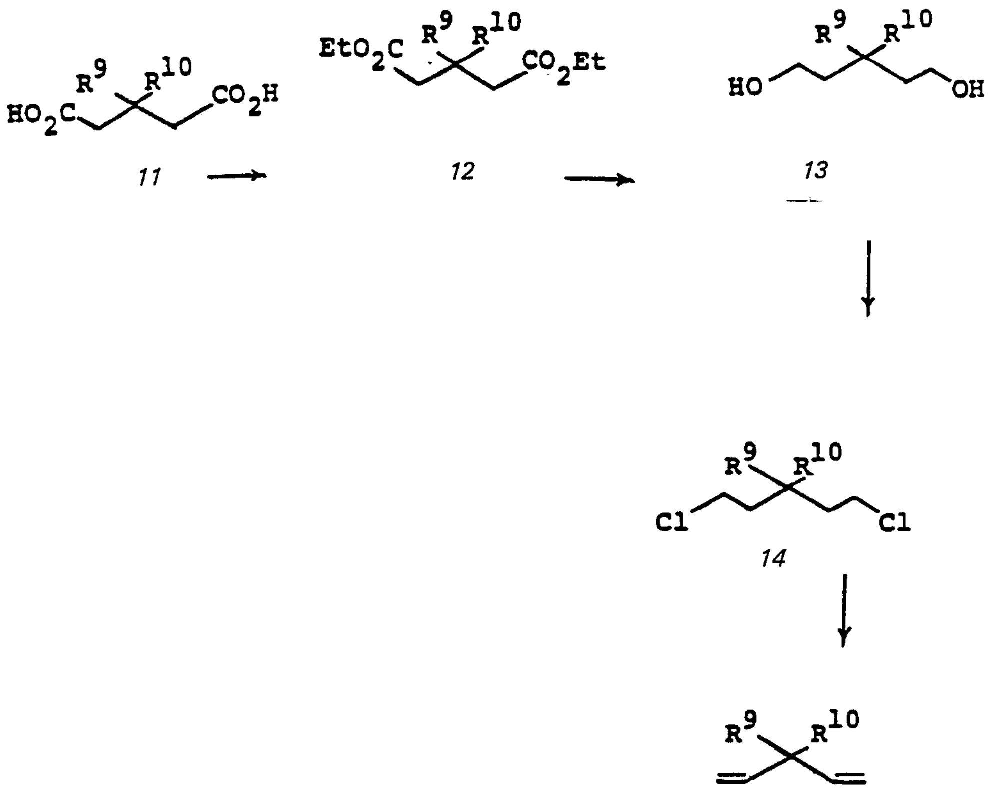

*11* *12* *13* *14* *1''*

In words relative to Diagram VI, the diester *12* is prepared by treating the diacid 11 with thionyl chloride at reflux for two hours followed by reacting with ethanol at 80°C for 4 hours. Reduction of the diester *12* with lithium aluminum hydride in ether at reflux for 4 hours followed by hydrolysis with 10% NaOH gives diol *13* which on further reaction with thionyl chloride gives dichloride *14*. Reaction of the dichloride 14 with base such as 2-methylquinoline, DBU or sodium hydroxide in polyethylene glycol gives the expected 3-substituted 1,4-pentadiene 1''.

The foregoing Diagrams (I—VI) describe the synthesis of racemic 1,1-disubstituted 6-[1-hydroxyethyl]-2-carbamimidoyl-1-carbadethiapen-2-em-3-carboxylic acids I.

The preferred comfiguration of final products I is represented by the following drawing:

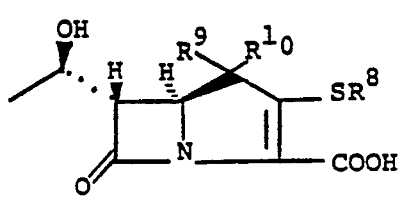

I

The corresponding configuration at the level of intermediate *3* is represented by the following drawing:

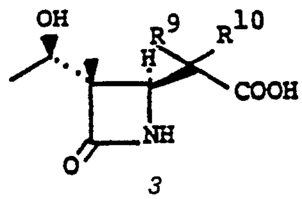

3

With respect to the chiral synthesis of I, the racemic azetidinone carboxylic acid *3* (R°' and R° are H or protecting groups, Diagram II) is resolved according to conventional optical resolution techniques, such as fractional crystallization of optically active ammonium salts, esters, or chromatographic separation of such esters.

Alternatively, the chiral azetidinone intermediates *3*—*6* (Diagram II) may also be conveniently prepared *via* alkylation of the corresponding unsubstituted chiral azetidinone *27, 31* and *35* (Diagram VII, below). Chiral precursors *24, 28* and *32* are known. Diagram VIII summarizes these reactions:

Diagram VIII

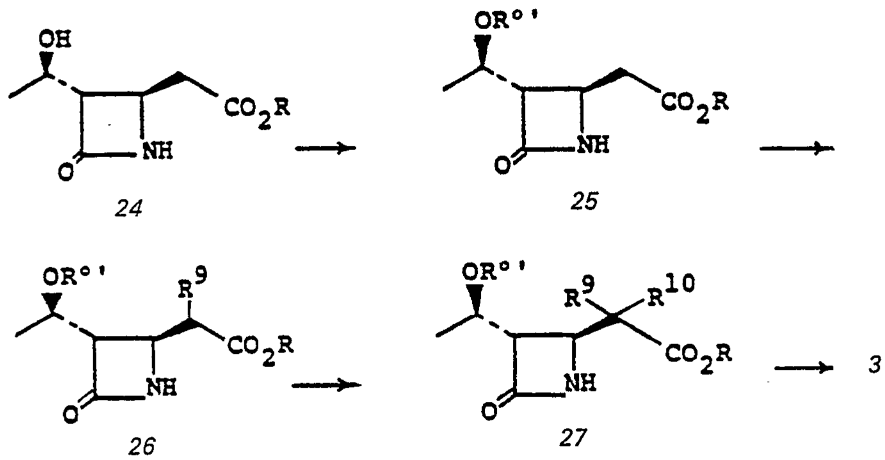

24 25 26 27 3

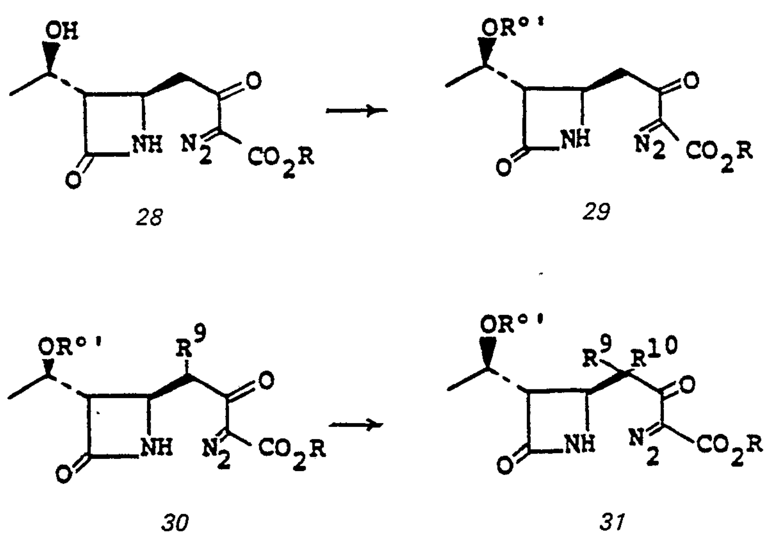

28 29 30 31

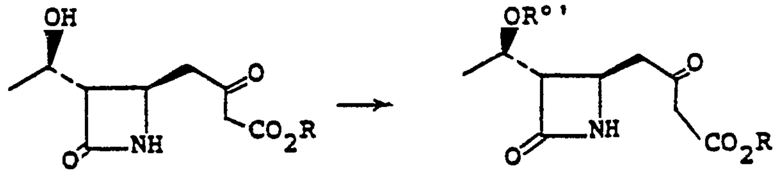

32 33

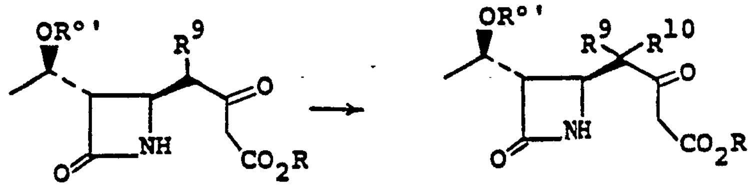

34 35

In words relative to Diagram VIII, the free hydroxy function of the azetidinone carboxylate *24* (R = $CH_3$) is selectively protected by treating *24* with 1—2 eq of a triorganosilylating agent such as t-butyldimethylchlorosilane in a solvent such as DMF, $CH_2Cl_2$, or the like, in the presence of 2—5 eq. of imidazole at room temperature for 1 to 8 hrs. to give *25* (R = $CH_3$, R°' = t-Butyldimethylsilyl, for example). Treatment of *25* at −78°C under a nitrogen atmosphere with 2—2.5 eq. of a base such as lithium diisopropylamide or the like in a solvent such as THF, ether or the like for from 10 min. to 30 min. yields dianion intermediate. The dianion so obtained is then treated with 2 to 100 q. of a reagent ($R^9X$, $R^{10}X$, X is a leaving group) calculated to establish $R^9/R^{10}$ (such reagents include halides, sulfonates, and sulfates, for example: $R^9$-halides, such as iodomethane, iodoethane, $R^9$ sulfonates, or $R^9$-sulfates such as dimethylsulfate, or the like) at −78° to −25°C for from 0.5 min. to 3 hrs; the reaction is then quenched with 1.0*N* hydrochloric acid to give chiral species *26*. By repeating the preceding procedure using *26* as starting material, the desired disubstituted azetidinone *27* is obtained; hydrolysis of *27* in the presence of 1 eq of NaOH, followed by acidic work up gives chiral species *3*.

Similarly prepared are *31* and *35* from *28* and *32*, respectively, by the procedure described above. However, it should be noted that the transformation *33* to *34* (or *34* to *35*) nominally requires an additional equivalent of base.

The compounds of the present invention (I) are valuable antibiotics active against various Gram-positive and Gram-negative bacteria and accordingly find utility in human and veterinary medicine. Representative pathogens which are sensitive to antibiotics I include: *Staphylococcus aureus, Escherichia coli, Klebsiella pneumoniae, Bacillus subtilis, Salmonella typhosa Pseudomonas* and *Bacterium proteus*. The antibacterials of the invention are not limited to utility as medicaments; they may be used in all manner of industry, for example: additives to animal feed, preservation of food, disinfectants, and in other industrial systems where control of bacterial growth is desired. For example, they may be employed in aqueous compositions in concentrations ranging from 0.1 to 100 parts of antibiotic per million parts of solution in order to destroy or inhibit the growth of harmful bacteria on medical and dental equipment and as bactericides in industrial applications, for example in waterbased paints and in the white water of paper mills to inhibit the growth of harmful bacteria.

The products of this invention may be used in any of a variety of pharmaceutical preparations. They may be employed in capsule, powder form, in liquid solution, or in suspension. They may be administered by a variety of means; those of principal interest include: orally, topically or parenterally by injection (intravenously or intramuscularly).

Such tablets and capsules, designed for oral administration, may be in unit dosage form, and may contain conventional excipients, such as binding agents, for example, syrup, acacia, gelatin, sorbitol, tragacanth, or polyvinylpyrrolidone; fillers, for example, lactose, sugar, cornstarch, calcium phosphate, sorbitol, or glycerine; lubricants, for example, magnesium stearate, talc, polyethylene glycol, silica; disintegrants, for example, potato starch, acceptable wetting agents such as sodium lauryl sulphate. The tablets may be coated according to methods well known in the art. Oral liquid preparations may be in the form of aqueous or oily suspensions, or solutions, or they may be presented as a dry product for reconstitution with water or other suitable vehicle before use. Such liquid preparations may contain conventional additives such as suspending agents, for example, sorbitol, methyl cellulose, glucose/sugar syrup, gelatin, hydroxyethylcellulose, or carboxymethyl cellulose. Suppositories will contain conventional suppository bases, such as cocoa butter or other glycerides.

Compositions for injection, the preferred route of delivery, may be prepared in unit dosage form in ampules, or in multidose containers. The compositions may take such forms as suspensions, solutions, or emulsions in oily or aqueous vehicles, and may contain formulatory agents. Alternatively, the active ingredient may be in powder form for reconstitution, at the time of delivery, with a suitable vehicle, such as sterile water.

The compositions may also be prepared in suitable forms for absorption through the mucous membranes of the nose and throat or bronchial tissues and may conveniently take the form of liquid sprays or inhalants, lozenges, or throat paints. For medication of the eyes or ears, the preparation may be presented in liquid or semi-solid form. Topical applications may be formulated in hydrophobic or hydrophilic bases as ointments, creams, lotions, paints, or powders.

The dosage to be administered depends to a large extent upon the condition and size of the subject being treated as well as the route and frequency of administration — the parenteral route by injection being preferred for generalized infections. Such matters, however, are left to the routine discretion of the therapist according to principles of treatment well known in the antibiotic art. In general, a daily dosage consists of from about 5 to about 600 mg of active ingredient per kg of body weight of the subject in one or more treatments per day. A preferred daily dosage for adult humans lies in the range of from about 10 to 240 mg of active ingredient per kg of body weight. Another factor influencing the precise dosage regimen, apart from the nature of the infection and peculiar identity of the individual being treated, is the molecular weight of the chosen species of this invention (I).

The compositions for human delivery per unit dosage, whether liquid or solid, may contain from 0.1% to 99% of active material, the preferred range being from about 10—60%. The composition will generally contain from about 15 mg to about 1500 mg of the active ingredient; however, in general, it is preferable to employ a dosage amount in the range of from about 250 mg to 1000 mg. In parenteral administration, the unit dosage is usually the pure compound I in sterile water solution or in the form of a soluble powder intended for solution. For zwitterionic species described under Structure I, the pH of such solutions typically will correspond to the zwitterionic point; however, consideration of individual properties of solubility and stability may require such aqueous solutions to have a pH other than that of the zwitterionic point, for example in the range of 5.5 to 8.2.

In the foregoing word description of the above, schematic reaction diagram for the total synthesis of the defined carbapenem antibiotics, it is to be understood that there is considerable latitude in selection of precise reaction parameters. Suggestion of this latitude and its breadth is generally indicated by the enumeration of equivalent solvent systems, temperature ranges, protecting groups, and range of identities of involved reagents. Further, it is to be understood that the presentation of the synthetic scheme as comprising distinct steps in a given sequence is more in the nature of a descriptive convenience than as a necessary requirement; for one will recognize that the mechanically dissected scheme represents a unified scheme of synthesis and that certain steps, in actual practice, are capable of being merged, conducted simultaneously, or effected in a reverse sequence without materially altering the progress of synthesis.

The following examples recite a precise scheme of total synthesis. It is to be understood that the purpose of this recitation is to further illustrate the total synthesis and not to impose any limitation. Temperature is in °C.

Example Section — Part I

EXAMPLE 1

Preparation of *3* (Method I)

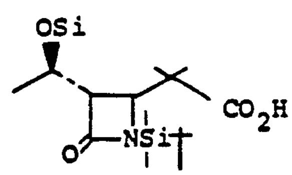

*3*

Step A

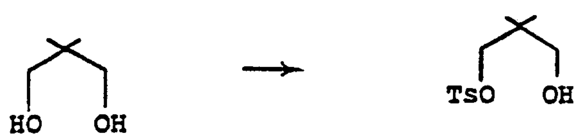

*1* *2*

Into a 1000-ml three-necked flask equipped with a mechanical stirrer is charged with 300 ml pyridine and 2,2-dimethyl-1,3-propane-diol (75 g). The flask is kept in an ice-bath. p-Toluenesulfonyl chloride (137.3 g) in pyridine (487 ml) is dropped into the flask through a dropping funnel. The mixture is stirred at 0°C overnight, then hydrolyzed with 100 ml water and crushed ice and acidified with conc. HCl. The mixture is extracted with 1.0 L ether. The organic layer is separated, dried over $MgSO_4$ then evaporated *in vacuo* to give product *2* (174 g).

Step B:

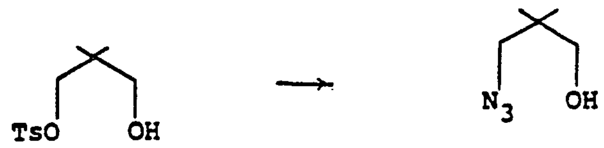

2 3

The tosylate (37 g), sodium azide (27.9 g) and polyethyleneglycol (mw 400, 100 ml) are placed in a 500 ml round bottomed flask attached with a distillation head. The mixture is heated at 135°C under vacuum (2—10 mm Hg) for 3.0 hours. The product is collected as colorless oil (17.2 g), 60 MHz NMR ($CDCl_3$): 0.95 (s), 2.70 (broad singlet), 3.21 (s), and 3.38 (s); IR (Neat): 2100 $cm^{-1}$ ($N_3$).

Step C:

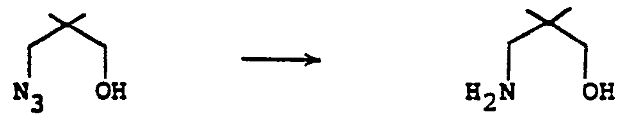

3 4

The azido propanol (37.8 g) in 120 ml cyclohexane is hydrogenated under 40 psi of hydrogen in the presence of 2.0 g of 10% Pd/C for 3 hours. The mixture is filtered from catalyst then evaporated *in vacuo* to give 35.7 g of product *4,* 60 MHz NMR ($CDCl_3$): 0.85 (s), 2.62 (s), 2.70 (s), 3.40 (s).

Step D:

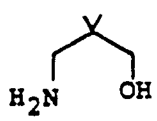

4

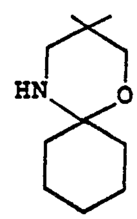

5

The amino alcohol *4* (75 g) and cyclohexanone (90 ml) in cyclohexane (400 ml) are heated at reflux. The water resulting from condensation is continuously removed by a Dean-Stark trap. After 4.5 hours, the mixture is evaporated *in vacuo* then distilled to give 94 g of product *5*, 60 MHz NMR ($CDCl_3$): 0.90 (s), 1.50—2.10 (m), 2.62 (s) and 3.40 (s).

Step E:

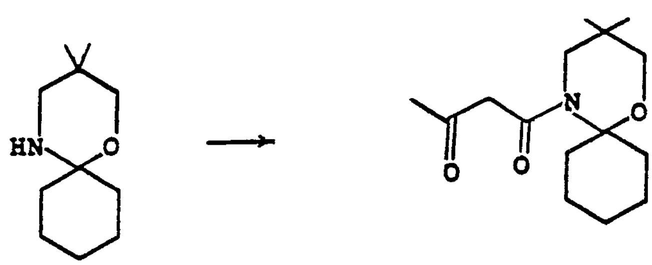

5 6

The starting material *5* (17.0 g) in ethanol (128 ml) is mixed with diketene (8.01 ml) and then stirred at 25°C for 4 hours. The mixture is evaporated *in vacuo* and the crude product is purified by silica gel column (4.4 × 30 cm) eluting with 35% EtOAc/cyclohexane to give product 6 (8.3 g). MS: m/e 267 ($M^+$), 252 ($M^+$—15), 224 ($M^+$—44): 60 MHz NMR ($CDCl_3$): 0.81 (s), 2.24 (s), 3.03 (s), 3.36 (s), 3.41 (m).

Step F:

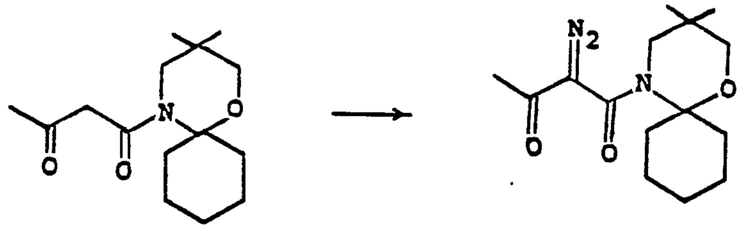

6 7

The β-ketoamide *6* (8.3 g) in acetonitrile (62 ml) is treated with EtOH (4.8 ml) and polymer-SO $N_3$ (14 g) at 25°C for 12 hours. The mixture is filtered from polymer, and the filtrate is evaporated *in vacuo* to give product *7* (6.4 g), IR ($CDCl_3$): 2128 ($N_2$), 1644 $cm^{-1}$; 60 MHz NMR ($CDCl_3$): 1.02 (s), 1.40—2.20 (m), 1.34 (s), 3.17 (s), 3.24 (s).

Step G:

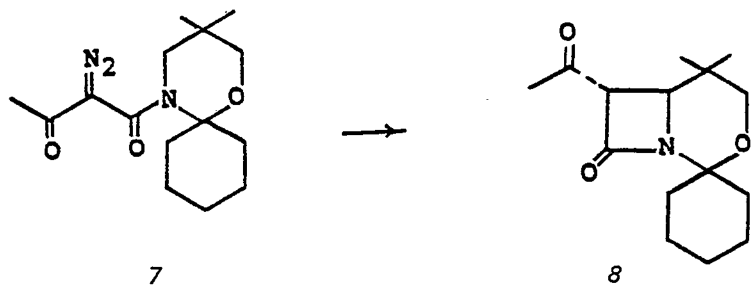

7 8

The diazo compound *7* (94 g) is dissolved in 940 ml 50% EtOAc/cyclohexane and heated at reflux in the presence of 270 mg of rhodium acetate for 6.0 hour. The mixture is washed with water, and brine. The organic layer is separated, dried over $MgSO_4$, then concentrated and chromatographed by a silica gel column (3.2 × 8″) eluting with 50% EtOAc/cyclohexane to give product *8* (94 g), MS: 265 ($M^+$), 222 ($M^+$—43); 60 MHz NMR ($CDCl_3$): 0.90 (s), 1.07 (s), 1.50—2.20 (m), 2.35 (s), 3.40—4.60 (m).

Step H:

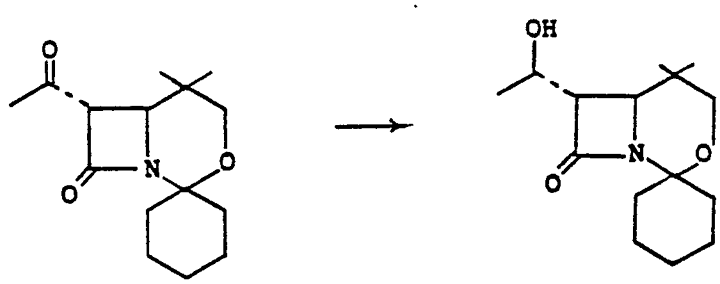

8 9

The ketone *8* (3.39 g) in 35 ml absolute ethanol is treated with sodium borohydride (0.49 g) at 0°C for 50 minutes, then mixed with 1 ml water and stirred at 25°C for 30 minutes. The mixture is titrated with saturated ammonium chloride until the organic layer is clear. The mixture is filtered from ppts. then evaporated *in vacuo*. The crude product is redissolved in EtOAc and washed with water, and brine. The organic layer is separated, dried over $MgSO_4$, concentrated, and chromatographed by a silica gel column eluting with 50% EtOAc/cyclohexane to give product *9* (1.40 g), MS: m/e 267 ($M^+$), 224 ($M^+$—43), 180 ($M^+$—87); 300 MHz NMR ($CDCl_3$): 0.86 (s), 0.88 (s), 0.90 (s), 1.08 (s), 1.15 (d), 1.17 (d), 1.30—2.90 (m), 2.95 (m), 3.11 (d), 3.29 (d), 3.35 (d), 3.57 (d, d), 4.05 (m), 4.15 (m).

Step I:

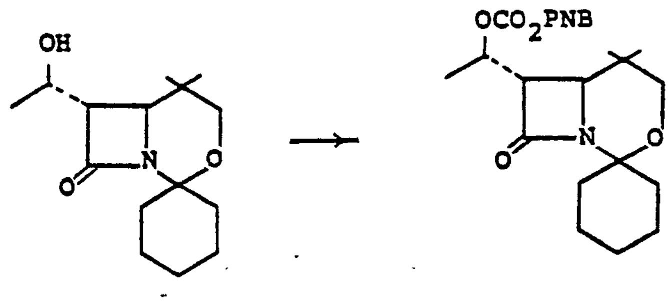

9 10

The alcohol *9* (16.1 g) is dissolved in 309 ml $CH_2Cl_2$. The solution is cooled to −20°C by an methanol-dry-ice bath. To the solution is added 4-N,N-dimethylaminopyridine (10.3 g) and p-nitrobenzylchloroformate (19.5 g). The mixture is stirred without cooling bath for 4 hours, then hydrolyzed with 200 ml 0.1*N* HCl, washed with water and brine. The organic layer is separated, dried over $Na_2SO_4$ then chromatographed by a silica gel column (3.2—12″) eluting with 30% EtOAc/cyclohexane to 22.0 g of product *10* MS m/e 446 ($M^+$), 418 ($M^+$—28). 300 MHz NMR ($CDCl_3$): 0.82 (s), 0.84 (s), 1.06 (s), 1.42 (d), 1.45 (d), 1.50—2.00 (m), 3.50—3.65 (m), 5.12 (quintets), 5.28 (d), 5.33 (d), 7.60 (m), 8.30 (m).

Step J

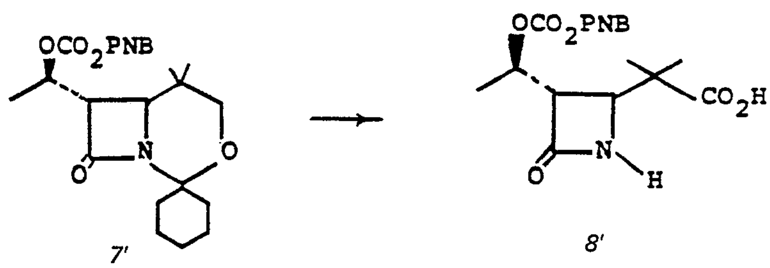

7′ 8′

The bicyclic azetidinone *7′* (6.0 g) in 60 ml acetone is treated with 4*N* Jones reagent (9.4 ml) at 0°C for 30 min. The reaction is quenched with 1 ml isopropanol at 0°C for 10 min, then mixed with 250 ml ethylacetate and washed with water and brine until blue color disappeared in the organic layer. The organic layer is separated, dried over $MgSO_4$, and evaporated in *in vacuo* to give crude product which is purified by a silica gel column (4.4 × 10 cm) eluting with ethylacetate to give 3.1 g of *8′* as crystalline solid.

Step K

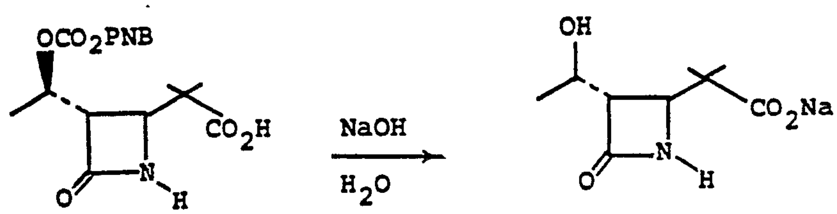


8′ 9′

The azetidinone carboxylic acid *8′* (3.0 g) is suspended in 50 ml water. The mixture is stirred and treated with 2.5*N* NaOH and maintained at pH 12.0 at room temperature for 30 min. The resulting solution is neutralized with 2.5*N* HCl to pH 7.5. After the mixture is extracted with EtOAc, the aqueous layer is concentrated and lyophilized to give white solid product *9′*.

Step L

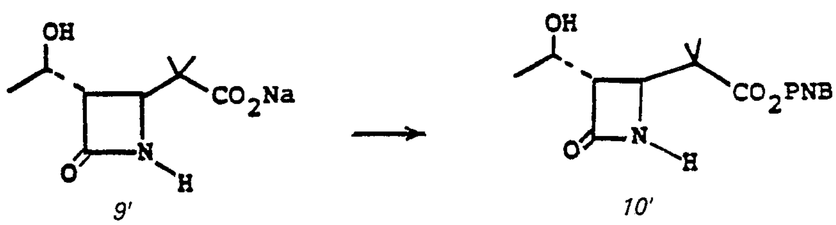

9′ 10′

The azetidinone carboxylic acid sodium salt *9'* (2.2 g) and p-nitrobenzylbromide (2.94 g) are stirred at room temperature in DMF (29.3 ml) for 5 hrs. The mixture is diluted with EtOAc and washed with water and brine. The organic layer is separated, dried over $MgSO_4$ and evaporated to give crude product which is purified by TLC plates eluting with 50% EtOAc/cyclohexane to give product *10'*.

Step M

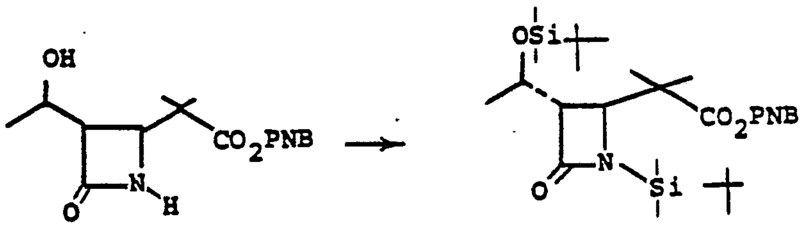

*10'* *11'*

The ester *10'* (1.33 g) is stirred with t-butyldimethylchlorosilane (2.49 g), triethylamine (4.61 ml) in DMF (16 ml) at room temperature overnight. The mixture is filtered from ppts and evaporated *in vacuo* to give crude product *11'* which is redissolved in ethylacetate and washed with water, brine, dried over $Na_2SO_4$, concentrated to 0.5 ml then purified by TLC eluting with 30% EtOAc/cyclohexane to give product *11'*.

Step N

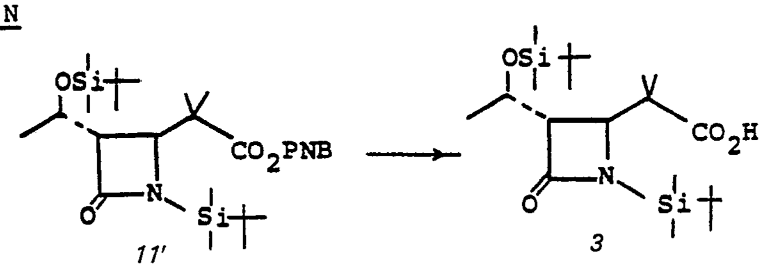

*11'* *3*

The bis-silyl azetidinone ester *11'* (1.60 g) in 30 ml EtOAc is hydrogenated under 50 psi hydrogen in the presence of 0.32 g 10% Pd/C for 30 min. The mixture is filtered from catalyst. The catalyst is washed with MeOH. The methanol and ethylacetate solutions are combined and evaporated *in vacuo* to give white solids. The crude product is re-dissolved in ethylacetate and washed with 0.1*N* HCl. The organic layer is separated, dried over $Na_2SO_4$ and evaporated to give white solid product *3*.

Example 2

Preparation of 3 (Method II)

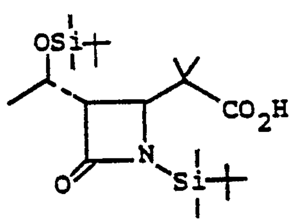

*3*

*Step A:*
Preparation of 3,3-Dimethyl-1,4-pentadiene

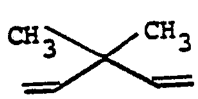

*1*

Procedure a

β,β-Dimethylglutaric acid (1.0 mole) is refluxed for 2 hours with thionyl chloride (68% excess). After removal of excess thionyl chloride, absolute ethanol (109% excess) is added slowly. The mixture is refluxed for 3 hours then distilled to collect the product, diethyl β,β-dimethylglutarate (98% yield).

To a suspension of lithium aluminum hydride (24 g) in ether (860 ml) is added dropwise with rapid stirring a solution of diethyl β,β-dimethylglutarate (124 g in 250 ml ether). The mixture is refluxed for 6

hours, then cooled to room temperature. Water (25 ml) is added slowly. The mixture is then titrated with 10% NaOH until a clear organic layer is obtained. The organic layer is separated, dried over anhydrous sodium sulfate then evaporated *in vacuo* to give the resulting diol as an oil (90% yield). The 3,3-dimethyl-1.5-pentanediol (0.5 mole) is treated with thionyl chloride (1.05 mole) at reflux for 3 hours. After removal of excess thionyl chloride *in vacuo*, the 3,3-dimethyl-1,5-dichloropentane is obtained (90% yield).

3,3-Dimethyl-1.5-dichloropentane (41 g) is added dropwise at 170°C to a mixture of 48 g of sodium hydroxide and 40 g of polyethylene glycol tetramer and the mxiture is distilled to give 3,3-dimethyl-1,4-pentadiene (66%).

Step B:

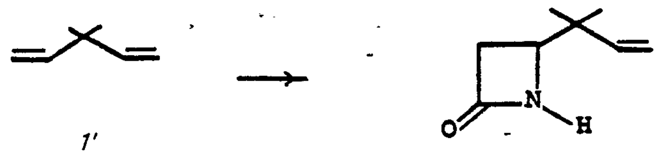

*1'* *2'*

In a sealed tube, 3,3-dimethyl-1,4-pentadiene (9.6 g) and chlorosulfonyl isocyanate (14.2 g) are allowed to stand at room temperature for 6 days. The resulting mixture is diluted with methylene chloride and added slowly to a stirred aqueous solution which contains 20 g of $Na_2SO_3$ and 50 g of $K_2HPO_4$ at 0—5°C for 30 minutes. The organic layer is separated and dried over $MgSO_2$. After evaporation, the crude product is chromatographed on silica gel GF eluting with EtOAc to give *2'*.

Step C:

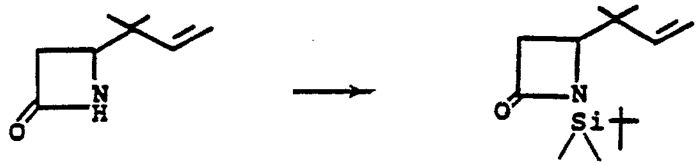

*2'* *3'*

t-Butyldimethylchlorosilane (7.51 g) is added in one portion to an ice-cold, stirred solution of 4-(1-methyl-prop-2-ene)-azetidin-2-one (6.54 g) and triethylamine (12 ml) in anhydrous dimethylformamide (100 ml). The reaction mixture is stirred at a temperature ranging from 0 to 5°C for 1 hour and then allowed to warm to room temperature. Most of the solvent is removed under vacuum to give a residue which is partitioned between diethyl ether (250 ml) and water. The ethereal phase is washed with 2.5N hydrochloric acid (50 ml), water (3 × 50 ml), and brine, dried with magnesium sulfate, filtered and evaporated under vacuum to provide a crude product which is purified by chromatography on silica gel (20% ether in petroleum ether) to yield *3'*.

Step D:

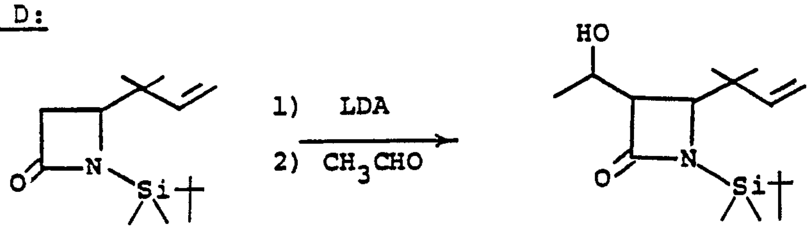


*3'* *4'*

n-Butyllithium in hexane (26.25 mmol) is added slowly by syringe to a solution of diisopropylamine (26.55 mmol) in anhydrous tetrahydrofuran (100 ml) at −78°C. The resulting solution is stirred for 15 min proir to the addition of a solution of *3'* (25.0 mmol) in anhydrous tetrahydrofuran (25 ml). After stirring for 15 min at −78°C acetaldehyde (75 mmol) is added by syringe and the resulting solution is stirred at −78°C for 5 min. Saturated aqueous ammonium chloride solution (15 ml) is added by syringe and the reaction mixture is allowed to warm to room temperature, then diluted with ether (250 ml) and washed 2.5N hydrochloric acid solution (2 × 50 ml), water (100 ml) and brine and dried over magnesium sulfate. Solvents are removed *in vacuo* and the residue is chromatographed on silica gel (1:1, ether:petroleum ether) to give the expected product *4'*.

Step E:

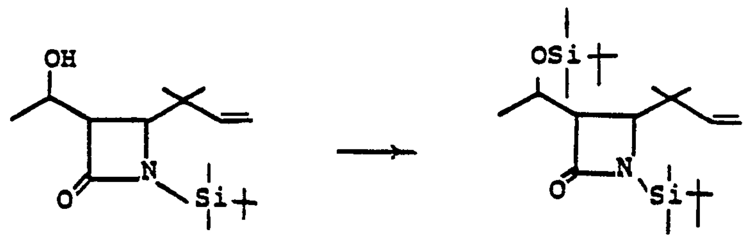

4' 5'

At 0°C, the alcohol *4'* (5.00 g) is dissolved in DMF (50 ml) and treated with t-butyl-dimethylchlorosilane (7.51 g) and triethylamine (12 ml). The mixture is allowed to warm to room temperature with constant stirring for 2 hours then filtered from solids, evaporated *in vacuo* to give oil residue which is re-dissolved in ethylacetate and washed with 0.1 *N* HCl, water, and brine. The organic layer is separated, dried over $MgSO_4$ and evaporated *in vacuo* to give crude product *5'*. HPLC purification of product (20% ethylacetate/cyclohexane) affords *5'*.

Step F:

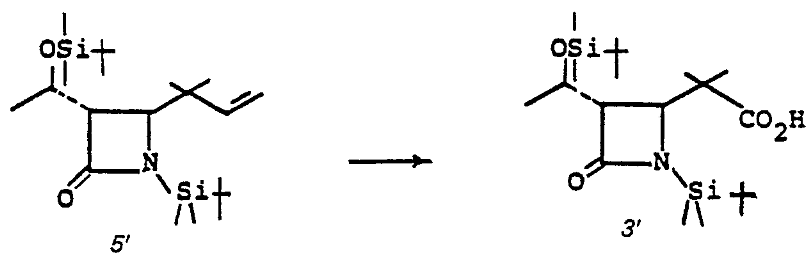

5' 3'

A solution of *5'* (3.0 mmol) in dry methylene chloride (30 ml) is cooled to −78°C (dry-ice acetone) and a stream of ozone is bubbled through until the reaction mixture becomes blue. The ozone flow is then stopped and the reaction is purged by bubbling through nitrogen until the blue color disappears. Solid *m*-chloroperbenzoic acid (3.0 mmol) is added and the cold bath is removed. When the reaction mixture reaches room temperature, the flask is fitted with a reflux condenser and the mixture is heated at reflux for three days. Removal of solvents *in vacuo* gives crude product which is chromatographed on silica gel (2% glacial acetic acid in methylene chloride) to *3'*.

EXAMPLE 3

Preparation of 1a:

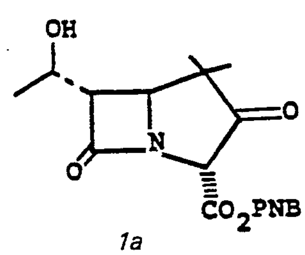

1a

Step A:

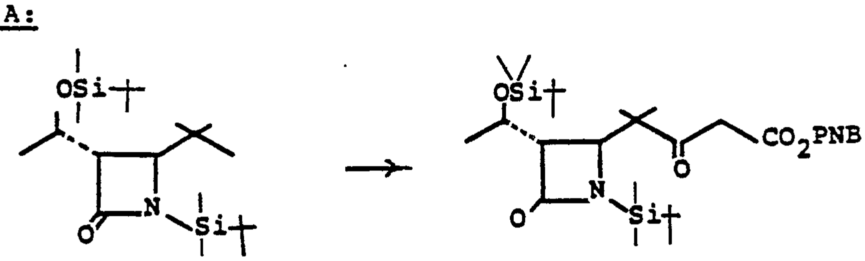

1' 2'

PNB = p-nitrobenzyl

The N,O-bis-silyl azetidinone carboxylic acid *1'* (500 mg) suspended in acetonitrile (14.8 ml) is treated with 1,1'-carbonyldiimidazole (229.6 mg) and stirred at room temperature for 30 min. The mixture is then treated with p-nitrobenzylmalonate magnesium salt (1.18 g) and heated at 60°C for 3 hrs. The mixture is diluted with $CH_2Cl_2$ and washed with water, brine, and dried over $Na_2SO_4$. TLC purification (75% EtOAc/cyclohexane) of the crude product provides 0.50 g of product *2'*.

Step B:

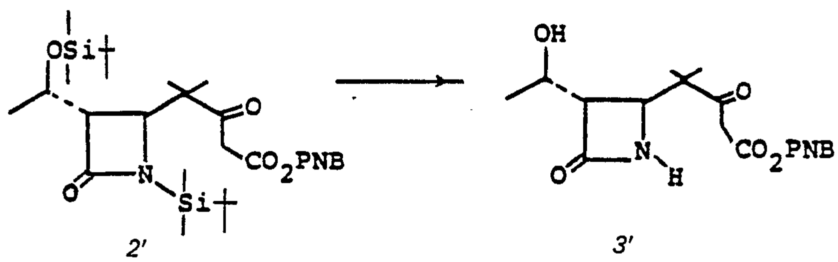

2' 3'

The bis-silyl β-keto ester *2'* (667 mg) in methanol (16 ml) is stirred with 2 ml 6*N* HCl at room temperature for 2 hrs. The mixture is diluted with ethylacetate, washed with 0.1*M* sodium phosphate buffer, brine, dried over $Na_2SO_4$ then evaporated *in vacuo* to give 0.6 g of crude product which is purified by TLC eluting with 100% ethylacetate to give product *3'*.

Step C:

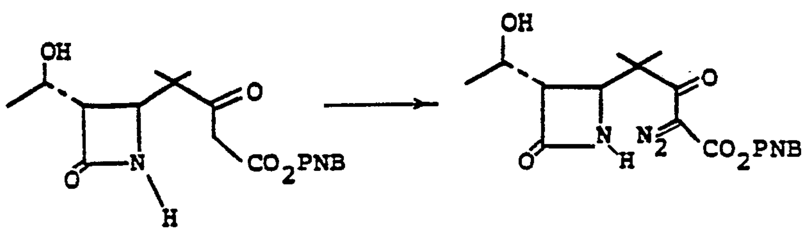

3' 4'

The β-keto ester *3'* (270 mg) in 3.2 ml acetonitrile p-toluene-sulfonazide (1.11 g, 3.33 meq/g), triethylamine (0.31 ml) are placed in a 25 ml round-bottom flask. The mixture is stirred under nitrogen atmosphere at room temperature for 1 hr.

The mixture is diluted with ethylacetate and washed with water, brine, and dried over $MgSO_4$. The crude product is purified by TLC eluting with 50% EtOAc/cyclohexane to give *4'*.

Step D:

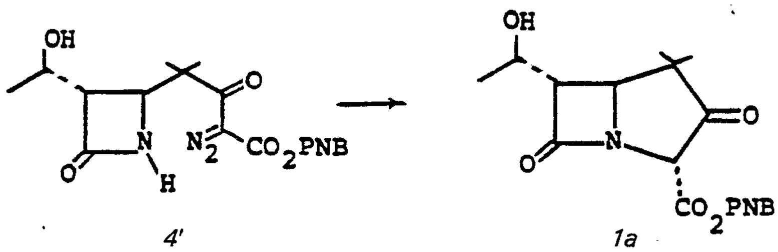

4' 1a

The diazo β-keto ester *4'* (38.6 mg) in toluene (1 ml) is heated at 80°C in the presence of rhodium acetate (1.7 mg) for 10 min. The mixture is diluted with ethylacetate (10 ml) and washed with water and brine. The organic layer is separated, dried over $MgSO_4$ and evaporated *in vacuo* to give bicyclic keto ester *1a*.

Example 4

Chiral Synthesis

Step A:

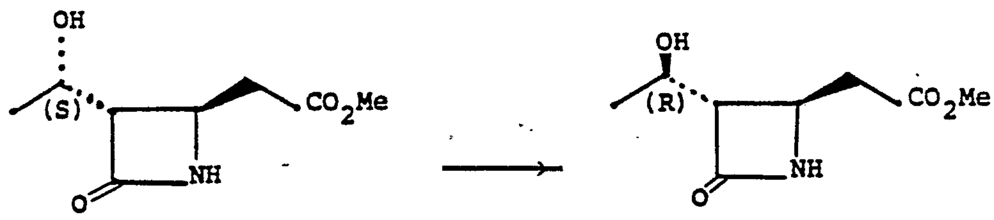

1 2

At 0°C, under $N_2$, the chiral starting material *1* (0.94 g), methylene chloride (8.2 ml), triphenylphosphine (3.34 g) and formic acid (1.15 g, 97%) are placed in a 50 ml three-necked flask. To the solution is slowly added di-isopropyl azodicarboxylate (2.58 g). The mixture is then stirred at room temperature overnight; thin layer chromatograph (TLC) shows that all the starting material is consumed. The mixture is cooled to 0°C and treated with methanol (11.48 ml), water (4.9 ml) and concentrated hydrochloric acid (1.7 ml) for 2 hr, then extracted with methylene chloride. The organic layer is separated, dried over $MgSO_4$ and evaporated to give product *2.*

Step B:

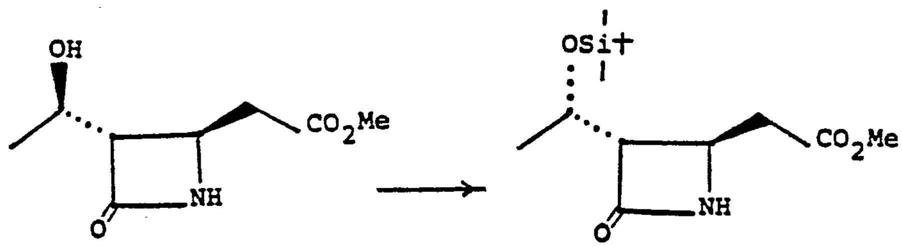

2 3

The free hydroxyl azetidinone *2* (374 mg) in DMF is treated with imidazole (688 mg) and t-butyldimethylchlorosilane (603 mg) at room temperature for 7 hrs. The mixture is evaporated *in vacuo* and the residue is re-dissolved in ethyl acetate and washed with 1*N* HCl, water and brine. The organic layer is separated, dried over $MgSO_4$, and evaporated *in vacuo* to give *3.*

Step C:

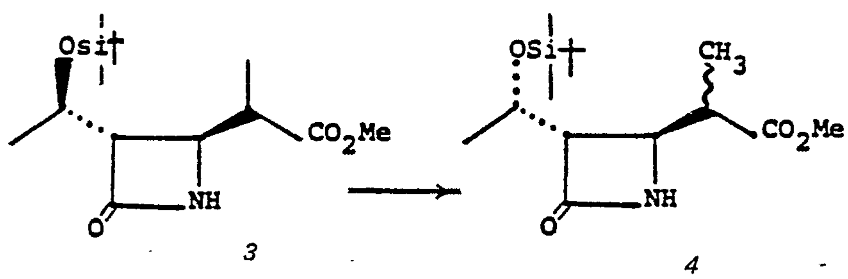

3 4

Under $N_2$, at −78°C, diisopropylamine (1.68 ml) in 12.5 ml THF is treated with n-butyllithium (12.5 ml, 1.6 *M* in hexane) for 10 min. To the solution is added THF solution of *3* (1.51 g in 5 ml THF) and the mixture is stirred for 20 min, then is treated with iodomethane (1.87 ml). After stirring 40 min at −78°C, the mixture is allowed to warm to 0°C then hydrolyzed with 0.1*N* HCl. The mixture is extracted with ethyl acetate. The organic layer is washed with water, brine then dried over $MgSO_4$ and evaporated *in vacuo* to give 2.1 g of mixture of *4.*

Step D:

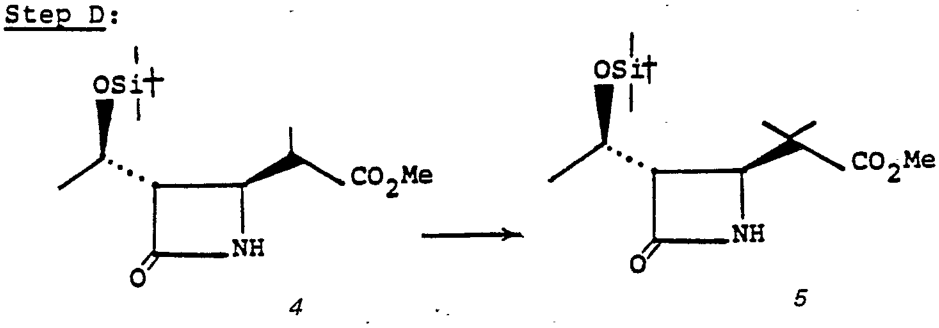

4 5

Under $N_2$, at −78°C, diisopropylamine (0.84 ml) in 6.3 ml THF is treated with n-BuLi (6.3 ml, 1.6*M* in hexane) for 10 min. To the solution is added THF solution of *3* (0.75 g in 5 ml THF) and the mixture is stirred for 20 min, then is treated with iodomethane (0.99 ml). After stirring 40 min at −78°C, the mixture is allowed to warm to 0°C then hydrolyzed with 0.1*N* HCl. The mixture is extracted with ethyl acetate. The organic layer is washed with water, brine then dried over $MgSO_4$ and evaporated *in vacuo* to give *5*.

Step E:

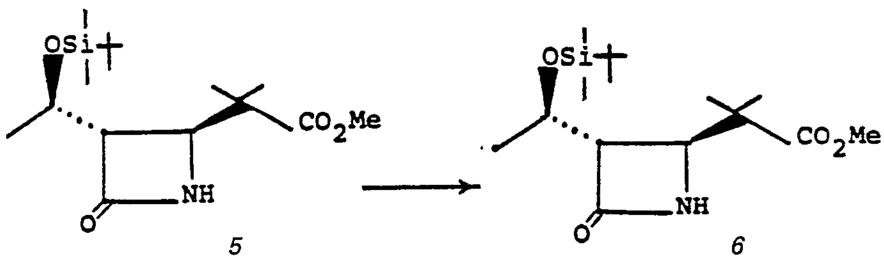

5 6

The methyl ester *5* (1.0 g) is treated with NaOH solution (2.5*N*, 1.4 ml) in methanol (5 ml) at room temperature for 5 hr. The mixture is acidified with 1*N* HCl to pH 1.0 then extracted with ethyl acetate. The organic layer is separated and dried over $MgSO_4$ and evaporated to give product *6*.

Step F:

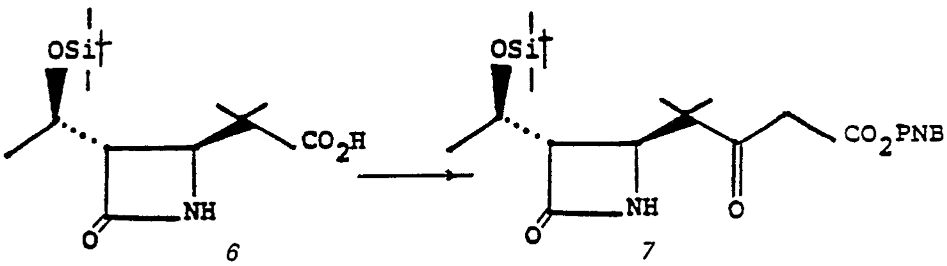

6 7

The carboxylic acid (300 mg) suspension in acetonitrile is treated with 1,1'-carbonyldiimidazole (194.6 mg) at room temperature for 30 min. The mixture becomes homogeneous within 5 min. To the solution is added magnesium p-nitrobenzylmalonate (1.00 g), then the mixture is heated at 65°C for 3 hr. The final reaction mixture is evaporated *in vacuo* to give oily residue which is re-dissolved in 10 ml ethyl acetate and washed with water and brine. The organic layer is separated, dried over $MgSO_4$ then evaporated *in vacuo* to give product 7.

Step G:

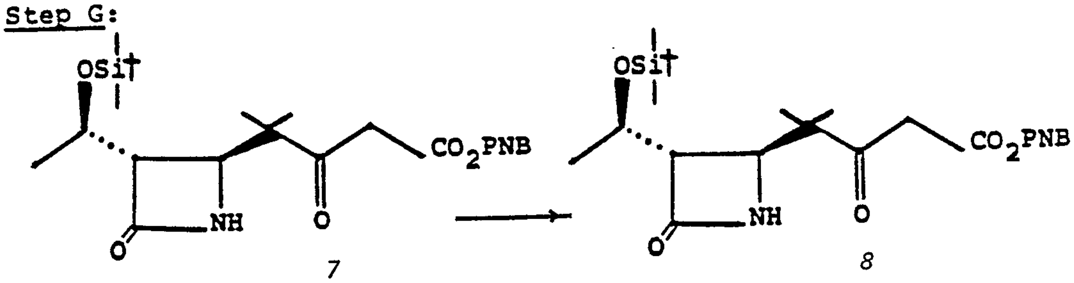

7 8

The starting material *7* (400 mg) is dissolved in 4 ml methanol and treated with 6*N* HCl (0.41 ml) at room temperature for 80 min. The mixture is diluted with ethyl acetate and washed with 0.1*N* pH 7.0 phosphate buffer and brine. The organic layer is separated, dried over $MgSO_4$ and evaporated *in vacuo* to give crude product *8* as solid which is triturated in petroleum ether then filtered to give *8*.

Step H:

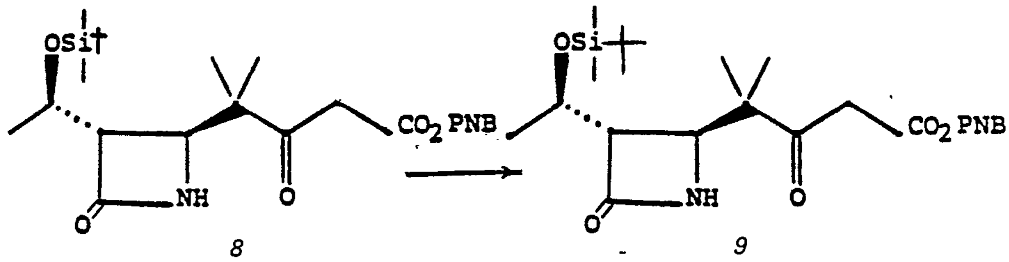

8 9

The β-keto ester *8* (269 mg) dissolved in 3.2 ml acetonitrile is gently stirred with Amberlite XE—301—$SO_2N_3$ (1.5 g, 3.33 meq of $N_3$/g) and triethylamine (0.46 ml) at room temperature for 1.5 hr. The mixture is filtered from polymer beads and the filtrate is evaporated *in vacuo* to give product *9*.

Step G:

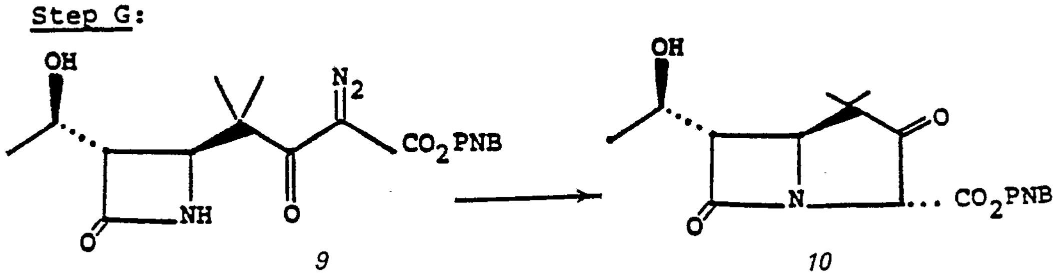

9 10

The diazo compound *9* (145 mg) is dissolved in 4.1 ml toluene and 2.5 ml ethyl acetate. The mixture is heated at 80—85°C in the presence of rhodium acetate (2.9 mg) for 15 min. The solution is allowed to cool to room temperature then diluted with 10 ml ethyl acetate and washed thoroughly with water, and brine. The organic layer is separated, dried over $MgSO_4$ then evaporated *in vacuo* to give product *10*.

## EXAMPLE 5

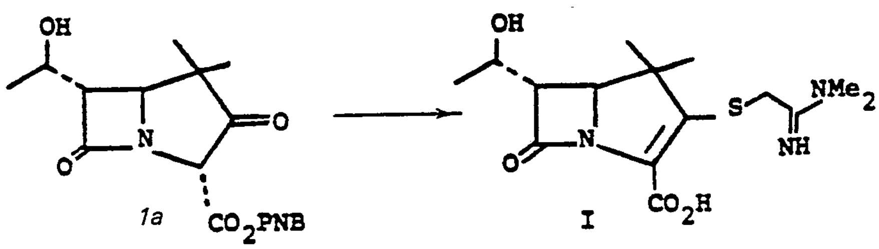

1a I

The bicyclic keto ester *1a* (33.3 mg) in acetonitrile (0.47 ml) at 0°C under $N_2$ atmosphere is treated with diphenyl chlorphosphate (20.97 μl) at 0°C and stirred for 30 min. To the mixture is added DMSO (0.20 ml) solution of N,N-dimethylmercaptoacetamidine hydrochloride (18.68 mg) and diisopropylethylamine (24.3 μl) and stirred for 1 min at 0°C. The mixture is then mixed with 10 ml ether and centrifuged to separate the oil product which is subsequently re-dissolved in 3.72 ml THF and 2.80 ml 0.1*M* pH 7.0 sodium phosphate buffer. The solution is hydrogenated under 50 psi hydrogen in the presence of 50.0 mg of 10% Pd/C at room temperature for 30 min.

Additional 50 mg of 10% Pd/C is added and the mixture is further hydrogenated for 30 minutes then filtered from catalysts. The filtrate is extracted with ether, concentrated to 4 ml then chromatographed by a Dowex-50X4 ($Na^+$cycle) column (2.2 × 6 cm) which is eluted with DI water to give product I. Lyopholization of the aqueous solution gives product I.

EXAMPLE 6

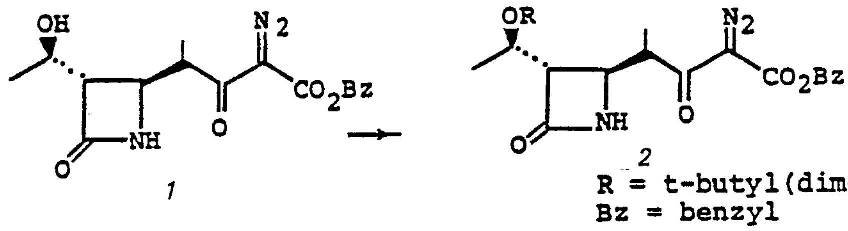

1

2

R = t-butyl(dimethyl)silyl
Bz = benzyl

The starting material *1* (2.10 g) is treated with t-butyldimethylchlorosilane (1.29 g) and imidazole (1.46 g) in DMF (8.6 ml) at room temperature for 3 hours. The mixture is evaporated *in vacuo* and the residue is redissolved in ethyl acetate and washed with water and brine. The organic layer is separated, dried over $Na_2SO_4$, and evaporated *in vacuo* to give product *2.*

EXAMPLE 7

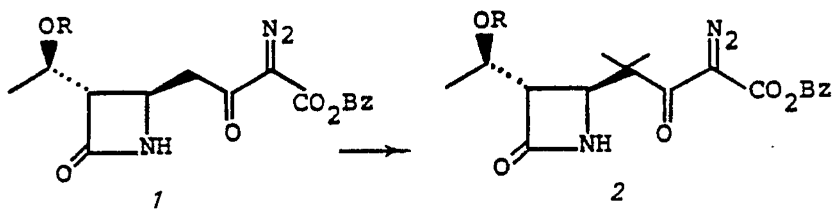

1

2

At −78°C, under $N_2$, diisopropylamine (168.2 μl) is treated with n-butyllithium (1.6*M*, 1.13 ml) in THF (0.84 ml) for 10 min. To the solution is added *1* (280 mg in 0.2 ml THF). The solution becomes deep-red upon the addition of *1*. After the mixture is stirred for 20 min, iodomethane (0.7 ml) is added and stirred for an additional 40 min at −78°C, then the mixture is allowed to warm to room temperature. The mixture is hydrolyzed with saturated $NH_4Cl$, and diluted with ethyl acetate. The organic layer is separated, dried over $Na_2SO_4$, and evaporated *in vacuo* to give product *2.*

Example 8

Following the foregoing text and examples, the following species I are obtained when the corresponding bicyclic keto ester is treated with $HSR^8$.

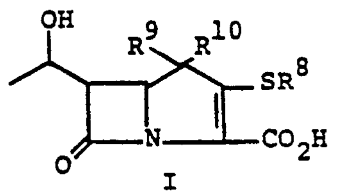

I

| | $R^8$ | $R^9$ | $R^{10}$ |
|---|---|---|---|
| 1) | 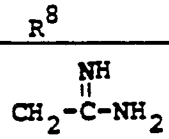 | $CH_3$ | $CH_3$ |
| 2) | 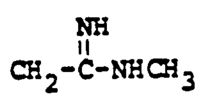 | $CH_3$ | $CH_3$ |
| 3) | 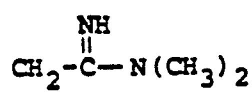 | $CH_3$ | $CH_3$ |
| 4) | 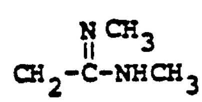 | $CH_3$ | $CH_3$ |

| | $R^8$ | $R^9$ | $R^{10}$ |
|---|---|---|---|
| 5) | 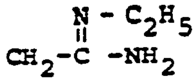 | $CH_3$ | $CH_3$ |
| 6) | 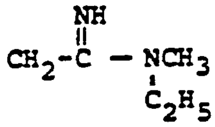 | $CH_3$ | $CH_3$ |
| 7) | 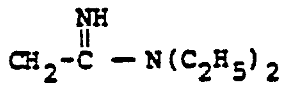 | $CH_3$ | $CH_3$ |
| 8) | 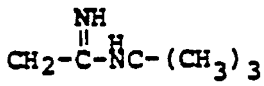 | $CH_3$ | $CH_3$ |
| 9) | 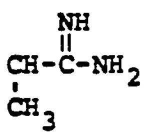 | $CH_3$ | $CH_3$ |
| 10) | 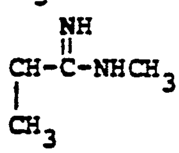 | $CH_3$ | $CH_3$ |
| 11) | 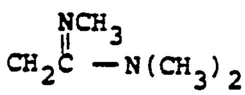 | $CH_3$ | $CH_3$ |
| 12) | 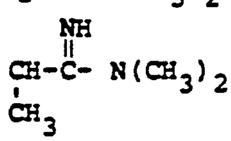 | $CH_3$ | $CH_3$ |
| 13) | 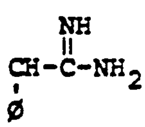 | $CH_3$ | $CH_3$ |
| 14) | 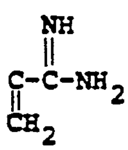 | $CH_3$ | $CH_3$ |
| 15) | 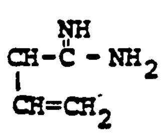 | $CH_3$ | $CH_3$ |
| 16) | 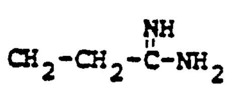 | $CH_3$ | $CH_3$ |
| 17) | 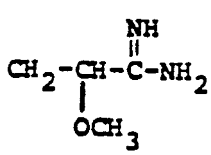 | $CH_3$ | $CH_3$ |

| | $R^8$ | $R^9$ | $R^{10}$ |
|---|---|---|---|
| 18) | 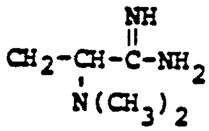 | $CH_3$ | $CH_3$ |
| 19) | 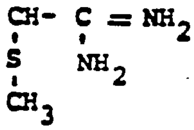 | $CH_3$ | $CH_3$ |
| 20) | 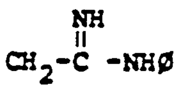 | $CH_3$ | $CH_3$ |
| 21) | 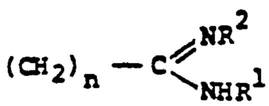 | $CH_3$ | $CH_3$ |
| | $n = 2-5$, $R^2 = H, CH_3$<br>$R^1 = H, CH_3$ | | |
| 22) | 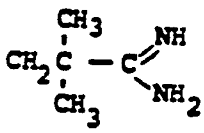 | $CH_3$ | $CH_3$ |
| 23) | 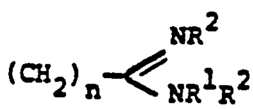 | $CH_3$ | $CH_3$ |
| | $n = 2-5$, $R^1, R^2 = H, CH_3$ | | |
| 24) | 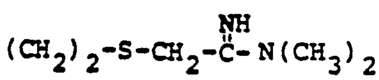 | $CH_3$ | $CH_3$ |
| 25) | 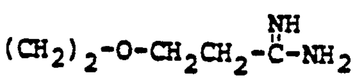 | $CH_3$ | $CH_3$ |
| 26) | 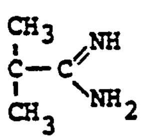 | $CH_3$ | $CH_3$ |
| 27) | 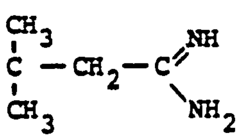 | CH3 | $CH_3$ |
| 28) | 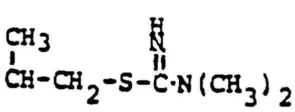 | $CH_3$ | $CH_3$ |

| | $R^8$ | $R^9$ | $R^{10}$ |
|---|---|---|---|
| 29) | 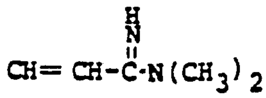 | $CH_3$ | $CH_3$ |
| 30) | 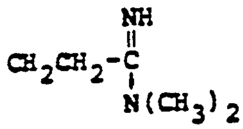 | $CH_3$ | $CH_3$ |
| 31) | 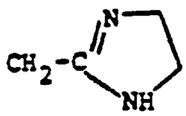 | $CH_3$ | $CH_3$ |
| 32) | 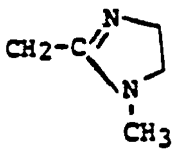 | $CH_3$ | $CH_3$ |
| 33) | 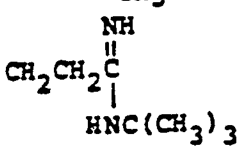 | $CH_3$ | $CH_3$ |
| 34) | 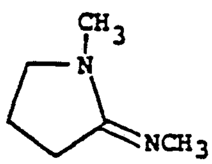 | $CH_3$ | $CH_3$ |
| 35) | 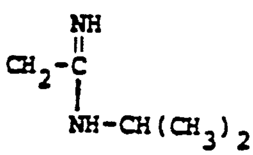 | $CH_3$ | $CH_3$ |
| 36) | 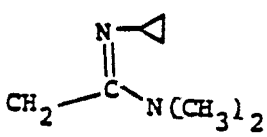 | $CH_3$ | $CH_3$ |
| 37) | 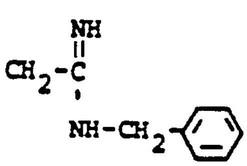 | $CH_3$ | $CH_3$ |
| 38) | 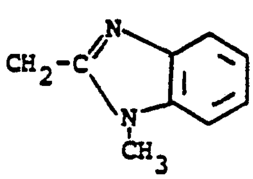 | $CH_3$ | $CH_3$ |

| | $R^8$ | $R^9$ | $R^{10}$ |
|---|---|---|---|
| 39) | 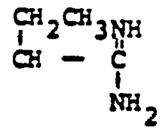 | $CH_3$ | $CH_3$ |
| 40) | 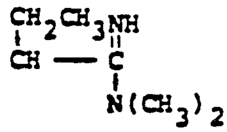 | $CH_3$ | $CH_3$ |
| 41) | 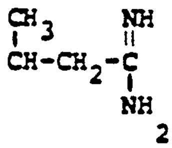 | $CH_3$ | $CH_3$ |
| 42) | 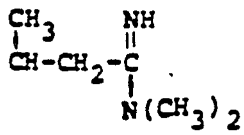 | $CH_3$ | $CH_3$ |
| 43) | 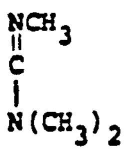 | $CH_3$ | $CH_3$ |
| 44) | 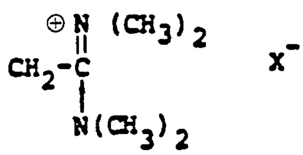 | $CH_3$ | $CH_3$ |
| 45) | 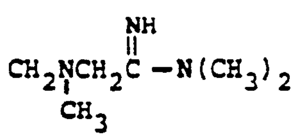 | $CH_3$ | $CH_3$ |
| 46) | 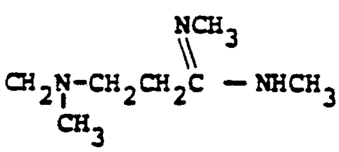 | $CH_3$ | $CH_3$ |
| 47) | 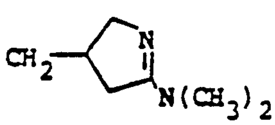 | $CH_3$ | $CH_3$ |
| 48) | 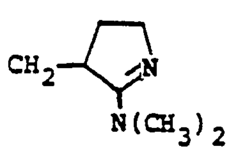 | $CH_3$ | $CH_3$ |

| | $R^8$ | $R^9$ | $R^{10}$ |
|---|---|---|---|
| 49) | 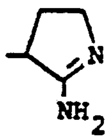 | $CH_3$ | $CH_3$ |
| 50) | 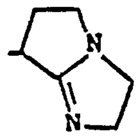 | $CH_3$ | $CH_3$ |
| 51) | 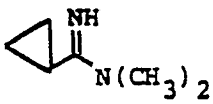 | $CH_3$ | $CH_3$ |
| 52) | 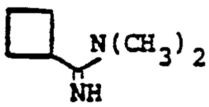 | $CH_3$ | $CH_3$ |
| 53) | 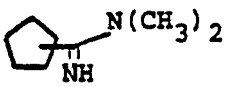 | $CH_3$ | $CH_3$ |
| 54) | 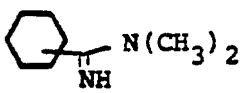 | $CH_3$ | $CH_3$ |
| 55) | 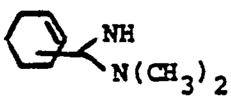 | $CH_3$ | $CH_3$ |
| 56) | 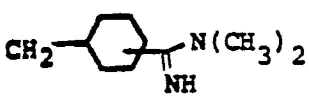 | $CH_3$ | $CH_3$ |
| 57) | 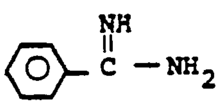 | $CH_3$ | $CH_3$ |
| 58) | 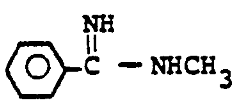 | $CH_3$ | $CH_3$ |
| 59) | 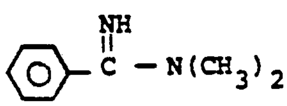 | $CH_3$ | $CH_3$ |
| 60) | 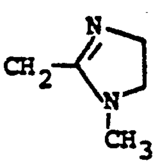 | $CH_3$ | $CH_3$ |
| 61) | 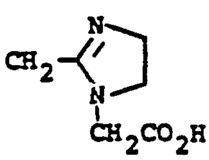 | $CH_3$ | $CH_3$ |

| | $R^8$ | $R^9$ | $R^{10}$ |
|---|---|---|---|
| 62) | 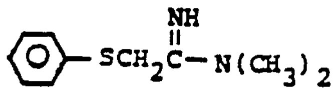 | $CH_3$ | $CH_3$ |
| 63) | 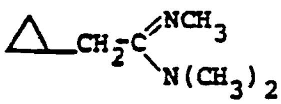 | $CH_3$ | $CH_3$ |
| 64) | 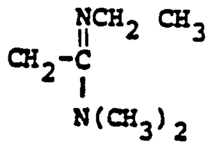 | $CH_3$ | $CH_3$ |
| 65) | 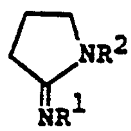 $R^1$ = H, CH3 $R^2$ = H, $CH_3$ | $CH_3$ | $CH_3$ |
| 66) | 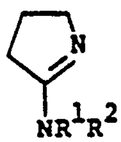 $R^1$ = H, $CH_3$ $R^2$ = H, $CH_3$ | $CH_3$ | $CH_3$ |
| 67) | 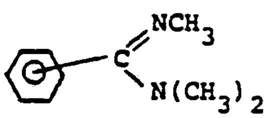 | $CH_3$ | $CH_3$ |
| 68) | 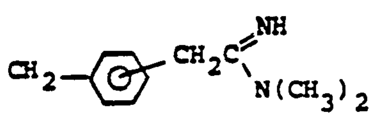 | $CH_3$ | $CH_3$ |
| 69) | 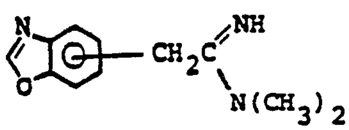 | $CH_3$ | $CH_3$ |
| 70) | 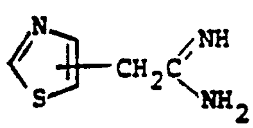 | $CH_3$ | $CH_3$ |
| 71) | 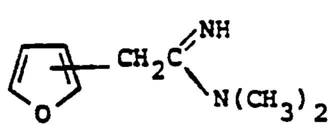 | $CH_3$ | $CH_3$ |
| 72) | 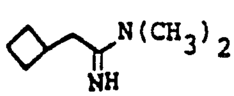 | $CH_3$ | $CH_3$ |

| | $R^8$ | $R^9$ | $R^{10}$ |
|---|---|---|---|
| 73) | 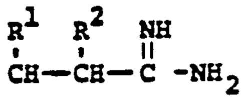<br>$R^1 = CH_3$<br>$R^2 = CH_3$, $NR_2$, OR<br>$R^3 = H$, $CH_3$ | $CH_3$ | $CH_3$ |
| 74) | 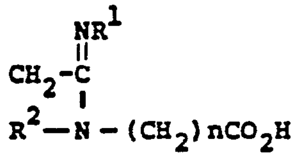<br>$R^1 = H$, $CH_3$<br>$R^2 = H$, $CH_3$<br>n = 1 or 2 | $CH_3$ | $CH_3$ |
| 75) | 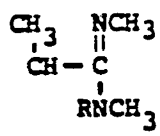 R = H, $CH_3$ | $CH_3$ | $CH_3$ |
| 76) | 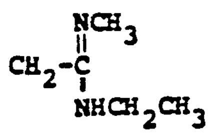 | $CH_3$ | $CH_3$ |
| 77) | 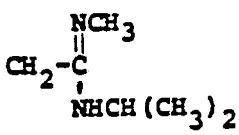 | $CH_3$ | $CH_3$ |
| 78) | 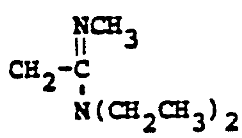 | $CH_3$ | $CH_3$ |
| 79) | 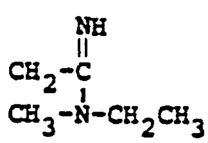 | $CH_3$ | $CH_3$ |
| 80) | 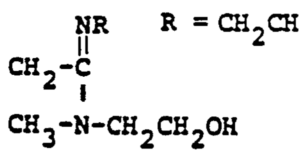 R = $CH_2CH_3$, $CH_3$ | $CH_3$ | $CH_3$ |
| 81) | 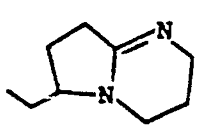 | CH3 | $CH_3$ |

| | $R^8$ | $R^9$ | $R^{10}$ |
|---|---|---|---|
| 82) | 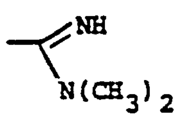 | $CH_3$ | $CH_3$ |
| 83) | 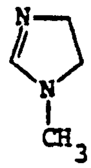 | $CH_3$ | $CH_3$ |

Compounds 84—166 correspond to the above compounds 1—83 except $R^9$ is ethyl.
Compounds 167—249 correspond to the above compounds 1—83 except $R^9$ is phenyl.
Compounds 250—332 correspond to the above compounds 1—83 except $R^9$ is $CH_2F$.
Compounds 333—415 correspond to the above compounds 1—83 except $R^9$ is cyclopropyl.
Compounds 416—498 correspond to the above compounds 1—83 except $R^9$ is trifluoromethyl.
Compounds 499—581 correspond to the above compounds 1—83 except $R^9$ and $R^{10}$ are ethyl.
Compounds 582—667 correspond to the above compounds 1—83 except $R^9$ and $R^{10}$ are joined to form $—CH_2CH_2CH_2—$.

Example Section — Part II

Example 1

Preparation of 3 (Method I)

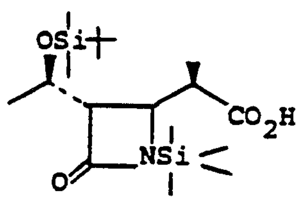

3

**Step A:**

$CH_3CHO + R^3CH_2CHO$

NaOH

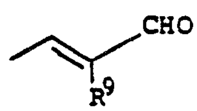

C

The α,β-unsaturated aldehydes (C) are prepared by modified procedures reported by M. B. Green and W. J. Hickinbottom in *J. Chem. Soc.* 3262 (1957); and W. J. Bailey and R. Barclay Jr., *J. Org. Chem., 21,* 328 (1956).

Acetaldehyde (1 eq.) and propionaldehyde ($R''CH_3$) (1 eq.) are placed in a three-necked round-bottom flask which is equipped with a mechanical stirrer, a dry-ice condenser, and a pressure equalized dropping-funnel. To the solution is added dropwise 1 eq. of 1*N* NaOH through the dropping funnel with constant stirring. After completion of the mixing, the mixture is stirred for 10 min, then poured into a beaker containing crushed ice. Extraction of the mixture with ether gives the crude product. The desired product (C) is obtained by fractional distillation through a Widmer column.

**Step B:**

**Preparation of 1'**

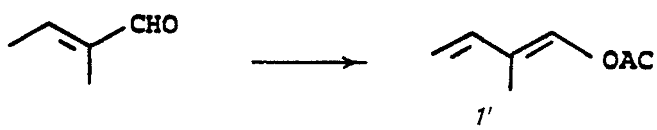

1'

Isopropenyl acetate (182 g), cupric acetate (0.40 g), 2-methyl-2-butanol (84 g) and p-toluenesulfonic acid (1.52 g) are placed in a 1.0 l three-necked flask equipped with a thermometer, a nitrogen inlet tube and a 10 in. Widmer column which is attached with a distillation head. The mixture is heated at 93—110°C until 73 ml of acetone is collected. After cooling to r.t. (22°C) the mixture is filtered from solids. The dark brown filtrate is cooled in an ice-bath and mixed with 3.4 g triethanolamine in 200 ml water. The two layer mixture is distilled quickly at 53 mm (b.p. 54°). The organic layer of the distillate is separated. The aqueous layer is extracted with 200 ml ether. The organic layers are combined and washed with 10% $K_2CO_3$, dried over $Na_2SO_4$ and evaporated *in vacuo.* The residue so obtained is mixed with 2.0 g N-phenyl-β-naphthylamine and distilled under reduced pressure to give *1'* (97 g), b.p. 81—91° (66 mm).

Following the procedure of Example 1, the following $R^9$ substituted species are obtained. (Table I).

TABLE I

| $R^9$ | R |
|---|---|
| 1. $CH_3$ | $CH_3\overset{O}{\overset{\|\|}{C}}-$ |
| 2. $CH_3CH_2$ | $CH_3\overset{O}{\overset{\|\|}{C}}-$ |
| 3. $CH_3CH_2CH_2$ | $CH_3\overset{O}{\overset{\|\|}{C}}-$ |
| 4. 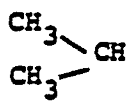 | $CH_3\overset{O}{\overset{\|\|}{C}}-$ |
| 5. 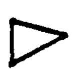 | $CH_3\overset{O}{\overset{\|\|}{C}}-$ |
| 6. Ph (Ph=phenyl) | $CH_3\overset{O}{\overset{\|\|}{C}}-$ |
| 7. $PhCH_2$ | $CH_3\overset{O}{\overset{\|\|}{C}}-$ |

STEP C

Preparation of 2' and 3'

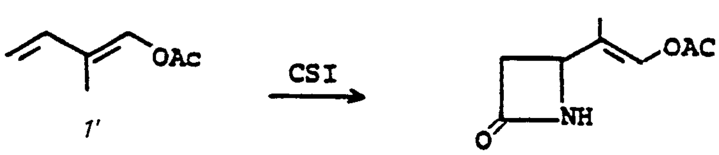


1'

3'

Chlorosulfonylisocyanate (CSI) (6.5 ml) is placed in a three-necked, 100 ml flask equipped with a thermometer, a magnetic stirring bar, a nitrogen inlet tube and a 25 ml pressure-equalizing dropping funnel. The CSI is chilled to −50°C and mixed with 12.5 ml ether through the dropping funnel. The ethereal solution of CSI is allowed to warm up to −25°C, to the solution is added dropwise 1-acetoxyl-2-methyl-1,3-butadiene (*1'*) (5.9 ml in 12.5 ml ether) in 30 min. The mixture is then stirred for 20 min at −20 ± 3°C. The white precipitate formed initially is redissolved at the end of the reaction.

In a 500 ml round bottom flask, a solution of 10 g sodium sulfite and 25 g potassium hydrogen phosphate in 100 ml water is prepared and is cooled in an ice bath. Ether (100 ml) and crushed ice (100 g) are added and the mixture is vigorously stirred in an ice bath. At the end of 20 minutes reaction time, the reaction mixture which contains *2'* is transferred into the dropping funnel and added dropwise to the hydrolysis mixture in 5 minutes. The hydrolysis is allowed to continue for an additional 30 minutes at 3°C. The organic layer is separated and the aqueous is extracted with 50 ml ether. The organic layers are combined, dried over $Na_2SO_4$ and evaporated to give crystalline product *3'* (2.3 g), m.p. 77—78.5°; m.s. 169 ($M^+$); IR 1760 $cm^{-1}$ (β-lactam); NMR (300 MHz, $CDCl_3$): 1.70 (d), 2.16 (s), 2.84 (qq), 3.18 (qq), 4.20 (m), 5.82 (broad, s) and 6.26 (s) ppm.

**Step D:**

**Preparation of 4':**

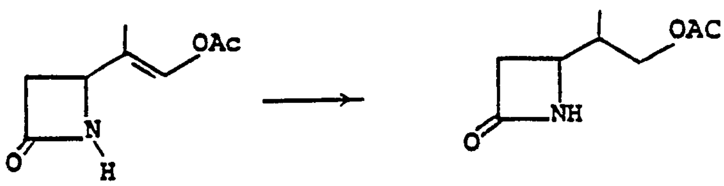

3' 4'

4-(1-methyl-2-acetoxyvinyl)azetidine-2-one (*3'*) (6.5 g) is hydrogenated on a Parr shaker at r.t. under 40 psi hydrogen in the presence of 10% Pd/C (0.6 g) in 200 ml ethylacetate for 2 hr. The mixture is filtered from the catalyst and the filtrate is evaporated *in vacuo* to give the crude product. Purification of the crude product by high pressure liquid chromatography (HPLC, silica gel column, 30% ethylacetate/$CH_2Cl_2$ solvent system) affords white crystalline product *4'* (6.04 g) after evaporation of solvent. The product shows following physical characteristics: ms 171 ($M^+$); IR (Neat) 1754 $cm^{-1}$; NMR (60 MHz, $CDCl_3$): 0.96 (d), 1.01 (d), 2.06 (d, OAc), 2.75—3.80 (m), 3.99 (d) and 6.80 (broad) ppm.

**Step E:**

**Preparation of 5'**

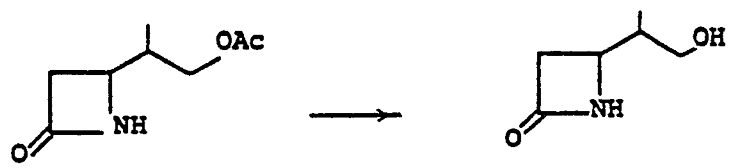

4' 5'

Under $N_2$ at 0°, a solution of 4-(1-methyl-2-acetoxyethyl)-2-azetidinone *4'* (1.2 g) in 10 ml methanol is treated with sodium methoxide (57 mg). After stirring for 1 hr, the solution is neutralized with glacial acetic acid (65 mg). Removal of methanol *in vacuo* gives crude 4-(1-methyl-2-hydroxyethyl)-2-azetidinone *5'* as an oil. The product is purified by chromatography on silica gel eluting with ethyl acetate to give 0.78 g of *5'*: IR (Neat) 1740 $cm^{-1}$; NMR ($CDCl_3$): 0.77 (d), 0.96 (d), 1.90 (m), 2.60—3.30 (m), 3.60 (m), 4.19 (s), and 7.23 (s). The product crystallizes as a colorless solid in the refrigerator.

**Step F:**

**Preparation of 6'**

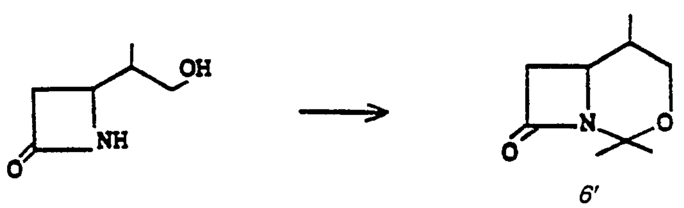

6'

A solution of 4-(1-methyl-2-hydroxyethyl)-2-azetidinone (0.5 g) and 2,2-dimethoxypropane (0.48 g) in 10 ml anhydrous methylene chloride is treated with boron trifluoride (55 mg) at room temperature for 90 min. The mixture is washed with 5 ml saturated $NaHCO_3$. The organic layer is separated, dried over $Na_2SO_4$ and allowed to evaporate *in vacuo* to give crude isomeric mixture of *6'* (0.48 g) as an oil.

Separation of isomers *6'*α and *6'*β is accomplished by high pressure liquid chromatography (HPLC, silica gel) eluting with 40% ethylacetate/hexanes. Evaporation of the solvents affords 250 mg of *6'*β as an oil and 200 mg of *6'*α as a white solid.

NMR (300 MHz, $CDCl_3$) of *6'*α: 0.81 (d), 1.31 (s), 1.68 (s), 1.62 (m), 2.52 (g), 3.05 (m), 3.42 (t), and 3.66 ppm (q), NMR (300 MHz, $CDCl_3$) of *6'*β: 1.10 (d), 1.38 (s), 1.67 (s), 1.90 (m), 2.80 (q), 2.86 (q), 3.62 (q). 3.78 (m) and 3.98 (q) ppm.

**Step G:**

**Preparation of 7'α**

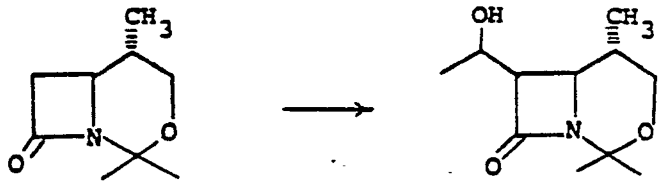

6'α 7'α

At −78°C, diisopropylamine (2.2 g) in 20 ml of anhydrous tetrahydrofuran is treated with n-butyllithium (1.6 M in n-hexane, 14 ml) for 5 min. To the solution is added 8-oxo-5α, 2,2-trimethyl-1-azabicyclo[4.2.0]octane (*6'α*) (3.4 g) and the mixture is stirred for 10 min. The resulting lithium enolate is treated with acetaldehyde (1.68 ml). The mixture is stirred for 1 min then is quenched with 24 ml saturated ammonium chloride at −78°C, then allowed to warm to room temperature (25°C). The mixture is extracted with ethylacetate (2 × 100 ml). The organic layer is separated, dried over $Na_2SO_4$ and allowed to evaporate *in vacuo* to give 4.5 g of the crude product 7'α.

The crude isomeric mixture of 7'α is purified and separated by HPLC (silica gel) eluting with 50% ethylacetate/methylene chloride to give 3.5 g of *trans*-7'α and 0.5 g of *cis*-7'α. Both isomers are crystalline solids.

**Step G':**

**Preparation of 7'β**

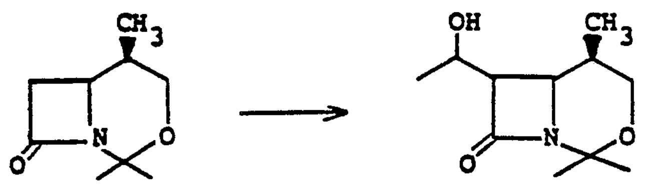

6'α 7'β

Following the procedure of Step G, except replacing the starting material 6'α with 6'β isomer, the products, trans-7'β (4.0 g) and *cis*-7'β (0.1 g), are obtained.

**Step H:**

**Preparation of 7"β**

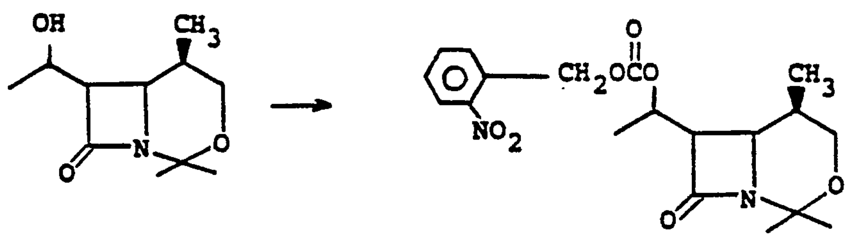

Under anhydrous conditions at 0°C a solution of R enriched trans-7'β (2.90 g) in 60 ml methylene chloride is treated with 4-dimethylaminopyridine (3.32 g) and o-nitrobenzylchloroformate (5.88 g). The mixture is allowed to warm to room temperature and stirred for 1 hr. The resulting mixture is washed with 0.1*N* HCl, water, brine and water. The organic layer is separated, dried over $Na_2SO_4$ and allowed to evaporate *in vacuo* to give crude products. The crude products are dissolved in 20 ml ether and chilled at −5°C give the o-nitrobenzyl alcohol (0.5 g) which is separated by filtration. The isomeric mixture comprising trans-7'β is purified and separated by HPLC (silica gel) eluting with 40% ethylacetate/cyclohexane to give 1.2 g of S-trans-7'β and 1.0 g of R-trans-7'β.

The spectrum data of R-trans-7''β: NMR (300 MHz, $CDCl_3$): 1.12 (d), 1.40 (s), 1.46 (d), 1.73 (s), 1.95 (m), 3.20 (q), 3.60 (q), 3.74 (q), 3.95 (q), 5.07 (m), 5.58 (q), 7.56 (t), 7.70 (m) and 8.19 (d) ppm.

The spectrum data of S-trans-7''β: NMR (300 MHZ, $CDCl_3$): 1.10 (d), 1.40 (s), 1.43 (d), 1.72 (s), 1.94 (m), 3.34 (q), 3.61 (q), 3.67 (q), 3.96 (q), 5.13 (m), 5.64 (q), 7.53 (m), 7.68 (m), and 8.17 (d) ppm.

Step H:

Preparation of 7''α

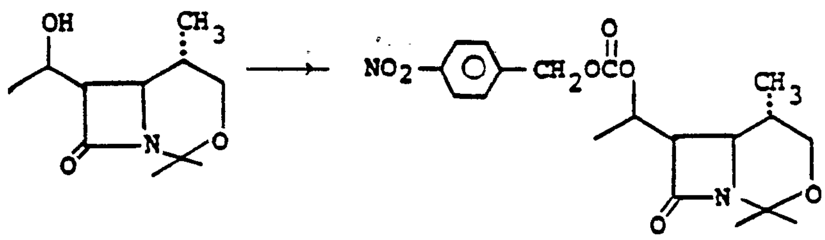

7''α

Following the procedure of Step H; except replacing the starting material trans-7'β with trans-7'α isomer, and using p-nitrobenzyloxychloroformate as carbonating agent, the products R-trans-7'α and S-trans-7''α are obtained.

The following chart summarizes the foregoing separation:

TABLE II

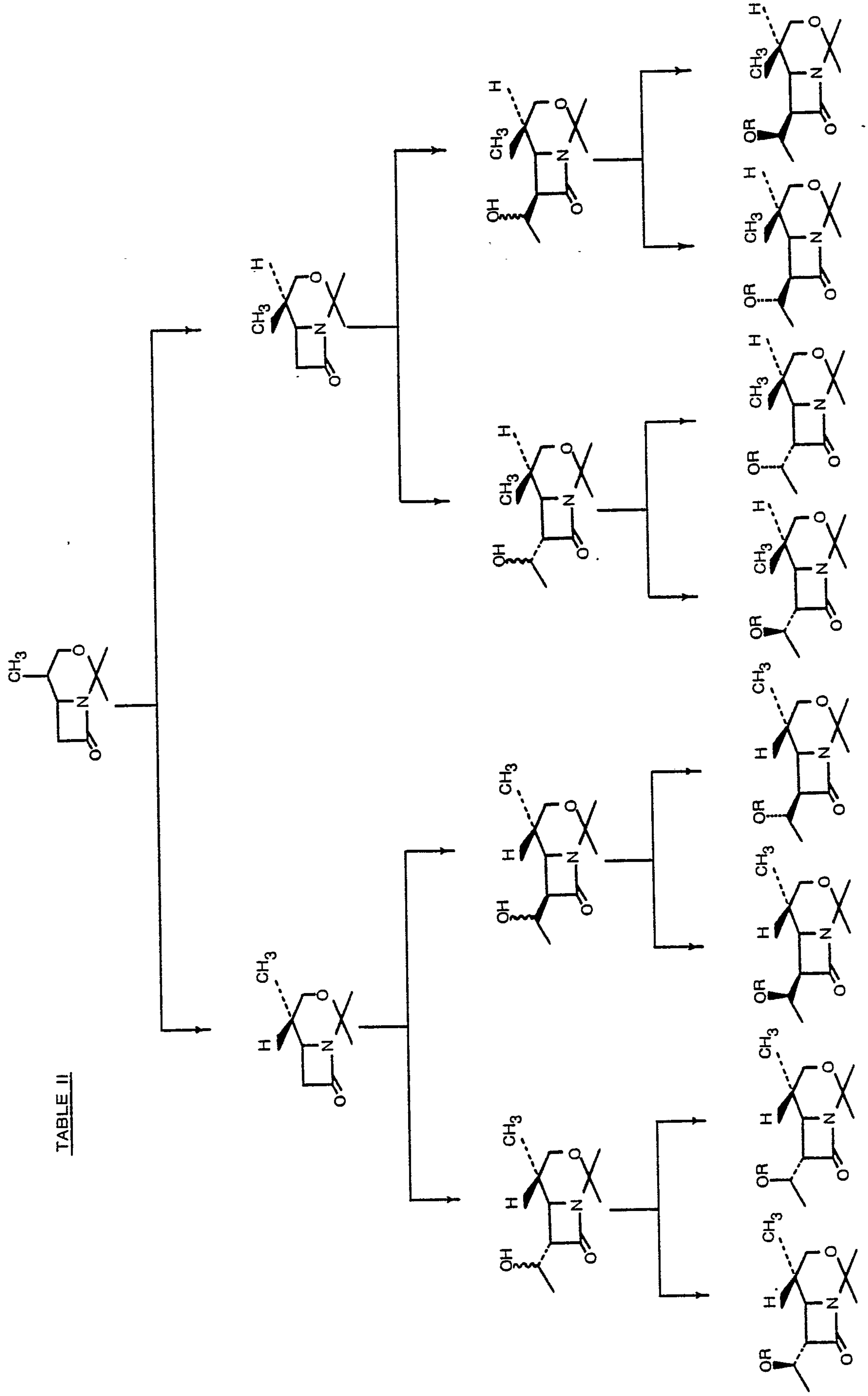

(R = p-nitrobenzyloxycarbonyl)

Step I:

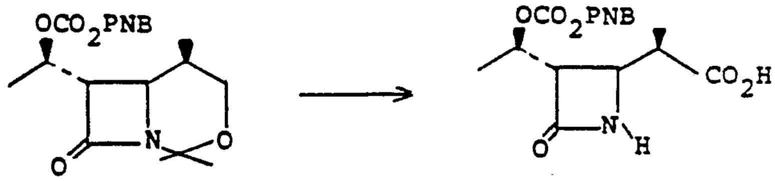

7' 8'

The bicyclic azetidinone 7' (6.0 g) in 60 ml acetone is treated with 4*N* Jones reagent (9.4 ml) at 0°C for 30 min. The reaction is quenched with 1 ml isopropanol at 0°C for 10 min, then mixed with 250 ml ethylacetate and washed with water and brine until blue color disappeared in the organic layer. The organic layer is separated, dried over $MgSO_4$, and evaporated *in vacuo* to give crude product which is purified by a silica gel column (4.4 × 10 cm) eluting with ethylacetate to give 3.1 g of 8' as a crystalline solid.

Step J:

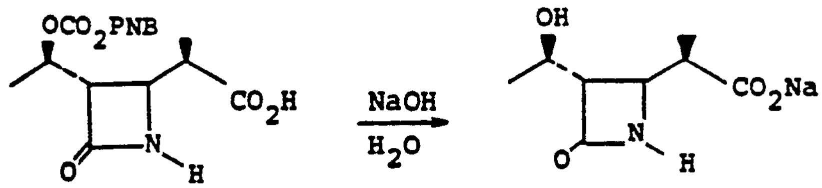


8' 9'

The azetidinone carboxylic acid 8' (3.0 g) is suspended in 50 ml water. The mixture is stirred and treated with 2.5*N* NaOH and maintained at pH 12.0 at room temperature for 30 min. The resulting homogenous solution is neutralized with 2.5*N* HCl to pH 7.5. After the mixture is extracted with EtOAc, the aqueous layer is concentrated and lyophilized to give 9' a white a solid, 60 MHz NMR ($CDCl_3$): 1.15 (d, 3H, J=6.0Hz), 1.23 (d, 3H, J=6.0Hz), 2.45 (m, 1H), 3.02 (q, 1H, J=2.0 and 5.8Hz), 3.68 (q, 1H, J=2.0 and 8.0Hz), 4.12 (m, 1H).

Step K:

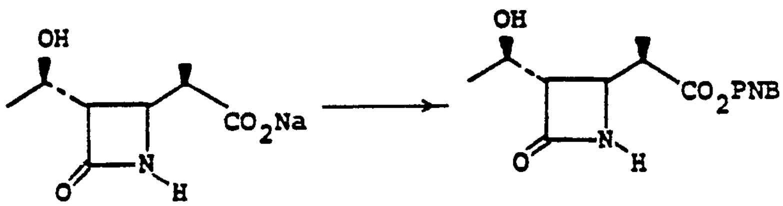

9' 10'

The azetidinone carboxylic acid sodium salt 9' (2.2 g) and p-nitrobenzylbromide (2.94 g) are stirred at room temperature in DMF (29.3 ml) for 5 hrs. The mixture is diluted with EtOAc and washed with water and brine. The organic layer is separated, dried over $MgSO_4$ and evaporated to give crude product which is purified by TLC plates eluting with 50% EtOAc/cyclohexane to give 1.34 g of white solid product 10', 60 MHz NMR ($CDCl_3$): 1.28 (d, 3H, J=7.0Hz), 1.23 (d, 3H, J=7.0Hz), 2.80 (m), 3.00 (q, 1H, J=2.0 and 6.0Hz), 3.84 (q, 1H, J=2.0 and 7.0Hz), 4.14 (m, 1H), 5.28 (s, 2H), 6.73 (broad singlet), 7.53 (d, 2H), and 8.23 (d, 2H).

Step L:

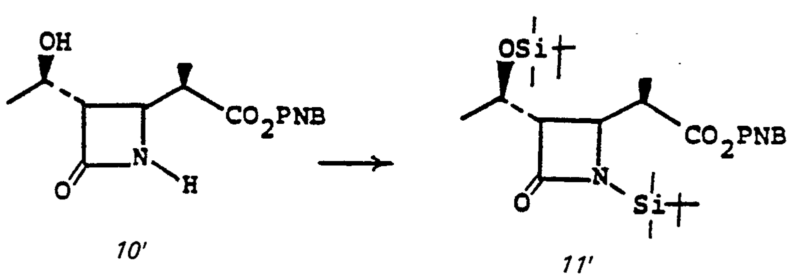

10' 11'

The ester *10'* (1.33 g) is stirred with t-butyldimethylchlorosilane (2.49 g), triethylamine (4.61 ml) in DMF (16 ml) at room temperature overnight. The mixture is filtered from solids and evaporated *in vacuo* to give crude product *11'* which is redissolved in ethylacetate and washed with water, brine, dried over $Na_2SO_4$, concentrated to 0.5 ml then purified by TLC eluting with 30% EtOAc/cyclohexane to give 1.65 g of product *11'* 60 MHz NMR ($CDCl_3$): 0.06 (s, 3H), 0.10 (s, 3H), 0.12 (s, 3H), 0.15 (s, 3H), 0.90 (s, 9H), 0.96 (s, 9H), 1.06 (d, 3H, J=7.8Hz), 1.10 (d, 3H, J=6.0Hz), 3.00 (m, 1H), 3.28 (q, 1H, J=2.2 and 7.8Hz), 3.71 (q, 1H, J=2.2 and 4.0Hz), 4.06 (m, 1H), 5.22 (s, 2H), 7.53 (d, 2H) and 8.22 (d, 2H).

**Step M:**

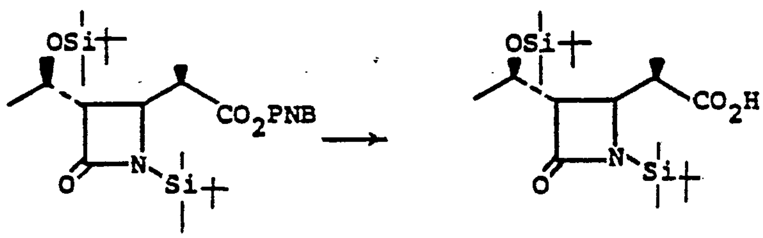

*11'* *3'*

The bis-silyl azetidinone ester *11'* (1.60 g) is 30 ml EtOAc is hydrogenated under 50 psi hydrogen in the presence of 0.32 g 10% Pd/C for 30 min. The mixture is filtered from catalyst. The catalyst is washed with MeOH. The methanol and ethylacetate solutions are combined and evaporated *in vacuo* to give white solids. The crude product is re-dissolved in ethylacetate and washed with 0.1*N* HCl. The organic layer is separated, dried over $Na_2SO_4$ and evaporated to give white solid product *3* (1.30 g), 60MHz NMR ($CDCl_3$): 0.03 (s, 3H), 0.05 (s, 3H), 0.10 (s, 3H), 0.15 (s, 3H), 0.91 (s, 9H), 0.99 (s, 9H), 1.20 (d, 3H, J=7.2Hz), 1.22 (d, 3H, J=6.2Hz), 2.94 (m, 1H), 3.31 (q, 1H, J=2.4 and 7.6Hz), 3.75 (q, 1H, J=2.4 and 4.0Hz), 4.09 (m, 1H), and 10.72 (s, 1H).

Example 2

Preparation of 3 (Method II)

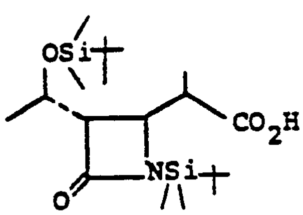

*3*

*Step A:* Preparation of 3-Methyl-1,4-Pentadiene *1'*

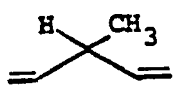

*1'*

Procedure a

β-Methylglutaric acid (1.0 mole, obtained from Aldrich Chemical Company), is refluxed for 2 hours with thionyl chloride (68% excess). After removal of excess thionyl chloride, absolute ethanol (109% excess) is added slowly. The mixture is refluxed for 3 hours then distilled to collect the product, diethyl β-Methylglutarate.

To a suspension of lithium aluminum hydride (24 g) in ether (860 ml) is added dropwise with rapid stirring a solution of diethyl β-methylglutarate (124 g in 250 ml ether). The mixture is refluxed for 6 hours, then cooled to room temperature. Water (25 ml) is added slowly. The mixture is then titrated with 10% NaOH until a clear organic layer is obtained. The organic layer is separated, dried over anhydrous sodium sulfate then evaporated *in vacuo* to give 3-methyl-1,5-pentanediol. The 3-methyl-1,5-pentanediol (0.5 mole) is treated with thionyl chloride (1.05 mole) at reflux for 3 hours. After removal of excess thionyl chloride *in vacuo,* the 3-methyl-1,5-dichloropentane is obtained.

2-methyl-1,5-dichloropentane (41 g) is added dropwise at 170°C to a mixture of 48 g of sodium hydroxide and 40 g of polyethylene glycol tetramer and the mixture is distilled to give 3-methyl-1,4-pentadiene.

Procedure b

At −40°C, 1,3-dichlorobutane (50 g) is mixed with aluminum chloride (5 g). Ethylene is bubbled through the solution for 4 hours. The mixture is allowed to warm to room temperature, then hydrolyzed with water and extracted with ethyl acetate to give 3-methyl-1,5-dichloropentane.

A mixture of 0.5 mole of 3-methyl-1,5-dichloropentane, 2-methylquinoline (2 moles), and sodium iodide (0.1 mole) is refluxed in a flask equipped with a Vigreaux column at the top of which is a condenser and take-off. The diolefin *1'* is collected during 8 hours reaction. The product is dried over anhydrous sodium sulfate.

**Step B:**

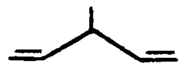

*1'*

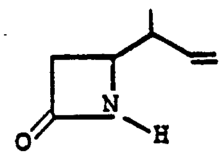

*2'*

In a sealed tube, 3-methyl-1,4-pentadiene (9.6 g) and chlorosulfonyl isocyanate (14.2 g) are allowed to stand at room temperature for 6 days. The resulting mixture is diluted with methylene chloride and added slowly to a stirred aqueous solution which contains 20 g of $Na_2SO_3$ and 50 g of $K_2HPO_4$ at 0—5°C for 30 minutes. The organic layer is separated and dried over $MgSO_4$. After evaporation, the crude product is chromatographed on silica gel GF eluting with EtOAc to give *2'*.

**Step C:**

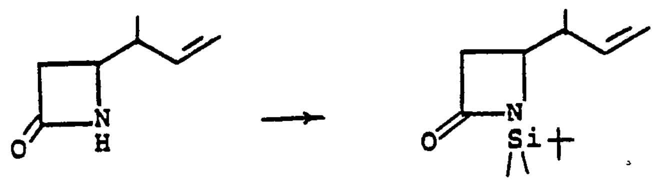

*2'* *3'*

t-Butyldimethylchlorosilane (7.5 g) is added in one portion to an ice-cold, stirred solution of 4-(1-methyl-prop-2-ene)-azetidin-2-one (6.54 g) and triethylamine (12 ml) in anhydrous dimethylformamide (100 ml). The reaction mixture is stirred at a temperature ranging from 0 to 5°C for 1 hour and then allowed to warm to room temperature. Most of the solvent is removed under vacuum to give a residue which is partitioned between diethyl ether (250 ml) and water. The ethereal phase is washed with 2.5N hydrochloric acid (50 ml), water (3 × 50 ml), and brine, dried with magnesium sulfate, filtered and evaporated under vacuum to provide a crude product which is purified by chromatography on silica gel (20% ether in petroleum ether) to yield *3'*.

**Step D:**

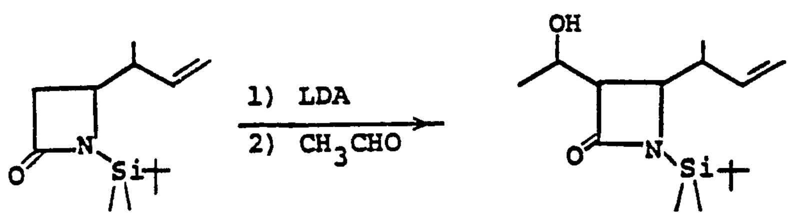


*3'* *4'*

n-Butyllithium in hexane (26.25 mmol) is added slowly by a syringe to a solution of diisopropylamine (26.25 mmol) in anhydrous tetrahydrofuran (100 ml) at −78°C. The resulting solution is stirred for 15 min prior to the addition of a solution of *3'* (25.0 mmol) in anhydrous tetrahydrofuran (25 ml). After stirring for 15 min at −78°C, acetaldehyde (75 mmol) is added by syringe and the resulting solution is stirred at −78°C for 5 min. Saturated aqueous ammonium chloride solution (15 ml) is added by syringe and the reaction mixture is allowed to warm to room temperature, then diluted with ether (250 ml) and washed with 2.5N hydrochloric acid solution (2 × 50 ml), water (100 ml) and brine over magnesium sulfate. Solvents are removed *in vacuo* and the residue is chromatographed on silica gel (1:1, ether: petroleum ether) to give the expected product *4'*.

**Step E:**

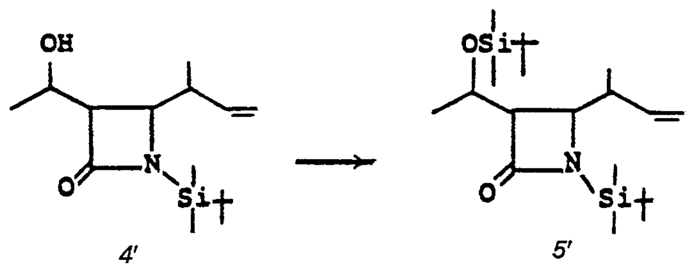

4' 5'

At 0°C, the alcohol *4'* (5.00 g) is dissolved in DMF (50 ml) and treated with t-butyl-dimethylchlorosilane (7.51 g) and triethylamine (12 ml). The mixture is allowed to warm to room temperature with constant stirring for 2 hours, then filtered from solids, evaporated *in vacuo* to give oil residue which is re-dissolved in ethylacetate and washed with 0.1 *N* HCl, water, and brine. The organic layer is separated, dried over $MgSO_4$ and evaporated *in vacuo* to give crude product *5'*. HPLC purification of product (20% ethylacetate/cyclohexane) affords *5'*.

**Step F:**

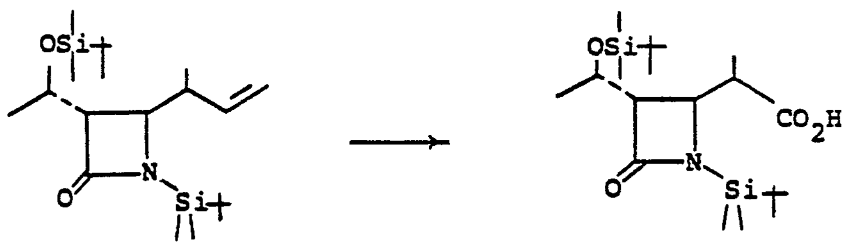

5' 3'

At a solution of *5'* (3.0 mmol) in dry methylene chloride (30 ml) is cooled to −78°C (dry ice-acetone) and a stream of ozone is bubbled through until the reaction mixture becomes blue. The ozone flow is then stopped and the reaction is purged by bubbling through nitrogen until the blue color disappears. Solid *m*-chloroperbenzoic acid (3.0 mmol) is added and the cold bath is removed. When the reaction mixture reaches room temperature, the flask is fitted with a reflux condenser and the mixture is heated at reflux for three days. Removal of solvents *in vacuo* gives crude product which is chromotographed on silica gel (2% glacial acetic acetic acid in methylene chloride) to *3'*.

Example 3

Preparation of 1a:

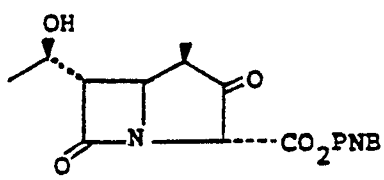

*1a*

**Step A:**

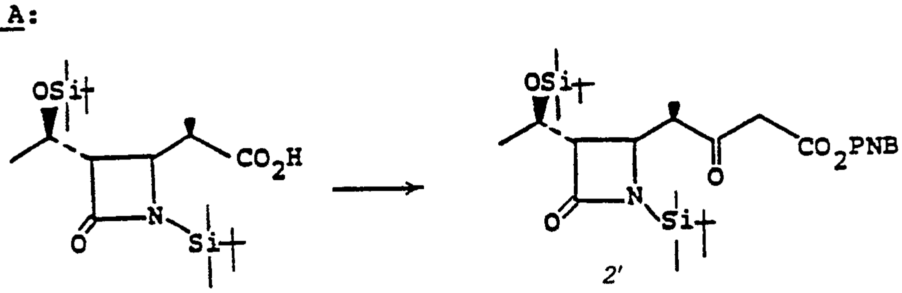

1' 2'

PNB = p-nitrobenzyl

The N,O-bis-silyl azetidinone carboxylic acid *1'* (500 mg) suspended in acetonitrile (14.8 ml) is treated with 1,1'-carbonyldiimidazole (229.6 mg) and stirred at room temperature for 30 min. The mixture is then treated with p-nitrobenzylmalonate magnesium salt (1.18 g) and heated at 60°C for 3 hrs. The mixture is diluted with $CH_2Cl_2$ and washed with water, brine, and dried over $Na_2SO_4$. TLC purification (75% EtOAc/cyclohexane) of the crude product provides 0.50 g of product *2'*, 60MHz NMR ($CDCl_3$): 0.05 (s), 0.15 (s), 0.28 (s), 0.90 (s), 0.97 (s), 1.11 (d, 3H, J=7.0Hz), 1.19 (d, 3H, J=6.0Hz), 2.80—3.30 (m), 3.50 (s, 2H), 3.50—4.20 (m), 5.18 (s, 2H), 7.37 (d) and 8.06 (d).

Step B:

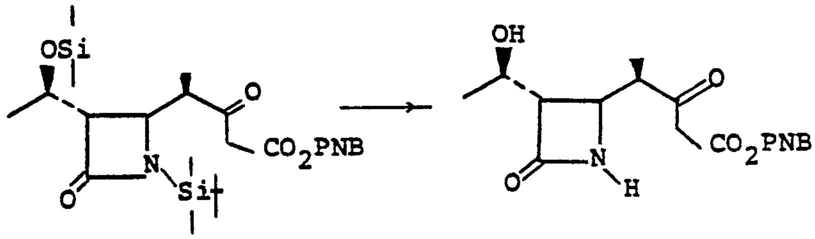

2' 3'

The bis-silyl β-keto ester *2'* (667 mg) is methanol (16 ml) is stirred with 2 ml 6*N* HCl at room temperature for 2 hrs. The mixture is diluted with ethylacetate, washed with 0.1*M* sodium phosphate buffer, brine, dried over $Na_2SO_4$ then evaporated to give 0.6 g of crude product which is purified by TLC eluting with 100% ethylacetate to give product *3'* (277 mg), IR (neat): 1754 $cm^{-1}$, 60MHz NMR ($CDCl_3$): 1.21 (d), 2.68—3.20 (m), 3.20—4.10 (m), 3.70 (s), 5.27 (s), 6.80 (broad, N*H*), 7.43 (d) and 8.13 (d).

Step C:

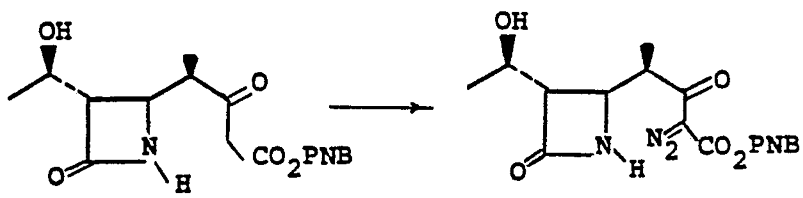

3' 4'

The β-keto ester *3'* (270 mg) in 3.2 ml acetonitrile p-toluene-sulfonazide (1.11 g, 3.33 meq/g), triethylamine (0.31 ml) are placed in a 25 ml round-bottomed flask. The mixture is stirred under nitrogen atmosphere at room temperature for 1 hr., then diluted with ethylacetate and washed with water, brine, and dried over $MgSO_4$. The crude product is purified by TLC eluting with 50% EtOAc/cyclohexane to give 221.6 mg of *4'*. IR ($CHCl_3$): 2140 ($C{=}N_2$), 1740 and 1650 $cm^{-1}$.

Step D:

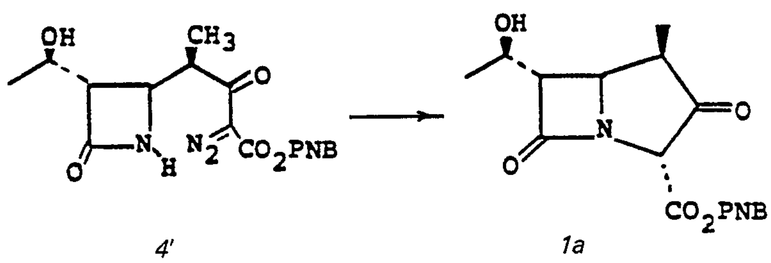

4' 1a

The diazo β-keto ester *4'* (38.6 mg) in toluene (1 ml) is heated at 80°C in the presence of rhodium acetate (1.7 mg) for 10 min. The mixture is diluted with ethylacetate (10 ml) and washed with water and brine. The organic layer is separated, dried over $MgSO_4$ and evaporated *in vacuo* to give bicyclic keto ester *1a* (30.6 mg).

Example 4

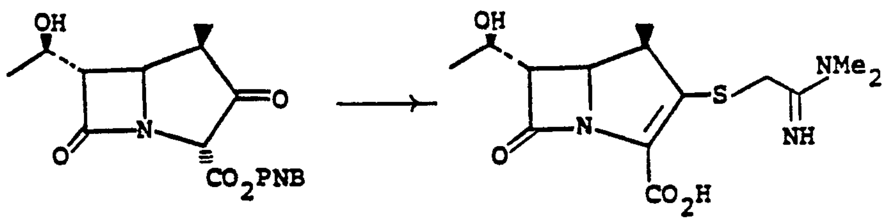

1a I

The bicyclic keto ester *1a* (33.3 mg) in acetonitrile (0.47 ml) under $N_2$ atmosphere is treated with diphenyl chlorophosphate (20.97 μl) and diisopropylethylamine (19.22 μl) at 0°C and stirred for 30 min. To

the mixture is added DMSO (0.20 ml) solution of N,N-dimethylmercaptoacetamidine hydrochloride (18.68 mg) and diisopropylethylamine (24.3 μl) and stirred for 1 min. at 0°C. The mixture is then mixed with 10 ml ether and centrifuged to separate the oil product which is subsequently re-dissolved in 3.72 ml THF and 2.80 ml 0.1*M* pH 7.0 sodium phosphate buffer. The solution is hydrogenated under 50 psi hydrogen in the presence of 50.0 mg of 10% Pd/C at room temperature for 30 min. Additional 50 mg of 10% Pd/C is added and the mixture is further hydrogenated for 30 minutes then filtered from catalysts. The filtrate is extracted with ether, concentrated to 4 ml then chromatographed by a Dowex-50X4 ($Na^+$Cycle) column (2.2 × 6 cm) which is eluted with DI water to give product I.

Lyophilization of the aqueous solution gives product *I* as white powder (6.20 mg), Uv $H_2O$ λ max 293 nm (ε=8,796); 300 MHz NMR ($D_2O$): 1.20 (d, 3H, J=8.0 Hz), 1.29 (d, 3H, J=6.0Hz), 3.19 (s, 3H), 3.35 (5.3H), 3.54 (q, 1H, J=2.2 and 6.0 Hz), 4.26 (m).

Example 5

Preparation of 9

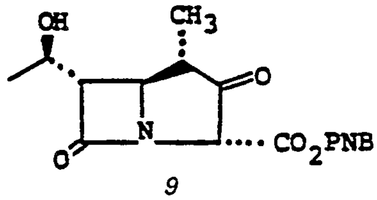

*9*

Step A:

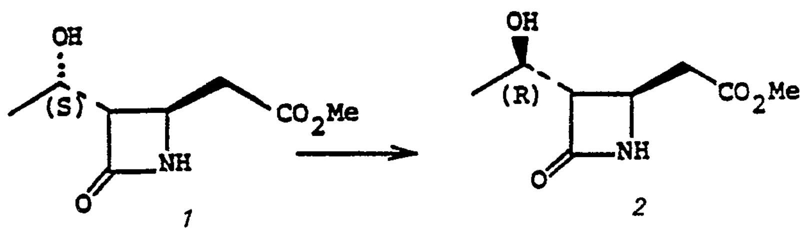

*1* *2*

At 0°C, under $N_2$, the chiral starting material *I* (0.94 g), methylene chloride (8.2 ml), triphenylphosphine (3.34 g) and formic acid (1.15 g, 97%) are placed in a 50 ml three-necked flask. To the solution is slowly added di-isopropyl azodicarboxylate (2.58 g). The mixture is stirred at room temperature overnight; TLC shows all the starting material consumed. The mixture is cooled to 0°C and treated with methanol (11.4 ml), water (4.9 ml) and concentrated hydrochloric acid (1.7 ml) for 2 hours, then extracted with methylene chloride. The organic layer is separated, dried over $MgSO_4$ and evaporated to give product *2*.

Step B:

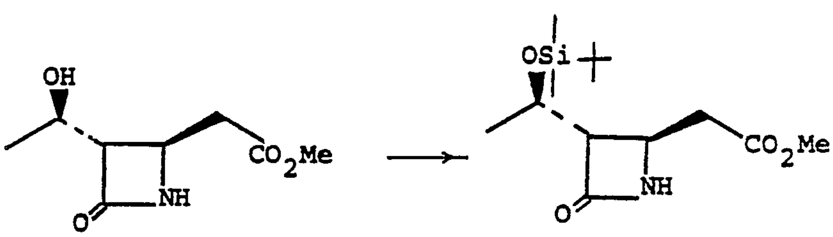

*2* *3*

The free hydroxyl azetidinone *2* (374 mg) in DMF is treated with imidazole (688 mg) and t-butyldimethylchlorosilane (603 mg) at room temperature for 7 hours. The mixture is evaporated *in vacuo* and the residue is re-dissolved in ethyl acetate and washed with 1*N* HCl, water and brine. The organic layer is separated, dried over $MgSO_4$ and evaporated *in vacuo* to give *3*.

Step C:

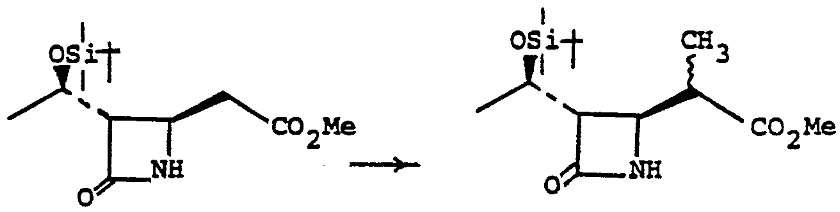

*3* *4*

Under $N_2$, at −78°C, diisopropylamine (1.68 ml) in 12.5 ml THF is treated with n-butyllithium (12.5 ml, 1.6 *M* in hexane) for 10 min. To the solution is added starting material *3* (1.51 g in 5 ml THF) and the mixture is stirred for 20 min, then is treated with iodomethane (1.87 ml). After stirring 40 min at −78°C, the mixture is allowed to warm to 0· then hydrolyzed with 0.1 *N* HCl. The mixture is extracted with ethyl acetate. The organic layer is washed with water, brine then dried over $MgSO_4$ and evaporated *in vacuo* to give 2.1 g of isomeric mixture of 4 which is chromatographically by HPLC separated to give pure α-methyl *4*. 60 MHz Nmr ($CDCl_3$): 0.10 (s, 6H), 0.90 (s, 9H), 1.25 (d, 6H), 2.60 (m), 3.71 (s, 3H), 4.18 (quintet, 1H), and 6.21 (broad singlet).

**Step D:**

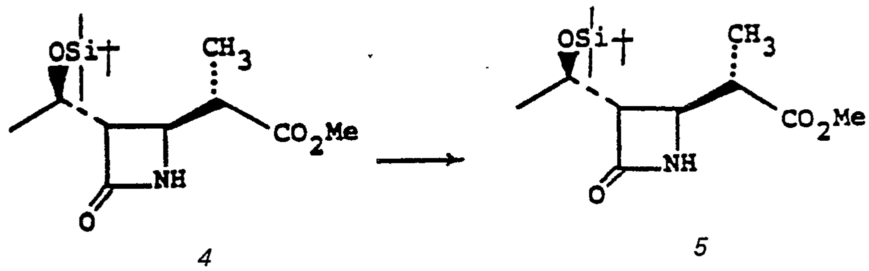

4 5

The methyl ester 4 (1.0 g) is treated with NaOH solution (2.5 *N*, 1.4 ml) in methanol (5 ml) and water (1 ml) at room temperature for 5 hours. The mixture is acidified with 1 *N* HCl to pH 1.0 then extracted with ethyl acetate. The organic layer is separated and dried over $MgSO_4$ and evaporated to give 0.70 g of *5* as white crystalline solids.

**Step E:**

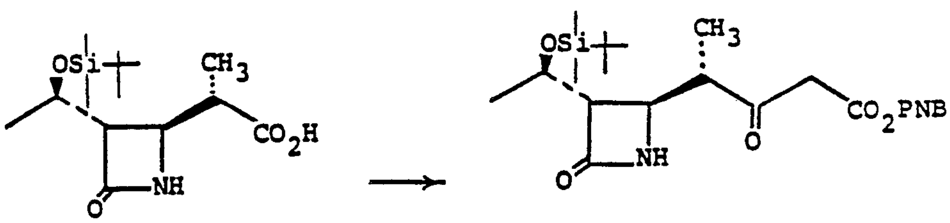

5 6

The carboxylic acid (300 mg) suspension in acetonitrile is treated with 1,1′-carbonyldiimidazole (194.6 mg) at room temperature for 30 min. The mixture becomes homogeneous within 5 min. To the solution is added magnesium p-nitrobenzylmalonate (1.00 g), then the mixture is heated at 65°C for 3 hours. The final reaction mixture is evaporated *in vacuo* to give an oily residue which is re-dissolved in 10 ml ethyl acetate and washed with water and brine. The organic layer is separated, dried over $MgSO_4$ then evaporated *in vacuo* to give 0.76 g of crude product which is purified by silica gel TLC plates eluting with 50% EtOAc/cyclohexane to give 0.41 g of product *6*, 60MHz NMR ($CDCl_3$): 0.10 (s, 6H), 0.90 (s, 9H), 1.26 (d, 6H), 2.76 (m), 3.64 (s, 2H), 4.12 (quintet, 1H), 5.27 (s, 2H), 6.40 (broad singlet, 1H), 7.43 (d, 2H) and 8.12 (d, 2H).

**Step F:**

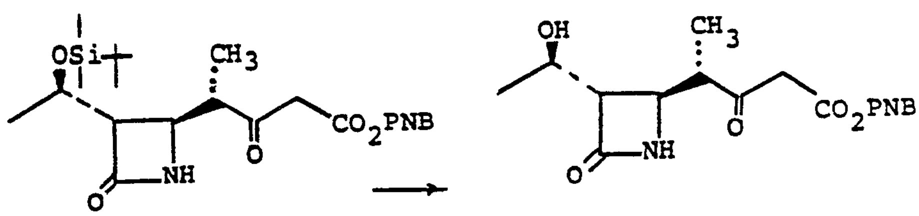

6 7

The starting material 6 (400 mg) is dissolved in 4 ml methanol and treated with 6*N* HCl (0.42 ml) at room temperature for 80 minutes. The mixture is diluted with ethyl acetate and washed with 0.1*N* pH 7.0 phosphate buffer and brine. The organic layer is separated, dried over $MgSO_4$ and evaporated *in vacuo* to give crude product 7 as solids which is triturated in pet. ether then filtered to give 269 mg of 7, 60 MHz NMR ($CDCl_3$): 1.11 (d), 1.34 (d), 1.96 (m), 2.80—4.20 (m), 3.60 (s), 5.24 (s), 7.43 (d) and 8.17 (d).

**Step G:**

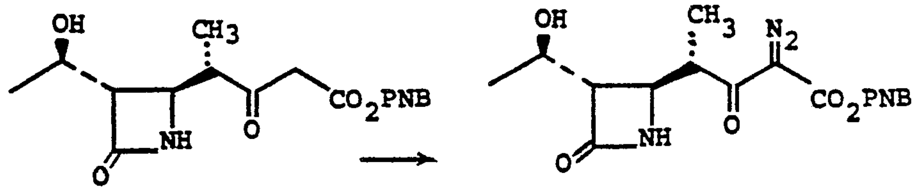

7 8

The β-keto ester 7 (269 g) dissolved in 3.2 ml acetonitrile is gently stirred with Amberlite XE-301-$SO_2N_3$ (1.5 g, 3.33 meq of $N_3$/g) and triethylamine (0.46 ml) at room temperature for 1.5 hours. The mixture is filtered from polymer beads and the filtrate is evaporated *in vacuo* to give oily residue which is purified by TLC (silica gel) eluting with ethyl acetate to give diazo keto ester 8 as crystalline solids (147.1 mg), IR (film): 2150 $cm^{-1}$ ($N_2$); 60 MHz NMR ($CDCl_3$): 1.14 (d), 1.23 (d), 2.80—4.20 [m], 5.26 (s), 6.44 (broad singlet), 7.38 (d) and 8.08 (d).

**Step H:**

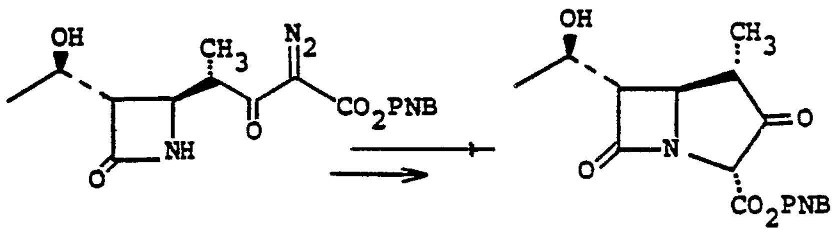

8 9

· The diazo compound 8 (145 mg) is dissolved in 4.1 ml toluene and 2.5 ml ethyl acetate. The mixture is heated at 80—85°C in the presence of rhodium acetate (2.9 mg) for 15 minutes. The solution is allowed to cool to room temperature then diluted with 10 ml ethyl acetate and washed thoroughly with water, and brine. The organic layer is separated, dried over $MgSO_4$ then evaporated *in vacuo* to give product *9* (113.3 mg) as solids. 60 MHz NMR ($CDCl_3$): 1.24 (d), 1.37 (d), 2.71 (m), 3.22 (q), 3.71 (q), 4.22 (quintet), 4.88 (s), 5.28 (s), 7.47 (d), and 8.16 (d).

Example 6

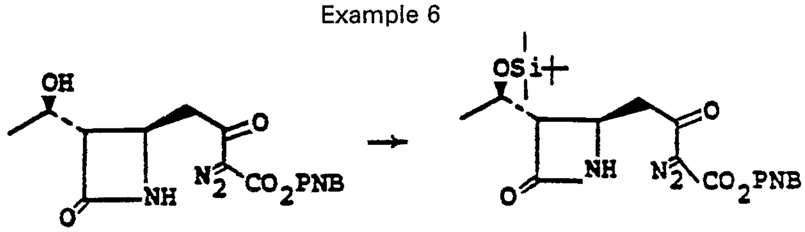

*1* 2

The starting material *1* (2.10 g) is treated with t-butyldimethylchlorosilane (1.29 g) and imidazole (1.46 g) in DMF (8.6 ml) at room temperature for 3 hours. The mixture is evaporated *in vacuo* and the residue is redissolved in ethyl acetate and washed with water and brine. The organic layer is separated, dried over $Na_2SO_4$, and evaporated *in vacuo* to give 574 mg product *2:* IR 2155 ($N_2$), 1754 $cm^{-1}$ (β-lactam); 60 MHz NMR ($CDCl_3$): 0.08 (s), 0.90 (s), 1.12 (d), 2.60—3.20 (m), 3.80—4.20 (m), 5.40 (s), 6.26 (broad singlet), 7.53 (d) and 8.21 (d).

Example 7

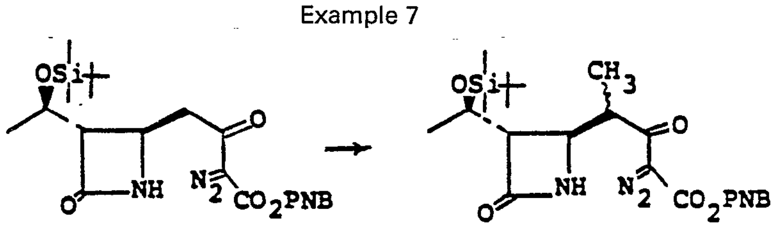

*1* 2

At −78°C, under $N_2$, diisopropylamine (168.2 μl) is treated with n-BuLi (1.6*M*, 1.13 ml) in THF (0.84 ml) for 10 min. To the solution is added *1* (280 mg in 0.2 ml THF). The solution became deep-red color upon the addition of 1. After the mixture is stirred for 20 min, iodomethane (0.7 ml) added and stirred for additional 40 min at −78°C. The solution is allowed to warm to room temperature then hydrolyzed with saturated $NH_4Cl$, extracted with ethyl acetate. The organic layer is separated, dried over $Na_2SO_4$, and evaporated *in vacuo* to give Product *2*: IR 2125 ($N_2$), 1754 $cm^{-1}$ (β-lactam): 60 MHz NMR ($CDCl_3$): 0.03 (s), 0.86 (s), 1.15 (d), 1.26 (d), 2.40—3.00 (m), 3.90—4.20 (m), 4.90 (s), 7.50 (d), 8.21 (d).

Example 8

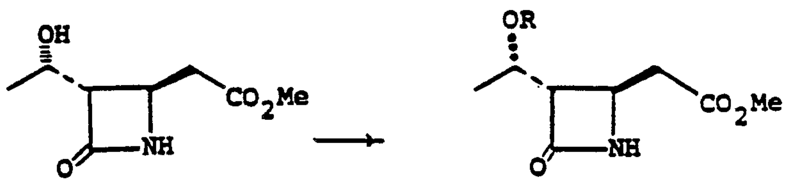

*1* *2*

The chiral starting material *1* (9.35 g) dissolved in 100 ml DMF is treated with t-butyldimethylchlorosilane (15.10 g) and imidazole (17.20 g) at room temperature for 4 hours. The mixture is evaporated *in vacuo* and the residue is redissolved in ethyl acetate and washed with 1.0 *N* HCl, water and brine. The organic layer is separated *in vacuo* to give 15.8 g of *2*. 60 MHz NMR ($CDCl_3$): 0.04 (s), 0.89 (s), 1.27 (d), 2.61 (d), 2.82 (m), 3.68 (s), 3.90—4.20 (m), 6.33 (s).

Example 9

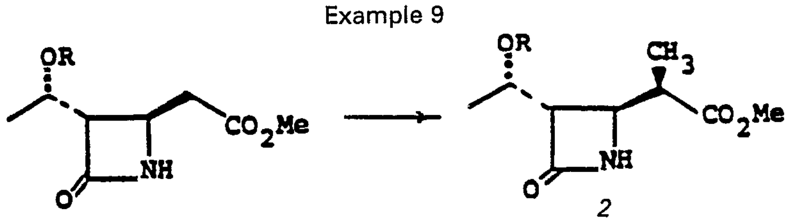

*2*

*1*

(R= t-butyl(dimethyl)silyl)

Under nitrogen, at −78°C, diisopropylamine (1.68 ml) in THF (12.5 ml) is treated with n-butyllithium (1.6*M*, 12.5 ml) and stirred for 10 min. To the solution is added *1* (1.51 g in 5 ml THF; and stirred for 20 min. The resulting orange solution is treated with iodomethane (1.87 ml) and stirred at −78°C for 40 min. then gradually warmed to 0°C. The mixture is hydrolized with 2 ml saturated $NH_4Cl$, diluted with ethyl acetate. The organic layer is separated, dried over $Na_2SO_4$ and evaporated *in vacuo* to give product *2*, 60 MHz NMR: 0.05(s), 0.92(s), 1.30(d), 1.31(d), 2.60—3.10(m), 3.69(s), 3.90—4.30(m), 6.72(broad singlet).

Example 10

Following the foregoing text and examples, the following species I are obtained when the corresponding bicyclic keto ester is treated with $HSR^8$.

TABLE I

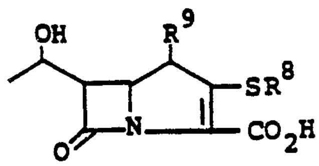

I

| | $R^8$ | $R^9$ |
|---|---|---|
| 1. | 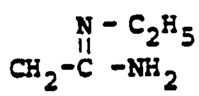 | $CH_3$ |
| 2. | 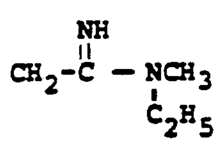 | $CH_3$ |

| | $R^8$ | $R^9$ |
|---|---|---|
| 3. | 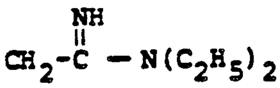 | $CH_3$ |
| 4. | 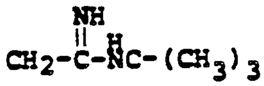 | $CH_3$ |
| 5. | 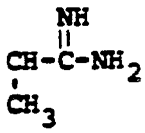 | $CH_3$ |
| 6. | 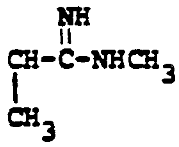 | $CH_3$ |
| 7. | 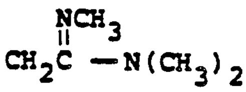 | $CH_3$ |
| 8. | 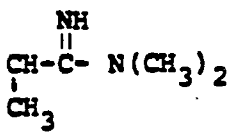 | $CH_3$ |
| 9. | 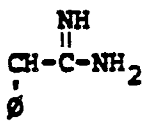 | $CH_3$ |
| 10. | 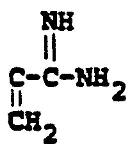 | $CH_3$ |
| 11. | 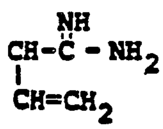 | $CH_3$ |
| 12. | 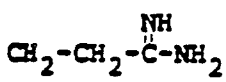 | $CH_3$ |
| 13. | 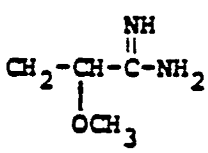 | $CH_3$ |
| 14. | 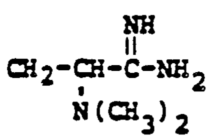 | $CH_3$ |
| 15. | 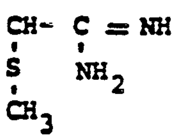 | $CH_3$ |

| | $R^8$ | | $R^9$ |
|---|---|---|---|
| 16. | 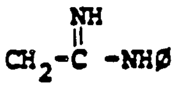 | | $CH_3$ |
| 17. | 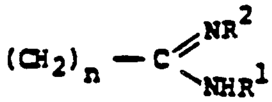 | $n = 2-5$, $R^2 = H$, $CH_3$<br>$R^1 = H$, $CH_3$ | $CH_3$ |
| 18. | 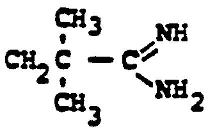 | | $CH_3$ |
| 19. | 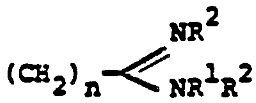 | $n = 2-5$, $R^1$, $R^2 = H$, $CH_3$ | $CH_3$ |
| 20. | 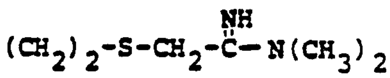 | | $CH_3$ |
| 21. | 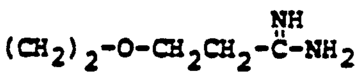 | | $CH_3$ |
| 22. | 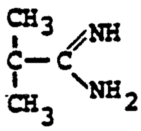 | | $CH_3$ |
| 23. | 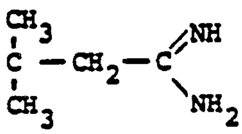 | | $CH_3$ |
| 24. | 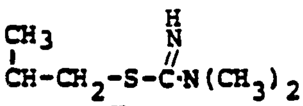 | | $CH_3$ |
| 25. | 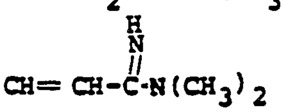 | | $CH_3$ |
| 26. | 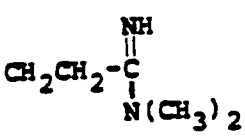 | | $CH_3$ |
| 27. | 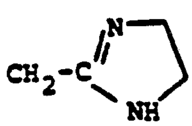 | | $CH_3$ |
| 28. | 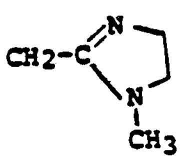 | | $CH_3$ |

| | $R^8$ | $R^9$ |
|---|---|---|
| 29. | 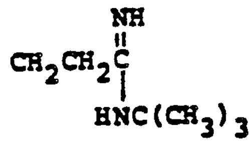 | $CH_3$ |
| 30. | 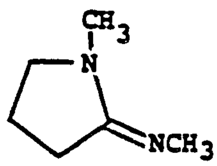 | $CH_3$ |
| 31. | 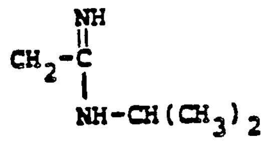 | $CH_3$ |
| 32. | 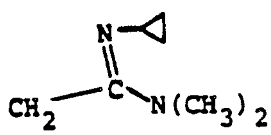 | $CH_3$ |
| 33. | 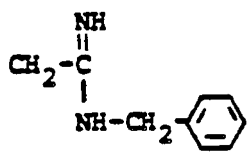 | $CH_3$ |
| 34. | 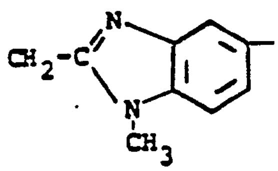 | $CH_3$ |
| 35. | 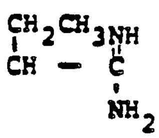 | $CH_3$ |
| 36. | 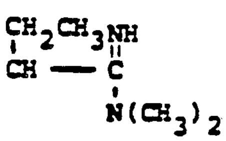 | $CH_3$ |
| 37. | 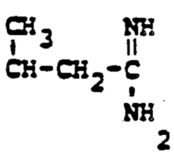 | $CH_3$ |
| 38. | 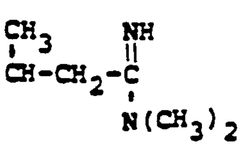 | $CH_3$ |

| | $R^8$ | $R^9$ |
|---|---|---|
| 39. | 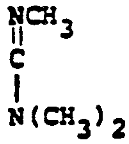 | $CH_3$ |
| 40. | 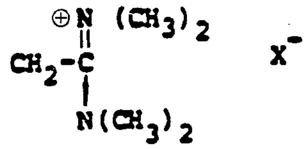 | $CH_3$ |
| 41. | 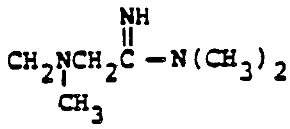 | $CH_3$ |
| 42. | 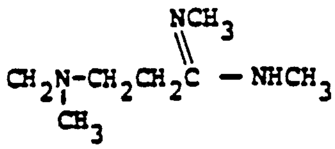 | $CH_3$ |
| 43. | 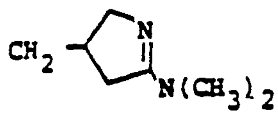 | $CH_3$ |
| 44. | 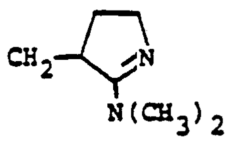 | $CH_3$ |
| 45. | 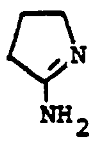 | $CH_3$ |
| 46. | 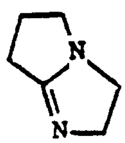 | $CH_3$ |
| 47. | 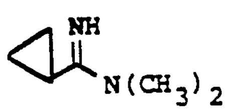 | $CH_3$ |
| 48. | 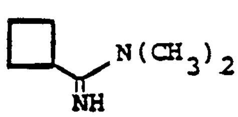 | $CH_3$ |
| 49. | 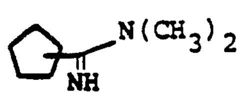 | $CH_3$ |
| 50. | 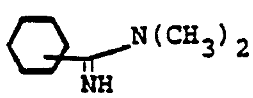 | $CH_3$ |

| | $R^8$ | $R^9$ |
|---|---|---|
| 51. | 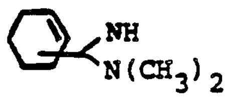 | $CH_3$ |
| 52. | 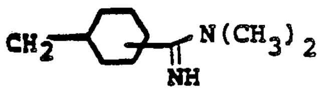 | $CH_3$ |
| 53. | 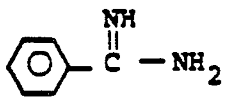 | $CH_3$ |
| 54. | 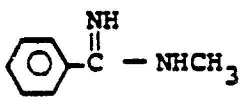 | $CH_3$ |
| 55. | 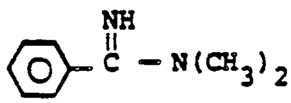 | $CH_3$ |
| 56. | 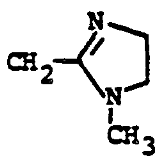 | $CH_3$ |
| 57. | 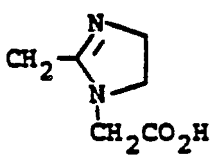 | $CH_3$ |
| 58. | 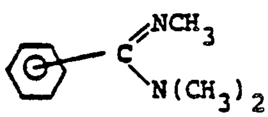 | $CH_3$ |
| 59. | 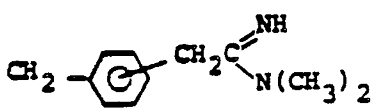 | CH3 |
| 60. | 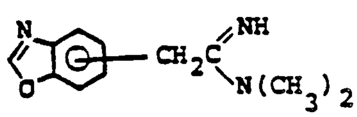 | $CH_3$ |
| 61. | 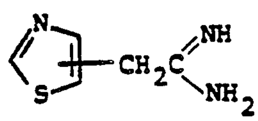 | $CH_3$ |
| 62. | 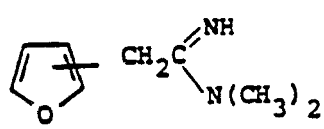 | $CH_3$ |

| | $R^8$ | | $R^9$ |
|---|---|---|---|
| 63. | 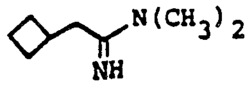 | | $CH_3$ |
| 64. | 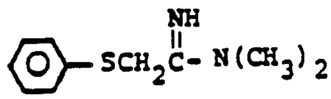 | | $CH_3$ |
| 65. | 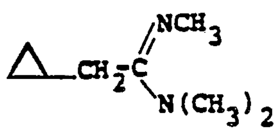 | | $CH_3$ |
| 66. | 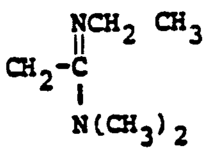 | | $CH_3$ |
| 67. | 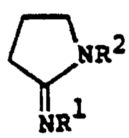 | $R^1$ = H, CH3<br>$R^2$ = H, $CH_3$ | $CH_3$ |
| 68. | 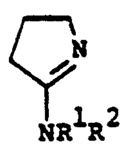 | $R^1$ = H, $CH_3$<br>$R^2$ = H, $CH_3$ | $CH_3$ |
| 69. | 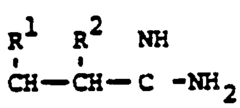 | $R^1$ = $CH_3$<br>$R^2$ = $CH_3$, $NR_2$, OR<br>$R^3$ = H, $CH_3$ | $CH_3$ |
| 70. | 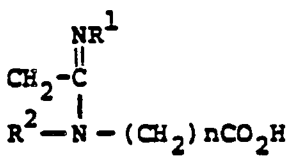 | $R^1$ = H, $CH_3$<br>$R^2$ = H, $CH_3$<br>n = 1 or 2 | $CH_3$ |
| 71. | 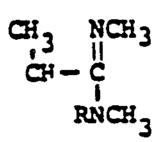 | R = H, $CH_3$ | $CH_3$ |
| 72. | 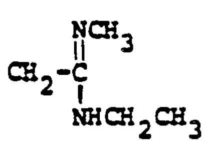 | | $CH_3$ |
| 73. | 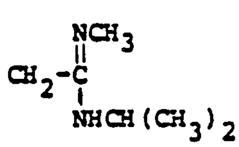 | | $CH_3$ |

| | $R^8$ | $R^9$ |
|---|---|---|
| 74. | 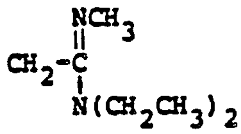 | $CH_3$ |
| 75. | 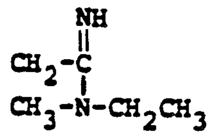 | $CH_3$ |
| 76. | 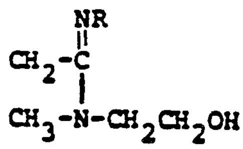 R = $CH_2CH_3$, $CH_3$ | $CH_3$ |
| 77. | 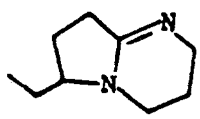 | $CH_3$ |
| 78. | 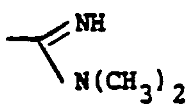 | $CH_3$ |
| 79. | 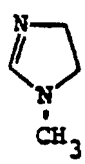 | $CH_3$ |
| 80. | 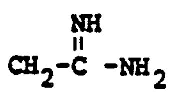 | $CH_3$ |
| 81. | 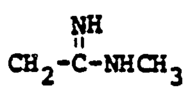 | $CH_3$ |
| 82. | 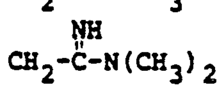 | $CH_3$ |
| 83. | 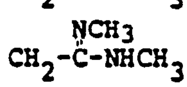 | $CH_3$ |

Compounds 84—166 correspond to the above compounds 1—87 except $R^9$ is ethyl; see Example 1, Step A wherein $R^9$ is ethyl.

Compounds 167—249 correspond to above compounds 1—83 except $R^9$ is phenyl; see Example 1, Step A wherein $R^9$ is phenyl.

Compounds 250—332 correspond to the above compounds 1—83 except $R^9$ is $CH_2F$; see Example 1, Step A wherein $R^9$ is $CH_2F$.

Compounds 333—415 correspond to the above compounds 1—83 except $R^9$ is cyclopropyl; see Example 1, Step A wherein $R^9$ is cyclopropyl.

Compounds 416—498 correspond to the above compounds 1—83 except $R^9$ except $R^9$ is trifluoromethyl; see Example 1, Step A wherein $R^9$ is trifluoromethyl.

Example Section — Part III

EXAMPLE 1

Preparation of 3 (Method I)

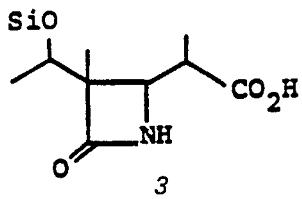

3

Step A:

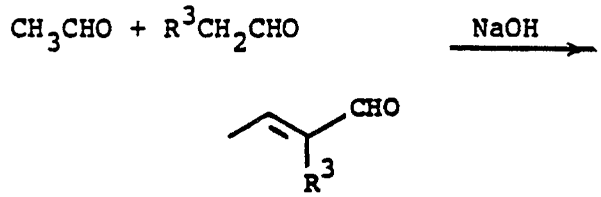


C

The α,β-unsaturated aldehydes (C) are prepared by modified procedures reported by M. B. Green and W. J. Hickinbottom in *J. Chem. Soc.* 3262 (1957); and W. J. Bailey and R. Barclay Jr., *J. Org. Chem., 21,* 328 (1956).

Acetaldehyde (1 eq.) and propionaldehyde ($R=CH_3$) (1 eq.) are placed in a three-necked round-bottom flask which is equipped with a mechanical stirrer, a dry-ice condenser, and a pressure equalized dropping-funnel. To the solution is added dropwise 1 eq. of 1*N* NaOH through the dropping funnel with constant stirring. After completion of the mixing, the mixture is stirred for 10 min, then poured into a beaker containing crushed ice. Extraction of the mixture with ether gives the crude product. The desired product (C) is obtained by fractional distillation through a Widmer column.

Step B:

Preparation of 1'

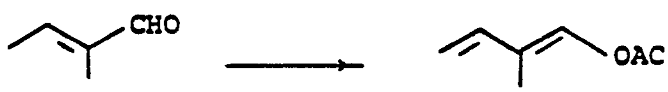

*1'*

Isopropenyl acetate (182 g), cupric acetate (0.40 g), 2-methyl-2-butenal (84 g) and p-toluenesulfonic acid (1.52 g) are placed in a 1.0—1 three-necked flask equipped with a thermometer, a nitrogen inlet tube and a 10-in. Widmer column which is attached with a distillation head. The mixture is heated at 93—110°C until 73 ml of acetone is collected. After cooling to r.t. (22°C) the mixture is filtered from solids. The dark brown filtrate is cooled in an ice-bath and mixed with 3.4 g triethanolamine in 200 ml water. The two layer mixture is distilled quickly at 53 mm (b.p. 54°). The organic layer of the distillate is separated. The aqueous layer is extracted with 200 ml ether. The organic layers are combined and washed with 10% $K_2CO_3$, dried over $Na_2SO_4$, and evaporated *in vacuo*. The residue so obtained is mixed with 2.0 g N-phenyl-β-naphthylamine and distilled under reduced pressure to give *1'* (97 g), b.p. 81—91° (66mm).

Following the procedure of Example 1, the following $R^9$ substituted species are obtained. (Table I).

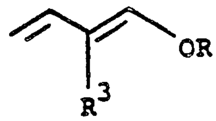

TABLE I

| $R^9$ | R |
|---|---|
| 1. $CH_3$ | $CH_3\overset{O}{\overset{\|}{C}}-$ |
| 2. $CH_3CH_2$ | $CH_3\overset{O}{\overset{\|}{C}}-$ |
| 3. $CH_3CH_2CH_2$ | $CH_3\overset{O}{\overset{\|}{C}}-$ |
| 4. 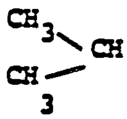 | $CH_3\overset{O}{\overset{\|}{C}}-$ |
| 5. 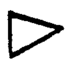 | $CH_3\overset{O}{\overset{\|}{C}}-$ |
| 6. Ph (Ph=phenyl) | $CH_3\overset{O}{\overset{\|}{C}}-$ |
| 7. $PhCH_2$ | $CH_3\overset{O}{\overset{\|}{C}}-$ |
| 8. $CH_2Br$ | $CH_3\overset{O}{\overset{\|}{C}}-$ |

**STEP C**
**Preparation of 2' and 3'**

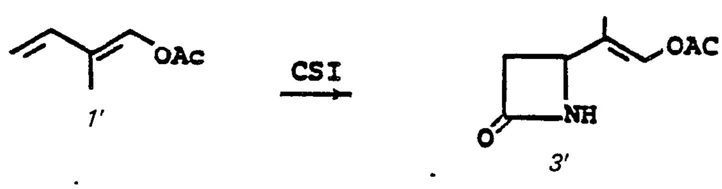


*1'* *3'*

Chlorosulfonylisocyanate (CSI) (6.5 ml) is placed in a three-necked, 100-ml flask equipped with a thermometer, a magnetic stirring bar a nitrogen inlet tube and a 25-ml pressure-equalizing dropping funnel. The CSI is chilled to −50°C and mixed with 12.5 ml ether through the dropping funnel. The etheral solution of CSI is allowed to warm up to −25°C, to the solution is added dropwise 1-acetoxyl-2-methyl-1,3-butadiene (*1'*) (5.9 ml in 12.5 ml ether) in 30 min. The mixture is then stirred for 20 min at −20 ± 30°C. The white precipitate formed initially is redissolved at the end of the reaction.

In a 500-ml round bottom flask, a solution of 10 g sodium sulfite and 25 g potassium hydrogen phosphate in 100 ml water is prepared and is cooled in an ice bath. Ether (100 ml) and crushed ice (100 g) are added and the mixture is vigorously stirred in an ice bath. At the end of 20 minutes reaction time, the reaction mixture which contains *2'* is transferred into the dropping funnel and added dropwise to the hydrolysis mixture in 5 minutes. The hydrolysis is allowed to continue for an additional 30 minutes at 3°C. The organic layer is separated and the aqueous is extracted with 50 ml ether. The organic layers are combined, dried over $Na_2SO_4$ and evaporated to give crystalline product *3'* (2.3 g), m.p. 77—78.5°C; m.s. 169($M^+$); IR 1760 $cm^{-1}$ (β-lactam); NMR (300 MHz, $CDCl_3$): 1.70 (d), 2.16(s), 2.84 (qq), 3.18 (qq), 4.20 (m), 5.82 (Broad s), and 6.26 (s) ppm.

Step D:

Preparation of 4':

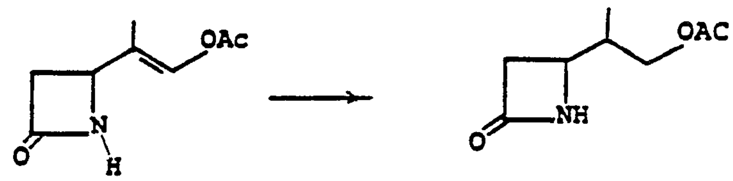

3'    4'

4-(1-methyl-2-acetoxyvinyl)azetidine-2-one (*3'*) (6.5 g) is hydrogenated on a Parr shaker at r.t. under 40 psi hydrogen in the presence of 10% Pd/C (0.6 g) in 200 ml ethylacetate for 2 hr. The mixture is filtered from the catalyst and the filtrate is evaporated *in vacuo* to give the crude product. Purification of the crude product by high pressure liquid chromatography (HPLC, silical gel column, 30% ethylacetate/$CH_2Cl_2$ solvent system) affords white crystalline product *4'* (6.04 g) after evaporation of solvent. The product shows following physical characdteristics: ms 171 ($M^+$); IR(Neat) 1754 $cm^{-1}$; NMR (60 MHz, $CDCl_3$): 0.96 (d), 1.01 (d), 2.06 (d, OAc), 2.75—3.80 (m), 3.99 (d) and 6.80 (broad) ppm.

Step E:

Preparation of 5'

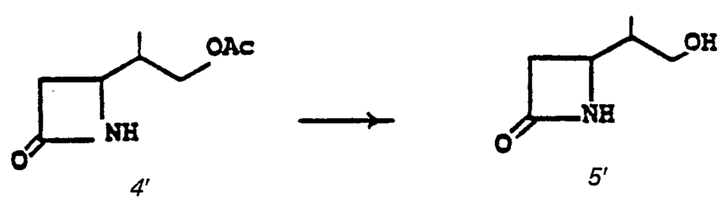

4'    5'

Under $N_2$ at 0°, a solution of 4-(1-methyl-2-acetoxyethyl)-2-azetidinone *4'* (1.2 g) in 10 ml methanol is treated with sodium methoxide (57 mg). After stirring for 1 hr, the solution is neutralized with glacial acetic acid (65 mg). Removal of methanol *in vacuo* gives crude 4-(1-methyl-2-hydroxyethyl)-2-azetidinone *5'* as an oil. The product is purified by chromatography on silica gel eluting with ethyl acetate to give 0.78 g of *5'*: IR (neat): 1740 $cm^{-1}$; NMR ($CDCl_3$): 0.77 (d), 0.96 (d), 1.90 (m), 2.60—3.30 (m), 3.60 (m), 4.19 (s), and 7.33 (s). The product crystallizes as a colorless solid in the refrigerator.

Step F:

Preparation of 6'

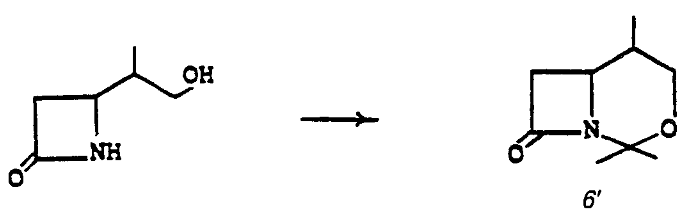

6'

A solution of 4-(1-methyl-2-hydroxyethyl)-2-azetidinone (0.5 g) and 2,2-dimethoxypropane (0.48 g) in 10 ml anhydrous methylene chloride is treated with boron trifluoride (55 mg) at room temperature for 90 min. The mixture is washed with 5 ml saturated $NaHCO_3$. The organic layer is separated, dried over $Na_2SO_4$ and allowed to evaporated *in vacuo* to give crude isomeric mixture of *6'* (0.48 g) as an oil.

Separation of isomers 6'α and 6'β is accomplished by high pressure liquid chromatography (HPLC, silica gel) eluting with 40% ethylacetate/hexanes. Evaporation of the solvents affords 250 mg of 6'β as an oil and 200 mg of 6'α as a white solid.

NMR (300 MHz, $CDCl_3$) of 6'α: 0.81 (d), 1.31 (s), 1.68 (s), 1.62 (m), 2.52 (q), 3.05 (m), 3.42 (t), and 3.66 ppm (q), NMR (300 MHz, $CDCl_3$) of 6'β: 1.10 (d), 1.38 (s), 1.67 (s), 1.90 (m), 2.80 (q), 2.86 (q), 3.62 (q), 3.78 (m) and 3.98 (q) ppm.

Step G:

Preparation of 7'a

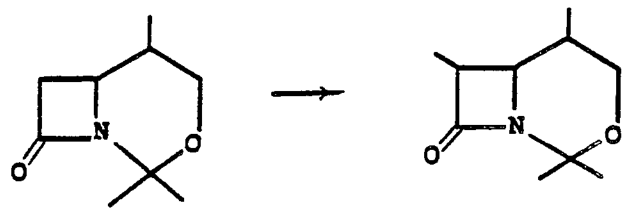

6' 7'a

A solution of diisopropylamine (10.5 mmol) in anhydrous THF (40 ml) is cooled to −78°C and stirred under $N_2$ atmosphere while n-butyllithium in hexane (10.5 mmol) is added slowly by syringe. After 15 minutes, a solution of *6'* (10.0 mmol) in anhydrous THF (12 ml) is added slowly. The mixture is stirred at −78°C for 20 minutes then treated with methyl iodide (30.0 mmol) for 10 minutes. The mixture is quenched with saturated ammonium chloride solution and allowed to warm to room temperature. The reaction mixture is diluted with ethylacetate and washed with water and brine. The organic layer is separated, dried over magnesium sulfate and evaporated *in vacuo* to give crude product *7'a*, which is purified by a silica gel column chromatography.

Step H:

Preparation of 7'

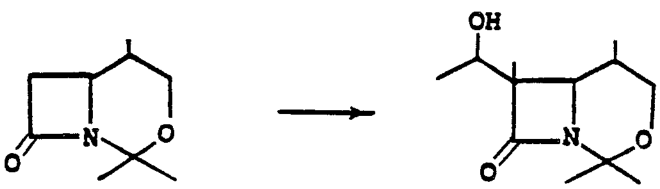

7'a 7'

At −78°C, diisopropylamine (2.2 g) in 20 ml of anhydrous tetrahydrofuran is treated with n-butyllithium (1.6M in n-hexane, 14 ml) for 5 min. To the solution is added 8-oxo-2,2,5,7-tetra-methyl-1-aza-3-oxa-bicyclo[4.2.0]octane *(7'a)* (3.4 g) and the mixture is stirred for 10 min. The resulting lithium enolate is treated with acetaldehyde (1.68 ml). The mixture is stirred for 1 min. then is quenched with 24 ml saturated ammonium chloride at −78°C, then allowed to warm to room temperature (25°C). The mixture is extracted with ethylacetate (2 × 100 ml).

Step I:

Preparation of 8'

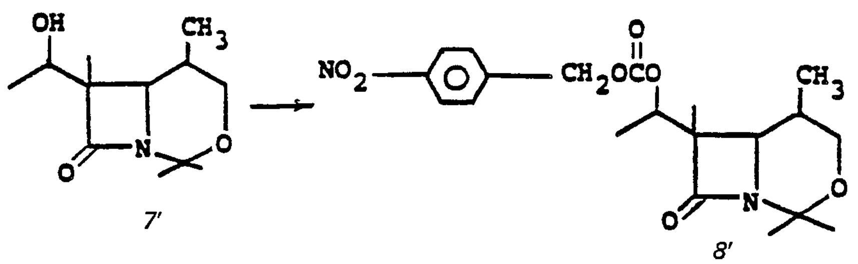

7' 8'

Under anhydrous conditions at 0°C a solution of 7' (2.90 g) in 60 ml methylene chloride is treated with 4-dimethylaminopyridine (3.32 g) and p-nitrobenzylchloroformate (5.88 g). The mixture is allowed to warm to room temperature and stirred for 1 hr. The resulting mixture is washed with 0.1*N* HCl, water, brine and water. The organic layer is separated, dried over $Na_2SO_4$ and allowed to evaporate *in vacuo* to give crude products. The crude product, is purified by HPLC (silica gel) eluting with 40% ethylacetate/cyclohexane to give *8'*.

Step J:

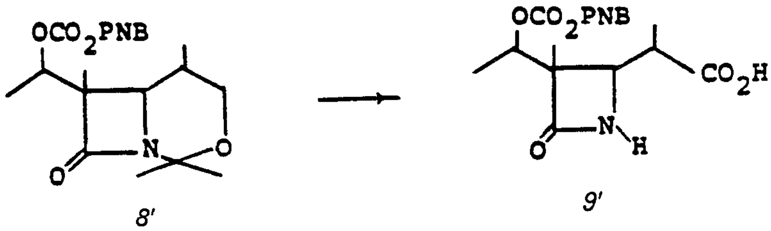

8' 9'

The bicyclic azetidinone *8'* (6.0 g) in 60 ml acetone is treated with *4N* Jones reagent (9.4 ml) at 0°C for 30 min. The reaction is quenched with 1 ml isopropanol at 0°C for 10 min, then mixed with 250 ml ethylacetate and washed with water and brine until blue color disappeared in the organic layer. The organic layer is separated, dried over $MgSO_4$, and evaporated *in vacuo* to give crude product which is purified by a silica gel column (4.4 × 10 cm) eluting with ethylacetate to give 3.1 g of *9'* as a crystalline solid.

Step K:

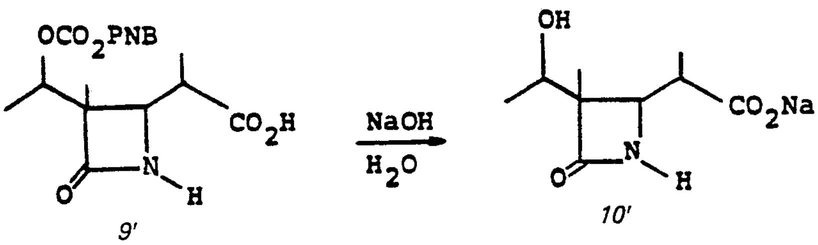


9' 10'

The azetidinone carboxylic acid *9'* (3.0 g) is suspended in 50 ml water. The mixture is stirred and treated with 2.5*N* NaOH and maintained at pH 12.0 at room temperature for 30 min. The resulting homogenous solution is neutralized with 2.5*N* HCl to pH 7.5. After the mixture is extracted with EtOAc, the aqueous layer is concentrated and lyophilized to give product *10'*.

Step L:

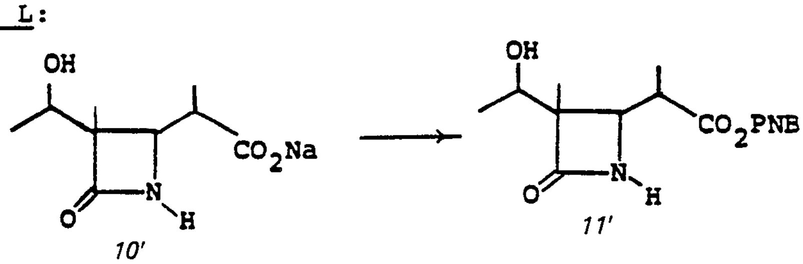

10' 11'

The azetidinone carboxylic acid sodium salt *10'* (2.2 g) and p-nitrobenzylbromide (2.94 g) are stirred at room temperature in DMF (29.3 ml) for 5 hrs. The mixture is diluted with EtOAc and washed with water and brine. The organic layer is separated, dried over $MgSO_4$ and evaporated to give crude product which is purified by TLC plates eluting with 50% EtOAc/cyclohexane to give product *11'*.

Step M:

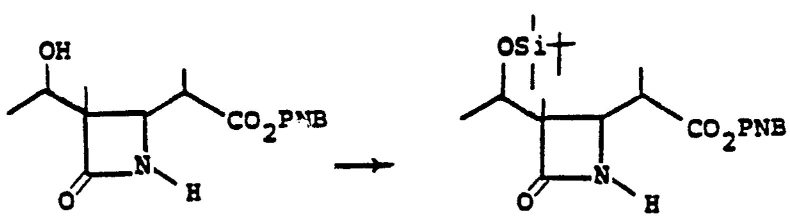

11' 12'

The ester *11'* (1.33 g) is stirred with t-butyldimethylchlorosilane (2.49 g), imidazole (2.2 g) in DMF (16 ml) at room temperature overnight. The mixture is filtered from solids and evaporated *in vacuo* to give crude product *12'* which is redissolved in ethylacetate and washed with water, brine, dried over $Na_2SO_4$, concentrated to 0.5 ml then purified by TLC eluting with 30% ETOAc/cyclohexane to give product *11'*.

Step N:

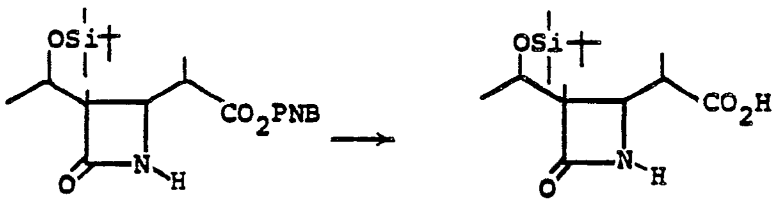

12'    3

The azetidinone ester *12'* (1.60 g) in 30 ml EtOAc is hydrogenated under 50 psi hydrogen in the presence of 0.32 g 10% Pd/C for 30 min. The mixture is filtered from catalyst. The catalyst is washed with MeOH. The methanol and ethylacetate solutions are combined and evaporated *in vacuo* to give white solids. The crude product is re-dissolved in ethylacetate and washed with 0.1*N* HCl. The organic layer is separated, dried over $Na_2SO_4$ and evaporated to give white solid product *3.*

Example 2

Preparation of chiral intermediate *3*:

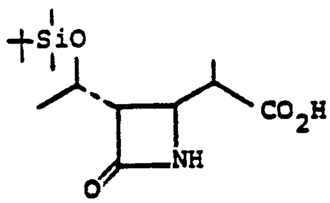

Step A:

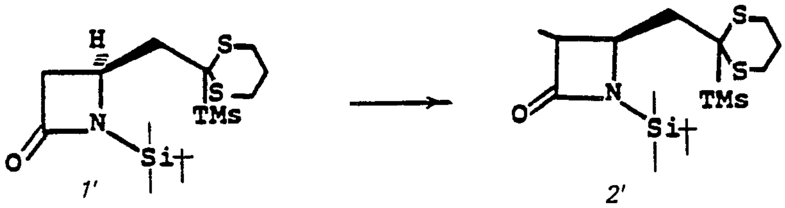

1'    2'

A solution of diisopropylamine (10.5 mmol) in anhydrous THF (40 ml) is cooled to −78°C and stirred under nitrogen atmosphere while n-butyllithium in hexane (10.5 mmol) is added slowly by syringe and stirred for 10 minutes. To the solution is added *1'* (10.0 mmol) in 12 ml THF. The resulting solution is stirred at −78°C for 20 minutes, then treated with excess Me (50 mmol). The mixture is allowed to warm to room temperature then quenched with saturated ammonium chloride. The mixture is extracted with ethyl acetate to give product *2'* which is purified by a silica gel column chromatography.

Step B:

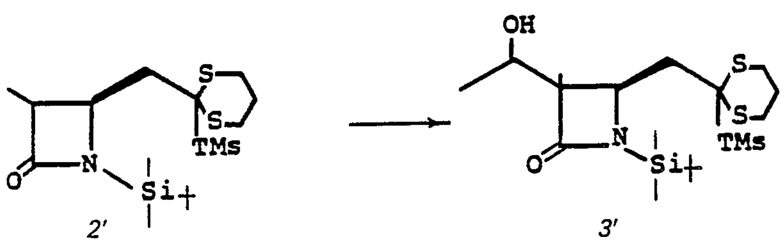

2'    3'

At −78°C, under nitrogen atmosphere, to a lithium diisopropylamide solution (10.5 mmol) in 40 ml THF) is added starting material *2'* (10 mmol). The mixture is stirred at −78°C for 10 minutes, then mixed with acetaldehyde (30 mmol). After 10 minutes reaction at −78°C, the mixture is quenched by the addition of saturated aqueous ammonium chloride solution, and allowed to warm to room temperature. The reaction mixture is extracted with ethyl acetate and washed with 2.5*N* HCl, water and brine. The organic layer is separated, dried over magnesium sulfate and evaporated *in vacuo* to give product *3'*.

Step C:

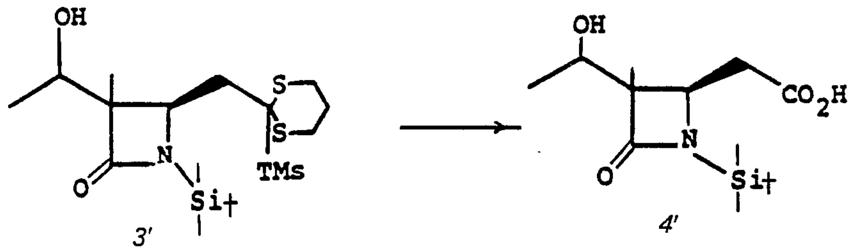

3' 4'

The starting material *3'* (2.3 mmol) in 5% aqueous methanol (12 ml) is mixed with mercuric oxide (3.5 mmol) and mercuric chloride (5.1 mmol). The mixture is heated at reflux for 45 minutes then cooled and filtered from solids. The filtrate is concentrated to 5 ml, then diluted with ethyl acetate and washed with saturated ammonium chloride, water and brine. The organic layer is separated, dried over magnesium sulfate and evaporated *in vacuo* to give crude silyl ketone intermediate which is purified by a silica gel column chromatography. The silyl ketone intermediate (1.0 mmol) in chloroform is treated with m-chloroperbenzoic acid (1.0 mmol) at reflux for 4 hours, then cooled, concentrated *in vacuo*, and the residue chromatographed on silica gel column to give *4'*.

Step D:

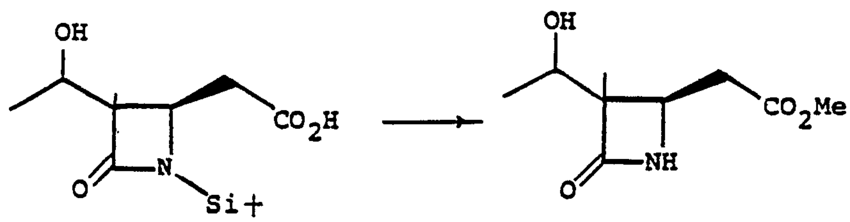

4' 5'

The starting material *4'* (1.0 mmol) in $CH_3CN$ (10 ml) is treated with 1,1'-carbonyldiimidazole (1.1 mmol) at room temperature for 30 minutes, then mixed with 1 ml of MeOH. After 1 hour reaction time, the mixture is evaporated *in vacuo* and the residue is redissolved in 5 ml MeOH. To the methanol solution is added 0.2 ml of 6*N* HCl and the mixture stirred at r.t. for 1 hour then evaporated *in vacuo*. The residue is extracted with ethyl acetate to give product *5'*.

Step E:

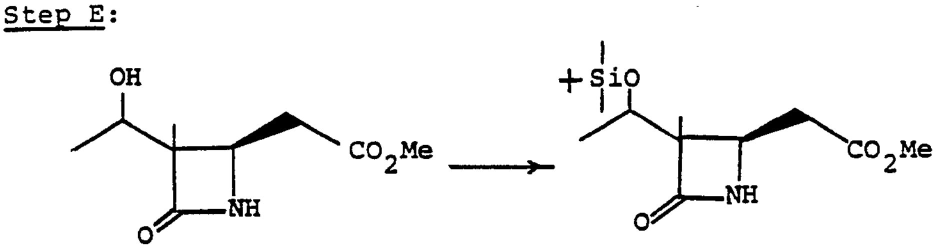

5' 6'

The starting material *5'* (5 mmol) in DMF (25 ml) is treated with t-butyldimethyl-chlorosilane (10 mmol) and imidazole (20 mmol) at r.t. for 5 hours. The mixture is evaporated *in vacuo* to give an oily residue which is re-dissolved in ethyl acetate and washed with 0.1*N* HCl, water and brine. The organic layer is separated, dried over magnesium sulfate and evaporated *in vacuo* to give crude product *6'* which is purified by a silica gel column chromatography.

Step F:

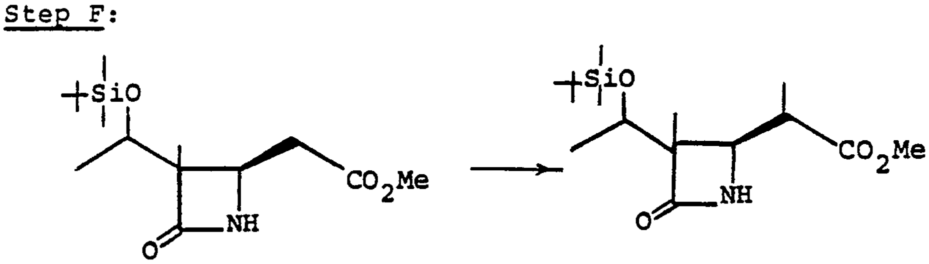

6' 7'

At −78°C, under nitrogen atmosphere, the starting material 6′ (1 mmol) in 5 ml THF is treated with lithium diisopropylamide (2.0 mmol) for 20 minutes. To the solution is added iodomethane (20 mmol). The mixture is allowed to warm to room temperature then is hydrolized with saturated ammonium chloride (1 ml) and diluted with ethyl acetate. The organic layer is separated, dried over magnesium sulfate and chromatographed on silica gel column to give product *7′*.

Step G:

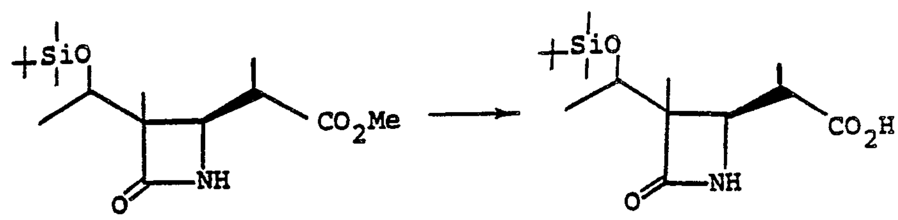

*8′* *9′*

The methyl ester *8′* (10 mmol) in 20 ml methanol is treated with 0.25*N* NaOH (10 mmol) at room temperature for 5 hours. The mixture is extracted with ether (50 ml), acidified with 1*N* HCl and then extracted with ethyl acetate. The ethyl acetate layer is dried over magnesium sulfate and evaporated *in vacuo* to give product *9′*.

Example 3

Preparation of 1a:

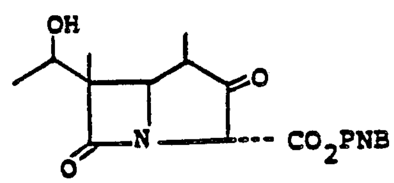

*1a*

Step A:

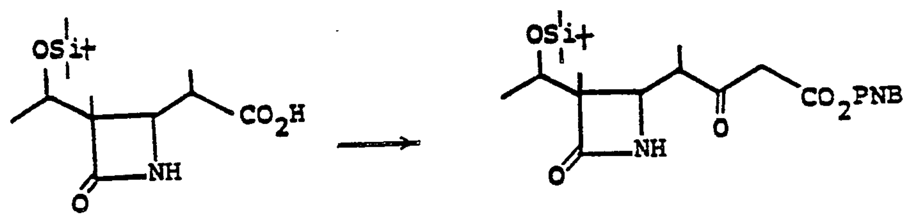

*1′* *2′*

PNB = p-nitrobenzyl

The azetidinone carboxylic acid *1′* (500 mg) suspended in acetonitrile (14.8 ml) is treated with 1,1′-carbonyldiimidazole (229.6 mg) and stirred at room temperature for 30 min. The mixture is then treated with p-nitrobenzylmalonate magnesium salt (1.18 g) and heated at 60°C for 3 hrs. The mixture is diluted with $CH_2Cl_2$ and washed with water, brine, and dried over $Na_2SO_4$. TLC purification (75% EtOAc/cyclohexane) of the crude product provides 0.50 g of product *2′*.

Step B:

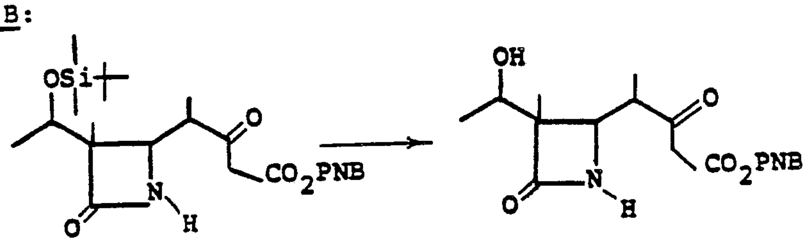

*2′* *3′*

The β-keto ester *2'* (667 mg) in methanol (16 ml) is stirred wioth 2 ml 6*N* HCl at room temperature for 2 hrs. The mixture is diluted with ethylacetate, washed with 0.1*M* sodium phosphate buffer, brine, dried over $Na_2SO_4$ then evaporated to give 0.6 g of crude product which is purified by TLC eluting with 100% ethylacetate to give product *3'*.

Step C:

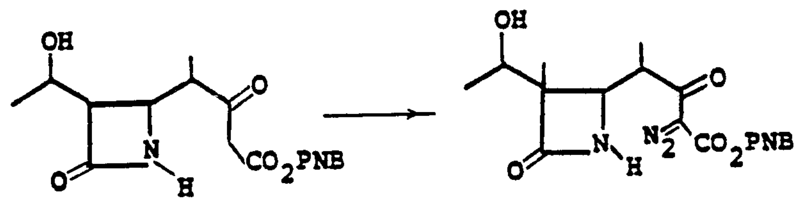

*3'* *4'*

The β-keto ester *3'* (270 mg) in 3.2 ml acetonitrile, p-toluene-sulfonylazide (1.11 g, 3.33 meq/g), and triethylamine (0.31 ml) are placed in a 25 ml round-bottomed flask. The mixture is stirred under nitrogen atmosphere at room temperature for 1 hr., then diluted with ethylacetate and washed with water, brine, and dried over $MgSO_4$. The crude product is purified by TLC eluting with 50% EtOAC/cyclohexane to give *4'*.

Step D:

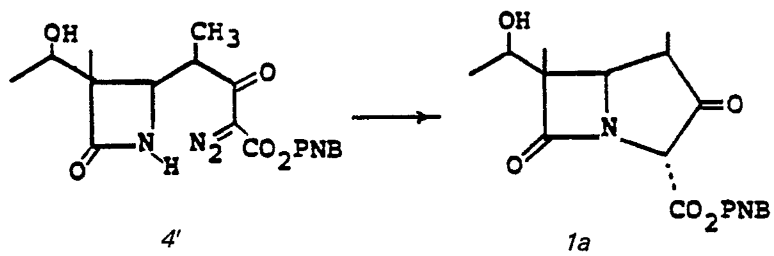

*4'* *1a*

The diazo β-keto ester *4'* (38.6 mg) in toluene (1 ml) is heated at 80°C in the presence of rhodium acetate (1.7 mg) for 10 min. The mixture is diluted with ethylacetate (10 ml) and washed with water and brine. The organic layer is separated, dried over $MgSO_4$ and evaporated *in vacuo* to give bicyclic keto ester *1a*.

Example 4

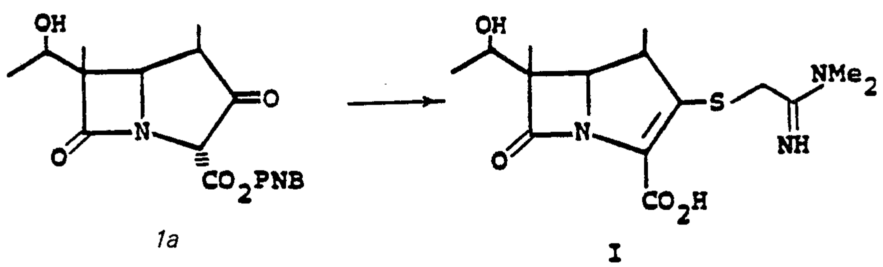

*1a* I

The bicyclic keto ester *1a* (33.3 mg) in acetonitrile (0.47 ml) under $N_2$ atmosphere is treated with diphenyl chlorophosphate (20.97 μl) and diisopropylethylamine (19.22 μl) at 0°C and stirred for 30 min. To the mixture is added DMSO (0.20 ml) solution of N,N-dimethylmercaptoacetamidine hydrochloride (18.68 mg) and diisopropylethylamine (24.3 μl) and stirred for 1 min. at 0°C. The mixture is then mixed with 10 ml ether and centrifuged to separate the oil product which is subsequently re-dissolved in 3.72 ml THF and 2.80 ml 0.1*M* 1pH 7.0 sodium phosphate buffer. The solution is hydrogenated under 50 psi hydrogen in the presence of 50.0 mg of 10% Pd/C at room temperature for 30 min. Additional 50 mg of 10% Pd/C is added and the mixture is further hydrogenated for 30 minutes then filtered from catalysts. The filtrate is extracted with ether, concentrated to 4 ml then chromatographed by a Dowex-50X4 ($Na^+$Cycle) column (2.2 × 6 cm) which is eluted with DI water to give product I.

Example 5

Preparation of chiral intermediates 3.

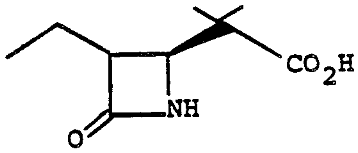

3

Step A:

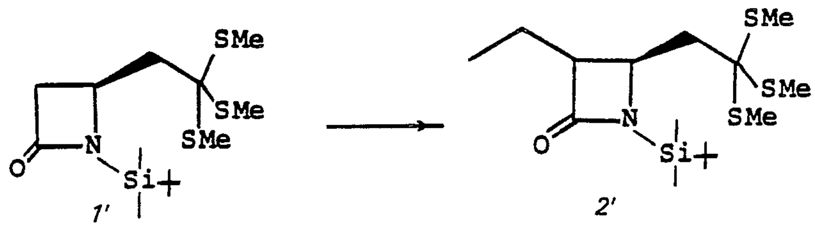

1'  2'

The starting material *1'* (5 mmol) is treated with lithium diisopropylamide (5.1 mmol) in THF (20 ml) at −78°C for 10 minutes. To the solution is added ethyl iodide (1 ml). The mixture is stirred for 30 minutes at −78°C then allowed to warm to room temperature. After quenching with saturated ammonium chloride, the mixture is extracted with ethyl acetate which is then dried over magnesium sulfate and purified by silica gel chromatography to give *2'*.

Step B:

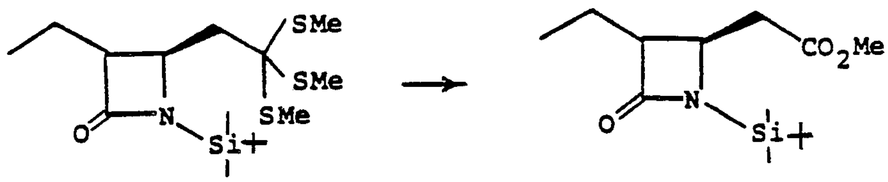

2'  3'

The starting material *2'* (1.0 mmol) in 20 ml methanol is treated with mercuric chloride (3.0 mmol) at 0°C for 3 minutes, then quenched with sodium bicarbonate (8.0 mmol). The mixture is filtered from solids and the filtrate is evaporated *in vacuo* and chromatographed on a silica gel column to give *3'*.

Step C:

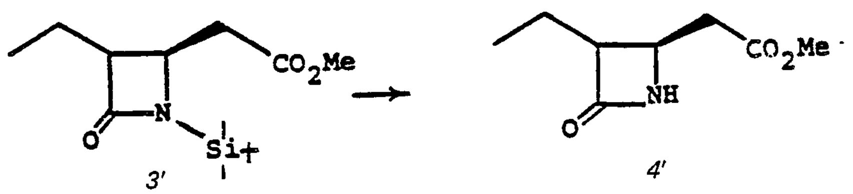

3'  4'

The starting material *3'* (2.0 mmol) in 5 ml methanol is treated with 6.0 *N* HCl (0.2 ml) at room temperature for 30 minutes, then evaporated *in vacuo* and chromatographed by a silica gel column to give *4'*.

Step D:

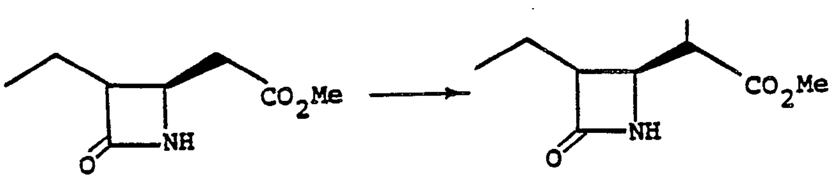

4'  5'

The methyl ester *4'* (1.0 mmol) is treated with lithium diisopropylamide (2.1 mmol) in THF (5 ml) at −78°C for 10 minutes, then mixed with excess iodomethane (1.0 ml). The mixture is allowed to warm to

room temperature, hydrolyzed with saturated ammonium chloride, then extracted with ethyl acetate. The organic layer is separated, dried over magnesium sulfate, and evaporated *in vacuo* to give *5'*.

Step E:

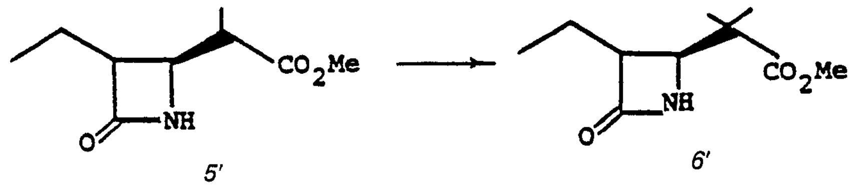

*5'* *6'*

Repeating reaction of step D, using *5'* as starting material, the desired product *6'* is obtained.

Step F:

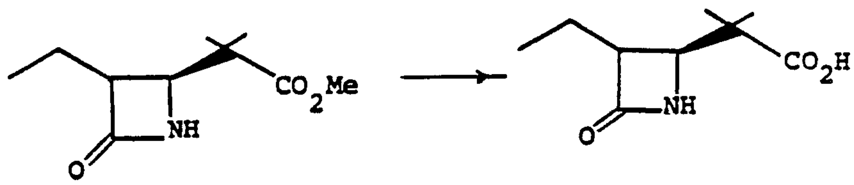

*6'* *3*

The methyl ester *6'* (1 mmol) in 2 ml methanol is treated with 0.25*N* NaOH (1 mmol) at room temperature for 5 hours. The mixture is extracted with ether, acidified with 1*N* HCl and extracted with ethyl acetate. The ethyl acetate layer is dried over magnesium sulfate and evaporated *in vacuo* to give product *3*.

Example 6

Preparation of 1a:

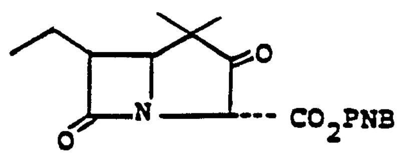

*1a*

Step A:

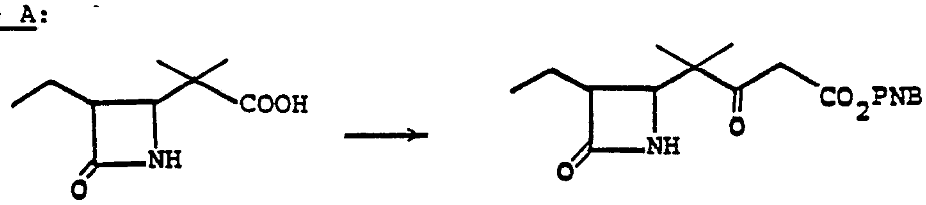

*1'* *2'*

PNB = p-nitrobenzyl

The azetidinone carboxylic acid *1'* (500 mg) suspended in acetonitrile (14.8 ml) is treated with 1,1'-carbonyldiimidazole (229.6 mg) and stirred at room temperature for 30 min. The mixture is then treated with p-nitrobenzylmalonate magnesium salt (1.18 g) and heated at 60°C for 3 hrs. The mixture is diluted with $CH_2Cl_2$ and washed with water, brine, and dried over $Na_2SO_4$. TLC purification (75% EtOAc/cyclohexane) of the crude product provides product *2'*.

Step B:

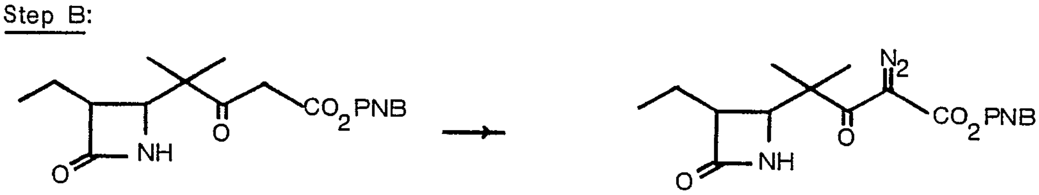

*2'* *3'*

The β-keto ester *2'* (270 mg) in 3.2 ml acetonitrile p-toluene-sulfonylazide (1.11 g, 3.33 meq/g), triethylamine (0.31 ml) are placed in a 25 ml round-bottomed flask. The mixture is stirred under nitrogen atmosphere at room temperature for 1 hr. The mixture is diluted with ethylacetate and washed with water, brine, and dried over $MgSO_4$. The crude product is purified by TLC eluting with 50% EtOAc/cyclohexane to give *3'*.

Step C:

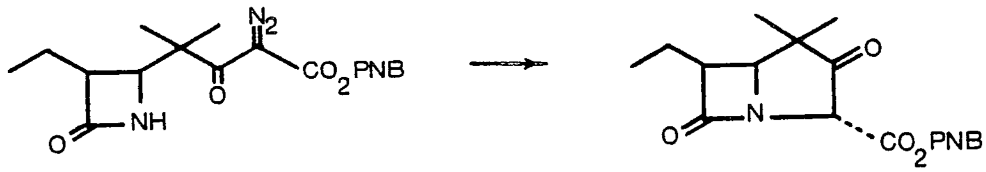

*3'* *1a*

The diazo β-keto ester *3'* (38.6 mg) in toluene (1 ml) is heated at 80°C in the presence of rhodium acetate (1.7 mg) for 10 min. The mixture is diluted with ethylacetate (10 ml) and washed with water and brine. The organic layer is separated, dried over $MgSO_4$ and evaporated *in vacuo* to give bicyclic keto ester *1a*.

Example 7

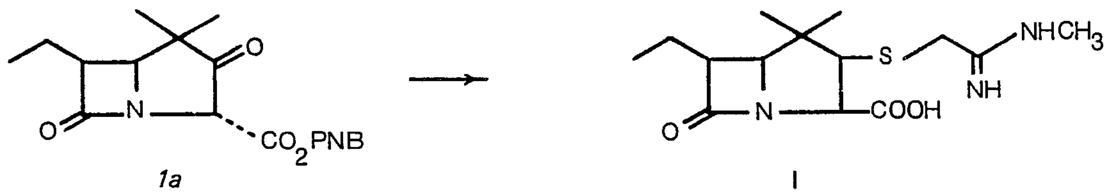

*1a* I

The bicyclic keto ester *1a* (33.3 mg) in acetonitrile (0.47 ml) under $N_2$ atmosphere is treated with diphenyl chlorphosphate (20.97 μl) at 0°C and stirred for 30 min. To the mixture is added DMSO (0.20 ml) solution of N-methylmercaptoacetamidine hydrochloride (18.68 mg) and diisopropylethylamine (24.3 μl) and stirred for 1 min. at 0°C. The mixture is then mixed with 10 ml ether and centrifuged to separate the oil product which is subsequently re-dissolved in 3.72 ml THF and 2.80 ml 0.1*M* pH 7.0 sodium phosphate buffer. The solution is hydrogenated under 50 psi hydrogen in the presence of 50.0 mg of 10% Pd/C at room temperature for 30 min. Additional 50 mg of 10% Pd/C is added and the mixture is further hydrogenated for 30 minutes then filtered from catalysts. The filtrate is extracted with ether, concentrated to 4 ml then chromatographed by a Dowex-50X4 ($Na^+$cycle) column (2.2 × 6 cm) which is eluted with DI water to give product I. Lyopholization of the aqueous solution gives product I.

Example 8

Following the procedure of Example 2, Steps A to G, and Example 5, Steps A to F, the following substituted azetidinone carboxylic acids are obtained when an equivalent amount of the indicated alkylating agents are substituted for the alkylating agents of Example 2, Steps A and F, and Example 5, Steps A, D and E.

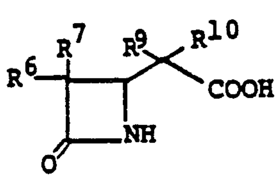
.

A

| Compound | $R^6$ | $R^7$ | $R^9$ | $R^{10}$ |
|---|---|---|---|---|
| 1 | H | $CH_2OH$ | $CH_3$ | H |
| 2 | H | $CH_2CF_3$ | $CH_3$ | H |

| Compound | $R^6$ | $R^7$ | $R^9$ | $R^{10}$ |
|---|---|---|---|---|
| 3 | H | 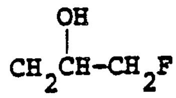 | $CH_3$ | H |
| 4 | H | 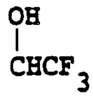 | $CH_3$ | H |
| 5 | H | 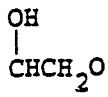 | $CH_3$ | H |
| 6 | H | 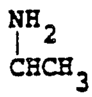 | $CH_3$ | H |
| 7 | H | 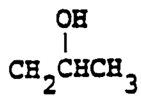 | $CH_3$ | H |
| 8 | H | $CH_2CH_2OH$ | $CH_3$ | H |
| 9 | H | 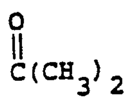 | $CH_3$ | H |
| 10 | H | 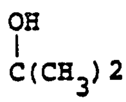 | $CH_3$ | H |
| 11 | H | $CH_2Ø$ | $CH_3$ | H |
| 12 | H | 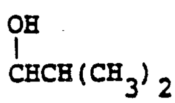 | $CH_3$ | H |
| 13 | H | 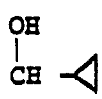 | $CH_3$ | H |
| 14 | H | 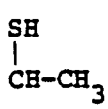 | $CH_3$ | H |

| Compound | $R^6$ | $R^7$ | $R^9$ | $R^{10}$ |
|---|---|---|---|---|
| 15 | H | 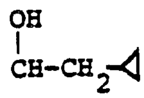 | $CH_3$ | H |
| 16 | H | 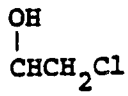 | $CH_3$ | H |
| 17 | H | 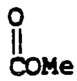 | $CH_3$ | H |
| 18 | H | 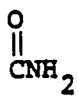 | $CH_3$ | H |
| 19 | H | 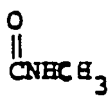 | $CH_3$ | H |
| 20 | H | $CH_2CH_2NH_2$ | $CH_3$ | H |
| 21 | H | 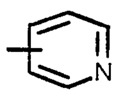 | $CH_3$ | H |
| 22 | H | $CH_2OH$ | $CH_3$ | $CH_3$ |
| 23 | H | $CH_2CF_3$ | $CH_3$ | $CH_3$ |
| 24 | H | 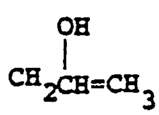 | $CH_3$ | $CH_3$ |
| 25 | H | 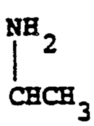 | $CH_3$ | $CH_3$ |

| Compound | $R^6$ | $R^7$ | $R^9$ | $R^{10}$ |
|---|---|---|---|---|
| 26 | H | 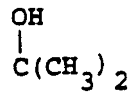 | $CH_3$ | $CH_3$ |
| 27 | H | $CH_2CH_2OH$ | $CH_3$ | $CH_3$ |
| 28 | H | 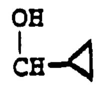 | $CH_3$ | $CH_3$ |
| 29 | H | 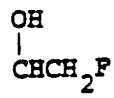 | $CH_3$ | $CH_3$ |
| 30 | H | 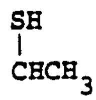 | $CH_3$ | $CH_3$ |
| 31 | H | 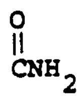 | $CH_3$ | $CH_3$ |
| 32 | H | 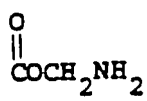 | $CH_3$ | $CH_3$ |
| 33 | H | 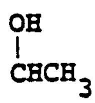 | $C_2H_5$ | H |
| 34 | H | 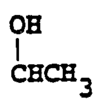 | $C_2H_5$ | $C_2H_5$ |
| 35 | H | 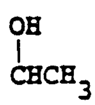 | $C_3H_7$ | H |

| Compound | $R^6$ | $R^7$ | $R^9$ | $R^{10}$ |
|---|---|---|---|---|
| 36 | H | $\overset{OH}{\overset{\vert}{C}}HCH_3$ | 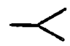 | H |
| 37 | H | $\overset{OH}{\overset{\vert}{C}}HCH_3$ | 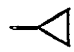 | H |
| 38 | $CH_3$ | $\overset{OH}{\overset{\vert}{C}}HCH_3$ | $CH_2CH$ | H |
| 39 | H | $CH_2OH$ | $CF_3$ | H |
| 40 | H | $CH_2OH$ | $CH_2\phi$ | H |
| 41 | H | $CH_2CH_2OH$ | 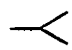 | $CH_3$ |
| 42 | $CH_3$ | $CH_2OH$ | $CH_3$ | $CH_3$ |
| 43 | $CH_3$ | $CH_3$ | $CH_3$ | $CH_3$ |
| 44 | $CH_3$ | $OCH_3$ | $CH_3$ | H |
| 45 | $CH_3$ | Cl | $CH_3$ | $CH_3$ |
| 46 | H | $\overset{OH}{\overset{\vert}{C}}HCH_3$ | $C_2F_5$ | H |
| 47 | $CH_3$ | $CH_2OH$ | $CH_3$ | $C_2H_5$ |
| 48 | 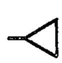 | H | $CH_3$ | $CH_2$-$\phi$ |

| Compound | $R^6$ | $R^7$ | $R^9 + R^{10}$ |
|---|---|---|---|
| 49 | H | OH<br>\|<br>$CHCH_3$ | 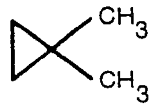 |
| 50 | H | OH<br>\|<br>$CHCH_3$ | 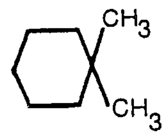 |
| 51 | H | OH<br>\|<br>$CHCH_3$ | 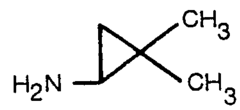 |
| 52 | H | OH<br>\|<br>$CHCH_3$ | $CH_2=$ |

| Compound | $R^6 + R^7$ | $R^9$ | $R^{10}$ |
|---|---|---|---|
| 53 | $CH_2=$ | $CH_3$ | H |
| 54 | HOCH= | $CH_3$ | $CH_3$ |
| 55 | 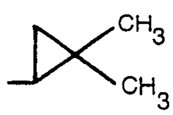 | $CH_3$ | H |
| 56 | 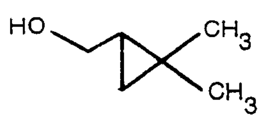 | $C_2H_5$ | $CH_3$ |

| Compound | $R^6 + R^7$ | $R^9 + R^{10}$ |
|---|---|---|
| 57 | $CH_2=$ | 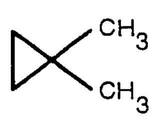 |
| 58 | HOCH= | 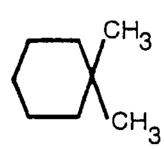 |
| 59 | 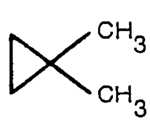 | $CH_2=$ |
| 60 | $HOCH_2CH=$ | 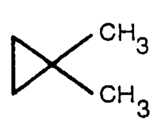 |

Example 9

Following the procedure of Example 3, Steps A to D and Example 4, the following species of present invention are obtained when azetidinones *A* of Example 8, are substituted in equivalent amounts, respectively, for the azetidinone of Example 3, Step *A*.

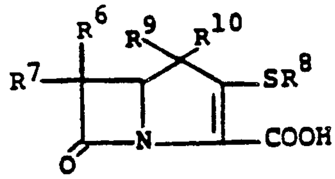

I

| Compound | $R^8$ | $R^6$ | $R^7$ | $R^9$ | $R^{10}$ |
|---|---|---|---|---|---|
| 1 | 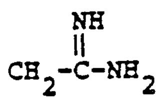 | 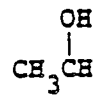 | $CH_3$ | $CH_3$ | H |
| 2 | 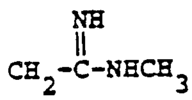 | 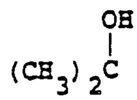 | H | $CH_3$ | $CH_3$ |
| 3 | 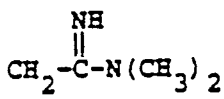 | $CH_3CH_2$ | H | $CH_3$ | H |
| 4 | 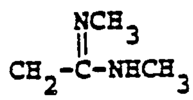 | $OHCH_2$ | H | $CH_3$ | H |
| 5 | 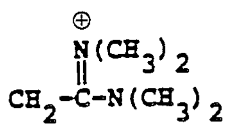 | $CH_3CH_2$ | H | $CH_3$ | H |
| 6 | 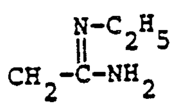 | $FCH_2CH_2$ | H | $C_2H_5$ | H |
| 7 | 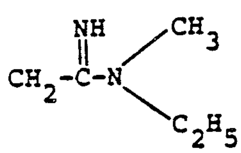 | $CH_3CH_2$ | $CH_3$ | $CH_3$ | $CH_3$ |
| 8 | 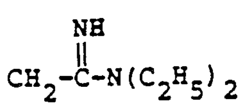 | $HOCH_2CH_2$ | $CH_3$ | 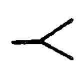 | H |

| Compound | $R^8$ | $R^6$ | $R^7$ | $R^9$ | $R^{10}$ |
|---|---|---|---|---|---|
| 9 | 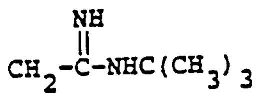 | $HOCH_2CH_2$ | $CH_3$ | 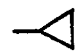 | H |
| 10 | 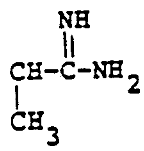 | 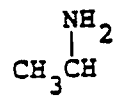 | H | $CH_3$ | H |
| 11 | 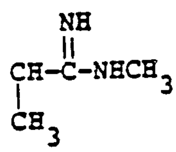 | 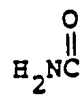 | H | $CH_3$ | H |
| 12 | 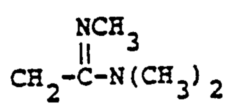 | 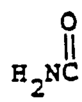 | H | $CH_3$ | $CH_3$ |
| 13 | 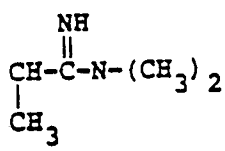 | 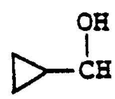 | H | $CH_3$ | H |
| 14 | 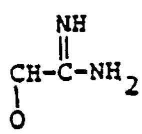 | 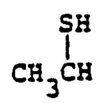 | $CH_3$ | $C_2H_5$ | $CH_3$ |
| 15 | 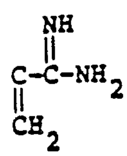 | $HOCH_2$ | $CH_3$ | $C_3H_7$ | H |
| 16 | 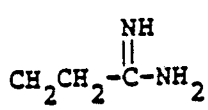 | H | H | $CH_3$ | H |
| 17 | 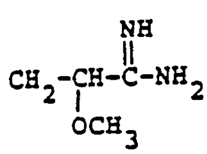 | $CH_2O$ | H | $CH_3$ | $CH_3$ |
| 18 | 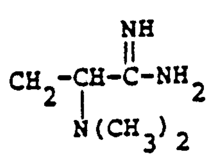 | $CO_2Me$ | H | $CH_3$ | $C_3H_7$ |

| Compound | $R^8$ | $R^6$ | $R^7$ | $R^9$ | $R^{10}$ |
|---|---|---|---|---|---|
| 19 | 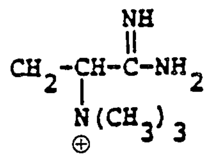 | $CF_3$ | $CH_3$ | $CH_2OH$ | H |
| 20 | 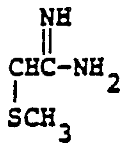 | $HOCH_2$ | $CH_3$ | 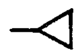 | $C_2H_5$ |
| 21 | 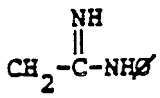 | 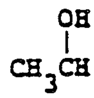 | H | $R^9$ + $R^{10}$: 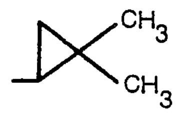 | |
| 22 | 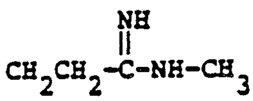 | 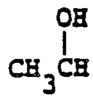 | H | $R^9$ + $R^{10}$: $CH_2$= | |
| 23 | 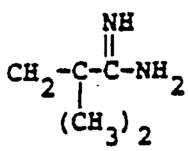 | $CH_2OH$ | $CH_3$ | $R^9$ + $R^{10}$: $HOCH_2$= | |
| 24 | 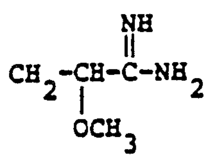 | 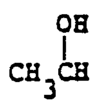 | H | $R^9$ + $R^{10}$: 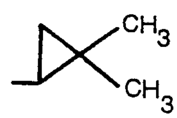 | |

| Compound | $R^8$ | $R^6$ | $R^7$ | $R^9$ | $R^{10}$ |
|---|---|---|---|---|---|
| 25 | $CH_2CH_2\overset{NCH_3}{\overset{\|}{C}}-NH_2$ | H | H | $CH_3$ | $CH_3$ |
| 26 | $CH_2CH_2-S-CH_2\overset{NH}{\overset{\|}{C}}-N(CH_3)_2$ | $R^6 + R^7$: 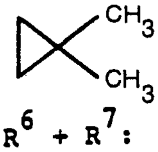 | | $CH_3$ | H |
| 27 | $CH_2CH_2-O-CH_2CH_2-\overset{NH}{\overset{\|}{C}}-NH_2$ | $R^6 + R^7$: 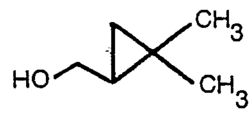 | | $CH_3$ | $CH_3$ |
| 28 | $\overset{CH_3}{\overset{\|}{C}}HCH_2-S-\overset{NCH_3}{\overset{\|}{C}}-N(CH_3)_2$ | $R^6 + R^7$: 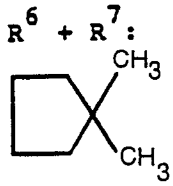 | | $CH_3$ | $C_2H_5$ |
| 29 | $CH{=}CH-\overset{NCH_3}{\overset{\|}{C}}-N(CH_3)_2$ | $R^6 + R^7$: $CH_2=$ | | $C_2H_5$ | $CH_3$ |
| 30 | $CH_2CH_2-\overset{NH}{\overset{\|}{C}}-N(CH_3)_2$ | $R^6 + R^7$: $HOCH_2CH=$ | | $CH_3$ | H |
| 31 | 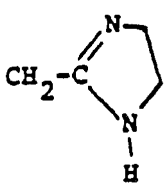 | $R^6 + R^7$: $HOCH_2CH=$ | | $R^9 + R^{10}$: 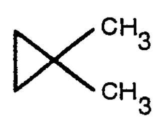 | |
| 32 | 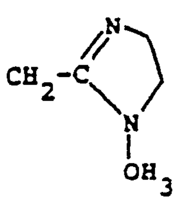 | $R^6 + R^7$: 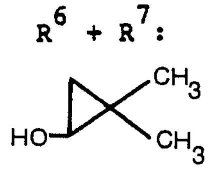 | | $R^9 + R^{10}$: $CH_2=$ | |

| Compound | $R^8$ | $R^6$ | $R^7$ | $R^9$ | $R^{10}$ |
|---|---|---|---|---|---|
| 33 | 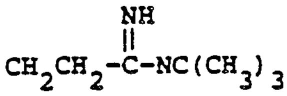 | 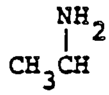 | $CH_3$ | $CH_3$ | $CH_3$ |
| 34 | 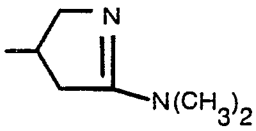 | 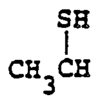 | H | $C_2H_5$ | H |
| 35 | 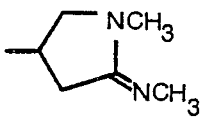 | 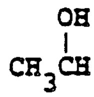 | CH | 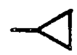 | H |
| 36 | 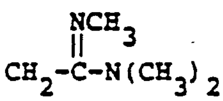 | 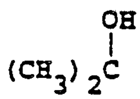 | H | $CH_3$ | H |
| 37 | 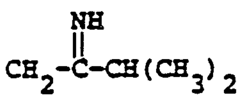 | 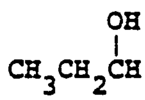 | H | $CH_3$ | $CH_3$ |
| 38 | 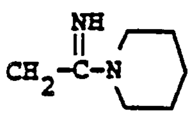 | $H_2NCH_2CH_2$ | H | $CH_3$ | H |
| 39 | 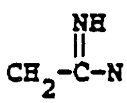 | 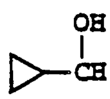 | H | $CH_3$ | H |
| 40 | 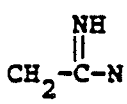 | 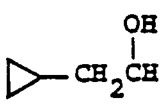 | H | $CH_3$ | $CH_3$ |
| 41 | 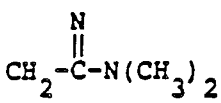 | 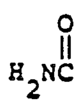 | $CH_3$ | $C_2H_5$ | H |
| 42 | 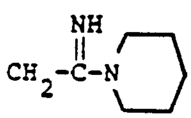 | 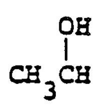 | $OCH_3$ | $C_3H_7$ | $CH_3$ |
| 43 | 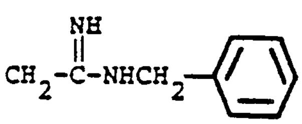 | 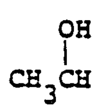 | $OCH_3$ | 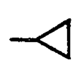 | $CH_3$ |

| Compound | $R^8$ | $R^6$ | $R^7$ | $R^9$ | $R^{10}$ |
|---|---|---|---|---|---|
| 44 | 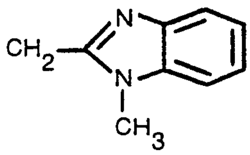 | OH<br>\|<br>$HOCH_2CH_2CH$ | H | $CH_2\phi$<br>$\phi$ = phenyl | H |
| 45 | $C_2H_5$ NH<br>\ ‖<br>$CH_2-C-NH_2$ | 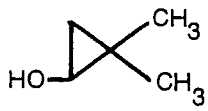 | H | $CH_3$ | $CH_3$ |
| 46 | $C_2H_5$ NH<br>\ ‖<br>$CHCH_2-C-NH_2$ | $CH_3CH_2$ | H | $CH_3$ | H |
| 47 | $CH_3$ NH<br>\| ‖<br>$CHCH_2-C-N(CH_3)_2$ | $CH_3CH_2$ | H | $CH_3$ | $CH_3$ |
| 48 | $NCH_3$<br>‖<br>$C-N(CH_3)_2$ | OH<br>\|<br>$(CH_3)_2C$ | H | $CH_3$ | H |
| 49 | ⊕<br>$N(CH_3)_2$<br>‖<br>$CH_2-C-N(CH_3)_2$ | $CH_2OH$ | H | $CH_3$ | $CH_3$ |
| 50 | NH<br>‖<br>$CH_2-N-CH_2C-N(CH_3)_2$<br>\|<br>$CH_3$ | $CH_2F$ | $CH_3$ | $CH_3$ | H |
| 51 | 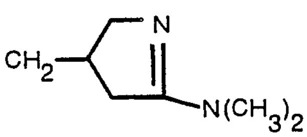 | $CH_3CH_2$ | H | $C_2H_5$ | H |
| 52 | 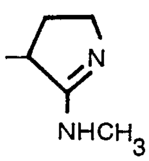 | $CH_3CH_2$ | H | $CH_2\phi$ | H |
| 53 | 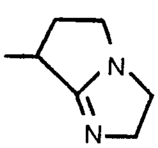 | OH<br>\|<br>$(CH_3)_2C$ | H | $CH_3$ | H |

| Compound | $R^8$ | $R^6$ | $R^7$ | $R^9$ | $R^{10}$ |
|---|---|---|---|---|---|
| 54 | 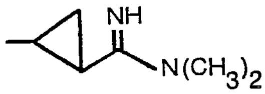 | OH<br>$OCH_2CH$ | H | $CH_3$ | $CH_3$ |
| 55 | 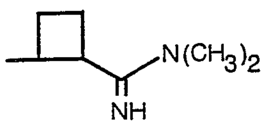 | SH<br>$(CH_3)_2C$ | H | $CH_3$ | H |
| 56 | 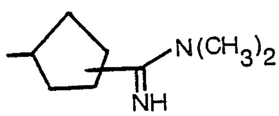 | $NH_2$<br>$(CH_3)_2C$ | H | $C_2H_5$ | H |
| 57 | 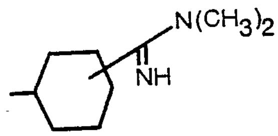 | $NH_2$<br>$(CH_3)_2C$ | H | $CH_3$ | $CH_3$ |
| 58 | 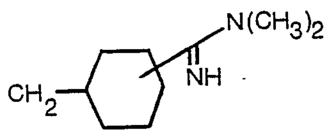 | $NH_2$<br>$(CH_3)_2C$ | H | $C_3H_7$ | H |
| 59 | 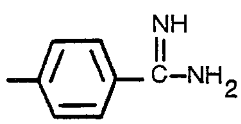 | $NH_2$<br>$(CH_3)_2C$ | H | 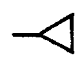 | H |
| 60 | 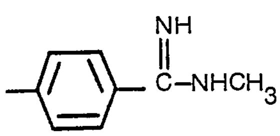 | $CH_3CH_2CH_2$ | H | $CH_3$ | H |
| 61 | 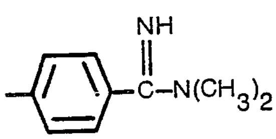 | OH<br>$CH_3CH_2CH$ | H | $CH_3$ | CH3 |
| 62 | 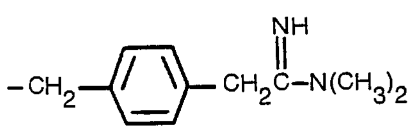 | $HOCH_2CH_2$ | H | $CH_3$ | H |
| 63 | 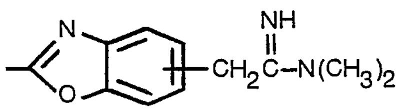 | $CH_2FCH_2$ | H | $CH_3$ | $CH_3$ |
| 64 | 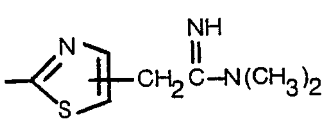 | $CH_3CHF$ | H | $CH_3$ | H |
| 65 | 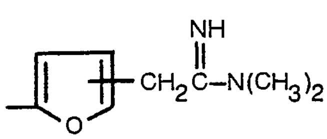 | OH<br>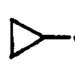$-CH_2CH$ | H | $CH_3$ | $CH_3$ |

| Compound | $R^8$ | $R^6$ | $R^7$ | $R^9$ | $R^{10}$ |
|---|---|---|---|---|---|
| 66 | 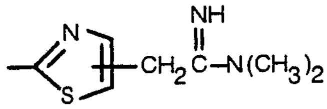 | 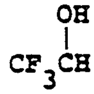 | H | $CH_3$ | H |
| 67 | 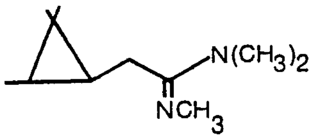 | 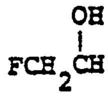 | H | $CH_3$ | $CH_3$ |
| 68 | 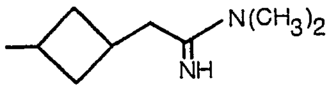 | 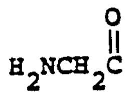 | H | $CH_3$ | H |
| 69 | 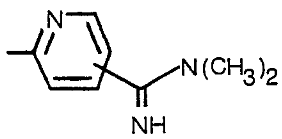 | 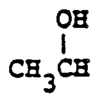 | $OCH_3$ | $CH_3$ | H |
| 70 | 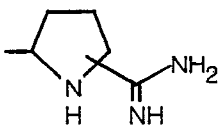 | 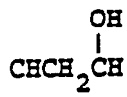 | $CH_3$ | $CH_3$ | $CH_3$ |
| 71 | 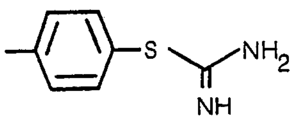 | $CH_3CH_2$ | H | $CH_3$ | H |
| 72 | 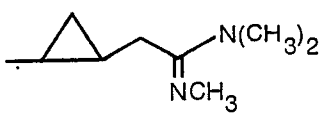 | $CH_3CH_2$ | H | $CH_3$ | $CH_3$ |
| 73 | 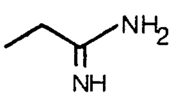 | $CH_3CH_2$ | H | $CH_3$ | $CH_3$ |
| 74 | 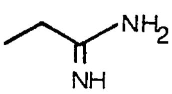 | 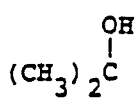 | H | $CH_3$ | H |
| 75 | 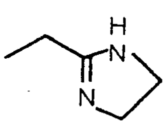 | $CH_3CH_2$ | H | $CH_3$ | H |
| 76 | 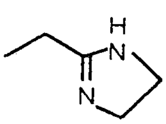 | 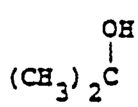 | H | $CH_3$ | H |

| Compound | $R^8$ | $R^6$ | $R^7$ | $R^9$ | $R^{10}$ |
|---|---|---|---|---|---|
| 77 | 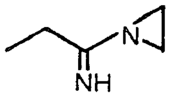 | $CH_3CH_2$ | H | $CH_3$ | $CH_3$ |
| 78 | 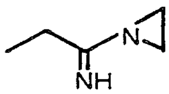 | OH<br>$(CH_3)_2C$ | H | $CH_3$ | H |
| 79 | 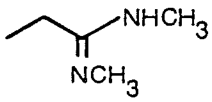 | $CH_3CH_2$ | H | $CH_3$ | H |
| 80 | 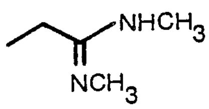 | $CH_3CH_3$ | H | $CH_3$ | H |
| 81 | 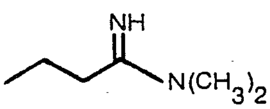 | $CH_3CH_2$ | H | $CH_3$ | H |
| 82 | 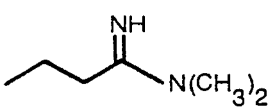 | $CH_3CH_2$ | H | $CH_3$ | H |
| 83 | 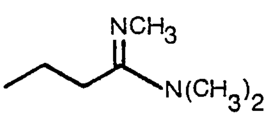 | $CH_3CH_2$ | H | $CH_3$ | H |
| 84 | 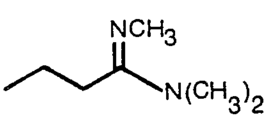 | OH<br>$(CH_3)_2C$ | H | $CH_3$ | H |
| 85 | 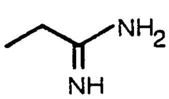 | $CH_3CH_2$ | H | $CH_3$ | H |
| 86 | 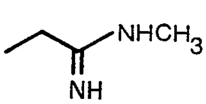 | OH<br>$(CH_3)_2C$ | H | $CH_3$ | $CH_3$ |

| Compound | $R^8$ | $R^6$ | $R^7$ | $R^9$ | $R^{10}$ |
|---|---|---|---|---|---|
| 87 | 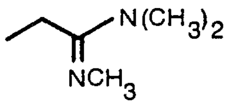 | $CH_3CH_2$ | H | $CH_3$ | H |
| 88 | 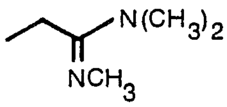 | $CH_3CH_2$ | H | $CH_3$ | $CH_3$ |
| 89 | 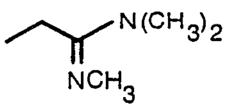 | 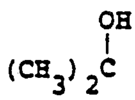 | H | $CH_3$ | H |
| 90 | 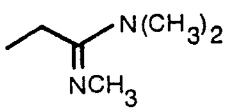 | 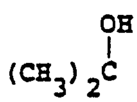 | H | $CH_3$ | $CH_3$ |
| 91 | 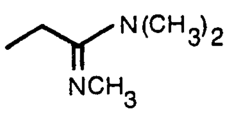 | $HOCH_2$ | H | $CH_3$ | H |
| 92 | 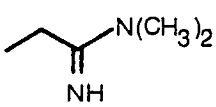 | $CH_2FCH_2$ | H | $CH_3$ | H |
| 93 | 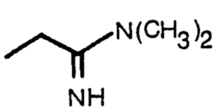 | 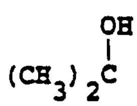 | H | $CH_3$ | H |
| 94 | 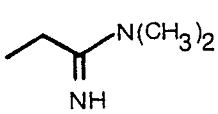 | 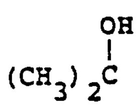 | H | $CH_3$ | $CH_3$ |
| 95 | 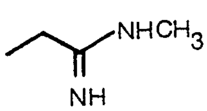 | 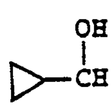 | H | $CH_3$ | H |
| 96 | 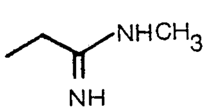 | $CH_3CH_2$ | H | $CH_3$ | H |

| Compound | $R^8$ | $R^6$ | $R^7$ | $R^9$ | $R^{10}$ |
|---|---|---|---|---|---|
| 97 | 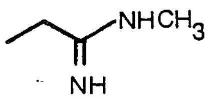 | $(CH_3)_2C(OH)$ | H | $CH_3$ | H |
| 98 | 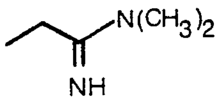 | $CH_3CH_2$ | H | $CH_3$ | H |
| 99 | 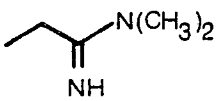 | $CH_3CH_2$ | H | $CH_3$ | $CH_3$ |
| 100 | 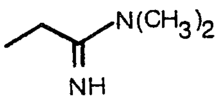 | $HOCH_2CH_2$ | H | $CH_3$ | H |
| 101 | 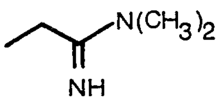 | $HOCH_2CH_2$ | H | $CH_3$ | $CH_3$ |
| 102 | 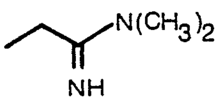 | $CH_3CHF$ | H | $CH_3$ | H |
| 103 | 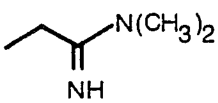 | HOCHCHF | H | $CH_3$ | H |

NOTE: Compounds such as No. 21, Example 9, above, wherein two radicals are joined are to be interpreted from the drawing as showing all but the point of common attachment. Thus, for example,

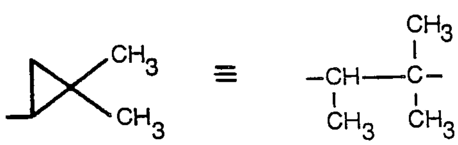

Example 11

Preparation of Pharmaceutical Compositions

One such unit dosage form is prepared by mixing 120 mg of compound A:

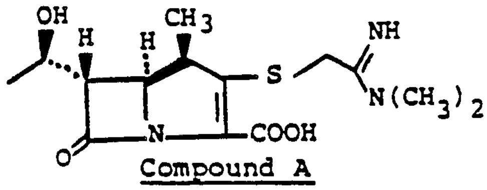

Compound A

with 20 mg of lactose and 5 mg of magnesium stearate and placing the 145 mg mixture into a No. 3 gelatin capsule. Similarly, by employing more of the active ingredient and less lactose, other dosage forms can be put up in No. 3 gelatin capsules, and, should it be necessary to mix more than 145 mg of ingredients together, larger capsules as well as compressed tablets and pills can be prepared. The following examples are illustrative of the preparation of pharmaceutical formulations:

| TABLET | PER TABLET |
|---|---|
| Compound A | 125 mg |
| Cornstarch, U.S.P. | 6 mg. |
| Dicalcium Phosphate | 192 mg. |
| Lactose, U.S.P | 190 mg. |
| Magnesium Stearate | Balance/800 mg. |

The active ingredient is blended with dicalcium phosphate, lactose and about half of the cornstarch. The mixture is then granulated with 15% cornstarch paste (6 mg) and rough-screened. It is dried at 45°C and screened again through No. 16 screens. The balance of the cornstarch and magnesium stearate is added and the mixture is compressed into tablets, approximately 0.5 inch in diameter each weighing 800 mg.

| PARENTAL SOLUTION | PER TABLET |
|---|---|
| Ampoule: | |
| Compound A | 500 mg. |
| Diluent: Sterile Water for Injection | 2 cc. |
| OPHTHALMIC SOLUTION | |
| Compound A | 100 mg. |
| Hydroxypropylmethyl Cellulose | 5 mg. |
| Sterile Water | to 1 ml. |
| OTIC SOLUTION | |
| Compound A | 100 mg. |
| Benzalkonium chloride | 0.1 mg. |
| Sterile Water | to 1 ml. |
| TOPICAL OINTMENT | |
| Compound A | 100 mg. |
| Polyethylene Glycol 4000 U.S.P. | 400 mg. |
| Polyethylene Glycol 400 U.S.P. | 1.0 gram |

**Claims for the Contracting States: BE CH DE FR GB IT LI LU NL SE**

1. A compound having the structural formula

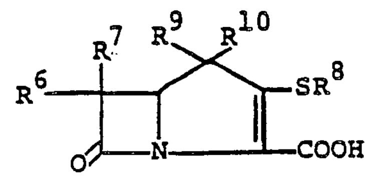

and pharmaceutically acceptable salt, ester and amide derivatives thereof; wherein:

$R^6$ and $R^7$, and $R^9$ and $R^{10}$ are independently hydrogen ($R^9$ and $R^{10}$ are not both hydrogen) or are selected from the group consisting of substituted and unsubstituted: alkyl, alkenyl, and alkynyl having 1—10 carbon atoms; cycloalkyl, cycloalkylalkyl, and alkylcycloalkyl having 3—6 carbon atoms in the cycloalkyl ring and 1—6 carbon atoms in the alkyl moiety; phenyl, phenylalkyl, phenylalkenyl, and phenylalkynyl wherein the aliphatic portion has 1—6 carbon atoms; or $R^6$ and $R^7$ are independently pyridyl or $R^6$ and $R^7$, and $R^9$ and $R^{10}$ may be joined to form a cyclicalkyl having, together with the carbon atom to which they are attached, 3—6 carbon atoms; wherein the substituent or substituents on $R^6$, $R^7$, $R^9$ or $R^{10}$ are independently selected from chloro, fluoro, hydroxy, bromo, alkylthio having 1—6 carbon atoms, and alkoxyl having 1—6 carbon atoms; and wherein $R^7$ can additionally be hydroxyl, methoxyl, mercapto, chloro, fluoro and bromo; and

when $R^7$ is hydrogen, $CH_3$ or $OCH_3$, $R^6$ can additionally have carboxyl, hydroxyimino, ureido or amino as substituents on the above-mentioned alkyl, cycloalkyl, cycloalkylalkyl and phenylalkyl groups; and

$R^8$ is carbamimidoyl selected from the group consisting of:

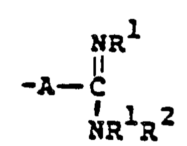
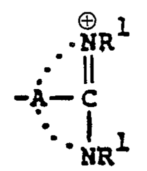
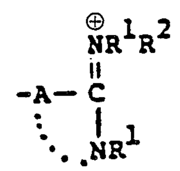

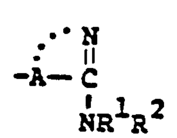
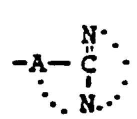
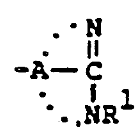

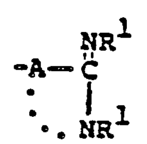
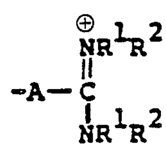
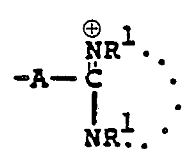

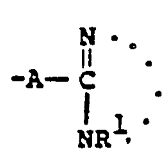
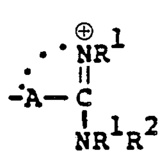
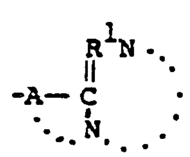

wherein: A is a single, direct bond, or A, the cyclic or acyclic connector, is selected from the group consisting of alkylene, alkenylene, and alkynylene having 1—10 carbon atoms which may be interrupted by a hetero atom selected from O, S or N, or by phenylene, cycloalkylene, cycloalkenylene, heterocyclylene or heteroarylene wherein such cyclic interruptions comprise 3—6 ring atoms selected from C, O, S and N; cycloalkylene having 3—10 carbon atoms; cycloalkenylene having 3—6 carbon atoms; and phenylene; $R^1$ and $R^2$ are independently selected from hydrogen and the previously defined, but monovalent, values for the group A; cycloalkylalkyl wherein the cycloalkyl moiety comprises 3 to 6 carbon atoms and the alkyl moiety comprises 1 to 6 carbon atoms; alkylcycloalkyl wherein the alkyl moiety comprises 1 to 6 carbon atoms and the cycloalkyl moiety comprises 3 to 6 carbon atoms; phenylalkyl having 7—10 carbon atoms;

wherein, if $R^1$ and $R^2$ are independently selected from straight and branched alkyl having from 1 to 6 carbon atoms; cycloalkyl having 3 to 6 carbon atoms; phenyl; or one of the above-defined cycloalkylalkyl,

alkylcycloalkyl and phenylalkyl groups, each of those radicals can be substituted by amino, hydroxy, cyano, carboxyl, nitro, chloro, bromo, fluoro, alkoxy and alkylthio having from 1 to 6 carbon atoms, mercapto, perhaloalkyl having 1 to 3 carbon atoms, guanidino, amidino or sulfamoyl; and

wherein A can additionally be selected from cycloalkylalkylene wherein the cycloalkyl moiety comprises 3 to 6 carbon atoms and the alkylene moiety comprises 1 to 10 carbon atoms; alkylcycloalkylene wherein the alkyl moiety comprises 1 to 6 carbon atoms and the cycloalkylene moiety comprises 3 to 6 carbon atoms; cycloalkenylalkylene wherein the cycloalkenyl moiety comprises 3 to 10 carbon atoms and the alkylene moiety comprises 1 to 6 carbon atoms; naphthylene; phenylalkylene and alkylphenylene wherein the alkyl has 1 to 6 carbon atoms; and

wherein the dotted lines indicate provision for cyclic structures formed by the joinder of the indicated nitrogen atom and the connector group A and by the joinder of the indicated nitrogen atoms;

with the exception of such compounds wherein:

a) $R^6$ is H, and
$R^7$ is $CH_2OH$ $R^8$ is 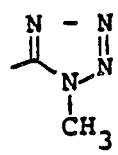
$R^9$ is $CH_3$
$R^{10}$ is H

b) $R^6$ is H
$R^7$ is 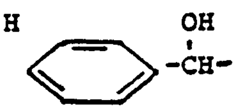 and
$R^9$ is $CH_3$ $R^8$ is $CH_2CH_2\overset{\overset{NH}{\|}}{C}-NH_2$
$R^{10}$ is 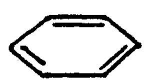

c) $R^6$ is H
$R^7$ is $CH_3-\overset{\overset{OH}{|}}{C}H-$ and
$R^9$ is $CH_3$ $R^8$ is 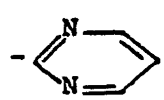
$R^{10}$ is $CH_3$

d) $R^6$ is H
$R^7$ is $CH_3-\overset{\overset{OH}{|}}{C}H-$
$R^9$ and $R^{10}$ are joined to form a cyclopropyl ring
and
$R^8$ is 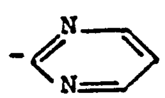.

2. A compound according to Claim 1 wherein $R^1$ and $R^2$ are independently selected from: hydrogen; substituted and unsubstituted: straight and branched alkyl having from 1 to 6 carbon atoms; cycloalkyl having from 3 to 6 carbon atoms, cycloalkylalkyl wherein the cycloalkyl moiety comprises 3 to 6 atoms and the alkyl moiety comprises 1 to 6 carbon atoms; phenyl, benzyl; wherein the substituent or substituents on the above-named radicals are selected from fluoro, hydroxy, mercapto, alkoxy having from 1 to 3 carbon atoms; and alkylthio having from 1 to 3 carbon atoms.

3. A compound according to Claim 1 wherein the connecting group A is selected from: a single, direct bond, straight and branched loweralkylene having from 1 to 6 carbon atoms, cycloalkylene having from 3 to 6 carbon atoms; phenylene; alkylheteroalkyl wherein the alkyl moiety comprises 1 to 3 carbon atoms and the heteroatom or atoms are sulfur, oxygen and nitrogen.

4. A compound according to Claim 1 wherein $-SR^8$ is selected from the group consisting of:

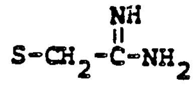

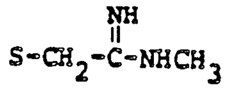

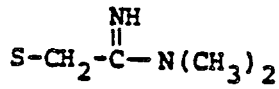

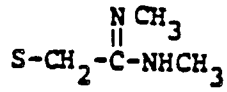

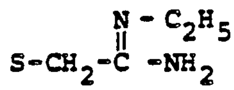

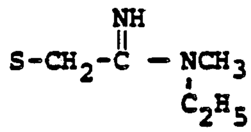

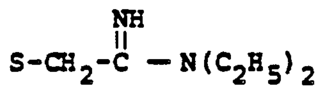

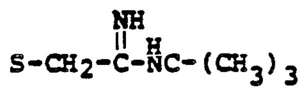

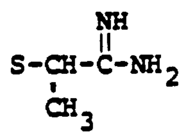

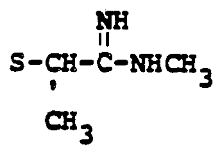

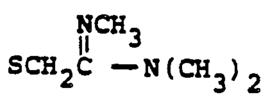

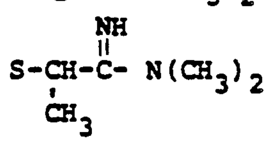

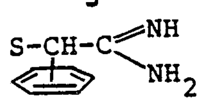

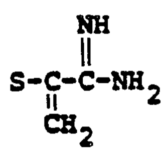

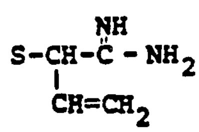

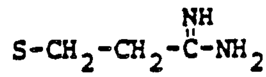

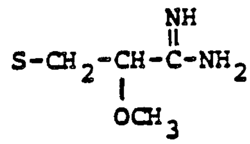

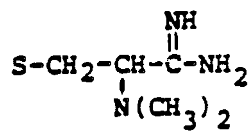

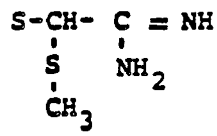

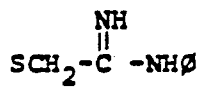

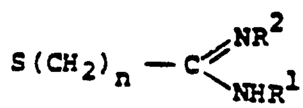

$n = 2\text{-}5$, $R^2 = H, CH_3$

$R^1 = H, CH_3$

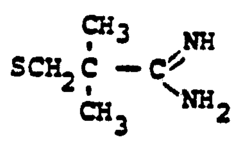

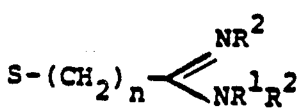

$n = 2\text{-}5$, $R^1, R^2 = H, CH_3$

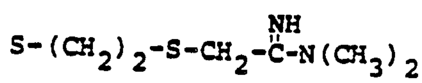

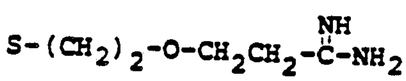

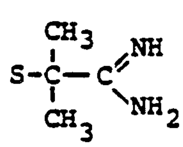

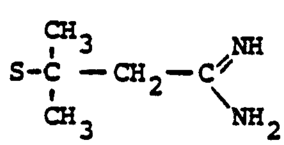

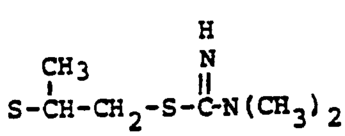

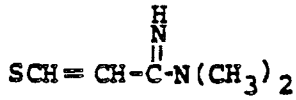

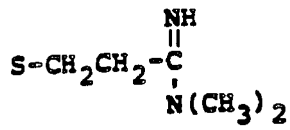

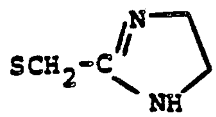

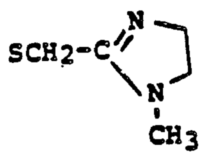

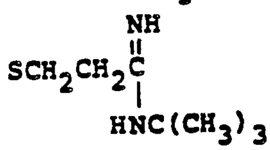

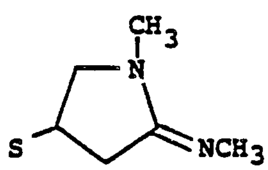

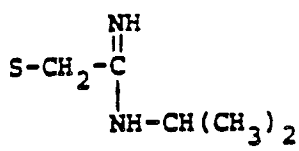

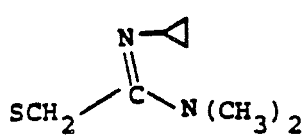

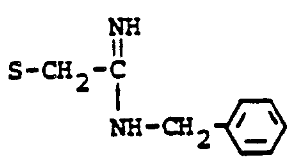

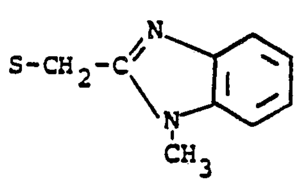

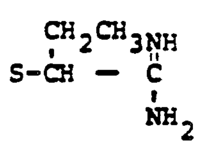

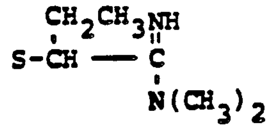

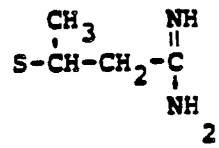

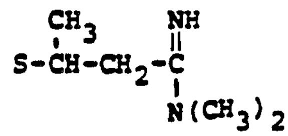

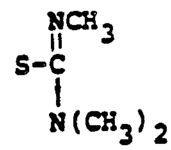

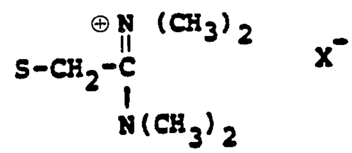

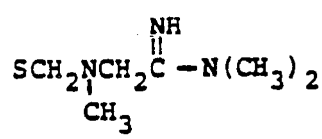

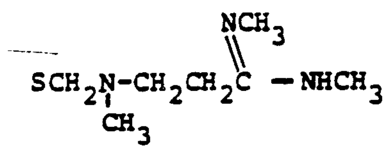

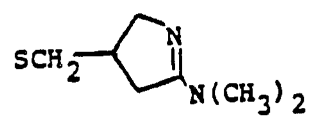

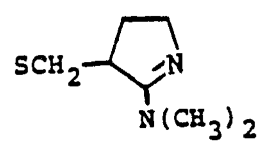

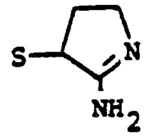

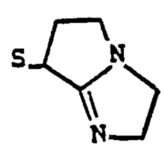

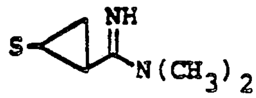

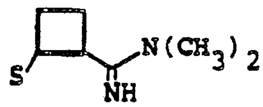

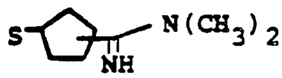

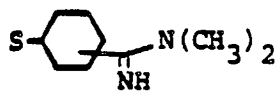

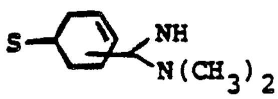

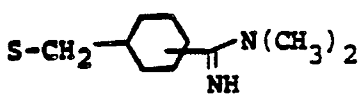

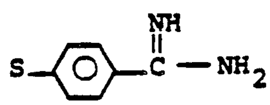

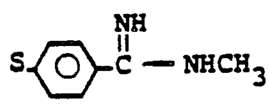

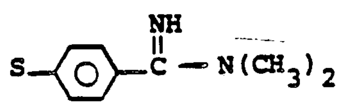

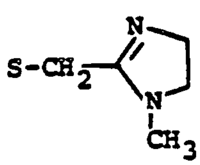

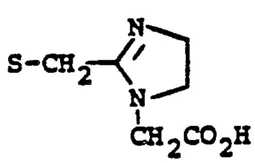

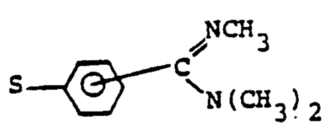

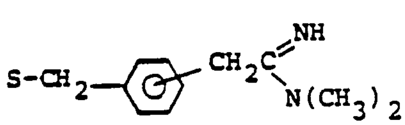

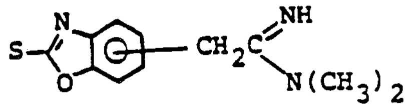

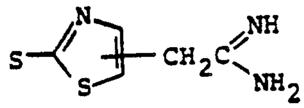

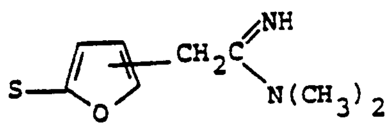

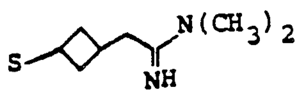

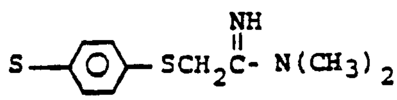

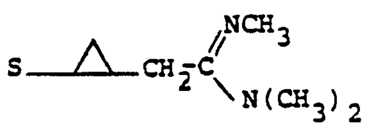

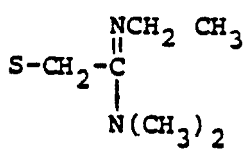

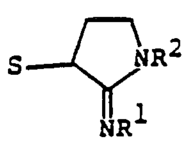

$R^1$ = H, CH3
$R^2$ = H, $CH_3$

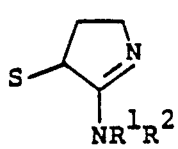

$R^1$ = H, $CH_3$
$R^2$ = H, $CH_3$

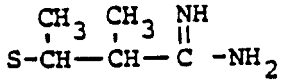

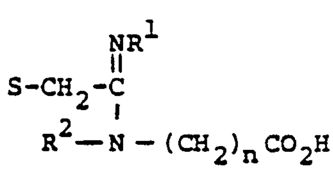

$R^1$ = H, $CH_3$
$R^2$ = H, $CH_3$
n = 1 or 2

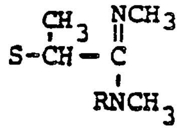

R = H, $CH_3$

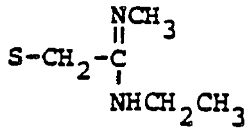

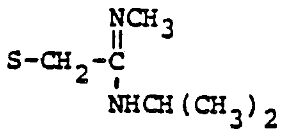

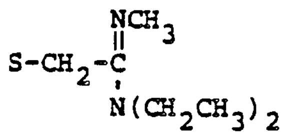

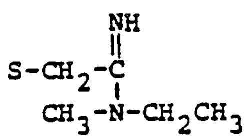

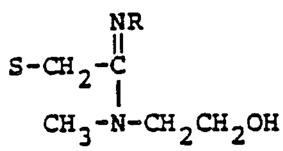

R = $CH_2CH_3$, $CH_3$

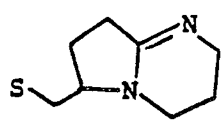

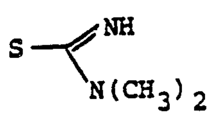

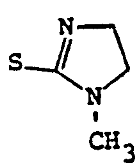

5. A compound according to Claims 1, 2, 3 or 4 wherein $R^9$ and $R^{10}$ are $C_{1-10}$ alkyl.

6. A compound according to Claim 5 wherein $R^9$ and $R^{10}$ are methyl.

7. A compound according to Claim 1, 2, 3 or 4 wherein $R^9$ and $R^{10}$ are independently selected from the group consisting of:

$CH_2CH_3$
$CH(CH_3)_2$
$CH_2C_6H_5$
$CH_2F$

$CF_3$

$CH_2Br$

$-CH=CH_2$

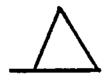

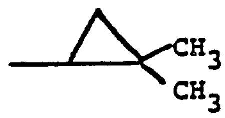

$CHF_2$

$CH_2Cl$

$CH_2OH$

$CH_2SMe$

$CH_2OMe$

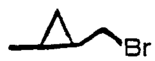

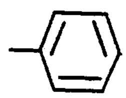

$-(CH_2)_n-$ n = 2,3,4,5

8. A compound according to Claim 1, wherein $R^7$ is selected from H, $OCH_3$ and $CH_3$.

9. A compound according to Claim 8 wherein $R^6$ is selected from the group consisting of $C_{1-10}$ alkyl, $C_{3-6}$ cycloalkyl-$C_{1-6}$ alkyl, $C_{1-10}$ alkyl substituted by one or more hydroxyl groups, or $C_{3-6}$ cycloalkyl-$C_{1-6}$ alkyl substituted by one or more hydroxyl groups.

10. A compound according to Claim 9 wherein $R^7$ is hydrogen.

11. A compound according to Claims 1, 8 or 9 wherein $R^6$ is selected from:

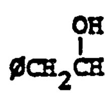 Ø = phenyl

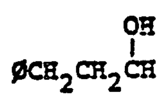

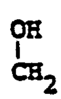

$ØCH_2$

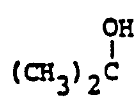

$CH_2=CHCH_2$

$(CH_3)_2C=CHCH_2$

$CH_3OCH_2\overset{OH}{\overset{|}{C}}H$

$CH_3$

$CH_3CH_2$

$(CH_3)_2CH$

$HO_2CCH_2$

$CF_3CF_2\overset{OH}{\overset{|}{C}}H$

$HO_2CCH_2\overset{OH}{\overset{|}{C}}H$

$CH_3CH(CH_3)\overset{OH}{\overset{|}{C}}H$

$(CH_3)_2CHCH_2\overset{OH}{\overset{|}{C}}H$

$CH_3CH_2\overset{OH}{\overset{|}{C}}H$

$HOCH_2CH_2$

$CF_3\overset{OH}{\overset{|}{C}}H$

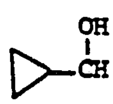

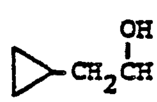

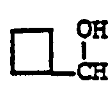

$HOCH_2CH_2CH_2$

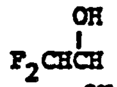

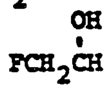

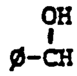

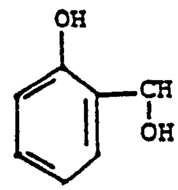

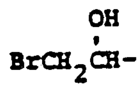

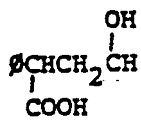

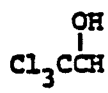

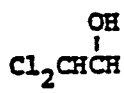

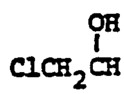

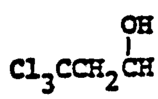

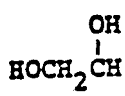

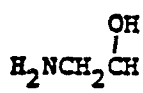

$-H$

$-CH(OH)CH_3$

$-CH_2CH_2CH_2CH_2OH$

$-CH(OH)CH_2CH_2OH$

$-CH(NH_2)CH_3$

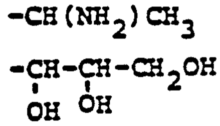

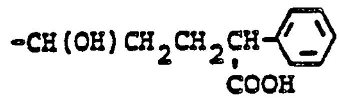

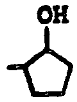

12. A compound according to Claim 1 wherein $R^7$ is substituted alkyl having 1 to 10 carbon atoms wherein the substituent or substituents are: alkoxyl having 1 to 6 carbon atoms or hydroxyl.

13. A compound according to Claim 1 wherein $R^7$ is hydrogen, hydroxyl, $OCH_3$, $CH_3$, hydroxyl- and polyhydroxyl-substituted alkyl having 1 to 10 carbon atoms.

14. A compound according to Claim 8 wherein $R^6$ is: hydrogen; substituted and unsubstituted: $C_{1-10}$alkyl, $C_{3-6}$cycloalkyl, $C_{3-6}$cycloalkyl-$C_{1-6}$ alkyl, phenyl-$C_{1-6}$ alkyl; wherein the substituent or substituents are hydroxyl.

15. A process for preparing a compound of claim 1 comprising the step of treating:

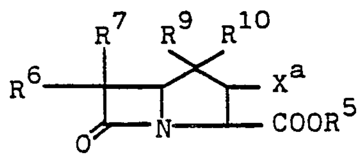

with $HSR^8$; wherein $X^a$ is a leaving group, $R^5$ is a protecting group and $R^6$, $R^7$, $R^9$ and $R^{10}$ are defined as in Claim 1.

16. An antibiotic composition comprising a therapeutically effective amount of a compound according to Claim 1 and a pharmaceutically effective carrier therefor.

**Claims for the Contracting State: AT**

1. A process for preparing a compound having the structural formula

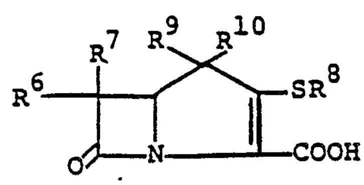

and pharmaceutically acceptable salt, ester and amide derivatives thereof; wherein:

$R^6$ and $R^7$, and $R^9$ and $R^{10}$ are independently hydrogen ($R^9$ and $R^{10}$ are not both hydrogen) or are selected from the group consisting of substituted and unsubstituted: alkyl, alkenyl, and alkynyl having 1—10 carbon atoms; cycloalkyl, cycloalkylalkyl, and alkylcycloalkyl having 3—6 carbon atoms in the cycloalkyl ring and 1—6 carbon atoms in the alkyl moiety; phenyl, phenylalkyl, phenylalkenyl, and phenylalkynyl wherein the aliphatic portion has 1—6 carbon atoms; or $R^6$ and $R^7$ are independently pyridyl or $R^6$ and $R^7$, and $R^9$ and $R^{10}$ may be joined to form a cyclicalkyl having, together with the carbon atom to which they are attached, 3—6 carbon atoms; wherein the substituent or substituents on $R^6$, $R^7$, $R^9$ or $R^{10}$ are independently selected from chloro, fluoro, hydroxy, bromo, alkylthio having 1—6 carbon atoms, and alkoxyl having 1—6 carbon atoms; and wherein $R^7$ can additionally be hydroxyl, methoxyl, mercapto, chloro, fluoro and bromo; and

when $R^7$ is hydrogen, $CH_3$ or $OCH_3$, $R^6$ can additionally have carboxyl, hydroxyimino, ureido or amino as substituents on the above-mentioned alkyl, cycloalkyl, cycloalkylalkyl and phenylalkyl groups; and

$R^8$ is carbamimidoyl selected from the group consisting of:

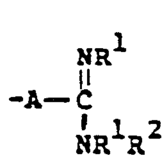

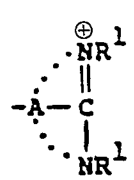

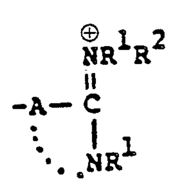

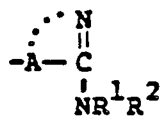 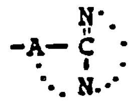 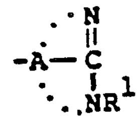

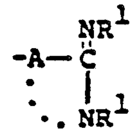 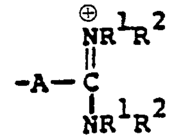 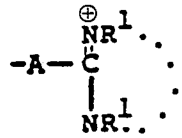

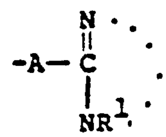 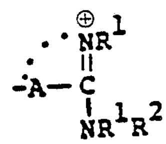 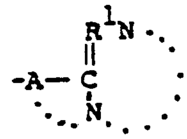

wherein: A is a single, direct bond, or A, the cyclic or acyclic connector, is selected from the group consisting of alkylene, alkenylene, and alkynylene having 1—10 carbon atoms which may be interrupted by a hetero atom selected from O, S or N, or by phenylene, cycloalkylene, cycloalkenylene, heterocyclylene or heteroarylene wherein such cyclic interruptions comprise 3—6 ring atoms selected from C, O, S and N; cycloalkylene having 3—10 carbon atoms; cycloalkenylene having 3—6 carbon atoms; and phenylene; $R^1$ and $R^2$ are independently selected from hydrogen and the previously defined, but monovalent, values for the group A; cycloalkylalkyl wherein the cycloalkyl moiety comprises 3 to 6 carbon atoms and the alkyl moiety comprises 1 to 6 carbon atoms; alkylcycloalkyl wherein the alkyl moiety comprises 1 to 6 carbon atoms and the cycloalkyl moiety comprises 3 to 6 carbon atoms; phenylalkyl having 7—10 carbon atoms;

wherein, if $R^1$ and $R^2$ are independently selected from straight and branched alkyl having from 1 to 6 carbon atoms; cycloalkyl having 3 to 6 carbon atoms; phenyl; or one of the above-defined cycloalkylalkyl, alkylcycloalkyl and phenylalkyl groups, each of those radicals can be substituted by amino, hydroxy, cyano, carboxyl, nitro, chloro, bromo, fluoro, alkoxy and alkylthio having from 1 to 6 carbon atoms, mercapto, perhaloalkyl having 1 to 3 carbon atoms, guanidino, amidino or sulfamoyl; and

wherein A can additionally be selected from cycloalkylalkylene wherein the cycloalkyl moiety comprises 3 to 6 carbon atoms and the alkylene moiety comprises 1 to 10 carbon atoms; alkylcycloalkylene wherein the alkyl moiety comprises 1 to 6 carbon atoms and the cycloalkylene moiety comprises 3 to 6 carbon atoms; cycloalkenylalkylene wherein the cycloalkenyl moiety comprises 3 to 10 carbon atoms and the alkylene moiety comprises 1 to 6 carbon atoms; naphthylene; phenylalkylene and alkylphenylene wherein the alkyl has 1 to 6 carbon atoms; and

wherein the dotted lines indicate provision for cyclic structures formed by the joinder of the indicated nitrogen atom and the connector group A and by the joinder of the indicated nitrogen atoms;

with the exception of such compounds wherein:

a) $R^6$ is H, and
$R^7$ is $CH_2OH$ $R^8$ is 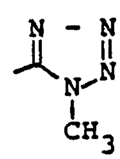
$R^9$ is $CH_3$
$R^{10}$ is H

b) $R^6$ is H
$R^7$ is 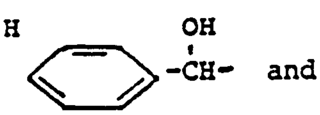 and
$R^9$ is $CH_3$ $R^8$ is 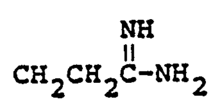
$R^{10}$ is 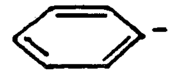

c) $R^6$ is H
$R^7$ is $CH_3-\overset{OH}{CH}-$ and
$R^9$ is $CH_3$ $R^8$ is - 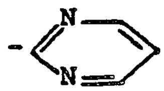
$R^{10}$ is $CH_3$

d) $R^6$ is H
$R^7$ is $CH_3-\overset{OH}{CH}-$
$R^9$ and $R^{10}$ are joined to form a cyclopropyl ring
and
$R^8$ is - 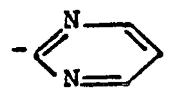.

comprising the step of treating

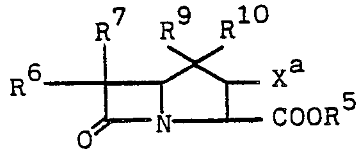

with $HSR^8$; wherein $X^a$ is a leaving group, $R^5$ is a protecting group and $R^6$, $R^7$, $R^9$ and $R^{10}$ are defined as above.

2. A process according to Claim 1 wherein $R^1$ and $R^2$ are independently selected from: hydrogen; substituted and unsubstituted: straight and branched alkyl having from 1 to 6 carbon atoms; cycloalkyl having from 3 to 6 carbon atoms, cycloalkylalkyl wherein the cycloalkyl moiety comprises 3 to 6 atoms and the alkyl moiety comprises 1 to 6 carbon atoms; phenyl, benzyl; wherein the substituent or substituents on the above-named radicals are selected from fluoro, hydroxy, mercapto, alkoxy having from 1 to 3 carbon atoms; and alkylthio having from 1 to 3 carbon atoms.

3. A process according to Claim 1 wherein the connecting group A is selected from: a single, direct bond, straight and branched loweralkylene having from 1 to 6 carbon atoms, cycloalkylene having from 3 to 6 carbon atoms; phenylene; alkylheteroalkyl wherein the alkyl moiety comprises 1 to 3 carbon atoms and the heteroatom or atoms are sulfur, oxygen and nitrogen.

4. A process according to Claim 1 wherein $-SR^8$ is selected from the group consisting of:

$$S-CH_2-\overset{NH}{\overset{\|}{C}}-NH_2$$

$$S-CH_2-\overset{NH}{\overset{\|}{C}}-NHCH_3$$

$$S-CH_2-\overset{NH}{\overset{\|}{C}}-N(CH_3)_2$$

$$S-CH_2-\overset{N\,CH_3}{\overset{\|}{C}}-NHCH_3$$

$$S-CH_2-\overset{N-C_2H_5}{\overset{\|}{C}}-NH_2$$

$$S-CH_2-\overset{NH}{\overset{\|}{C}}-\underset{C_2H_5}{NCH_3}$$

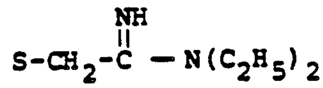

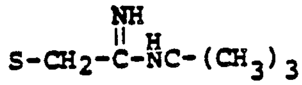

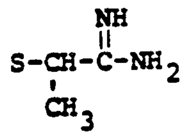

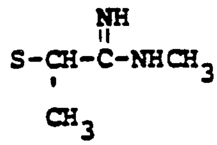

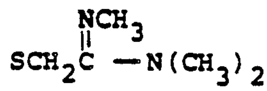

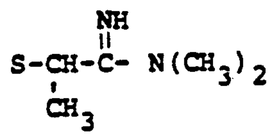

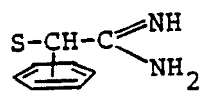

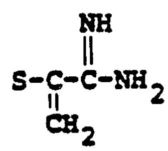

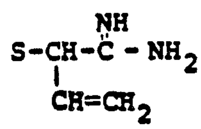

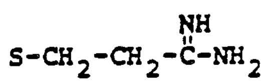

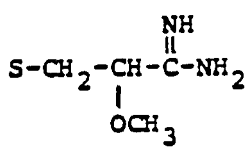

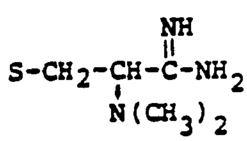

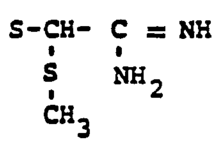

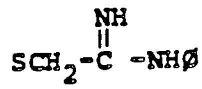

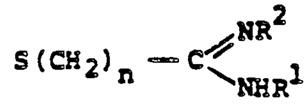

$n = 2\text{-}5,\ R^2 = H,\ CH_3$

$R^1 = H,\ CH_3$

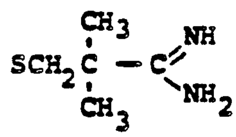

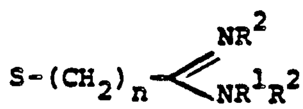

$n = 2\text{-}5,\ R^1,\ R^2 = H,\ CH_3$

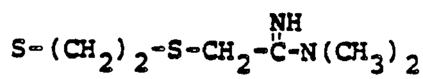

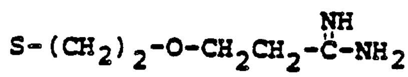

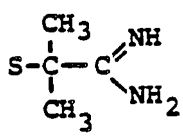

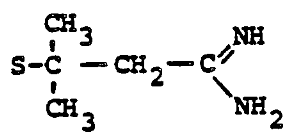

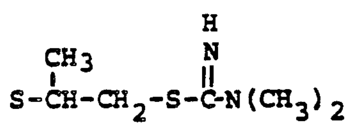

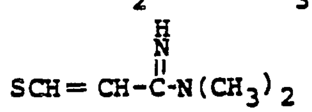

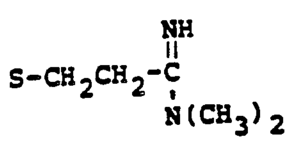

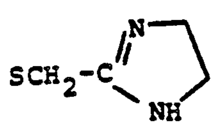

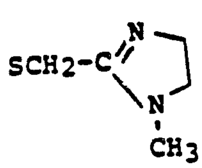

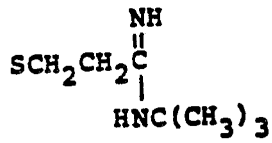

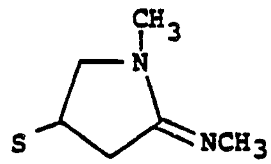

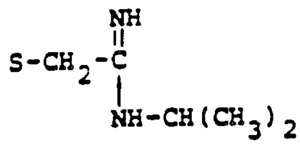

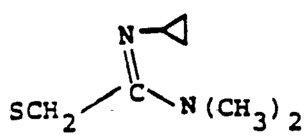

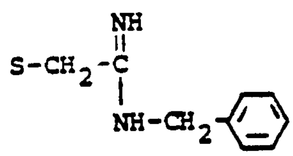

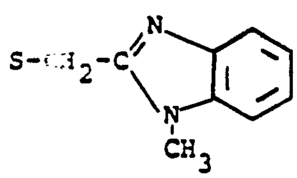

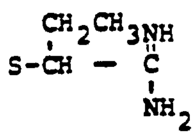

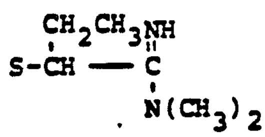

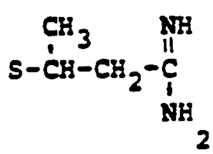

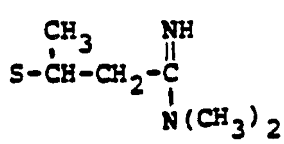

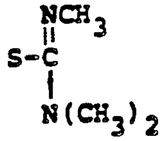

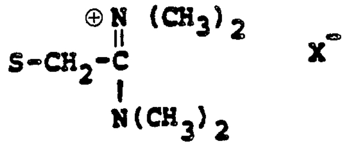

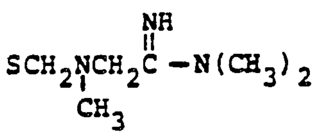

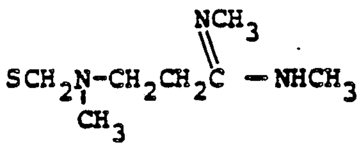

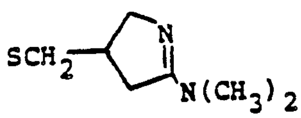

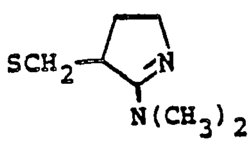

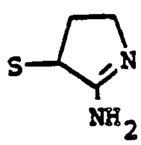

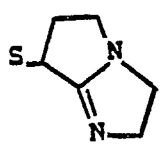

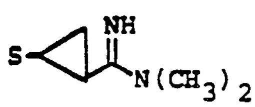

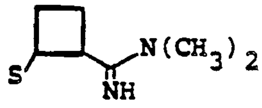

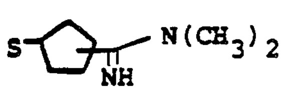

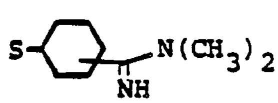

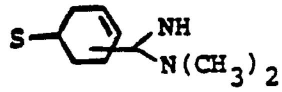

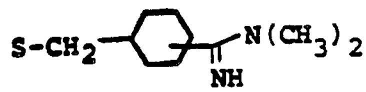

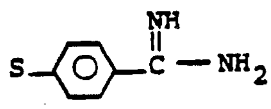

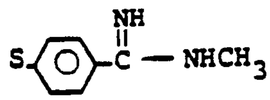

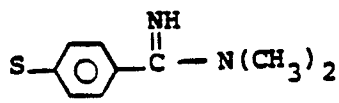

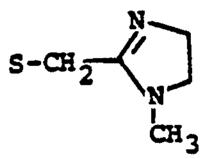

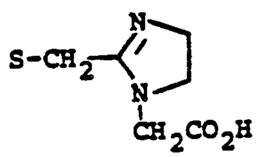

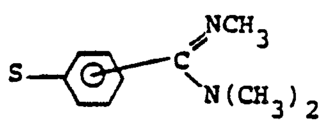

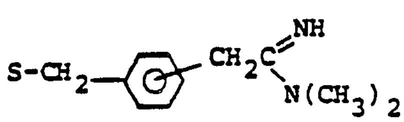

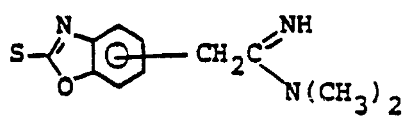

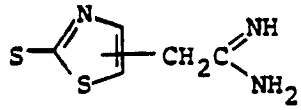

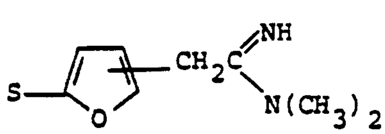

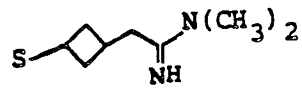

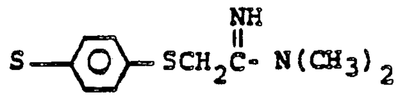

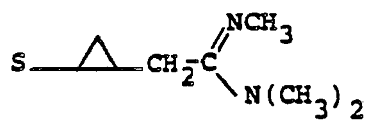

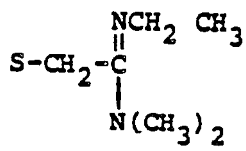

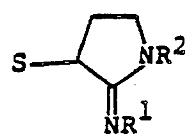

$R^1$ = H, CH3
$R^2$ = H, $CH_3$

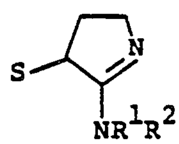

$R^1$ = H, $CH_3$
$R^2$ = H, $CH_3$

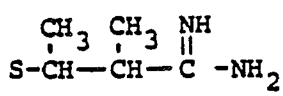

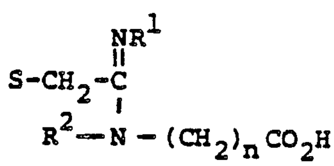

$R^1$ = H, $CH_3$
$R^2$ = H, $CH_3$
n = 1 or 2

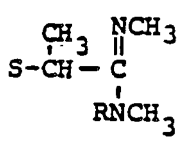

R = H, $CH_3$

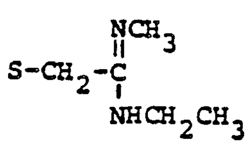

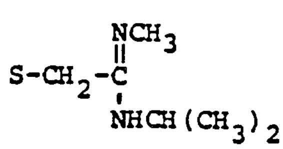

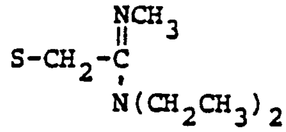

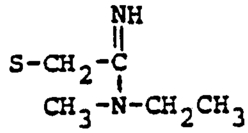

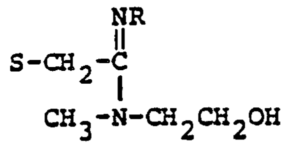

$R = CH_2CH_3$, $CH_3$

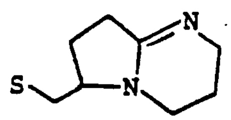

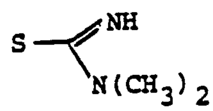

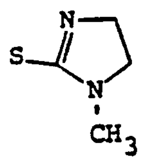

5. A process according to Claims 1, 2, 3 or 4 wherein $R^9$ and $R^{10}$ are $C_{1-10}$ alkyl.

6. A process according to Claim 5 wherein $R^9$ and $R^{10}$ are methyl.

7. A process according to Claim 1, 2, 3 or 4 wherein $R^9$ and $R^{10}$ are independently selected from the group consisting of:

$CH_2CH_3$
$CH(CH_3)_2$
$CH_2C_6H_5$
$CH_2F$
$CF_3$
$CH_2Br$
$-CH=CH_2$

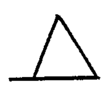

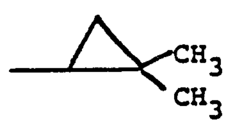

$CHF_2$
$CH_2Cl$
$CH_2OH$
$CH_2SMe$
$CH_2OMe$

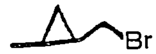

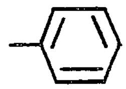

$-(CH_2)_n-$ $n = 2,3,4,5$

8. A process according to Claim 1, wherein $R^7$ is selected from H, $OCH_3$ and $CH_3$.

9. A process according to Claim 8 wherein $R^6$ is selected from the group consisting of $C_{1-10}$ alkyl, $C_{3-6}$ cycloalkyl-$C_{1-6}$ alkyl, $C_{1-10}$ alkyl substituted by one or more hydroxyl groups, or $C_{3-6}$ cycloalkyl-$C_{1-6}$ alkyl substituted by one or more hydroxyl groups.

10. A process according to Claim 9 wherein $R^7$ is hydrogen.

11. A process according to Claims 1, 8 or 9 wherein $R^6$ is selected from:

OH
$ØCH_2CH$ Ø = phenyl

OH
$ØCH_2CH_2CH$

OH
$CH_2$

$ØCH_2$

OH
$(CH_3)_2C$

$CH_2=CHCH_2$

$(CH_3)_2C=CHCH_2$

OH
$CH_3OCH_2CH$

$CH_3$

$CH_3CH_2$

$(CH_3)_2CH$

$HO_2CCH_2$

OH
$CF_3CF_2CH$

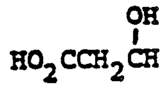

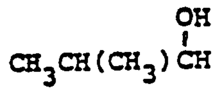

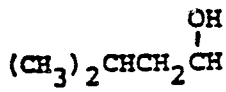

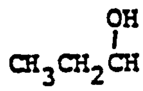

$HOCH_2CH_2$

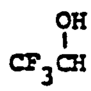

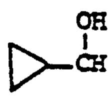

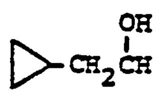

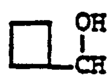

$HOCH_2CH_2CH_2$

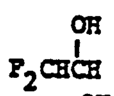

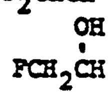

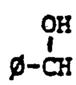

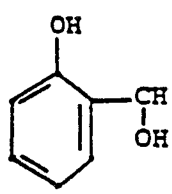

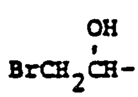

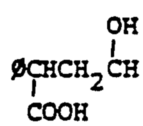

OH
$Cl_3CCH$

OH
$Cl_2CHCH$

OH
$ClCH_2CH$

OH
$Cl_3CCH_2CH$

OH
$HOCH_2CH$

OH
$H_2NCH_2CH$

$-H$

$-CH(OH)CH_3$

$-CH_2CH_2CH_2CH_2OH$

$-CH(OH)CH_2CH_2OH$

$-CH(NH_2)CH_3$

$-CH-CH-CH_2OH$
OH OH

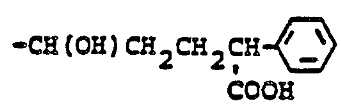

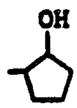

12. A process according to Claim 1 wherein $R^7$ is substituted alkyl having 1 to 10 carbon atoms wherein the substituent or substituents are: alkoxyl having 1 to 6 carbon atoms or hydroxyl.

13. A process according to Claim 1 wherein $R^7$ is hydrogen, hydroxyl, $OCH_3$, $CH_3$, hydroxyl- and polyhydroxyl-substituted alkyl having 1 to 10 carbon atoms.

14. A process according to Claim 8 wherein $R^6$ is: hydrogen; substituted and unsubstituted: $C_{1-10}$alkyl, $C_{3-6}$cycloalkyl, $C_{3-6}$cycloalkyl-$C_{1-6}$ alkyl, phenyl-$C_{1-6}$ alkyl; wherein the substituent or substituents are hydroxyl.

**Patentansprüche fur die Vertragsstaaten: BE CH DE FR GB IT LI LU NL SE**

1. Eine Verbindung mit der Strukturformel:

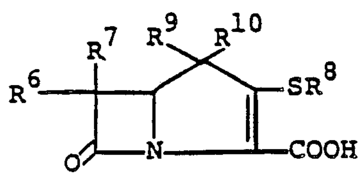

und pharmazeutisch annehmbare Salz-, Ester- und Amidderivate davon, worin:

$R^6$ und $R^7$ sowie $R^9$ und $R^{10}$ unabhängig voneinander Wasserstoff ($R^9$ und $R^{10}$ sind nicht beide Wasserstoff) sind oder aus der Gruppe ausgewählt sind, die besteht aus substituiertem und unsubstituiertem: Alkyl, Alkenyl und Alkinyl mit 1—10 Kohlenstoffatomen; Cycloalkyl, Cycloalkylalkyl und Alkylcycloalkyl mit 3—6 Kohlenstoffatomen im Cycloalkylring und 1—6 Kohlenstoffatomen im Alkylrest; Phenyl, Phenylalkyl, Phenylalkenyl und Phenylalkinyl, wobei der aliphatische Teil 1—6 Kohlenstoffatome aufweist; oder $R^6$ und $R^7$ unabhängig voneinander Pyridyl sind, oder $R^6$ und $R^7$ sowie $R^9$ und $R^{10}$ unter Bildung eines cyclischen Alkyls verbunden sein können, das gemeinsam mit dem Kohlenstoffatom, an das die Reste gebunden sind, 3—6 Kohlenstoffatome aufweist; wobei der Substituent oder die Substituenten an den Resten $R^6$, $R^7$, $R^9$ oder $R^{10}$ unabhängig voneinander ausgewählt sind aus Chlor, Fluor, Hydroxy, Brom, Alkylthio mit 1—6 Kohlenstoffatomen und Alkoxy mit 1—6 Kohlenstoffatomen; und wobei $R^7$ zusätzlich Hydroxyl, Methoxyl, Mercapto, Chlor, Fluor und Brom sein kann; und

falls $R^7$ Wasserstoff, $CH_3$ oder $OCH_3$ ist, $R^6$ zusätzlich Carboxyl, Hydroxyimino, Ureido oder Amino als Substituenten an den oben erwähnten Alkyl-, Cycloalkyl-, Cycloalkylalkyl- und Phenylalkylgruppen aufweisen kann;

und $R^8$ Carbamimidoyl, ausgewählt aus der Gruppe bestehend aus:

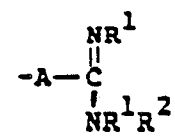
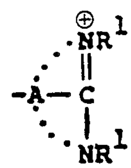
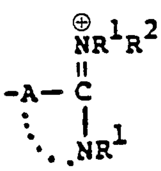

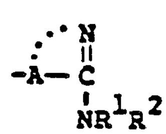
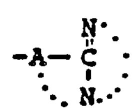
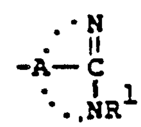

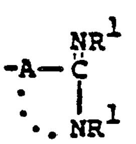
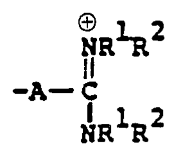
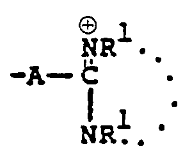

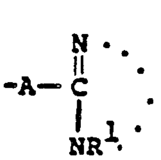
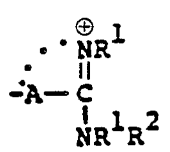
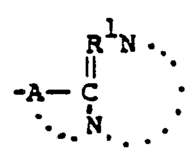

wobei: A eine einfache, direkte Bindung ist, oder A als cyclisches oder acyclisches Bindeglied aus der Gruppe ausgewählt ist, die aus Alkylen, Alkenylen und Alkinylen mit 1—10 Kohlenstoffatomen besteht, welche durch ein Heteroatom, ausgewählt aus O, S oder N, oder durch Phenylen, Cycloalkylen, Cycloalkenylen, Heterocyclylen oder Heteroarylen unterbrochen sein können, wobei solche cyclischen Unterbrechungen 3—6 Ringatome, ausgewählt aus C, O, S und N, enthalten; Cycloalkylen mit 3—10 Kohlenstoffatomen; Cycloalkenylen mit 3—6 Kohlenstoffatomen; und Phenylen ist;

$R^1$ und $R^2$ unabhängig voneinander aus Wasserstoff und den vorher definierten, jedoch einwertigen Bedeutungen für die Gruppe A ausgewählt sind; Cycloalkylalkyl, worin der Cycloalkylrest 3 bis 6 Kohlenstoffatome enthält, und der Alkylrest 1 bis 6 Kohlenstoffatome enthält; Alkylcycloalkyl, wobei der Alkylrest 1 bis 6 Kohlenstoffatome enthält, und der Cycloalkylrest 3 bis 6 Kohlenstoffatome enthält; oder Phenylalkyl mit 7—10 Kohlenstoffatomen sind; wobei, falls $R^1$ und $R^2$ unabhängig voneinander aus geradem und verzweigtem Alkyl mit 1 bis 6 Kohlenstoffatomen; Cycloalkyl mit 3 bis 6 Kohlenstoffatomen; Phenyl; oder einer der oben definierten Cycloalkylalkyl-, Alkylcycloalkyl- und Phenylalkylgruppen ausgewählt sind, jeder dieser Reste durch Amino, Hydroxy, Cyano, Carboxyl, Nitro, Chlor, Brom, Fluor, Alkoxy und Alkylthio mit 1 bis 6 Kohlenstoffatomen, Mercapto, Perhalogenalkyl mit 1 bis 3 Kohlenstoffatomen, Guanidino, Amidino oder Sulfamoyl substituiert sein kann; und wobei A zusätzlich aus Cycloalkylalkylen, worin der Cycloalkylrest 3 bis 6 Kohlenstoffatome enthält, und der Alkylrest 1 bis 10 Kohlenstoffatome enthält; Alkylcycloalkylen, worin der Alkylrest 1 bis 6 Kohlenstoffatome enthält, und der Cycloalkylenrest 3 bis 6 Kohlenstoffatome enthält; Cycloalkenylalkylen, worin der Cycloalkenylrest 3 bis 10 Kohlenstoffatome, und der Alkylrest 1 bis 6 Kohlenstoffatome enthält; Naphthylen; Phenylalkylen und Alkylphenylen, worin das Alkyl 1 bis 6 Kohlenstoffatome enthält, ausgewählt sein kann; und wobei die punktierten Linien die Vorkehrung für cyclische Strukturen anzeigen, welche durch die Vereinigung des

angegebenen Stickstoffatoms und der verbindenden Gruppe A und durch die Vereinigung der angegebenen Stickstoffatome gebildet werden;
mit der Ausnahme solcher Verbindungen, worin:

a) $R^6$ H ist,
$R^7$ $CH_2OH$ ist,
$R^9$ $CH_3$ ist,
$R^{10}$ H ist, und $R^8$ 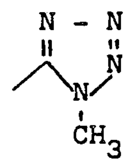 ist;

b) $R^6$ H ist,
$R^7$ 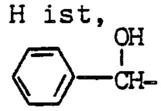 ist,
$R^9$ $CH_3$ ist,
$R^{10}$ 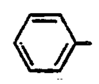 ist, und $R^8$ $CH_2CH_2C(=NH)-NH_2$ ist;

c) $R^6$ H ist,
$R^7$ $CH_3-CH(OH)-$ ist,
$R^9$ $CH_3$ ist,
$R^{10}$ $CH_3$ ist, und $R^8$ 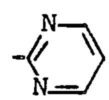 ist;

d) $R^6$ H ist,
$R^7$ $CH_3-CH(OH)-$ ist,
$R^9$ und $R^{10}$ unter Bildung eines Cyclopropylringes verbunden sind und
$R^8$ 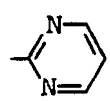 ist.

2. Eine Verbindung nach Anspruch 1, worin $R^1$ und $R^2$ unabhängig voneinander ausgewählt sind aus: Wasserstoff; substituiertem und unsubstituiertem: geradem und verzweigtem Alkyl mit 1 bis 6 Kohlenstoffatomen; Cycloalkyl mit 3 bis 6 Kohlenstoffatomen, Cycloalkylalkyl, wobei der Cycloalkylrest 3 bis 6 Kohlenstoffatome enthält, und der Alkylrest 1 bis 6 Kohlenstoffatome enthält; Phenyl, Benzyl; wobei der Substituent oder die Substituenten an den oben erwähnten Resten ausgewählt sind aus Fluor, Hydroxy, Mercapto, Alkoxy mit 1 bis 3 Kohlenstoffatomen; und Alkylthio mit 1 bis 3 Kohlenstoffatomen.

3. Eine Verbindung nach Anspruch 1, wobei die verbindende Gruppe A ausgewählt ist aus: einer einfachen, direkten Bindung, geradem und verzweigtem Niedrigalkylen mit 1 bis 6 Kohlenstoffatomen, Cycloalkylen mit 3 bis 6 Kohlenstoffatomen; Phenylen; Alkylheteroalkyl, wobei der Alkylrest 1 bis 3 Kohlenstoffatome enthält, und das Heteroatom oder die Heteroatome Schwefel, Sauerstoff und Stickstoff ist bzw. sind.

4. Eine Verbindung nach Anspruch 1, worin —$SR^8$ aus der Gruppe ausgewählt ist, die besteht aus:

$S-CH_2-C(=NH)-NH_2$

$S-CH_2-C(=NH)-NHCH_3$

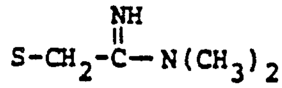

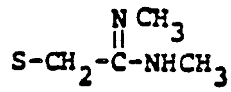

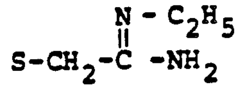

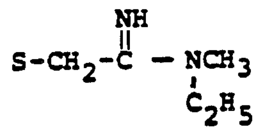

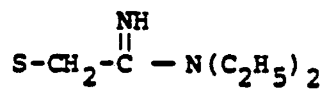

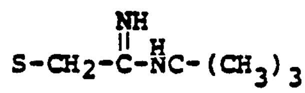

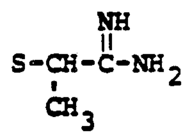

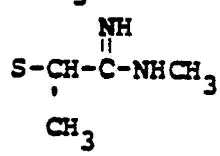

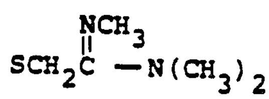

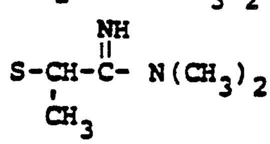

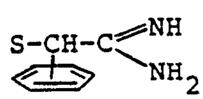

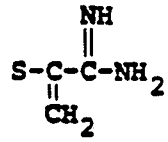

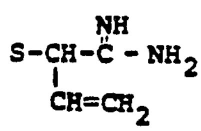

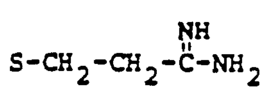

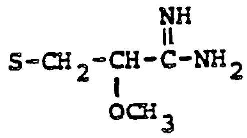

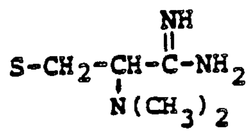

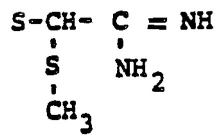

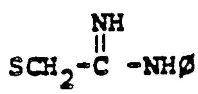

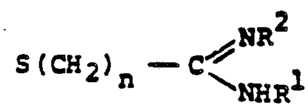

$n = 2\text{-}5$, $R^2 = H, CH_3$
$R^1 = H, CH_3$

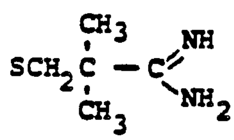

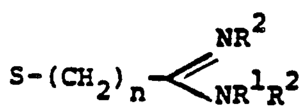

$n = 2\text{-}5$, $R^1, R^2 = H, CH_3$

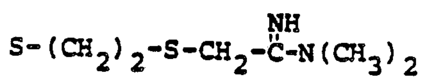

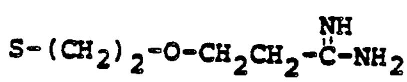

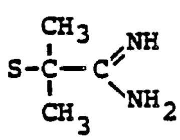

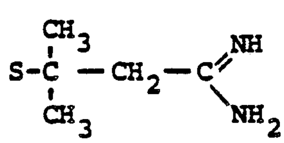

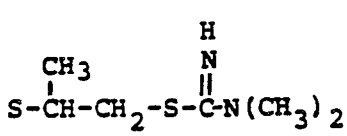

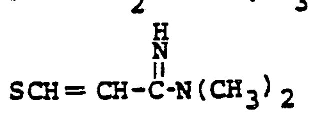

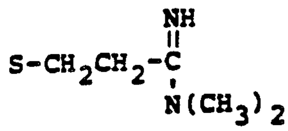

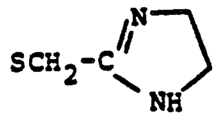

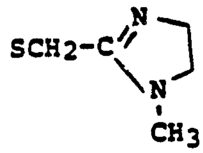

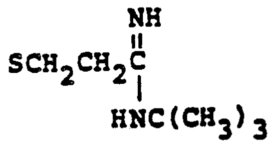

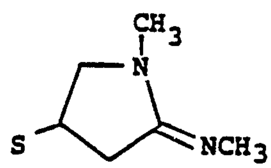

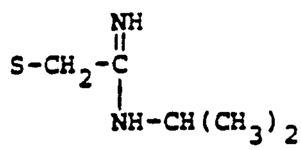

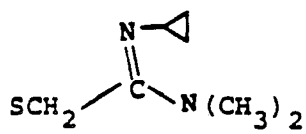

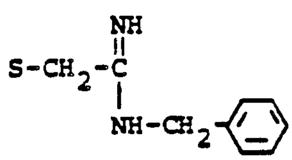

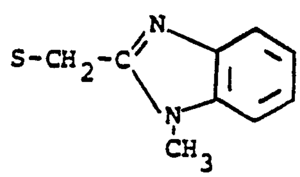

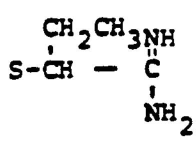

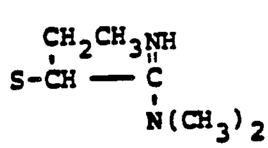

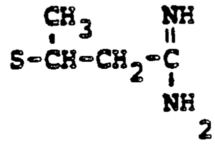

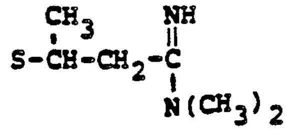

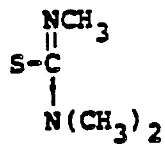

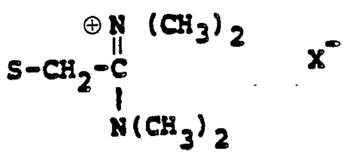

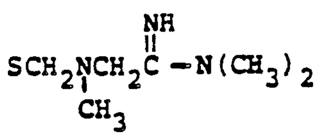

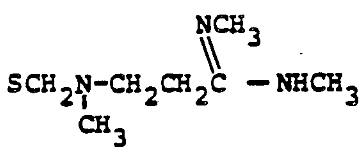

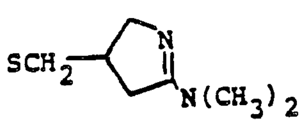

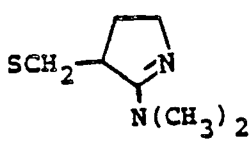

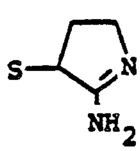

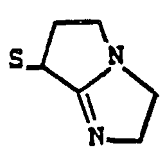

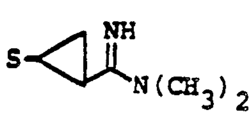

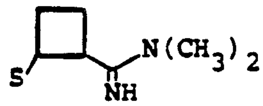

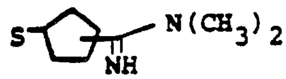

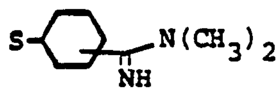

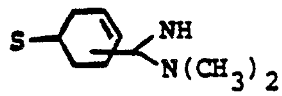

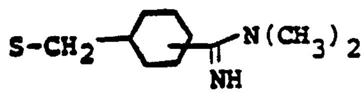

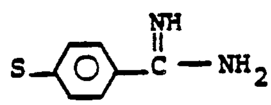

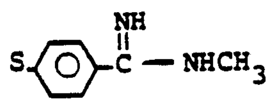

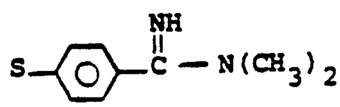

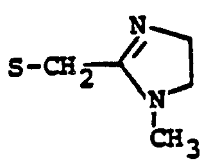

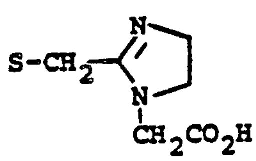

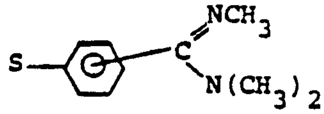

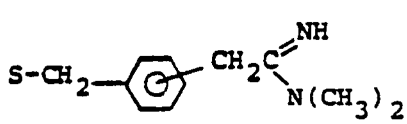

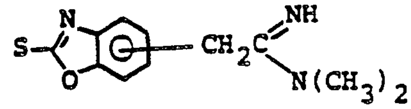

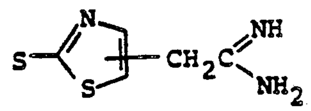

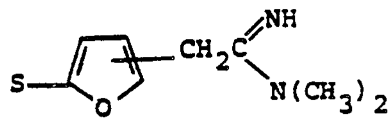

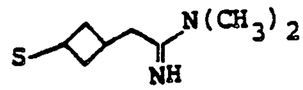

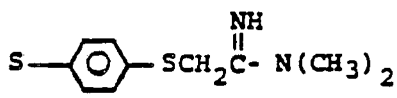

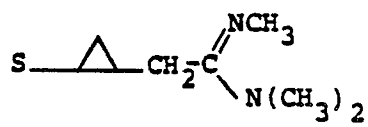

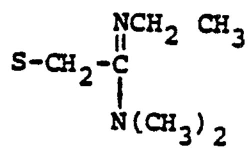

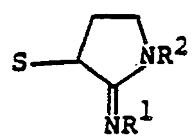

$R^1$ = H, CH3
$R^2$ = H, $CH_3$

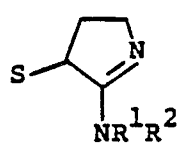

$R^1$ = H, $CH_3$
$R^2$ = H, $CH_3$

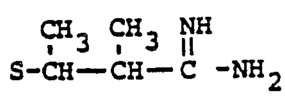

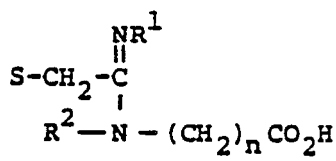

$R^1$ = H, $CH_3$
$R^2$ = H, $CH_3$
n = 1 oder 2

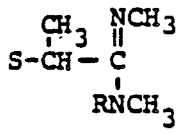

R = H, $CH_3$

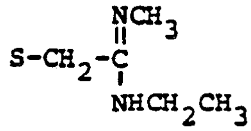

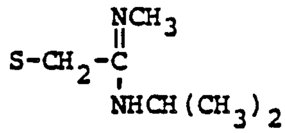

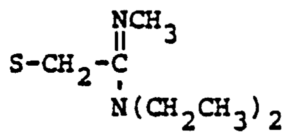

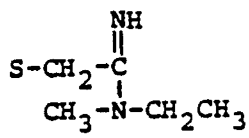

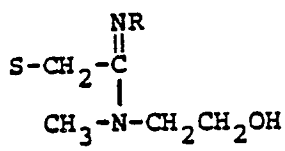

R = $CH_2CH_3$, $CH_3$

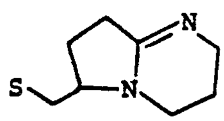

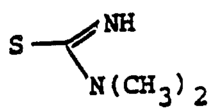

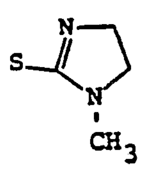

5. Eine Verbindung nach einem der Ansprüche 1, 2, 3 oder 4, worin $R^9$ und $R^{10}$ $C_{1-10}$-Alkyl sind.

6. Eine Verbindung nach Anspruch 5, worin $R^9$ und $R^{10}$ Methyl sind.

7. Eine Verbindung nach einem der Ansprüche 1, 2, 3 oder 4, worin $R^9$ und $R^{10}$ unabhängig voneinander aus der Gruppe ausgewählt sind, die besteht aus:

$CH_2CH_3$

$CH(CH_3)_2$

$CH_2C_6H_5$

$CH_2F$

$CF_3$

$CH_2Br$

$-CH=CH_2$

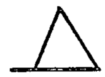

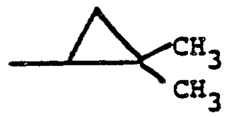

$CHF_2$

$CH_2Cl$

$CH_2OH$

$CH_2SMe$

$CH_2OMe$

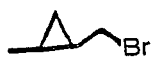

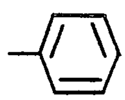

$-(CH_2)_n-$ $n = 2,3,4,5$

8. Eine Verbindung nach Anspruch 1, worin $R^7$ aus H, $OCH_3$ und $CH_3$ ausgewählt ist.

9. Eine Verbindung nach Anspruch 8, worin $R^6$ aus der Gruppe ausgewählt ist, die aus $C_{1-10}$-Alkyl, $C_{3-6}$-Cycloalkyl-$C_{1-6}$-alkyl, durch eine oder mehrere Hydroxylgruppen substituiertem $C_{1-10}$-Alkyl, oder durch eine oder mehrere Hydroxylgruppen substituiertem $C_{3-6}$-Cycloalkyl-$C_{1-6}$-alkyl besteht.

10. Eine Verbindung nach Anspruch 9, worin $R^7$ Wasserstoff ist.

11. Eine Verbindung nach einem der Ansprüche 1, 8 oder 9, worin $R^6$ ausgewählt ist aus:

OH<br>|<br>ØCH$_2$CH  Ø = Phenyl

OH<br>|<br>ØCH$_2$CH$_2$CH

OH<br>|<br>CH$_2$

ØCH$_2$

OH<br>|<br>(CH$_3$)$_2$C

$CH_2=CHCH_2$

$(CH_3)_2C=CHCH_2$

OH
$CH_3OCH_2CH$

$CH_3$

$CH_3CH_2$

$(CH_3)_2CH$

$HO_2CCH_2$

OH
$CF_3CF_2CH$

OH
$HO_2CCH_2CH$

OH
$CH_3CH(CH_3)CH$

OH
$(CH_3)_2CHCH_2CH$

OH
$CH_3CH_2CH$

$HOCH_2CH_2$

OH
$CF_3CH$

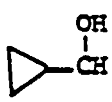

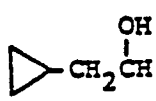

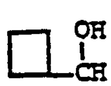

$HOCH_2CH_2CH_2$

OH
$F_2CHCH$

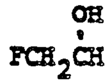

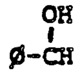

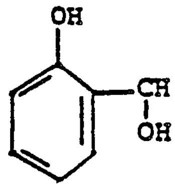

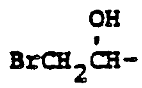

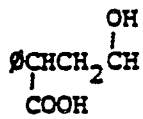

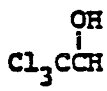

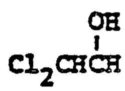

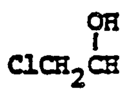

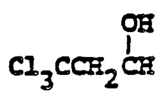

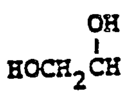

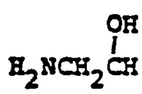

$-H$

$-CH(OH)CH_3$

$-CH_2CH_2CH_2CH_2OH$

$-CH(OH)CH_2CH_2OH$

$-CH(NH_2)CH_3$

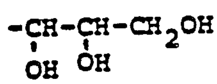

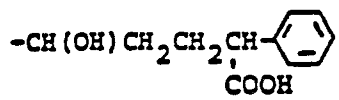

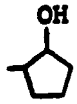

12. Eine Verbindung nach Anspruch 1, worin $R^7$ substituiertes Alkyl mit 1 bis 10 Kohlenstoffatomen ist, wobei der Substituent oder die Substituenten Alkoxyl mit 1 bis 6 Kohlenstoffatomen oder Hydroxyl ist bzw. sind.

13. Eine Verbindung nach Anspruch 1, wobei $R^7$ Wasserstoff, Hydroxyl, $OCH_3$, $CH_3$ oder Hydroxyl- bzw. Polyhydroxyl-substituiertes Alkyl mit 1 bis 10 Kohlenstoffatomen ist.

14. Eine Verbindung nach Anspruch 8, worin $R^6$ Wasserstoff; oder substituiertes bzw. unsubstituiertes: $C_{1-10}$-Alkyl, $C_{3-6}$-Cycloalkyl, $C_{3-6}$-Cycloalkyl-$C_{1-6}$-alkyl oder Phenyl-$C_{1-6}$-alkyl ist; wobei der Substituent oder die Substituenten Hydroxyl ist bzw. sind.

15. Ein Verfahren zur Herstellung einer Verbindung von Anspruch 1, umfassend die Stufe der Behandlung von:

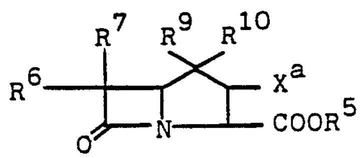

mit $HSR^8$; worin $X^a$ eine austretende Gruppe ist, $R^5$ eine Schutzgruppe ist, und $R^6$, $R^7$, $R^9$ und $R^{10}$ wie in Anspruch 1 definiert sind.

16. Eine antibiotische Zusammensetzung, enthaltend eine therapeutisch wirksame Menge einer verbindung nach Anspruch 1 und einen pharmazeutisch wirksamen Träger dafür.

**Patentansprüche fur den Vertragsstaat: AT**

1. Ein Verfahren zur Herstellung einer Verbindung mit der Strukturformel:

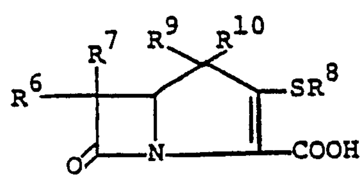

und pharmazeutisch annehmbarer Salz-, Ester- und Amidderivate davon, worin:

$R^6$ und $R^7$ sowie $R^9$ und $R^{10}$ unabhängig voneinander Wasserstoff ($R^9$ und $R^{10}$ sind nicht beide Wasserstoff) sind oder aus der Gruppe ausgewählt sind, die besteht aus substituiertem und unsubstituiertem: Alkyl, Alkenyl und Alkinyl mit 1—10 Kohlenstoffatomen; Cycloalkyl, Cycloalkylalkyl und Alkylcycloalkyl mit 3—6 Kohlenstoffatomen im Cycloalkylring und 1—6 Kohlenstoffatomen im Alkylrest; Phenyl, Phenylalkyl, Phenylalkenyl und Phenylalkinyl, wobei der aliphatische Teil 1—6 Kohlenstoffatome aufweist; oder $R^6$ und $R^7$ unabhängig voneinander Pyridyl sind, oder $R^6$ und $R^7$ sowie $R^9$ und $R^{10}$ unter Bildung eines cyclischen Alkyls verbunden sein können, das gemeinsam mit dem Kohlenstoffatom, an das die Reste gebunden sind, 3—6 Kohlenstoffatome aufweist; wobei der Substituent oder die Substituenten an den Resten $R^6$, $R^7$, $R^9$ oder $R^{10}$ unabhängig voneinander ausgewählt sind aus Chlor, Fluor, Hydroxy, Brom, Alkylthio mit 1—6 Kohlenstoffatomen und Alkoxy mit 1—6 Kohlenstoffatomen; und wobei $R^7$ zusätzlich Hydroxyl, Methoxyl, Mercapto, Chlor, Fluor und Brom sein kann; und

falls $R^7$ Wasserstoff, $CH_3$ oder $OCH_3$ ist, $R^6$ zusätzlich Carboxyl, Hydroxyimino, Ureido oder Amino als Substituenten an den oben erwähnten Alkyl-, Cycloalkyl-, Cycloalkylalkyl- und Phenylalkylgruppen aufweisen kann;

und $R^8$ Carbamimidoyl, ausgewählt aus der Gruppe bestehend aus:

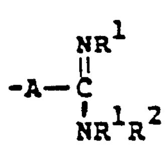 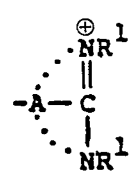 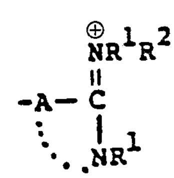

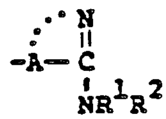 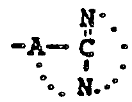 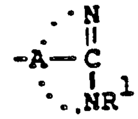

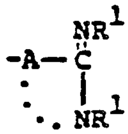 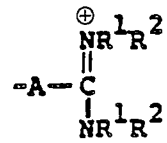 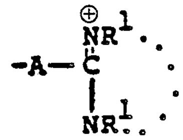

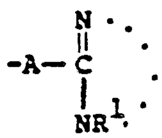 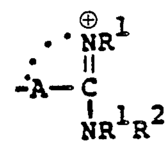 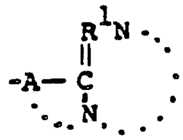

wobei: A eine einfache, direkte Bindung ist, oder A als cyclisches oder acyclisches Bindeglied aus der Gruppe ausgewählt ist, die aus Alkylen, Alkenylen und Alkinylen mit 1—10 Kohlenstoffatomen besteht, welche durch ein Heteroatom, ausgewählt aus O, S oder N, oder durch Phenylen, Cycloalkylen, Cycloalkenylen, Heterocyclylen oder Heteroarylen unterbrochen sein können, wobei solche cyclischen Unterbrechungen 3—6 Ringatome, ausgewählt aus C, O, S und N, enthalten; Cycloalkylen mit 3—10 Kohlenstoffatomen; Cycloalkenylen mit 3—6 Kohlenstoffatomen; und Phenylen ist;

$R^1$ und $R^2$ unabhängig voneinander aus Wasserstoff und den vorher definierten, jedoch einwertigen Bedeutungen für die Gruppe A ausgewählt sind; Cycloalkylalkyl, worin der Cycloalkylrest 3 bis 6 Kohlenstoffatome enthält, und der Alkylrest 1 bis 6 Kohlenstoffatome enthält; Alkylcycloalkyl, wobei der Alkylrest 1 bis 6 Kohlenstoffatome enthält, und der Cycloalkylrest 3 bis 6 Kohlenstoffatome enthält; oder Phenylalkyl mit 7—10 Kohlenstoffatomen sind; wobei, falls $R^1$ und $R^2$ unabhängig voneinander aus geradem und verzweigtem Alkyl mit 1 bis 6 Kohlenstoffatomen; Cycloalkyl mit 3 bis 6 Kohlenstoffatomen; Phenyl; oder einer der oben definierten Cycloalkylalkyl-, Alkylcycloalkyl- und Phenylalkylgruppen ausgewählt sind, jeder dieser Reste durch Amino, Hydroxy, Cyano, Carboxyl, Nitro, Chlor, Brom, Fluor, Alkoxy und Alkylthio mit 1 bis 6 Kohlenstoffatomen, Mercapto, Perhalogenalkyl mit 1 bis 3 Kohlenstoffatomen, Guanidino, Amidino oder Sulfamoyl substituiert sein kann; und wobei A zusätzlich aus Cycloalkylalkylen, worin der Cycloalkylrest 3 bis 6 Kohlenstoffatome enthält, und der Alkylrest 1 bis 10 Kohlenstoffatome enthält; Alkylcycloalkylen, worin der Alkylrest 1 bis 6 Kohlenstoffatome enthält, und der Cycloalkylenrest 3 bis 6 Kohlenstoffatome enthält; Cycloalkenylalkylen, worin der Cycloalkenylrest 3 bis 10 Kohlenstoffatome, und der Alkylrest 1 bis 6 Kohlenstoffatome enthält; Naphthylen; Phenylalkylen und Alkylphenylen, worin das Alkyl 1 bis 6 Kohlenstoffatome enthält, ausgewählt sein kann; und wobei die punktierten Linien die Vorkehrung für cyclische Strukturen anzeigen, welche durch die Vereinigung des angegebenen Stickstoffatoms und der verbindenden Gruppe A und durch die Vereinigung der angegebenen Stickstoffatome gebildet werden;

mit der Ausnahme solcher Verbindungen, worin:

a) $R^6$ H ist,
$R^7$ $CH_2OH$ ist,
$R^9$ $CH_3$ ist,
$R^{10}$ H ist, und $R^8$ 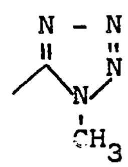 ist;

b) $R^6$ H ist,
$R^7$ 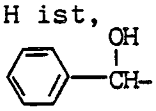 ist,
$R^9$ $CH_3$ ist,
$R^{10}$ 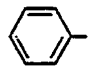 ist, und $R^8$ $CH_2CH_2C(=NH)-NH_2$ ist;

c) $R^6$ H ist,

$R^7$ $CH_3\text{-}\overset{OH}{CH}\text{-}$ ist,

$R^9$ $CH_3$ ist,

$R^{10}$ $CH_3$ ist, und $R^8$ 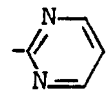 ist;

d) $R^6$ H ist,

$R^7$ $CH_3\text{-}\overset{OH}{CH}\text{-}$ ist,

$R^9$ und $R^{10}$ unter Bildung eines Cyclopropylringes verbunden sind und

$R^8$ 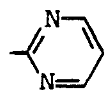 ist.

umfassend die Stufe der Behandlung von

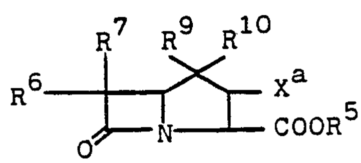

mit $HSR^8$; worin $X^a$ eine austretende Gruppe ist, $R^5$ eine Schutzgruppe ist, und $R^6$, $R^7$, $R^9$ und $R^{10}$ wie oben definiert sind.

2. Ein Verfahren nach Anspruch 1, worin $R^1$ und $R^2$ unabhängig voneinander ausgewählt sind aus: Wasserstoff; substituiertem und unsubstituiertem: geradem und verzweigtem Alkyl mit 1 bis 6 Kohlenstoffatomen; Cycloalkyl mit 3 bis 6 Kohlenstoffatomen, Cycloalkylalkyl, wobei der Cycloalkylrest 3 bis 6 Kohlenstoffatome enthält, und der Alkylrest 1 bis 6 Kohlenstoffatome enthält; Phenyl, Benzyl; wobei der Substituent oder die Substituenten an den oben erwähnten Resten ausgewählt sind aus Fluor, Hydroxy, Mercapto, Alkoxy mit 1 bis 3 Kohlenstoffatomen; und Alkylthio mit 1 bis 3 Kohlenstoffatomen.

3. Ein Verfahren nach Anspruch 1, wobei die verbindende Gruppe A ausgewählt ist aus: einer einfachen, direkten Bindung, geradem und verzweigtem Niedrigalkylen mit 1 bis 6 Kohlenstoffatomen, Cycloalkylen mit 3 bis 6 Kohlenstoffatomen; Phenylen; Alkylheteroalkyl, wobei der Alkylrest 1 bis 3 Kohlenstoffatome enthält, und das Heteroatom oder die Heteroatome Schwefel, Sauerstoff und Stickstoff ist bzw. sind.

4. Ein Verfahren nach Anspruch 1, worin $—SR^8$ aus der Gruppe ausgewählt ist, die besteht aus:

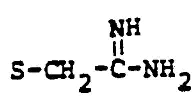

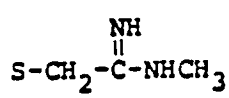

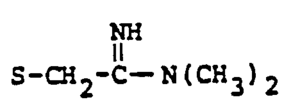

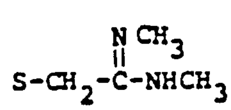

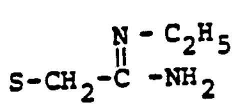

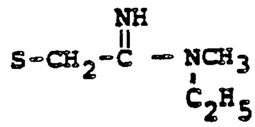

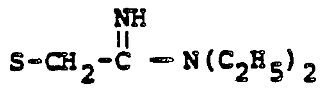

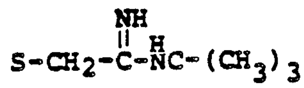

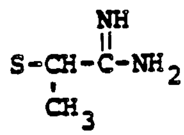

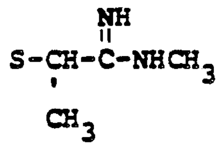

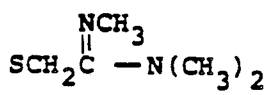

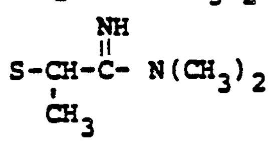

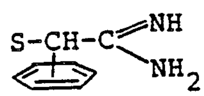

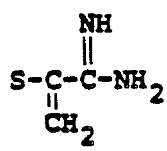

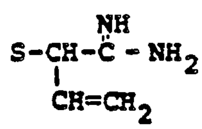

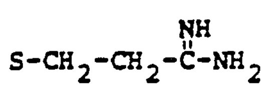

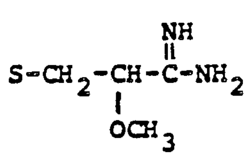

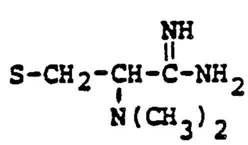

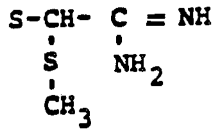

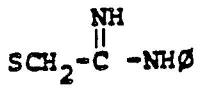

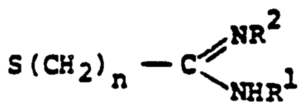

$n = 2\text{-}5$, $R^2 = H, CH_3$

$R^1 = H, CH_3$

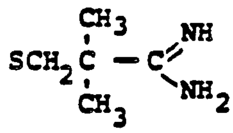

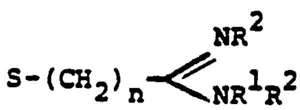

$n = 2\ \text{-}5$, $R^1, R^2 = H, CH_3$

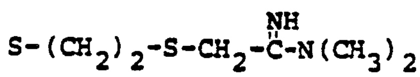

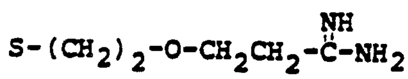

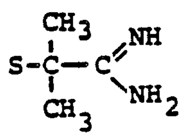

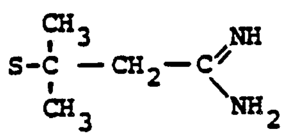

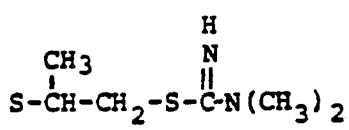

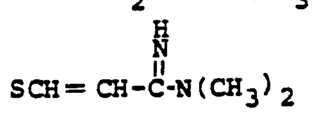

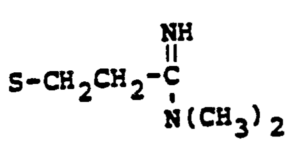

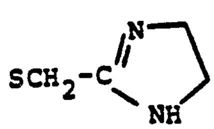

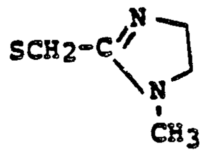

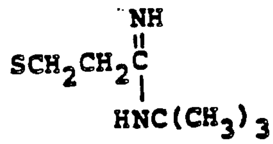

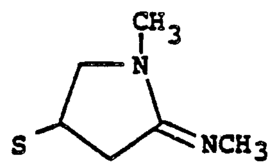

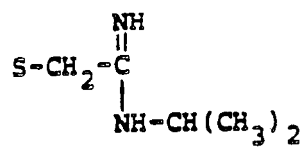

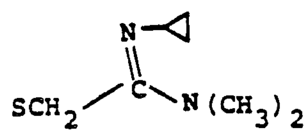

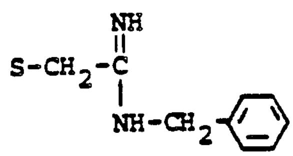

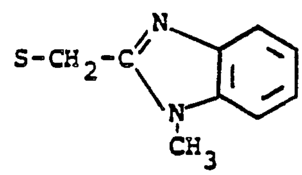

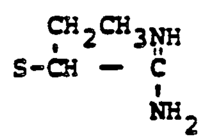

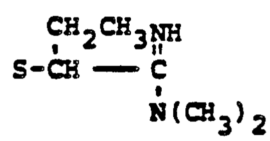

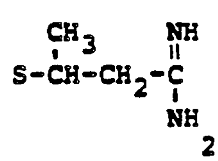

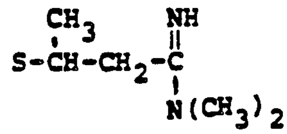

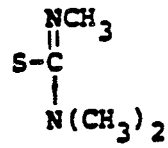

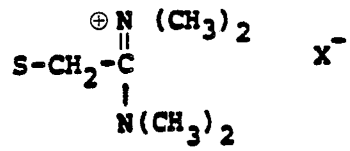

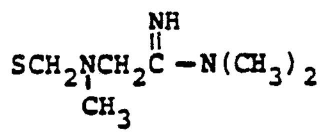

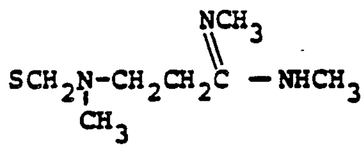

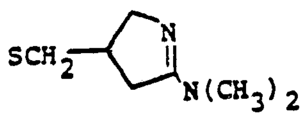

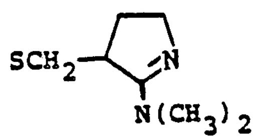

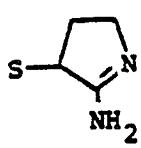

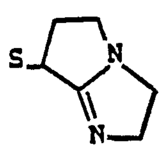

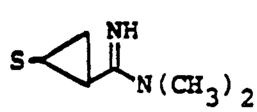

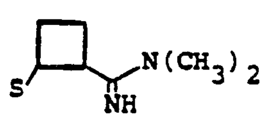

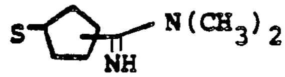

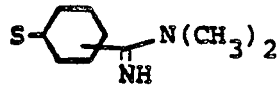

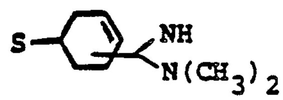

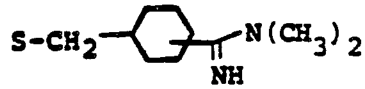

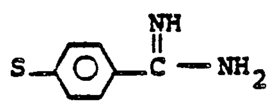

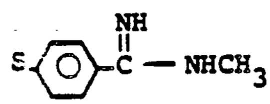

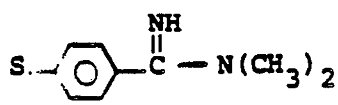

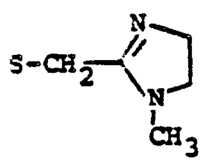

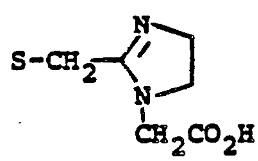

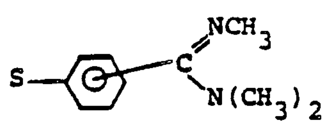

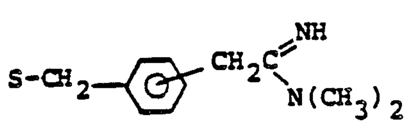

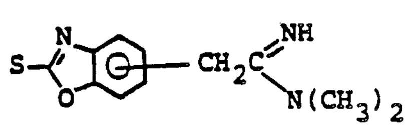

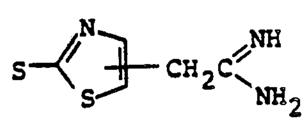

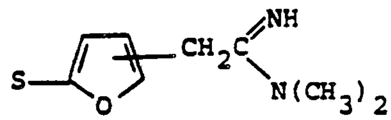

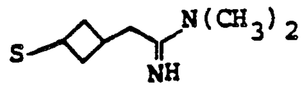

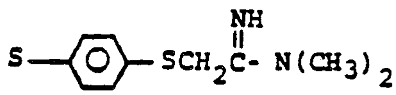

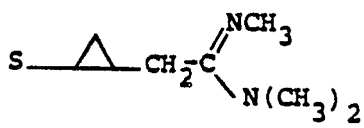

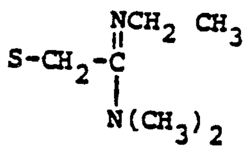

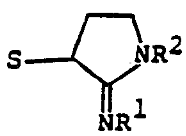

$R^1$ = H, CH3
$R^2$ = H, $CH_3$

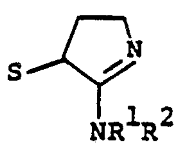

$R^1$ = H, $CH_3$
$R^2$ = H, $CH_3$

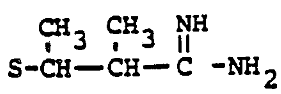

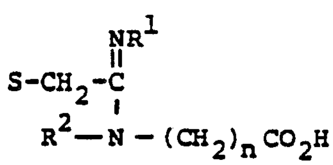

$R^1$ = H, $CH_3$
$R^2$ = H, $CH_3$
n = 1 oder 2

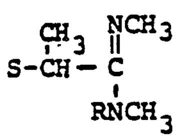

R = H, $CH_3$

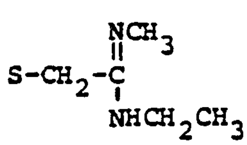

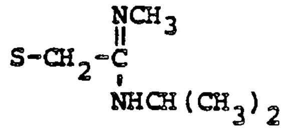

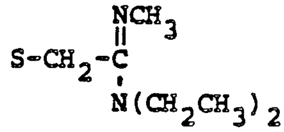

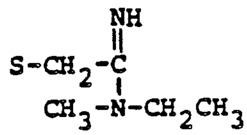

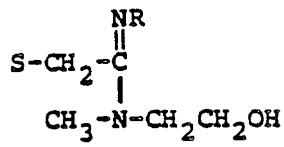

R = $CH_2CH_3$, $CH_3$

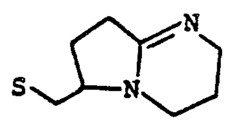

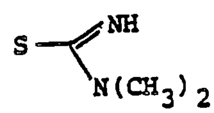

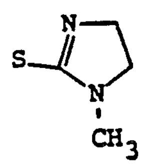

5. Ein Verfahren nach einem der Ansprüche 1, 2, 3 oder 4, worin $R^9$ und $R^{10}$ $C_{1-10}$-Alkyl sind.

6. Ein Verfahren nach Anspruch 5, worin $R^9$ und $R^{10}$ Methyl sind.

7. Ein Verfahren nach einem der Ansprüche 1, 2, 3 oder 4, worin $R^9$ und $R^{10}$ unabhängig voneinander aus der Gruppe ausgewählt sind, die besteht aus:

$CH_2CH_3$
$CH(CH_3)_2$
$CH_2C_6H_5$
$CH_2F$
$CF_3$
$CH_2Br$
$-CH=CH_2$

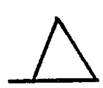

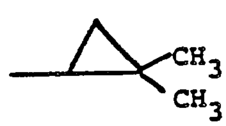

$CHF_2$

$CH_2Cl$

$CH_2OH$

$CH_2SMe$

$CH_2OMe$

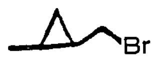

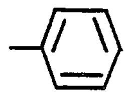

$-(CH_2)_n-$ $n = 2,3,4,5$

8. Ein Verfahren nach Anspruch 1, worin $R^7$ aus H, $OCH_3$ und $CH_3$ ausgewählt ist.

9. Ein Verfahren nach Anspruch 8, worin $R^6$ aus der Gruppe ausgewählt ist, die aus $C_{1-10}$-Alkyl, $C_{3-6}$-Cycloalkyl-$C_{1-6}$-alkyl, durch eine oder mehrere Hydroxylgruppen substituiertem $C_{1-10}$-Alkyl, oder durch eine oder mehrere Hydroxylgruppen substituiertem $C_{3-6}$-Cycloalkyl-$C_{1-6}$-alkyl besteht.

10. Ein Verfahren nach Anspruch 9, worin $R^7$ Wasserstoff ist.

11. Ein Verfahren nach einem der Ansprüche 1, 8 oder 9, worin $R^6$ ausgewählt ist aus:

$ØCH_2CH(OH)$ Ø = Phenyl

$ØCH_2CH_2CH(OH)$

OH
|
$CH_2$
——
$ØCH_2$

$(CH_3)_2C(OH)$

$CH_2=CHCH_2$

$(CH_3)_2C=CHCH_2$

$CH_3OCH_2CH(OH)$

$CH_3$

$CH_3CH_2$

$(CH_3)_2CH$

$HO_2CCH_2$

OH
$CF_3CF_2CH$

OH
$HO_2CCH_2CH$

OH
$CH_3CH(CH_3)CH$

OH
$(CH_3)_2CHCH_2CH$

OH
$CH_3CH_2CH$

$HOCH_2CH_2$

OH
$CF_3CH$

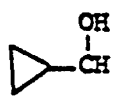

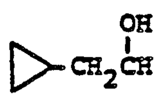

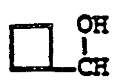

$HOCH_2CH_2CH_2$

OH
$F_2CHCH$

OH
$FCH_2CH$

OH
ø-CH

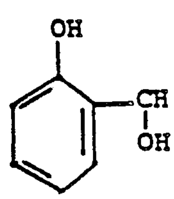

OH

$BrCH_2CH-$

OH

$ØCHCH_2CH$

COOH

OH

$Cl_3CCH$

OH

$Cl_2CHCH$

OH

$ClCH_2CH$

OH

$Cl_3CCH_2CH$

OH

$HOCH_2CH$

OH

$H_2NCH_2CH$

$-H$

$-CH(OH)CH_3$

$-CH_2CH_2CH_2CH_2OH$

$-CH(OH)CH_2CH_2OH$

$-CH(NH_2)CH_3$

$-CH-CH-CH_2OH$

OH OH

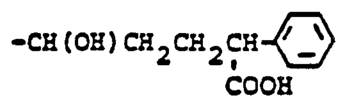

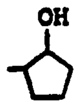

12. Ein Verfahren nach Anspruch 1, worin $R^7$ substituiertes Alkyl mit 1 bis 10 Kohlenstoffatomen ist, wobei der Substituent oder die Substituenten Alkoxyl mit 1 bis 6 Kohlenstoffatomen oder Hydroxyl ist bzw. sind.

13. Ein Verfahren nach Anspruch 1, wobei $R^7$ Wasserstoff, Hydroxyl, $OCH_3$, $CH_3$ oder Hydroxyl- bzw. Polyhydroxyl-substituiertes Alkyl mit 1 bis 10 Kohlenstoffatomen ist.

14. Ein Verfahren nach Anspruch 8, worin $R^6$ Wasserstoff; oder substituiertes bzw. unsubstituiertes: $C_{1-10}$-Alkyl, $C_{3-6}$-Cycloalkyl, $C_{3-6}$-Cycloalkyl-$C_{1-6}$-alkyl oder Phenyl-$C_{1-6}$-alkyl ist; wobei der Substituent oder die Substituenten Hydroxyl ist bzw. sind.

**Revendications pour les Etats contractants: BE CH DE FR GB IT LI LU NL SE**

1. Composé répondant à la formule développée:

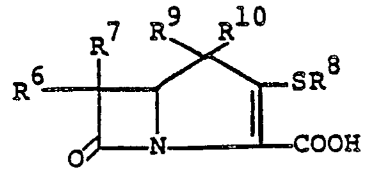

et les convenant en pharmacie, esters et amides qui en dérivent; où:

$R^6$ et $R^7$, et $R^9$ et $R^{10}$ sont indépendamment un hydrogène ($R^9$ et $R^{10}$ ne sont pas tous deux un hydrogène) ou sont choisis dans le groupe constitué par les formes substituées et non substituées de: alkyle, alcényle et alcynyle ayant 1 à 10 atomes de carbone; cycloalkyle, cycloalkylalkyle et alkylcycloalkyle ayant 3 à 6 atomes de carbone dans le cycle cycloalkyle et 1 à 6 atomes de carbone dans le fragment alkyle; phényle, phénylalkyle, phénylalcényle et phénylalcynyle dont la portion aliphatique a 1 à 6 atomes de carbone; ou $R^6$ et $R^7$ sont indépendamment un pyridyle ou $R^6$ et $R^7$, et $R^9$ et $R^{10}$ peuvent être unis pour former un cycloalkyle ayant, avec l'atome de carbone auquel ils sont fixés, 3 à 6 atomes de carbone; où le substituant ou les substituants sur $R^6$, $R^7$, $R^9$ ou $R^{10}$ sont indépendamment choisis parmi chloro, fluoro, hydroxy, bromo, alkylthio ayant 1 à 6 atomes de carbone et alcoxy ayant 1 à 6 atomes de carbone; et où $R^7$ peut de plus être un hydroxxy, un méthoxy, un mercapto, un chloro, un fluoro et un bromo; et lorsque $R^7$ est un hydrogène, $CH_3$ ou $OCH_3$, $R^6$ peut de plus avoir un carboxy, un hydroximino, un uréido ou un amino comme substituant sur les groupes alkyle, cycloalkyle, cycloalkylalkyle et phénylalkyle précités; et

$R^8$ est un carbamimidoyle choisi dans le groupe constitué par:

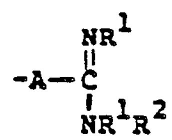
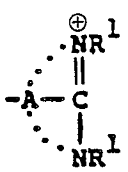
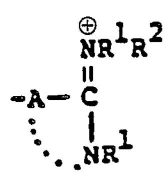
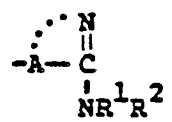
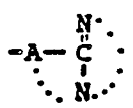
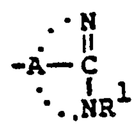
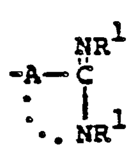
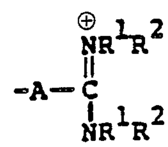
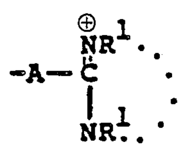
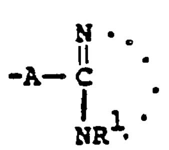
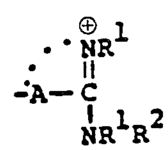
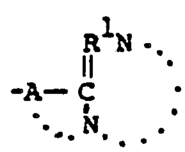

où: A est une simple liaison directe ou A, le connecteur cyclique ou acyclique, est choisi dans le groupe constitué par alkylène, alcénylène et alcynylène ayant 1 à 10 atomes de carbone pouvant être interrompu par un hétéroatome choisi parmi O, S ou N, ou par un phénylène, cycloalkylène, cycloalcénylène, hétérocyclylène ou hétéroarylène, où ces interruptions cycliques comprennent 3 à 6 atomes cycliques choisis parmi C, O, S et N; cycloalkylène ayant 3 à 10 atomes de carbone; cycloalcénylène ayant 3 à 6 atomes de carbone; et phénylène;

$R^1$ et $R^2$ sont indépendamment choisis parmi un hydrogène et les valeurs précédemment définies pour le groupe A mais monovalentes; un cycloalkylalkyle dont le fragment cycloalkyle comprend 3 à 6 atomes de carbone et le fragment alkyle comprend 1 à 6 atomes de carbone; un alkylcycloalkyle dont le fragment alkyle comprend 1 à 6 atomes de carbone et le fragment cycloalkyle comprend 3 à 6 atomes de carbone; un phénylalkyle ayant 7 à 10 atomes de carbone; où, lorsque $R^1$ et $R^2$ sont indépendamment choisis parmi un

alkyle droit ou ramifié ayant 1 à 6 atomes de carbone; un cycloalkyle ayant 3 à 6 atomes de carbone; un phényle; ou un des groupes cycloalkylalkyle, alkylcycloalkyle et phénylalkyle definis ci-dessus, chacun de ces radicaux peut être substitué par des groupes amino, hydroxy, cyano, carboxy, nitro, chloro, bromo, fluoro, alcoxy et alkylthio ayant 1 à 6 atomes de carbone, mercapto, perhalogénoalkyle ayant 1 à 3 atomes de carbone, guanidino, amidino ou sulfamoyle;

et où A peut de plus être choisi parmi un cycloalkylalkylène dont le fragment cycloalkyle comprend 3 à 6 atomes de carbone et le fragment alkylène 1 à 10 atomes de carbone; un alkylcycloalkylène dont le fragment alkyle comprend 1 à 6 atomes de carbone et le fragment cycloalkylène comprend 3 à 6 atomes de carbone; un cycloalcénylalkylène dont le fragment cycloalcényle comprend 3 à 10 atomes de carbone et le fragment alkylène comprend 1 à 6 atomes de carbone; un naphtylène; un phénylalkylène et un alkylphénylène dont le fragment alkyle à 1 à 6 atomes de carbone;

et où les pointillés indiquent la possibilité de structures cycliques formées par l'union de l'atome d'azote indiqué et du groupe connecteur A et par l'union des atomes d'azote indiqués; à l'exception des composés dans lesquels:

a) $R^6$ est H et

$R^7$ est $CH_2OH$ $R^8$ est 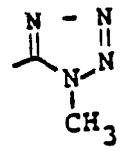

$R^9$ est $CH_3$

$R^{10}$ est H

b) $R^6$ est H

$R^7$ est 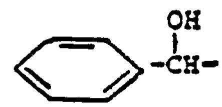 et

$R^9$ est $CH_3$ $R^8$ est $CH_2CH_2C(=NH)-NH_2$

$R^{10}$ est 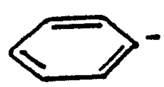

c) $R^6$ est H,

$R^7$ est $CH_3-CH(OH)-$ et

$R^9$ est $CH_3$ $R^8$ est 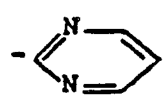

$R^{10}$ est $CH_3$

d) $R^6$ est H

$R^7$ est $CH_3-CH(OH)-$

$R^9$ et $R^{10}$ sont réunis pour former un cycle cyclo

et

$R^8$ est 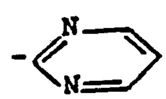

2. Composé selon la revendication 1, où $R^1$ et $R^2$ sont indépendamment choisis parmi: un hydrogène; les formes substituées et non substituées des radicaux suivants: alkyle droit ou ramifié ayant 1 à 6 atomes de carbone; cycloalkyle ayant 3 à 6 atomes de carbone, cycloalkylalkyle dont le fragment cycloalkyle comprend 3 à 6 atomes de carbone et le fragment alkyle comprend 1 à 6 atomes de carbone; phényle, benzyle; où le substituant ou les substituants des radicaux précités sont choisis parmi fluoro, hydroxy, mercapto, alcoxy ayant 1 à 3 atomes de carbone; et alkylthio ayant 1 à 3 atomes de carbone.

3. Composé selon la revendication 1, où le groupe connecteur A est choisi parmi: une simple liaison unique, un alkylène inférieur droit ou ramifié ayant 1 à 6 atomes de carbone, un cycloalkylène ayant 3 à 6

atomes de carbone; un phénylène; un alkylhétéroalkyle dont le fragment alkyle comprend 1 à 3 atomes de carbone et ou les hétéroatomes sont le soufre, l'oxygène et l'azote.

4. Composé selon la revendication 1, où —$SR^8$ est choisi dans le groupe constitué par

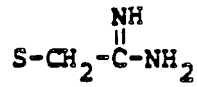

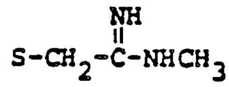

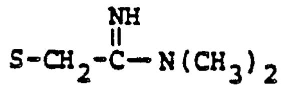

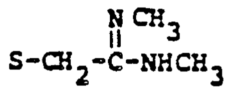

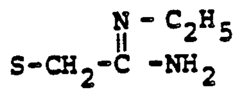

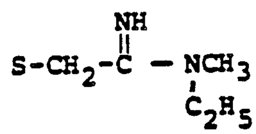

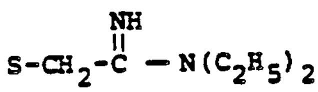

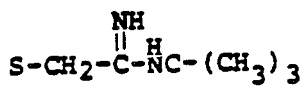

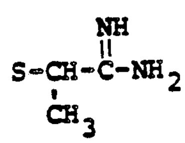

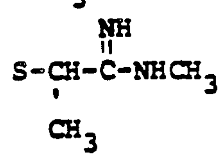

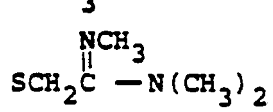

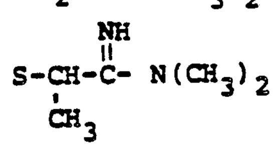

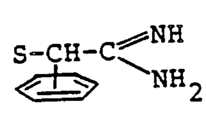

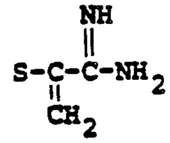

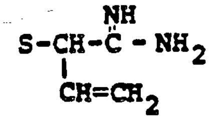

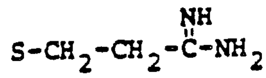

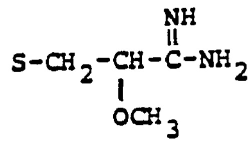

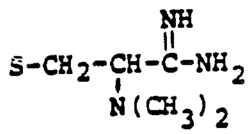

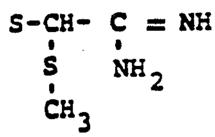

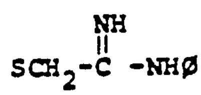

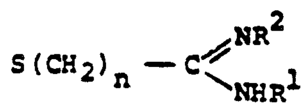

$n = 2\text{-}5$, $R^2 = H, CH_3$

$R^1 = H, CH_3$

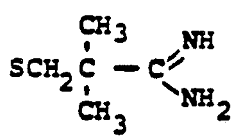

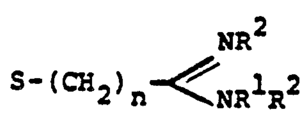

$n = 2\text{-}5$, $R^1, R^2 = H, CH_3$

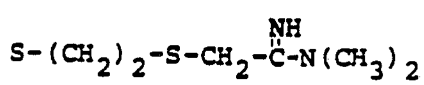

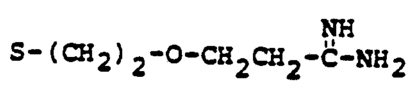

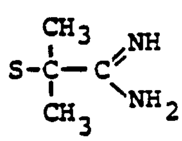

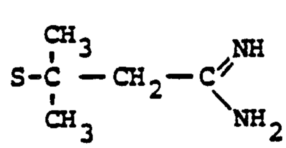

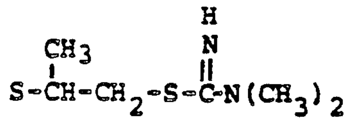

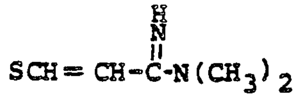

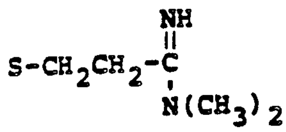

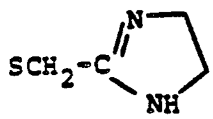

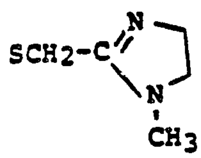

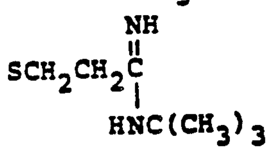

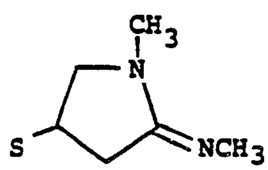

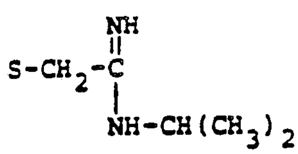

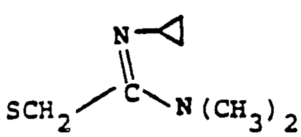

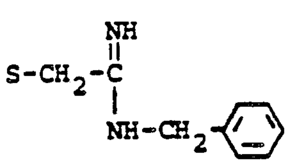

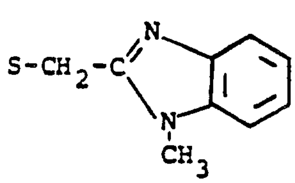

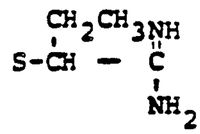

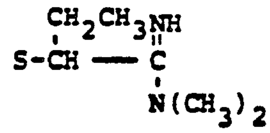

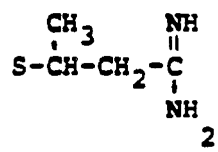

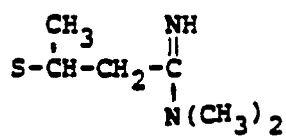

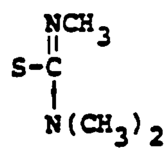

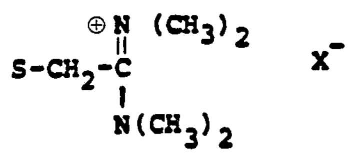

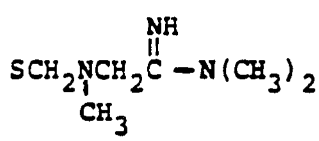

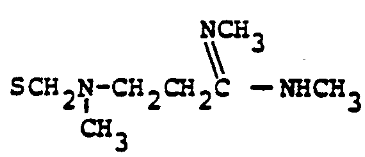

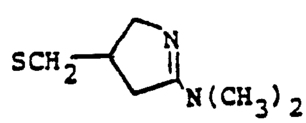

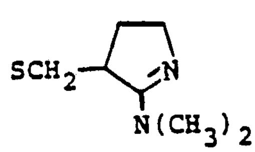

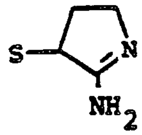

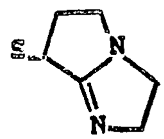

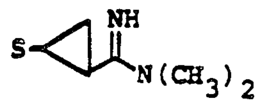

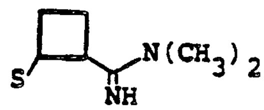

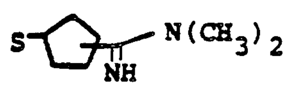

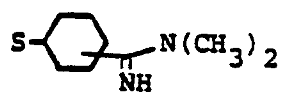

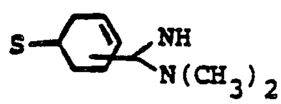

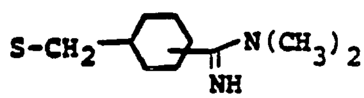

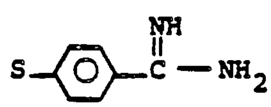

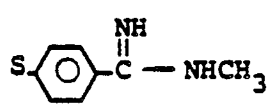

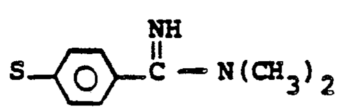

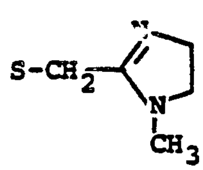

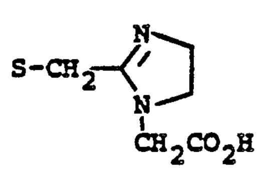

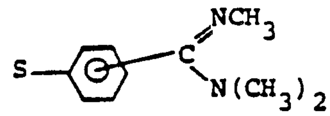

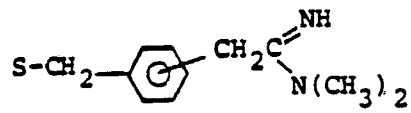

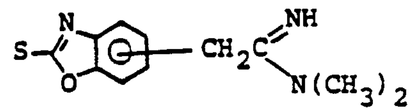

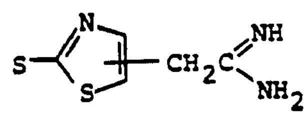

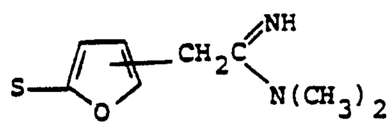

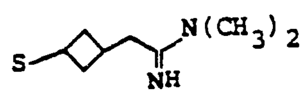

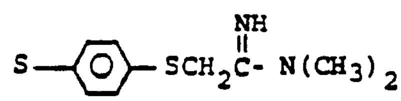

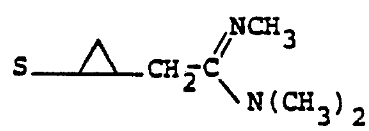

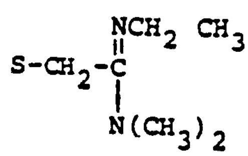

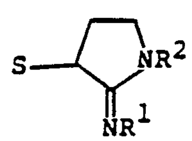

$R^1$ = H, CH3
$R^2$ = H, $CH_3$

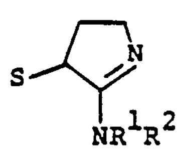

$R^1$ = H, $CH_3$
$R^2$ = H, $CH_3$

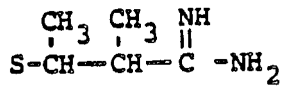

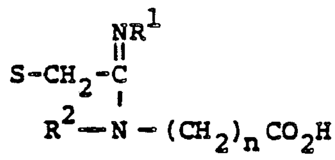

$R^1$ = H, $CH_3$
$R^2$ = H, $CH_3$
n = 1 ou 2

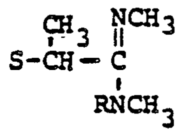

R = H, $CH_3$

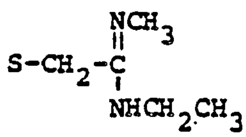

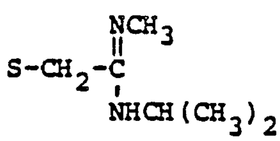

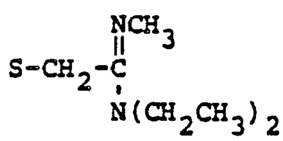

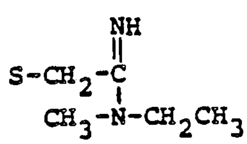

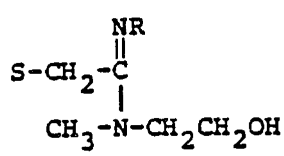

R = $CH_2CH_3$, $CH_3$

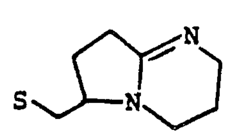

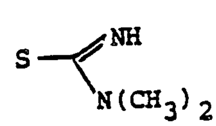

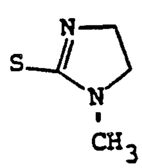

5. Composé selon la revendication 1, 2, 3 ou 4, où $R^9$ et $R^{10}$ sont un alkyle en $C_{1-10}$.

6. Composé selon la revendication 5, où $R^9$ et $R^{10}$ sont un méthyle

7. Composé selon la revendication 1, 2, 3 ou 4 où $R^9$ et $R^{10}$ sont indépendamment choisis dans le groupe constitué par:

$CH_2CH_3$

$CH(CH_3)_2$

$CH_2C_6H_5$

$CH_2F$

$CF_3$

$CH_2Br$

$-CH{=}CH_2$

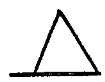

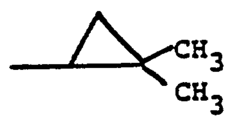

$CHF_2$

$CH_2Cl$

$CH_2OH$

$CH_2SMe$

$CH_2OMe$

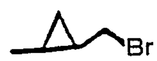

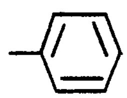

$-(CH_2)_n-$ n = 2,3,4,5

8. Composé selon la revendication 1, où $R^7$ est choisi parmi H, $OCH_3$ et $CH_3$.

9. Composé selon la revendication 8, où $R^6$ est choisi dans le groupe constitué par un alkyle en $C_{1-10}$, un cycloalkyl($C_{3-6}$)alkyle en $C_{1-6}$, un alkyle en $C_{1-10}$ substitué par un ou plusieurs groupes hydroxy ou un cycloalkyl($C_{3-6}$)alkyle en $C_{1-6}$ substitué par un ou plusieurs groupes hydroxy.

10. Composé selon la revendication 9, où $R^7$ est un hydrogène.

11. Composé selon la revendication 1, 8 ou 9, où $R^6$ est choisi parmi:

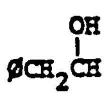 Ø = phényl

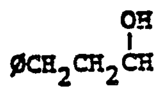

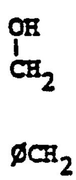

OH
$(CH_3)_2C$

$CH_2=CHCH_2$

$(CH_3)_2C=CHCH_2$

OH
$CH_3OCH_2CH$

$CH_3$

$CH_3CH_2$

$(CH_3)_2CH$

$HO_2CCH_2$

OH
$CF_3CF_2CH$

OH
$HO_2CCH_2CH$

OH
$CH_3CH(CH_3)CH$

OH
$(CH_3)_2CHCH_2CH$

OH
$CH_3CH_2CH$

$HOCH_2CH_2$

OH
$CF_3CH$

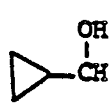

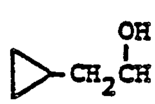

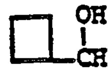

$HOCH_2CH_2CH_2$

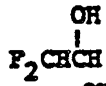

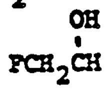

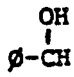

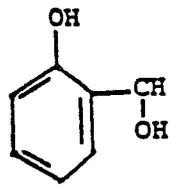

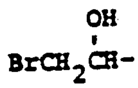

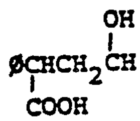

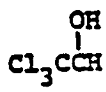

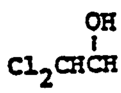

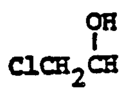

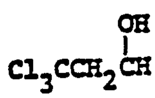

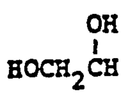

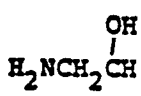

$-H$

$-CH(OH)CH_3$

$-CH_2CH_2CH_2CH_2OH$

$-CH(OH)CH_2CH_2OH$

$-CH(NH_2)CH_3$

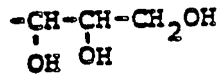

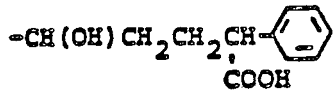

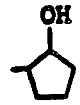

12. Composé selon la revendication 1, où $R^7$ est un alkyle substitué ayant 1 à 10 atomes de carbone dont le substituant ou les substituants sont: alcoxy ayant 1 à 6 atomes de carbone ou hydroxy.

13. Composé selon la revendication 1, où $R^7$ est un hydrogène, un hydroxy, $OCH_3$, $CH_3$, un alkylhydroxy- ou -polyhydroxy-substitué ayant 1 à 10 atomes de carbone.

14. Composé selon la revendication 8, où $R^6$ est: un hydrogène; un radical substitué ou non substitué choisi parmi: alkyle en $C_{1-10}$, cycloalkyle en $C_{3-6}$, cycloalkyl($C_{3-6}$)alkyle en $C_{1-6}$), phényl-alkyle en $C_{1-6}$; où le substituant ou les substituants sont hydroxy.

15. Procédé pour préparer un composé selon la revendication 1, comprenant le stade de traitement de

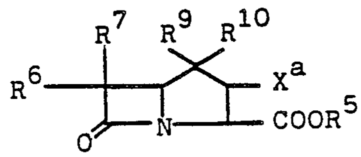

avec $HSR^8$; où $X^a$ est un groupe labile, $R^5$ est un groupe protecteur et $R^6$, $R^7$, $R^9$ et $R^{10}$ sont comme défini dans la revendication 1.

16. Composition antibiotique comprenant une quantité thérapeutique efficace d'un composé selon la revendication 1 et un support convenant en pharmacie de celui-ci.

**Revendications pour l'Etat contractant: AT**

1. Procédé pour préparer un composé répondant à la formule développée:

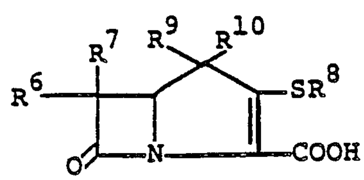

et les convenant en pharmacie, esters et amides qui en dérivent; où:

$R^6$ et $R^7$, et $R^9$ et $R^{10}$ sont indépendamment un hydrogène ($R^9$ et $R^{10}$ ne sont pas tous deux un hydrogène) ou sont choisis dans le groupe constitué par les formes substituées et non substituées de: alkyle, alcényle et alcynyle ayant 1 à 10 atomes de carbone; cycloalkyle, cycloalkylalkyle et alkylcycloalkyle ayant 3 à 6 atomes de carbone dans le cycle cycloalkyle et 1 à 6 atomes de carbone dans le fragment alkyle; phényle, phénylalkyle, phénylalcényle et phénylalcynyle dont la portion aliphatique a 1 à 6 atomes de carbone; ou $R^6$ et $R^7$ sont indépendamment un pyridyle ou $R^6$ et $R^7$, et $R^9$ et $R^{10}$ peuvent être unis pour former un cycloalkyle ayant, avec l'atome de carbone auquel ils sont fixés, 3 à 6 atomes de carbone; où le substituant ou les substituants sur $R^6$, $R^7$, $R^9$ ou $R^{10}$ sont indépendamment choisis parmi chloro, fluoro, hydroxy, bromo, alkylthio ayant 1 à 6 atomes de carbone et alcoxy ayant 1 à 6 atomes de carbone; et où $R^7$ peut de plus être un hydroxy, un méthoxy, un mercapto, un chloro, un fluoro et un bromo; et lorsque $R^7$ est un hydrogène, $CH_3$ ou $OCH_3$, $R^6$ peut de plus avoir un carboxy, un hydroximino, un uréido ou un amino comme substituant sur les groupes alkyle, cycloalkyle, cycloalkylalkyle et phénylalkyle précités; et

$R^8$ est un carbamimidoyle choisi dans le groupe constitué par:

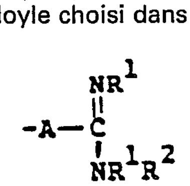

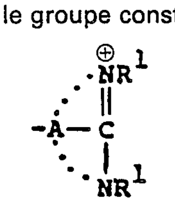

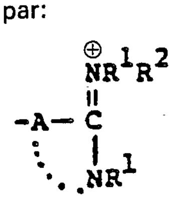

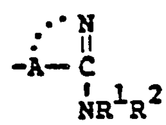 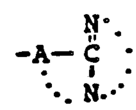 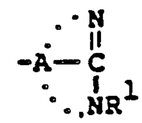

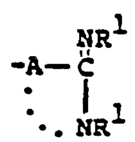 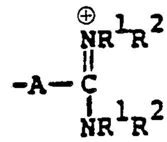 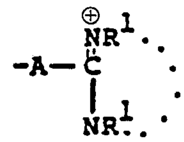

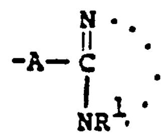 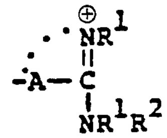 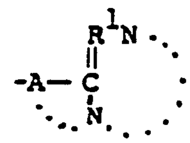

où: A est une simple liaison directe ou A, le connecteur cyclique ou acylclique, est choisi dans le groupe constitué par alkylène, alcénylène et alcynylène ayant 1 à 10 atomes de carbone pouvant être interrompu par un hétéroatome choisi parmi O, S ou N, ou par un phénylène, cycloalkylène, cycloalcénylène, hétérocyclylène ou hétéroarylène, où ces interruptions cycliques comprennent 3 à 6 atomes cycliques choisis parmi C, O, S et N; cycloalkylène ayant 3 à 10 atomes de carbone; cycloalcénylène ayant 3 à 6 atomes de carbone; et phénylène;

$R^1$ et $R^2$ sont indépendamment choisis parmi un hydrogène et les valeurs précédemment définies pour le groupe A mais monovalentes; un cycloalkylalkyle dont le fragment cycloalkyle comprend 3 à 6 atomes de carbone et le fragment alkyle comprend 1 à 6 atomes de carbone; un alkylcycloalkyle dont le fragment alkyle comprend 1 à 6 atomes de carbone et le fragment cycloalkyle comprend 3 à 6 atomes de carbone; un phénylalkyle ayant 7 à 10 atomes de carbone; où, lorsque $R^1$ et $R^2$ sont indépendamment choisis parmi un alkyle droit ou ramifié ayant 1 à 6 atomes de carbone; un cycloalkyle ayant 3 à 6 atomes de carbone; un phényle; ou un des groupes cycloalkylalkyle, alkylcycloalkyle et phénylalkyle definis ci-dessus, chacun de ces radicaux peut être substitué par des groupes amino, hydroxy, cyano, carboxy, nitro, chloro, bromo, fluoro, alcoxy et alkylthio ayant 1 à 6 atomes de carbone, mercapto, perhalogénoalkyle ayant 1 à 3 atomes de carbone, guanidino, amidino ou sulfamoyle;

et où A peut de plus être choisi parmi un cycloalkylalkylène dont le fragment cycloalkyle comprend 3 à 6 atomes de carbone et le fragment alkyle 1 à 10 atomes de carbone; un alkylcycloalkylène dont le fragment alkyle comprend 1 à 6 atomes de carbone et le fragment cycloalkylène comprend 3 à 6 atomes de carbone; un cycloalcénylalkylène dont le fragment cycloalcényle comprend 3 à 10 atomes de carbone et le fragment alkyle comprend 1 à 6 atomes de carbone; un naphtylène; un phénylalkylène et un alkylphénylène dont le fragment alkyle à 1 à 6 atomes de carbone;

et où les pointillés indiquent la possibilité de structures cycliques formées par l'union de l'atome d'azote indiqué et du groupe connecteur A et par l'union des atomes d'azote indiqués; à l'exception des composés dans lesquels:

a) $R^6$ est H et

$R^7$ est $CH_2OH$ $R^8$ est 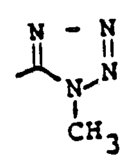

$R^9$ est $CH_3$

$R^{10}$ est H

b) $R^6$ est H

$R^7$ est 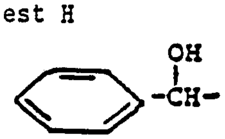 et

$R^9$ est $CH_3$ $R^8$ est 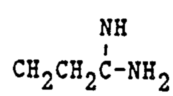

$R^{10}$ est 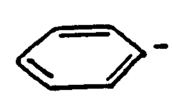

c) $R^6$ est H,

$R^7$ est 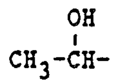 et

$R^9$ est $CH_3$ $R^8$ est 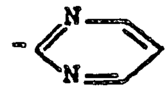

$R^{10}$ est $CH_3$

d) $R^6$ est H

$R^7$ est 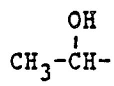

$R^9$ et $R^{10}$ sont réunis pour former un cycle cyclo

et

$R^8$ est 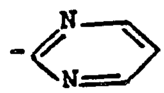

comprenant le stade de traitement de

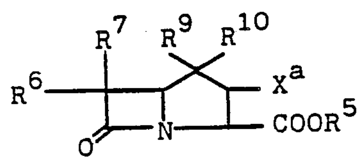

avec $HSR^8$; où $X^a$ est un groupe labile, $R^5$ est un groupe protecteur et $R^6$, $R^7$, $R^9$ et $R^{10}$ sont définis comme ci-dessus.

2. Procédé selon la revendication 1, où $R^1$ et $R^2$ sont indépendamment choisis parmi: un hydrogène; les formes substituées et non substituées des radicaux suivants: alkyle droit ou ramifié ayant 1 à 6 atomes de carbone; cycloalkyle ayant 3 à 6 atomes de carbone, cycloalkylalkyle dont le fragment cycloalkyle comprend 3 à 6 atomes de carbone et le fragment alkyle comprend 1 à 6 atomes de carbone; phényle, benzyle; où le substituant ou les substituants des radicaux précités sont choisis parmi fluoro, hydroxy, mercapto, alcoxy ayant 1 à 3 atomes de carbone; et alkylthio ayant 1 à 3 atomes de carbone.

3. Procédé selon la revendication 1, où le groupe connecteur A est choisi parmi: une simple liaison unique, un alkylène inférieur droit ou ramifié ayant 1 à 6 atomes de carbone, un cycloalkylène ayant 3 à 6 atomes de carbone; un phénylène; un alkylhétéroalkyle dont le fragment alkyle comprend 1 à 3 atomes de carbone et le ou les hétéroatomes sont le soufre, l'oxygène et l'azote.

4. Procédé selon la revendication 1, où $—SR^8$ est choisi dans le groupe constitué par:

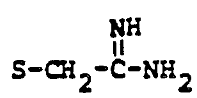

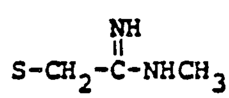

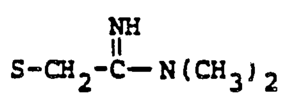

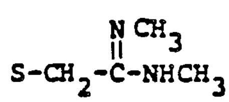

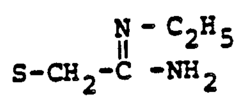

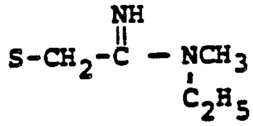

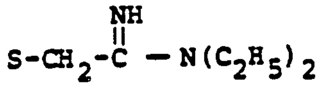

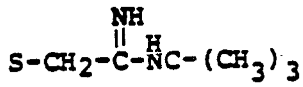

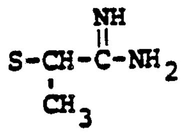

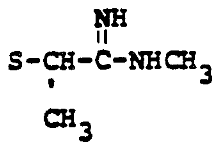

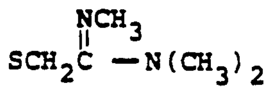

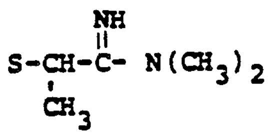

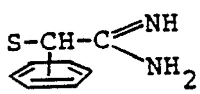

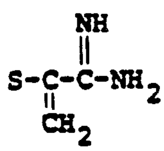

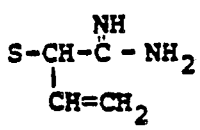

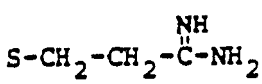

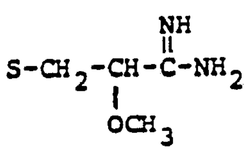

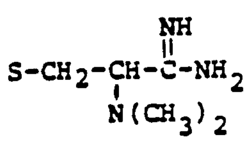

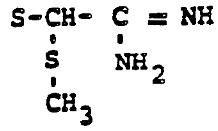

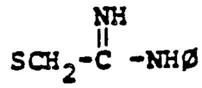

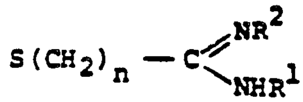

$n = 2\text{-}5$, $R^2 = H, CH_3$
$R^1 = H, CH_3$

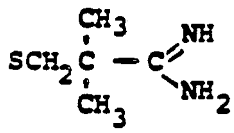

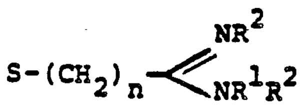

$n = 2\text{ -}5$, $R^1, R^2 = H, CH_3$

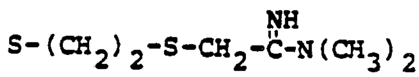

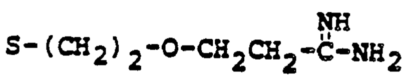

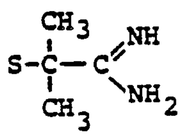

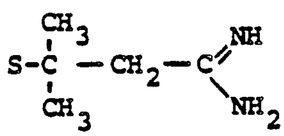

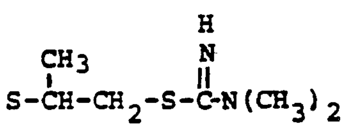

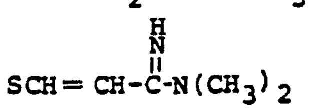

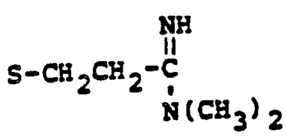

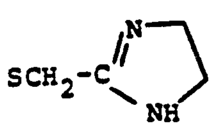

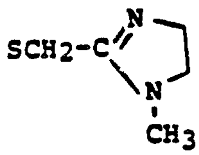

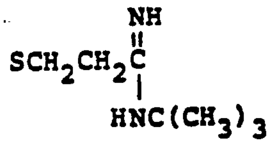

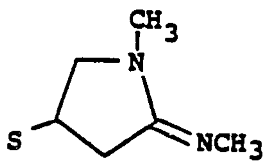

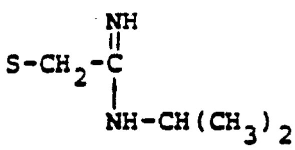

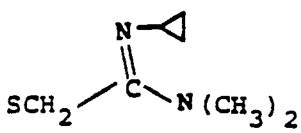

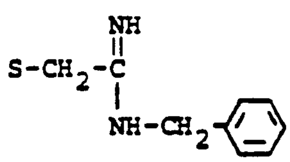

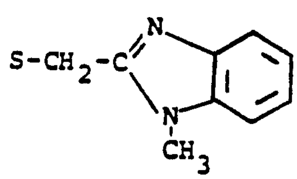

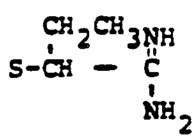

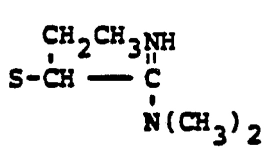

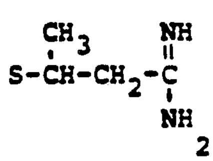

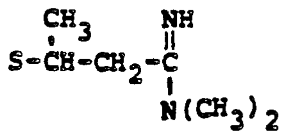

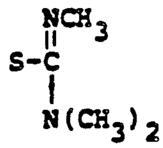

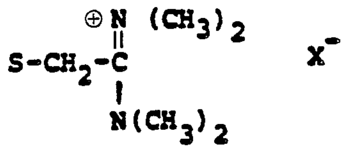

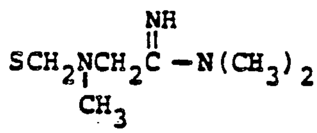

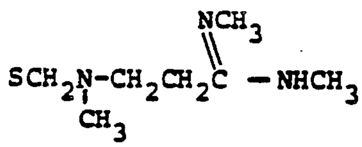

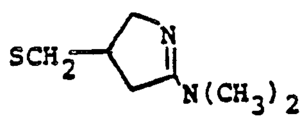

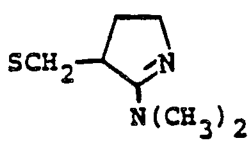

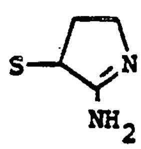

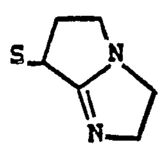

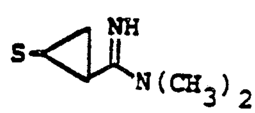

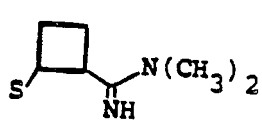

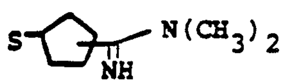

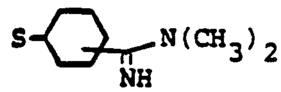

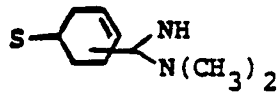

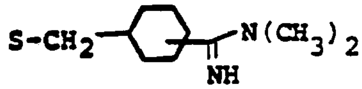

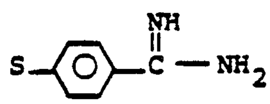

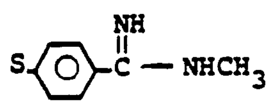

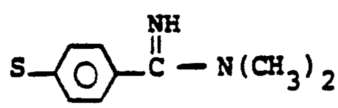

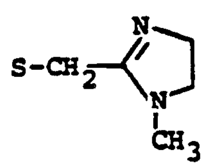

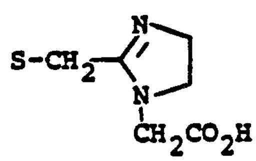

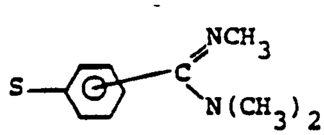

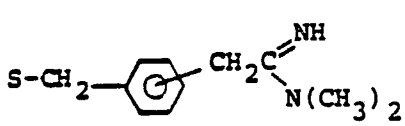

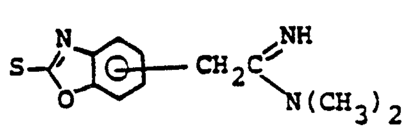

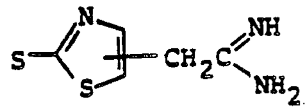

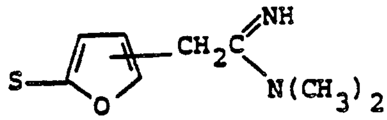

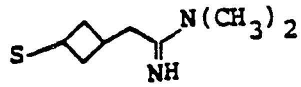

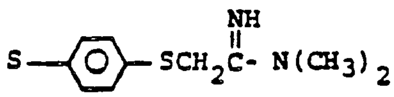

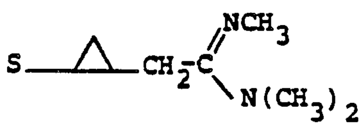

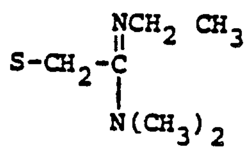

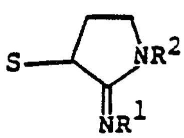

$R^1$ = H, CH3
$R^2$ = H, $CH_3$

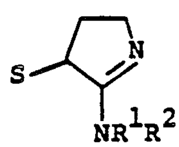

$R^1$ = H, $CH_3$
$R^2$ = H, $CH_3$

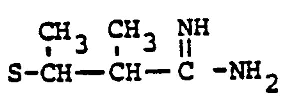

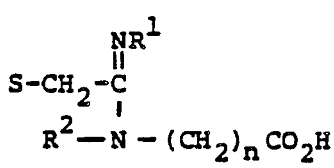

$R^1$ = H, $CH_3$
$R^2$ = H, $CH_3$
n = 1 ou 2

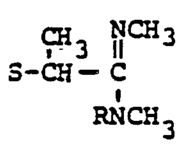

R = H, $CH_3$

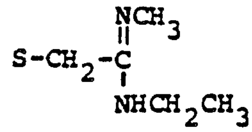

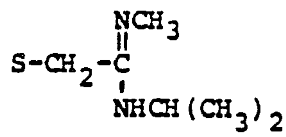

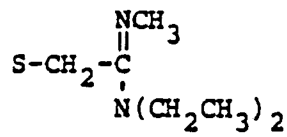

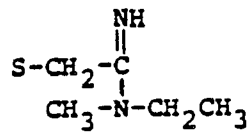

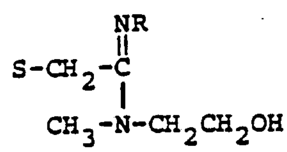

R $= CH_2CH_3$, $CH_3$

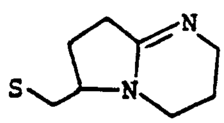

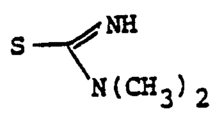

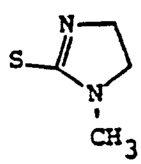

5. Procédé selon la revendication 1, 2, 3 ou 4, où $R^9$ et $R^{10}$ sont un alkyle en $C_{1-10}$.
6. Procédé selon la revendication 5, où $R^9$ et $R^{10}$ sont un méthyle
7. Procédé selon la revendication 1, 2, 3 ou 4 où $R^9$ et $R^{10}$ sont indépendamment choisis dans le groupe constitué par:

$CH_2CH_3$
$CH(CH_3)_2$
$CH_2C_6H_5$
$CH_2F$
$CF_3$
$CH_2Br$
$-CH=CH_2$

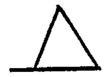

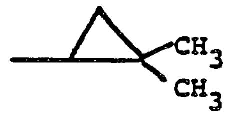

$CHF_2$

$CH_2Cl$

$CH_2OH$

$CH_2SMe$

$CH_2OMe$

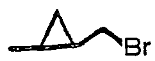

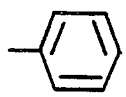

$-(CH_2)_n-$ $n = 2,3,4,5$

8. Procédé selon la revendication 1, où $R^7$ est choisi parmi H, $OCH_3$ et $CH_3$.

9. Procédé selon la revendication 8, où $R^6$ est choisi dans le groupe constitué par un alkyle en $C_{1-10}$, un cycloalkyl($C_{3-6}$)alkyle en $C_{1-6}$, un alkyle en $C_{1-10}$ substitué par un ou plusieurs groupes hydroxy ou un cycloalkyl($C_{3-6}$)alkyle en $C_{1-6}$ substitué par un ou plusieurs groupes hydroxy.

10. Procédé selon la revendication 9, où $R^7$ est un hydrogène.

11. Procédé selon la revendication 1, 8 ou 9, où $R^6$ est choisi parmi:

OH
|
ØCH$_2$CH   Ø = phényl

OH
|
ØCH$_2$CH$_2$CH

OH
|
CH$_2$

ØCH$_2$

OH
|
$(CH_3)_2C$

$CH_2{=}CHCH_2$

$(CH_3)_2C{=}CHCH_2$

OH
|
$CH_3OCH_2CH$

$CH_3$

$CH_3CH_2$

$(CH_3)_2CH$

$HO_2CCH_2$

$OH$
$CF_3CF_2CH$

$OH$
$HO_2CCH_2CH$

$OH$
$CH_3CH(CH_3)CH$

$OH$
$(CH_3)_2CHCH_2CH$

$OH$
$CH_3CH_2CH$

$HOCH_2CH_2$

$OH$
$CF_3CH$

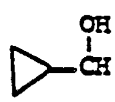

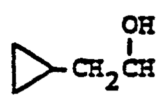

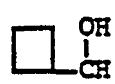

$HOCH_2CH_2CH_2$

$OH$
$F_2CHCH$

$OH$
$FCH_2CH$

$OH$
$\phi-CH$

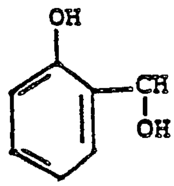

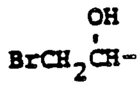

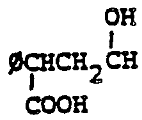

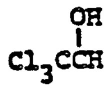

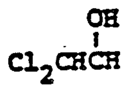

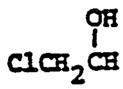

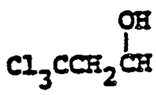

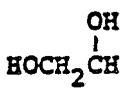

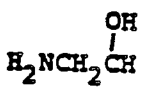

$-H$

$-CH(OH)CH_3$

$-CH_2CH_2CH_2CH_2OH$

$-CH(OH)CH_2CH_2OH$

$-CH(NH_2)CH_3$

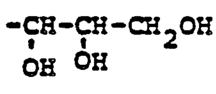

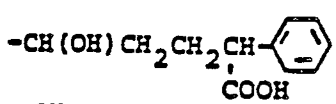

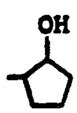

12. Procédé selon la revendication 1, où $R^7$ est un alkyle substitué ayant 1 à 10 atomes de carbone dont le substituant ou les substituant sont: alcoxy ayant 1 à 6 atomes de carbone ou hydroxy.

13. Procédé selon la revendication 1, où $R^7$ est un hydrogène, un hydroxy, $OCH_3$, $CH_3$, un alkylhydroxy- ou -polyhydroxy-substitué ayant 1 à 10 atomes de carbone.

14. Procédé selon la revendication 8, où $R^6$ est: un hydrogène; un radical substitué ou non substitué choisi parmi: alkyle en $C_{1-10}$, cycloalkyle en $C_{3-6}$, cycloalkyl($C_{3-6}$)alkyle en $C_{1-6}$), phényl-alkyle en $C_{1-6}$; où le substituant ou les substituant sont hydroxy.